# EUROPEAN PATENT APPLICATION

(11) **EP 4 400 493 A1**
(43) Date of publication of application: **17.07.2024**
(21) Application number: 22866750.7
(22) Date of filing: 09.09.2022
(51) Int. Cl.: C07D 239/94, C07D 239/93, C07D 239/88, C07D 215/38, C07D 215/36, C07D 215/233, C07D 471/04, A61K 31/47, A61K 31/517, A61K 31/519, A61P 35/00, A61P 31/04, A61P 31/12

(54) **HYDROXAMIC ACID COMPOUND HAVING ENPP1 INHIBITORY ACTIVITY AND USE THEREOF**

(30) Priority: 10.09.2021 CN 202111061250
(71) Applicant: Haihe Biopharma Co., Ltd., Shanghai 201203 (CN)
(72) Inventor: GAO, Shanyun, Shanghai 201203 (CN); LI, Jingjing, Shanghai 201203 (CN); ZHANG, Chaobo, Shanghai 201203 (CN); XU, Yanxiao, Shanghai 201203 (CN); TU, Wangyang, Shanghai 201203 (CN); YU, Bing, Shanghai 201203 (CN); ZHANG, Yixiang, Shanghai 201203 (CN); LI, Leping, Shanghai 201203 (CN)
(74) Representative: Plasseraud IP
(86) International application number: PCT/CN2022/118080
(87) International publication number: WO 2023/036289

(57) **Abstract**

The present disclosure involves a compound of formula (I) having ENPP1 inhibitory activity, a pharmaceutical composition thereof, and use thereof. The present disclosure particularly involves a compound of formula (I), as well as a pharmaceutical composition containing the compound and a method for preventing or treating diseases using the compound, particularly for diseases or disorders mediated by ENPP1 abnormal activity.

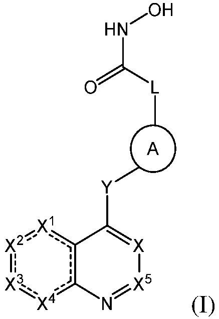

## Description

### TECHNICAL FIELD

The present disclosure involves a hydroxamic acid compound, use thereof for inhibiting ENPP1, and a method of using the compound for preventing or treating diseases.

### BACKGROUND

As the first line of the body to defend against pathogens, the human immune system plays an undeniable role in combating tumor cells. STING (Stimulator of Interferon Gene) is an interferon gene stimulator composed of 378 amino acids, which plays an important role in the immune response to tumor cells. STING can be activated by binding to cGAMP (cyclic GMP-AMP synthase), which in turn recruits TANK-binding kinase 1 (TBK1), leading to phosphorylation of interferon regulatory factor 3 (IRF3). The phosphorylated IRF3 will produce type I interferon (IFN) and other cytokines, which together with IFN trigger the immune response of human adaptive immune system to tumor cells and infectious diseases.

Ectonucleotide pyrophosphatase/phosphodi-esterase 1 (ENPP1) is one of the seven enzymes in the ENPP family and a type II transmembrane glycoprotein. Research has shown that ENPP1 has high hydrolysis ability and can decompose various ligands, including phosphodiester bonds and pyrophosphate bonds. ATP is also one of the main targets of ENPP1. ENPP1 not only hydrolyzes ATP into AMP and PPi, but also hydrolyzes cGAMP, inhibiting the amount of cGAMP in the human body and reducing STING activity. Therefore, the activity of ENPP1 inhibits anti-tumor and anti-infectious diseases immune response of the human immune system through STING, and inhibiting the activity of ENPP1 contributes to the stability of cGAMP and the increase of STING activity, thus improving the resistance of the human immune system to tumors and infectious diseases. In addition, ENPP1 has been confirmed to have a more prominent expression than usual in human breast tumors, which not only indicates that ENPP1 is a potential predictive marker of breast cancer, but also highlights the possibility and reliability of ENPP1 as an anticancer drug target.

In addition to the prominent expression of ENPP1 in tumors, studies have shown that the expression of ENPP1 is related to a variety of infectious diseases caused by bacteria or viruses. Therefore, ENPP1 can also be used for the treatment of infectious diseases.

Although cancer patients have multiple treatment options, there is still a need for effective and safe therapeutic agents and optimal application in combination therapy thereof.

### SUMMARY OF THE INVENTION

The present disclosure provides a compound of formula (I), a pharmaceutically acceptable salt thereof, a pharmaceutical composition thereof and a combination thereof. The inventor unexpectedly discovered that the compound of formula (I) is a good ENPP1 inhibitor. The inventor also unexpectedly found that the compound of the present disclosure has good physical and chemical stability, good bioavailability (for example, low clearance rate), and good drug property.

The present disclosure further provides a method for treating, preventing or improving ENPP1-mediated diseases or disorders, including administering an effective amount of ENPP1 inhibitors to an individual in need.

Specifically, the present disclosure provides a hydroxamic acid compound of formula (I):

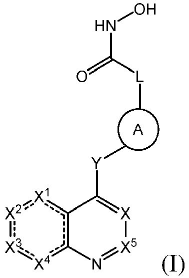

Wherein, each of the variables X, X¹, X², X³, X⁴, X⁵ and rings A, Y and L are defined herein, comprising a stereoisomer, a geometric isomer, a tautomer, a pharmaceutically acceptable salt, a polymorphic form, a solvate, a hydrate or a prodrug thereof; they can be used to treat or prevent ENPP1-mediated diseases or disorders, especially cancer or infectious diseases or disorders.

The present disclosure also provides a method for preparing the compound of the present disclosure, an intermediate for preparing the compound of the present disclosure, and a method for preparing the intermediate.

The present disclosure also provides a composition comprising at least one compound or the pharmaceutically acceptable salt thereof of the present disclosure.

The present disclosure also provides a pharmaceutical composition comprising a therapeutically effective amount of the compound or the pharmaceutically acceptable salt thereof of the present disclosure, as well as one or more pharmaceutically acceptable carriers, diluents or excipients.

In an embodiment, the present disclosure provides a pharmaceutical composition comprising a therapeutically effective amount of the compound or the pharmaceutically acceptable salt thereof of the present disclosure.

In another embodiment, the present disclosure provides a combination, especially a pharmaceutical combination, comprising a therapeutically effective amount of the compound or the pharmaceutically acceptable salt thereof of the present disclosure, as well as one or more other therapeutic agents.

The compound of the present disclosure may be used alone, in combination with other compounds of the present disclosure, or in combination with one or more, preferably one or two other substances simultaneously or sequentially.

The compound of the present disclosure is an ENPP1 inhibitor, which can regulate the STING activity in vivo, and therefore can be used to treat or prevent ENPP1-mediated diseases or obstacles, especially cancer or infectious diseases or obstacles. Therefore, the present disclosure can provide selective extracellular inhibition of ENPP1 activity to increase the extracellular level of cGAMP and activate the interferon gene stimulatory factor (STING) pathway. For example, use of the compound of the present disclosure for enhancing STING-mediated responses in a subject, and a method for using the compound of the present disclosure for regulating immune responses in a subject.

The compound of the present disclosure can be used for therapy.

The compound of the present disclosure can be used to prepare medicaments or drugs, which are used to treat or prevent ENPP1-mediated diseases or obstacles, especially cancer or infectious diseases or obstacles.

The present disclosure also involves a method for inhibiting ENPP1 receptor activity in individuals, wherein, the method includes administering a therapeutically effective amount of ENPP1 inhibitors such as the compound or the pharmaceutically acceptable salt thereof of the present disclosure to an individual in need.

In an embodiment of the present disclosure, the present disclosure provides a method for treating or preventing ENPP1-mediated diseases or disorders, including administering an effective amount of a first therapeutic agent and an optional second therapeutic agent to patients in need, wherein the first therapeutic agent is the compound or the pharmaceutically acceptable salt thereof of the present disclosure, and the second therapeutic agent is one or more other therapeutic agents.

In another embodiment of the present disclosure, the present disclosure involves a method for treating or preventing ENPP1-mediated diseases or disorders, such as cancer or infectious diseases or conditions, which includes administering a therapeutically effective amount of the compound or the pharmaceutically acceptable salt thereof of the present disclosure to an individual.

A preferred method of the present disclosure is to treat specific types of cancer, including breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer.

Another preferred method of the present disclosure is to treat ENPP1-mediated infectious diseases, including herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.

In addition, the present disclosure provides a product or a kit comprising a combination preparation of the compound, or the pharmaceutically acceptable salt thereof as defined above of the present disclosure, or the pharmaceutical composition thereof and one or more other active agents used simultaneously, separately, or sequentially in anti-cancer therapy.

### EMBODIMENTS

Specifically, the present disclosure provides the following embodiments:
On the one hand, the present disclosure provides a compound of formula (I): 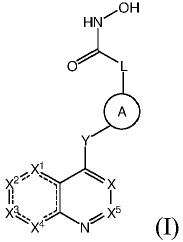
or a pharmaceutically acceptable salt thereof, wherein,
- - - - : is a single or double bond;
X is N or CR⁰;
X¹ is N, O, S, CR¹ or a bond;
X² is N, O, S, NR² or CR²;
X³ is N, O, S, NR³ or CR³;
X⁴ is N, O, S, NR⁴ or CR⁴;
X⁵ is N or CR⁵;
Y is O, S, -S(=O)-, -S(=O)₂-, -C(=O)-, NR⁶ or CR⁷R⁸;
L is a bond, NR^{a}, -NR^{x}-CHR^{y}- or (CR⁹R¹⁰)ₘ; m is 1 or 2;
R⁰ and R⁵ are each independently selected from hydrogen, halogen, CN, OH, NO₂, NR^{b}R^{c}, C₁-C₆ alkyl, C₃-C₆ cycloalkyl, -SO₂R^{a}, -C(O)OR^{a}, -C(O)NR^{b}R^{c} and C₁-C₆ alkoxy; wherein each of the alkyl, the cycloalkyl and the alkoxy is optionally substituted with halogen;
R¹ and R² are each independently selected from hydrogen, halogen, CN, OH, NO₂, NR^{b}R^{c}, C₁-C₄ alkyl, C₃-C₆ cycloalkyl and C₁-C₄ alkoxy; wherein each of the alkyl, the cycloalkyl and the alkoxy is optionally substituted with halogen;
R³ and R⁴ are each independently selected from hydrogen, halogen, CN, OH, NO₂, NR^{b}R^{c}, =O, C₁-C₄ alkyl, C₃-C₆ cycloalkyl and C₁-C₄ alkoxy; wherein each of the alkyl, the cycloalkyl and the alkoxy is optionally substituted with halogen;
R⁶ is selected from hydrogen, C₁-C₄ alkyl and C₃-C₆ cycloalkyl; wherein each of the alkyl and the cycloalkyl is optionally substituted with halogen;
R⁷ and R⁸ are each independently selected from hydrogen, halogen, OH, CN, NO₂, NR^{a}, C₁-C₆ alkyl, C₃-C₆ cycloalkyl and C₁-C₆ alkoxy; wherein each of the alkyl, the cycloalkyl and the alkoxy is optionally substituted with halogen; or R⁷ and R⁸ together with the carbon atom bound thereto form C₃-C₆ cycloalkyl optionally substituted with halogen, or form 4- to 12-membered heterocycloalkyl containing 1-2 heteroatoms selected from N, NR^{a}, O and S(O)ₚ;
R⁹ and R¹⁰ are each independently selected from hydrogen, halogen, CN, OH, NO₂, NR^{a}R^{b}, C₁-Cs alkyl, C₃-C₈ cycloalkyl, C₂-C₈ alkenyl, C₂-C₈ alkynyl, C₁-C₈ alkoxy, 4- to 12-membered heterocycloalkyl containing carbon atoms and 1-2 heteroatoms selected from N, NR^{a}, O and S(O)ₚ, 6- to 10-membered aryl and 5- to 10-membered heteroaryl; each of the alkyl, the alkenyl, the alkynyl, the alkoxy, the cycloalkyl and the heterocycloalkyl is substituted with 0-3 substituents independently selected from halogen, hydroxyl and CN; wherein each of the aryl and the heteroaryl is substituted with 0-3 substituents independently selected from halogen, C₁-C₄ alkyl, C₃-C₆ cycloalkyl and OR^{a};
or R⁹ and R¹⁰ together with the carbon atom bound thereto form C₃-C₆ cycloalkyl optionally substituted with halogen, or form 4- to 12-membered heterocycloalkyl containing 1-2 heteroatoms selected from N, NR^{a}, O and S(O)ₚ;
ring A is selected from C₄-C₁₀ cycloalkyl, 4- to 12-membered heterocycloalkyl, 6- to 10-membered aryl and 5- to 10-membered heteroaryl; wherein, ring A is optionally substituted one or more times by substituents independently selected from halogen, CN, OH, NO₂, NR^{b}R^{c}, C₁-C₄ alkyl, C₁-C₄ alkoxy, C₃-C₆ cycloalkyl, 4- to 7-membered heterocycloalkyl, 6- to 10-membered aryl, 5- to 10-membered heteroaryl and C₁-C₃ haloalkyl;
   or
L together with ring A forms C₄-C₈ cycloalkyl, 5- to 10-membered partially saturated heterocycle containing at least one heteroatom selected from N, O and S, 6- to 10-membered aryl or 5- to 7-membered heteroaryl; wherein the cycloalkyl, the heterocycle, the aryl and the heteroaryl are optionally substituted one or more times by substituents independently selected from halogen, C₁-C₄ alkyl and C₁-C₄ alkoxy; or
Y together with ring A forms 5- to 10-membered partially saturated heterocycle, 6- to 10-membered aryl or 5- to 10-membered heteroaryl; wherein the heterocycle, the aryl and the heteroaryl are optionally substituted one or more times by substituents independently selected from halogen, C₁-C₄ alkyl and C₁-C₄ alkoxy;
R^{a}, R^{b} and R^{c} are each independently selected from hydrogen, C₁-C₄ alkyl, C₃-C₆ cycloalkyl and benzyl; wherein each of the alkyl and the cycloalkyl is optionally substituted with halogen;
or R^{b} and R^{c} together with the nitrogen atom bound thereto form 3- to 6-membered heterocycloalkyl optionally substituted with halogen;
R^{x} and R^{y} are each independently selected from hydrogen and C₁-C₄ alkyl; or R^{x} and R^{y} together with the carbon and nitrogen atoms bound thereto form 4- to 8-membered heterocycloalkyl; and
p is 1 or 2;
with the proviso that when X₁ to X₄ are each CRⁿ, Y is O, S, -S(=O)-, -S(=O)₂-, -C(=O)- or CH₂; wherein Rⁿ is R¹ for X¹, R² for X², R³ for X³, and R⁴ for X⁴.

In a specific embodiment of the present disclosure, X¹ is N, O, S or CR¹; preferably, X¹ is N or CR¹.

In another specific embodiment of the present disclosure, X¹ is N.

In another specific embodiment of the present disclosure, X¹ is CR¹.

In another specific embodiment of the present disclosure, X¹ is a bond or CR¹.

In another specific embodiment of the present disclosure, X¹ is a bond.

In some embodiments of the present disclosure, X¹ is CR¹, and Y is O, S, -S(=O)-, -S(=O)₂-, - C(=O)- or CR⁷R⁸; preferably, Y is O, S, -S(=O)-, -S(=O)₂-, -C(=O)- or CH₂.

In other embodiments of the present disclosure, when X¹ is CR¹, and X² is CR², X³ is CR³, and X⁴ is CR⁴, Y is O, S, -S(=O)-, -S(=O)₂-, -C(=O)- or CH₂.

Whenever Rⁿ is mentioned herein, it represents R¹ for X¹, R² for X², R³ for X³, and R⁴ for X⁴.

In other embodiments of the present disclosure, R⁰ and R⁵ are each independently selected from hydrogen, halogen, CN, OH, NO₂, NH₂, C₁-C₆ alkyl, C₃-C₆ cycloalkyl and C₁-C₆ alkoxy, wherein each of the alkyl, the cycloalkyl and the alkoxy is optionally substituted with 0-3 halogens. Preferably, R⁰ and R⁵ are each independently selected from hydrogen, halogen, CN, OH, NO₂, NH₂, C₁-C₄ alkyl, C₃-C₆ cycloalkyl and C₁-C₄ alkoxy; wherein each of the alkyl, the cycloalkyl and the alkoxy is optionally substituted with 0-3 halogens. More preferably, R⁰ and R⁵ are each independently selected from hydrogen, halogen, CN, OH, NO₂, NH₂ and C₁-C₃ alkyl.

In other embodiments of the present disclosure, R¹ is selected from hydrogen, halogen, CN, OH, NO₂, NH₂, C₁-C₄ alkyl, C₁-C₃ alkoxy and C₁-C₃ haloalkyl; preferably selected from hydrogen, halogen, CN, OH, NH₂, C₁-C₃ alkyl; more preferably, R¹ is selected from hydrogen, halogen, CN, OH.

In other embodiments of the present disclosure, R² is selected from hydrogen, halogen, CN, OH, NO₂, NH₂, C₁-C₄ alkyl, C₁-C₃ alkoxy and C₁-C₃ haloalkyl; preferably selected from hydrogen, halogen, OH, C₁-C₃ alkyl and C₁-C₃ alkoxy, each of the alkyl, the cycloalkyl and the alkoxy is optionally substituted with 0-3 halogens. More preferably, R² is selected from hydrogen, halogen, C₁-C₃ alkyl and C₁-C₃ alkoxy.

In other embodiments of the present disclosure, R³ and R⁴ are each independently selected from hydrogen, halogen, CN, OH, NO₂, NH₂, C₁-C₄ alkyl, =O, C₃-C₆ cycloalkyl and C₁-C₃ alkoxy, wherein, each of the alkyl, the cycloalkyl and the alkoxy is optionally substituted with 0-3 halogens; preferably, R³ and R⁴ are each independently selected from hydrogen, halogen, CN, OH, NO₂, NH₂, =O, C₁-C₄ alkyl, C₃-C₆ cycloalkyl, C₁-C₃ alkoxy and C₁-C₃ haloalkyl. More preferably, R³ is selected from hydrogen, halogen, OH, =O, C₁-C₃ alkyl and C₁-C₃ alkoxy, and R⁴ is selected from hydrogen, halogen, C₁-C₃ alkyl, C₁-C₃ alkoxy, C₃-C₆ cycloalkyl and C₁-C₃ haloalkyl.

In some embodiments of the present disclosure, R³ and R⁴ are each independently selected from =O.

In some specific embodiments, X³ is -C(=O)-, and X⁴ is CH.

In other specific embodiments, X⁴ is -C(=O)-, and X³ is CH.

In other embodiments of the present disclosure,R¹, R², R³ and R⁴ are each independently selected from hydrogen, halogen, CN, OH, NO₂, NH₂, C₁-C₄ alkyl, C₃-C₆ cycloalkyl, C₁-C₄ alkoxy and C₁-C₄ haloalkyl; preferably, selected from hydrogen, halogen, CN, OH, NO₂, NH₂, C₁-C₃ alkyl, C₃-C₄ cycloalkyl, C₁-C₃ alkoxy and C₁-C₂ haloalkyl; more preferably, selected from H, F, Cl, CN, OH, NO₂, NH₂, methyl, ethyl, propyl, isopropyl, methoxy, ethoxy, fluoromethyl, difluoromethyl, trifluoromethyl, fluoroethyl, 2,2-difluoromethyl, 1,2-difluoromethyl, 2,2,2-trifluoroethyl, and cyclopropyl.

In other embodiments of the present disclosure, R¹ is hydrogen or halogen; R² is selected from hydrogen, halogen, OH, C₁-C₃ alkyl and C₁-C₃ alkoxy; R³ is selected from hydrogen, halogen, OH, =O, NR^{b}R^{c}, C₁-C₃ alkyl and C₁-C₃ alkoxy; and R⁴ is selected from hydrogen, halogen, C₁-C₃ alkyl, C₁-C₃ alkoxy, C₃-C₆ cycloalkyl and C₁-C₄ haloalkyl; preferably, R⁵ is selected from hydrogen, halogen, CN and C₁-C₆ alkyl.

In other embodiments of the present disclosure, R¹ is hydrogen or halogen; R² is selected from hydrogen, halogen, OH, C₁-C₃ alkyl and C₁-C₃ alkoxy; R³ is selected from hydrogen, halogen, OH, =O, C₁-C₃ alkyl, and C₁-C₃ alkoxy; and R⁴ is selected from hydrogen, halogen, C₁-C₃ alkyl and C₁-C₃ alkoxy. Preferably, R⁰ and R⁵ are each independently selected from hydrogen, halogen, CN and C₁-C₆ alkyl.

In other embodiments of the present disclosure, R⁰ is selected from hydrogen, halogen, CN, OH, NO₂, NH₂, C₁-C₄ alkyl, C₃-C₆ cycloalkyl, and C₁-C₄ alkoxy; R¹ is hydrogen or halogen; R² is selected from hydrogen, halogen, OH, C₁-C₃ alkyl and C₁-C₃ alkoxy; R³ is selected from hydrogen, halogen, OH, C₁-C₃ alkyl and C₁-C₃ alkoxy; R⁴ is selected from hydrogen, halogen, C₁-C₃ alkyl and C₁-C₃ alkoxy; and R⁵ is selected from hydrogen, halogen, CN, C₁-C₄ alkyl.

In other embodiments of the present disclosure, R¹ is hydrogen or halogen; R² is selected from hydrogen, halogen, C₁-C₃ alkyl and C₁-C₃ alkoxy; R³ is selected from hydrogen, halogen, OH, =O, NR^{b}R^{c}, C₁-C₃ alkyl and C₁-C₃ alkoxy; R⁴ is selected from hydrogen, halogen and C1-C3 alkyl. Preferably, R⁵ is selected from hydrogen, halogen, CN and C₁-C₆ alkyl.

In other embodiments of the present disclosure, R¹ is hydrogen or halogen; R² is selected from hydrogen, halogen and C₁-C₃ alkoxy; R³ is selected from hydrogen, halogen, OH and C₁-C₃ alkoxy; and R⁴ is selected from hydrogen and halogen. Preferably, R⁵ is selected from hydrogen, halogen, CN and C₁-C₆ alkyl.

In other embodiments of the present disclosure, R¹ is hydrogen or halogen; R² is selected from hydrogen, halogen, OH and C₁-C₃ alkoxy; R³ is selected from hydrogen, halogen, OH, C₁-C₃ alkyl and C₁-C₃ alkoxy; R⁴ is selected from hydrogen, halogen and C₁-C₃ alkoxy; preferably, R⁵ is selected from hydrogen, halogen, CN and C₁-C₆ alkyl. More preferably, R³ is selected from hydrogen, halogen, OH and C₁-C₃ alkoxy.

In other embodiments of the present disclosure, R² is selected from hydrogen, halogen, C₁-C₃ alkyl and C₁-C₃ alkoxy; R³ is selected from hydrogen, OH and halogen; R⁴ is selected from hydrogen, halogen, C₁-C₃ alkyl, C₁-C₃ alkoxy, C₃-C₄ cycloalkyl and C₁-C₃ haloalkyl; preferably, R⁵ is selected from hydrogen, halogen, CN and C₁-C₆ alkyl.

In a specific embodiment of the present disclosure, X is N, or X⁵ is N.

In another specific embodiment of the present disclosure, X⁵ is N.

In another specific embodiment of the present disclosure, X is N.

In some embodiments of the present disclosure, Y is O, S, -S(=O)-, -S(=O)₂-, -C(=O)-, NR⁶ or CR⁷R⁸; wherein, R⁶ is selected from hydrogen, C₁-C₄ alkyl and C₃-C₆ cycloalkyl, and R⁷ and R⁸ are each independently selected from hydrogen, halogen, OH, CN, NO₂, C₁-C₆ alkyl, C₃-C₆ cycloalkyl and C₁-C₆ alkoxy. Preferably, Y is O, NR⁶ or CR⁷R⁸.

In some embodiments of the present disclosure, Y is O, S, -S(=O)-, -S(=O)₂-, -C(=O)- or CR⁷R⁸, preferably O, -C(=O)- or CR⁷R⁸, more preferably O or CR⁷R⁸. Further preferably, R⁷ and R⁸ are each independently selected from hydrogen and C₁-C₃ alkyl.

In some embodiments of the present disclosure, Y is O, S, -S(=O)-, -S(=O)₂-, -C(=O)- or CH₂.

In some embodiments of the present disclosure, Y is NR⁶, and R⁶ is selected from hydrogen, C₁-C₄ alkyl and C₃-C₆ cycloalkyl. Preferably, R⁶ is selected from hydrogen and C₁-C₃ alkyl.

In some embodiments of the present disclosure, Y together with ring A forms 5- to 10-membered partially saturated heterocycle, 6- to 10-membered aryl or 5- to 10-membered heteroaryl; wherein the heterocycle, the aryl and the heteroaryl are optionally substituted one or more times by substituents independently selected from halogen, C₁-C₄ alkyl and C₁-C₄ alkoxy; for example,
Y is NR⁶, R⁶ is a C₁-C₄ alkyl, and R⁶ together with the nitrogen atom bound thereto and ring A forms 5- to 10-membered partially saturated heterocycle, 6- to 10-membered aryl or 5- to 10-membered heteroaryl; or
Y is CR⁷R⁸, and R⁷ or R⁸ together with the nitrogen atom bound thereto and ring A forms 5- to 10-membered partially saturated heterocycle, 6- to 10-membered aryl or 5- to 10-membered heteroaryl.

In some embodiments of the present disclosure, Y together with ring A forms 7- to 10-membered partially saturated bicyclic heterocycle, naphthyl or 7- to 10-membered bicyclic heteroaryl. Preferably, Y together with ring A forms dihydrobenzofuranyl, dihydroisobenzofuranyl, dihydroindolyl, dihydrobenzothiophenyl, dihydrobenzothiazolyl, dihydrobenzopyranyl, tetrahydroquinolyl, tetrahydroisoquinolyl, tetrahydropyridino[3,4-b]pyrazinyl More preferably, Y together with ring A forms dihydroindolyl.

In some embodiments of the present disclosure, Y is CR⁷R⁸, wherein, R⁷ and R⁸ are each independently selected from hydrogen, halogen, OH, CN, NO₂, NH₂, C₁-C₆ alkyl, C₃-C₆ cycloalkyl and C₁-C₆ alkoxy, preferably, selected from hydrogen and C₁-C₆ alkyl; wherein each of the alkyl, the cycloalkyl and the alkoxy is optionally substituted with 0-3 halogens.

In other embodiments of the present disclosure, Y is CR⁷R⁸, wherein, R⁷ and R⁸ together with the carbon atom bound thereto form C₃-C₆ cycloalkyl optionally substituted with 0-3 halogens, or form 5- to 6-membered heterocycloalkyl containing 1-2 heteroatoms selected from N, NR^{a}, O and S(O)ₚ; R^{a} is selected from hydrogen, C₁-C₃ alkyl, p is 1 or 2.

In other embodiments of the present disclosure, Y is O, S, -S(=O)-, -S(=O)₂-, -C(=O)-, NR⁶ or CR⁷R⁸; R⁶ is selected from hydrogen, C₁-C₄ alkyl and C₃-C₆ cycloalkyl; and R⁷ and R⁸ are each independently selected from hydrogen and C₁-C₃ alkyl. More preferably, R⁶ is selected from hydrogen and C₁-C₃ alkyl, R⁷ and R⁸ are each independently selected from hydrogen and C₁-C₃ alkyl;

preferably, Y is O, S, -S(=O)-, -S(=O)₂-, -C(=O)-, -NH-, -N(CH₃)-, -N(ethyl)-, -N(propyl)-, - N(cyclopropyl)-, -N(cyclobutyl)-, -N(cyclopentyl)-, -CH₂-, -CHF-, -CH(OH)-, -CH(CH₃)-, - CH(OCH₃)-, -CH(OEt)- or -C(CH₃)₂-; more preferably, Y is O, S, -S(=O)-, -S(=O)₂-, -C(=O)-, -NH-, -N(CH₃)-, -CH(CH₃)- or -CH₂-.

In some embodiments of the present disclosure, Y is O, S, -S(=O)-, -S(=O)₂-, -C(=O)-, NH or CH₂; preferably, Y is O, S, -S(=O)-, -S(=O)₂-, NH or CH₂; more preferably, Y is O, S or NH.

In other embodiments of the present disclosure, Y is O, NR⁶ or CR⁷R⁸; preferably, Y is O, NH, N(C₁-C₃ alkyl), CH₂, CH(C₁-C₃ alkyl) or C(C₁-C₃ alkyl)(C₁-C₃ alkyl). More preferably, Y is O, NH, -NCH₃, CH₂ or -CHCH₃.

In another specific embodiment of the present disclosure, Y is O.

In another specific embodiment of the present disclosure, Y is NH.

In some embodiments of the present disclosure, L is a bond, NR^{a}, -NR^{x}-CHR^{y}-, CR⁹R¹⁰ or (CR⁹R¹⁰)₂. It can be understood that in (CR⁹R¹⁰)₂, the two -CR⁹R¹⁰- units are each capable of being the same or different, for example, in -CH₂-CH(CH₃).

In other embodiments, L is -NR^{x}-CHR^{y}-, wherein, R^{x} and R^{y} are each independently selected from hydrogen, C₁-C₄ alkyl, or R^{x} and R^{y} together with the carbon and nitrogen atoms bound thereto form 4- to 8-membered heterocycloalkyl. Preferably, forming 4- to 6-membered heterocycloalkyl; for example, forming 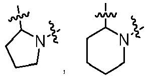 together.

In some embodiments of the present disclosure, R^{a} is selected from hydrogen, C₁-C₄ alkyl, C₃-C₆ cycloalkyl and benzyl, wherein each of the alkyl and the cycloalkyl is optionally substituted with halogen. Preferably, R^{a} is selected from hydrogen, C₁-C₄ alkyl, C₃-C₆ cycloalkyl, C₁-C₄ haloalkyl, C₃-C₆ halocycloalkyl and benzyl. More preferably, R^{a} is selected from hydrogen, C₁-C₃ alkyl and benzyl.

In some embodiments of the present disclosure, R⁹ and R¹⁰ are each independently selected from hydrogen, halogen, CN, OH, NH₂, C₁-C₈ alkyl, C₃-C₈ cycloalkyl, C₁-C₈ alkoxy, 4-to 12-membered heterocycloalkyl containing carbon atoms and 1-2 heteroatoms selected from N, NR^{a}, O and S(O)ₚ, preferably selected from hydrogen, halogen, OH, NH₂, C₁-C₈ alkyl, C₃-C₈ cycloalkyl, C₁-C₈ alkoxy, 4- to 8-membered heterocycloalkyl containing carbon atoms and 1-2 heteroatoms selected from N, NR^{a}, O and S(O)ₚ.

In other embodiments of the present disclosure, R⁹ and R¹⁰ together with the carbon atom bound thereto form C₃-C₆ cycloalkyl substituted with 0-3 halogens, or 4- to 12-membered heterocycloalkyl containing 1-2 heteroatoms selected from N, NR^{a}, O and S(O)ₚ. Preferably, R⁹ and R¹⁰ together with the carbon atom bound thereto form C₃-C₆ cycloalkyl, or 4- to 8-membered heterocycloalkyl containing 1-2 heteroatoms selected from N, NR^{a}, O and S(O)ₚ.

In a specific embodiment of the present disclosure, L is a bond.

In some embodiments of the present disclosure, L is a bond or (CR⁹R¹⁰)ₘ; m is 1 or 2. Preferably, L is CR⁹R¹⁰ or (CR⁹R¹⁰)₂; more preferably, L is CR⁹R¹⁰.

In some embodiments of the present disclosure, L is NR^{a} or (CR⁹R¹⁰)ₘ; m is 1 or 2. Preferably, L is NR^{a} or CR⁹R¹⁰; more preferably, L is NH or CR⁹R¹⁰.

In some embodiments of the present disclosure, L is NR^{a}, and R^{a} is selected from hydrogen, C₁-C₄ alkyl and C₃-C₆ cycloalkyl; wherein each of the alkyl and the cycloalkyl is optionally substituted with 0-3 halogens. Preferably, R^{a} is selected from hydrogen and C₁-C₃ alkyl.

In some embodiments of the present disclosure, L is (CR⁹R¹⁰)ₘ; m is 1 or 2;
R⁹ and R¹⁰ are each independently selected from hydrogen, halogen, CN, OH, NO₂, NH₂, C₁-Cs alkyl, C₃-C₈ cycloalkyl, C₂-C₈ alkenyl, C₂-C₈ alkynyl, C₁-C₈ alkoxy, 4- to 12-membered heterocycloalkyl containing carbon atoms and 1-2 heteroatoms selected from N, NR^{a}, O and S(O)ₚ, 6- to 10-membered aryl and 5- to 10-membered heteroaryl; each of the alkyl, the alkenyl, the alkynyl, the alkoxy, the cycloalkyl and the heterocycloalkyl is substituted with 0-3 substituents independently selected from halogen, hydroxyl and CN; wherein each of the aryl and the heteroaryl is substituted with 0-3 substituents independently selected from halogen, C₁-C₄ alkyl, C₃-C₆ cycloalkyl and OR^{a};
or, R⁹ and R¹⁰ together with the carbon atom bound thereto form C₃-C₆ cycloalkyl substituted with 0-3 halogens, or form 4- to 12-membered heterocycloalkyl containing 1-2 heteroatoms selected from N, NR^{a}, O and S(O)ₚ.

In some embodiments of the present disclosure, L is NH or CR⁹R¹⁰, and Y is O, S, -S(=O)-, - S(=O)₂-, -C(=O)-, NR⁶ or CR⁷R⁸; R⁶, R⁷ and R⁸ are each independently selected from hydrogen, C₁-C₄ alkyl and C₃-C₆ cycloalkyl; preferably, R⁶, R⁷ and R⁸ are each independently selected from hydrogen and C₁-C₃ alkyl; and more preferably, L is CR⁹R¹⁰, and R⁶, R⁷ and R⁸ are each independently selected from hydrogen and C₁-C₃ alkyl.

In a preferred embodiment of the present disclosure, wherein L is NR^{a} or CR⁹R¹⁰;
preferably, L is CR⁹R¹⁰; wherein, R⁹ and R¹⁰ are each independently selected from hydrogen, halogen, CN, OH, NO₂, NH₂, C₁-C₆ alkyl, C₃-C₆ cycloalkyl and C₁-C₆ alkoxy, 4- to 12-membered heterocycloalkyl containing carbon atoms and 1-2 heteroatoms selected from N, O and S, or R⁹ and R¹⁰ together with the carbon atom bound thereto form C₃-C₆ cycloalkyl substituted with 0-3 halogens (for example, cyclopropane, cyclobutane, cyclopentane or cyclohexane) or form 4- to 12-membered heterocycloalkyl containing 1-2 heteroatoms selected from N, NR^{a}, O and S(O)ₚ (for example, oxocyclobutane, azacyclobutane, thiocyclobutane, tetrahydrofuran ring, pyrrolidine, tetrahydrothiophene ring, pyrazolidine, imidazolidine, thiazolidine, oxazolidine, piperidine ring, tetrahydropyran ring, piperazine ring, hexahydropyrimidine ring, oxazinane, pyridazinane, morpholine ring, thiomorpholine ring, thiophane, tetrahydropyran ring, thiocyclohexane, oxocycloheptane, azacycloheptane or thiocycloheptane);
or L is CR⁹R¹⁰, wherein, R⁹ and R¹⁰ are each independently selected from hydrogen, halogen, CN, OH, C₁-C₆ alkyl and C1-C6 alkoxy, or R⁹ and R¹⁰ together with the carbon atom bound thereto form C₃-C₆ cycloalkyl (for example, cyclopropane, cyclobutane, cyclopentane or cyclohexane);
or L is selected from -CH₂-, -CH(OH)-, -CHF-, -CH(CN)-, -CH(CH₃)-, -CH(OCH₃)-, - CH(OEt)-, -C(CH₃)₂-, -CH(CH(CH₃)₂)-, -CH(CH₂CH(CH₃)₂)-, -C(CH₂CH(CH₃)₂)₂-, - C(CH(CH₃)₂)₂-, -C(CH₂CH₃)₂- and 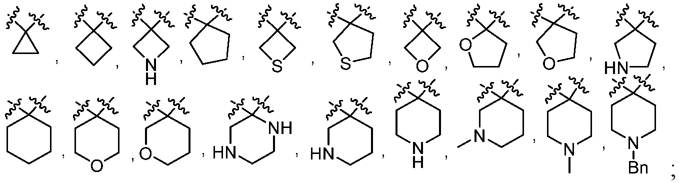
preferably, L is selected from -CH₂-, -CH(OH)-, -CHF-, -CH(CH₃)-, -CH(OCH₃)-, -CH(OEt)-, -C(CH₃)₂-, -CH(CH(CH₃)₂)-, -CH(CH₂CH(CH₃)₂)-, -C(CH₂CH(CH₃)₂)₂-, -C(CH₂CH₃)₂- and

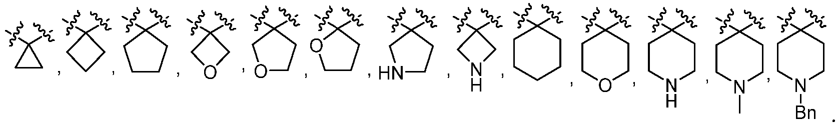

In other embodiments of the present disclosure, L is (CR⁹R¹⁰)₂. Preferably, L is -CH₂CH₂-, - CH₂CH(CH₃)-, -CH(CH₃)CH₂-, -CH₂CHF- or -CH₂CH(OCH₃)-.

In some embodiments of the present disclosure, ring A is selected from C₄-C₈ cycloalkyl, 4- to 8-membered heterocycloalkyl, 6- to 10-membered aryl and 5- to 10-membered heteroaryl; preferably, ring A can be optionally substituted one or more times, for example once or twice, by substituents independently selected from halogen, CN, OH, NO₂, NR^{b}R^{c}, C₁-C₄ alkyl, C₁-C₄ alkoxy, C₃-C₆ cycloalkyl, 4- to 7-membered heterocycloalkyl, 6- to 10-membered aryl, 5- to 10-membered heteroaryl and C₁-C₃ haloalkyl; R^{b} and R^{c} are each independently selected from hydrogen, C₁-C₄ alkyl and C₃-C₆ cycloalkyl, wherein each of the alkyl and the cycloalkyl is substituted with 0-3 halogens, or R^{b} and R^{c} together with the nitrogen atom bound thereto form 3- to 6-membered heterocycloalkyl substituted with 0-3 halogens. Further, ring A is optionally substituted one or more times, for example once or twice, by substituents independently selected from halogen, CN, OH, NH₂, C₁-C₄ alkyl, C₁-C₄ alkoxy and C₁-C₃ haloalkyl. Still further, ring A is optionally substituted once or twice by substituents independently selected from halogen, CN, C₁-C₄ alkyl and C₁-C₄ alkoxy.

Preferably, ring A can be optionally substituted one or more times, for example once or twice, by substituents independently selected from F, Cl, CN, OH, NO₂, NH₂, C₁-C₄ alkyl, C₁-C₄ alkoxy, C₃-C₆ cycloalkyl and C₁-C₃ haloalkyl; further preferably, ring A can be optionally substituted one or more times, for example once or twice, by substituents independently selected from F, Cl, CN, OH, NO₂, NH₂, C₁-C₃ alkyl, C₃-C₆ cycloalkyl and C₁-C₃ alkoxy.

In a preferred embodiment of the present disclosure, ring A is optionally substituted or unsubstituted cyclopentane, cyclohexane, pyrrolidine, pyrazoline, imidazoline, thiazoline, pyrrole ring, furan ring, thiophene ring, pyrazole ring, thiazole ring, oxazole ring, imidazole ring, tetrahydrofuran ring, tetrahydrothiophene ring, pyran ring, pyrazine ring, tetrahydropyran ring, piperidine ring, hexahydropyrimidine, dihydrofuran ring, dihydrothiophene ring, dihydroimidazole ring, dihydropyrazole ring, dihydropyrrole ring, dihydroindole ring, dihydroisoindole ring, dihydropyran ring, dihydropyridine ring, dihydropiperazine ring, piperazine ring, benzene ring, pyridine ring, pyrimidine ring, naphthalene ring, quinoline ring, isoquinoline ring, indole ring, isoindole ring, indazole ring or benzimidazole ring;
preferably, ring A is optionally substituted or unsubstituted cyclopentane, cyclohexane, pyrrolidine, imidazoline, pyrazole ring, pyrrole ring, imidazole ring, furan ring, thiophene ring, pyran ring, tetrahydrofuran ring, tetrahydrothiophene ring, tetrahydropyran ring, piperidine ring, pyrazine ring, piperazine ring, benzene ring, pyridine ring, pyrimidine ring or naphthalene ring;
more preferably, ring A is optionally substituted or unsubstituted cyclopentane, cyclohexane, pyrrolidine, piperidine ring, pyrimidine ring, benzene ring, pyridine ring, piperazine ring, imidazole ring, pyrazole ring or naphthalene ring;
further preferably, ring A is cyclopentane, cyclohexane, pyrrolidine, piperidine ring, pyrimidine ring, benzene ring, pyridine ring, piperazine ring, imidazole ring, tetrahydropyrrole ring, pyrazole ring or naphthalene ring that has been substituted once or twice by F, Cl, CN, OH, NO₂, NH₂, C₁-C₃ alkyl or C₁-C₃ alkoxy.

In some embodiments of the present disclosure, ring A is optionally substituted one or more times, for example once or twice, by substituents independently selected from halogen, CN, OH, NO₂, NH₂, C₁-C₃ alkyl, C₁-C₃ alkoxy and C₁-C₃ haloalkyl; preferably, ring A is optionally substituted one or more times, for example once or twice, by substituents independently selected from F, Cl, CN, OH, NO₂, NH₂, C₁-C₃ alkyl and C₁-C₃ alkoxy.

In some embodiments of the present disclosure, ring A is selected from: 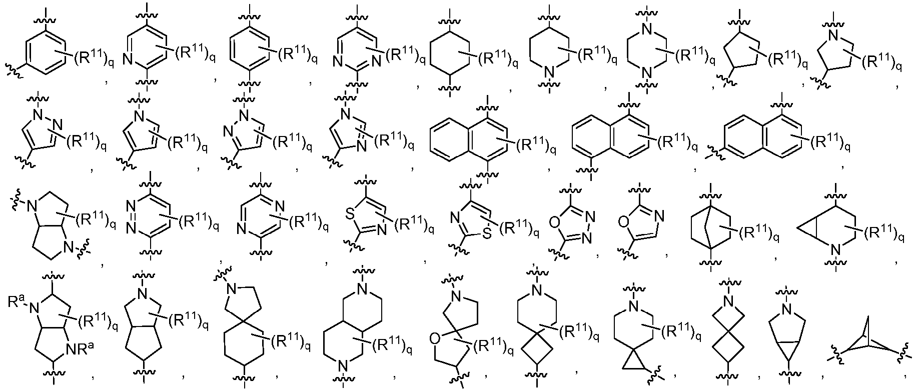 wherein, each R¹¹ is capable of being the same or different, and independently selected from halogen, CN, OH, NO₂, NH₂, C₁-C₄ alkyl, C₁-C₄ alkoxy and C₁-C₄ haloalkyl; and q is 0, 1 or 2. Preferably, R¹¹ is selected from F, Cl, CN, C₁-C₄ alkyl or C₁-C₄ alkoxy; R^{a} is selected from hydrogen and C₁-C₄ alkyl, and q is 0, 1, or 2.

In some embodiments of the present disclosure, L together with ring A forms C₄-C₈ cycloalkyl, 5- to 10-membered partially saturated heterocycle containing at least one heteroatom selected from N, O and S, 6- to 10-membered aryl or 5- to 7-membered heteroaryl; the cycloalkyl, the heterocycle, the aryl and the heteroaryl are optionally substituted one or more times by substituents independently selected from halogen, C₁-C₄ alkyl and C₁-C₄ alkoxy. Preferably, the cycloalkyl, the heterocycle, the aryl and the heteroaryl are optionally substituted one or more times by substituents independently selected from halogen, C₁-C₃ alkyl and C₁-C₃ alkoxy.

In some embodiments of the present disclosure, L together with ring A forms 5- to 10-membered partially saturated heterocycle containing at least one heteroatom selected from N, O and S, 6-to 10-membered aryl or 5- to 7-membered heteroaryl, the heterocycle, the aryl and the heteroaryl are optionally substituted once or twice by substituents independently selected from halogen, C₁-C₄ alkyl and C₁-C₄ alkoxy.

In some embodiments of the present disclosure, L together with ring A forms optionally substituted or unsubstituted dihydrobenzofuran ring, dihydroisobenzofuran ring, dihydrobenzothiophene ring, dihydroindole ring, dihydroisoindole ring, dihydrobenzimidazole ring, dihydrobenzoxazole ring, dihydrobenzothiazole ring, dihydrobenzopyran ring, dihydroisobenzopyran ring, tetrahydroquinoline ring, tetrahydroisoquinoline ring, 2,3-dihydro-1H-pyrrolo[2,3-b]pyridine ring, 2,3-dihydro-1H-pyrrolo[3,2-b]pyridine ring, tetrahydropyridino[3,4-b]pyrazine ring, dihydroindene ring, tetrahydronaphthalene ring, 2,3-dihydrofurano[2,3-b]pyridine ring, 2,3-dihydrofurano[3,2-*b*]pyridine ring; preferably, each of which is optionally substituted one or more times by substituents independently selected from halogen, C₁-C₄ alkyl and C₁-C₄ alkoxy.

More preferably, L together with ring A forms optionally substituted or unsubstituted dihydrobenzofuran ring, dihydroisobenzofuran ring, dihydroindole ring, dihydroisoindole ring, dihydrobenzopyran ring, dihydroisobenzopyran ring, tetrahydroquinoline ring, tetrahydroisoquinoline ring, dihydroindene ring, tetrahydronaphthalene ring, 2,3-dihydrofurano[2,3-*b*]pyridine ring, 2,3-dihydro-1H-pyrrolo[2,3-*b*]pyridine ring; further preferably, each of which is optionally substituted one or more times by substituents independently selected from halogen, C₁-C₄ alkyl and C₁-C₄ alkoxy.

In a preferred embodiment of the present disclosure, L together with ring A forms the following 5- to 10-membered partially saturated heterocycle containing at least one heteroatom selected from N, O and S, 6- to 10-membered aryl or 5- to 7-membered heteroaryl: 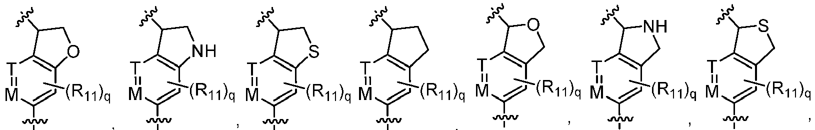 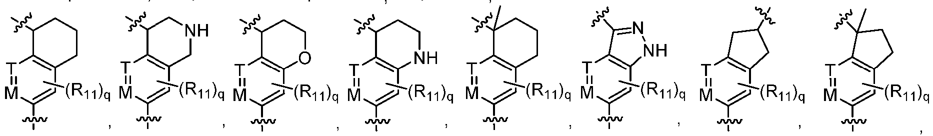 wherein, M and T are each capable of being the same or different, and are each independently N or C; each R¹¹ is capable of being the same or different, and independently selected from halogen, CN, OH, NH₂, C₁-C₃ alkyl, C₁-C₃ alkoxy and C₁-C₃ haloalkyl; and q is 0, 1 or 2. Preferably, R¹¹ is selected from F, Cl, CN, C₁-C₃ alkyl and C₁-C₃ alkoxy; and q is 0, 1 or 2.

In a preferred embodiment of the present disclosure, Y together with ring A forms a group selected from the following groups: 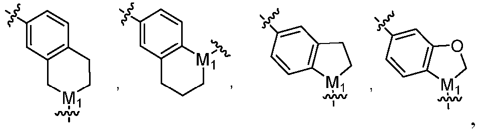 wherein, each M₁ is independently N, CH or C(C₁-C₄ alkyl). Preferably, Y is NR⁶, R⁶ is C₁-C₄ alkyl, and R⁶ together with the nitrogen atom bound thereto and ring A forms 5- to 10-membered partially saturated heterocycle, 6- to 10-membered aryl or 5- to 10-membered heteroaryl, wherein the heterocycle, the aryl and the heteroaryl are optionally substituted one or more times by substituents independently selected from halogen, C₁-C₄ alkyl and C₁-C₄ alkoxy.

Further, Y together with ring A forms a group selected from the following groups:

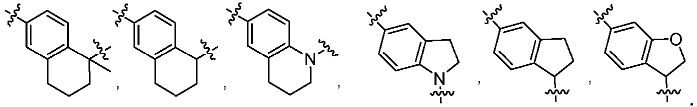

Further, the present disclosure provides a compound of formula (I) or a pharmaceutically acceptable salt thereof: 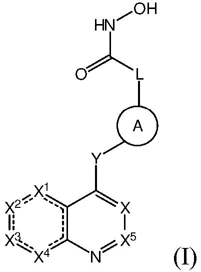
or a pharmaceutically acceptable salt thereof, wherein,
- - - - - : is a single or double bond;
X is N or CR⁰;
X¹ is N, O, S, CR¹ or a bond;
X² is N, O, S, NR² or CR²;
X³ is N, O, S, NR³ or CR³;
X⁴ is N, O, S, NR⁴ or CR⁴;
X⁵ is N or CR⁵;
Y is O, S, -S(=O)-, -S(=O)₂-, -C(=O)-, NR⁶ or CR⁷R⁸;
L is a bond, NR^{a}, -NR^{x}-CHR^{y}- or (CR⁹R¹⁰)ₘ; m is 1 or 2;
R⁰ and R⁵ are each independently selected from hydrogen, halogen, CN, OH, NO₂, NH₂, C₁-C₄ alkyl, C₃-C₆ cycloalkyl, -SO₂R^{a}, -C(O)OR^{a}, -C(O)NR^{b}R^{c} and C₁-C₄ alkoxy, wherein each of the alkyl, the cycloalkyl and the alkoxy is optionally substituted with halogen;
R¹ and R² are each independently selected from H, halogen, CN, OH, NO₂, NH₂, C₁-C₄ alkyl, C₁-C₃ alkoxy and C₁-C₃ haloalkyl;
R³ and R⁴ are each independently selected from H, halogen, CN, OH, NO₂, NH₂, =O, C₁-C₄ alkyl, C₃-C₆ cycloalkyl, C₁-C₃ alkoxy and C₁-C₃ haloalkyl;
R⁶ is selected from hydrogen, C₁-C₄ alkyl and C₃-C₆ cycloalkyl;
R⁷ and R⁸ are each independently selected from hydrogen, C₁-C₄ alkyl, C₃-C₆ cycloalkyl and C₁-C₄ alkoxy;
R⁹ and R¹⁰ are each independently selected from hydrogen, halogen, CN, OH, NH₂, NO₂, C₁-Cs alkyl, C₃-C₈ cycloalkyl, C₂-C₈ alkenyl, C₂-C₈ alkynyl, C₁-C₈ alkoxy, 4- to 12-membered heterocycloalkyl containing carbon atoms and 1-2 heteroatoms selected from N, NR^{a}, O and S(O)ₚ, 6- to 10-membered aryl and 5- to 10-membered heteroaryl; each of the alkyl, the alkenyl, the alkynyl, the cycloalkyl, the alkoxy and the heterocycloalkyl is substituted with 0-3 substituents independently selected from halogen, OH and CN; wherein each of the aryl and the heteroaryl is substituted with 0-3 substituents independently selected from halogen, C₁-C₄ alkyl, C₃-C₆ cycloalkyl and OR^{a};
or R⁹ and R¹⁰ together with the carbon atom bound thereto form C₃-C₆ cycloalkyl optionally substituted with halogen, or form 4- to 12-membered heterocycloalkyl containing 1-2 heteroatoms selected from N, NR^{a}, O and S(O)ₚ;
ring A is selected from C₄-C₈ cycloalkyl, 4- to 8-membered heterocycloalkyl, 6- to 10-membered aryl and 5- to 10-membered heteroaryl; wherein, ring A is optionally substituted one or more times by substituents independently selected from halogen, CN, OH, NO₂, NR^{b}R^{c}, C₁-C₄ alkyl, C₁-C₄ alkoxy, C₃-C₆ cycloalkyl, 4- to 7-membered heterocycloalkyl, 5- to 10-membered aryl, 5- to 10-membered heteroaryl and C₁-C₃ haloalkyl;
   or
L together with ring A forms C₄-C₈ cycloalkyl, 5- to 10-membered partially saturated heterocycle containing at least one heteroatom selected from N, O and S, 6- to 10-membered aryl or 5- to 7-membered heteroaryl; wherein the cycloalkyl, the heterocycle, the aryl and the heteroaryl are optionally substituted one or more times by substituents independently selected from halogen, C₁-C₃ alkyl and C₁-C₃ alkoxy; or
Y together with ring A forms 5- to 10-membered partially saturated heterocycle, 6- to 10-membered aryl or 5- to 10-membered heteroaryl; wherein the heterocycle, the aryl and the heteroaryl are optionally substituted one or more times by substituents independently selected from halogen, C₁-C₄ alkyl and C₁-C₄ alkoxy;
R^{a}, R^{b} and R^{c} are each independently selected from hydrogen, C₁-C₄ alkyl, C₃-C₆ cycloalkyl and benzyl, wherein each of the alkyl and the cycloalkyl is optionally substituted with halogen, or R^{b} and R^{c} together with the nitrogen atom bound thereto form 3- to 6-membered heterocycloalkyl optionally substituted with halogen;
R^{x} and R^{y} are each independently selected from hydrogen and C₁-C₄ alkyl, or R^{x} and R^{y} together with the carbon and nitrogen atoms bound thereto form 4- to 8-membered heterocycloalkyl; and
p is 1 or 2;
with the proviso that when X₁ to X₄ are each CRⁿ, Y is O, S, -S(=O)-, -S(=O)₂-, -C(=O)- or CH₂, wherein Rⁿ is R¹ for X¹, R² for X², R³ for X³, and R⁴ for X⁴.

In a specific embodiment of the present disclosure, wherein,
R¹ is hydrogen or halogen;
R² is selected from hydrogen, halogen, OH, C₁-C₃ alkyl and C₁-C₃ alkoxy;
R³ is selected from hydrogen, halogen, OH, NR^{b}R^{c}, =O, C₁-C₃ alkyl and C₁-C₃ alkoxy;
R⁴ is selected from hydrogen, halogen, C₁-C₃ alkyl, C₁-C₃ alkoxy, C₃-C₆ cycloalkyl and C₁-C₄ haloalkyl;
R⁵ is selected from hydrogen, halogen, CN and C₁-C₆ alkyl;

In another specific embodiment of the present disclosure, wherein,
R¹ is hydrogen or halogen;
R² is selected from hydrogen, halogen, OH, C₁-C₃ alkyl and C₁-C₃ alkoxy;
R³ is selected from hydrogen, halogen, OH, =O, C₁-C₃ alkyl and C₁-C₃ alkoxy;
R⁴ is selected from hydrogen, halogen, C₁-C₃ alkyl, C₁-C₃ alkoxy, C₃-C₆ cycloalkyl and C₁-C₄ haloalkyl;
R⁵ is selected from hydrogen, halogen, CN and C₁-C₆ alkyl;
and R⁶, R⁷ and R⁸ are each independently selected from hydrogen and C₁-C₃ alkyl.

In another specific embodiment of the present disclosure, at least one of X¹, X², X³ and X⁴ is independently selected from N, O, S and NRⁿ (if any); wherein Rⁿ is R¹ for X¹, R² for X², R³ for X³, and R⁴ for X⁴.

In another specific embodiment of the present disclosure, a compound of formula (I) or a pharmaceutically acceptable salt thereof is provided, wherein, wherein, at least one of X¹, X², X³ and X⁴ is independently selected from N and NRⁿ (if any); wherein Rⁿ is R¹ for X¹, R² for X², R³ for X³, and R⁴ for X⁴. Specifically, the present disclosure provides a compound of formula (Ia): 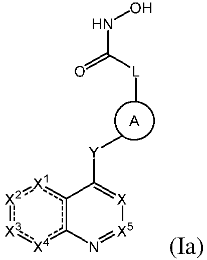
or a pharmaceutically acceptable salt thereof; wherein,
at least one of X¹, X², X³ and X⁴ is independently selected from N or NRⁿ; that is, in X¹, X², X³ and X⁴, at least X¹ is N, or at least X² is N or NR², or at least X³ is N or NR³, or at least X⁴ is N or NR⁴;
each of the variables X⁵, X, Y, A and L is defined as formula (I).

In a specific embodiment of the present disclosure, at least one of X¹, X², X³ and X⁴ is independently selected from Nor NR¹¹, and R¹ is hydrogen or halogen;
R² is selected from hydrogen, halogen, C₁-C₃ alkyl and C₁-C₃ alkoxy;
R³ is selected from hydrogen, halogen, OH, =O, NR^{b}R^{c}, C₁-C₃ alkyl and C₁-C₃ alkoxy;
R⁴ is selected from hydrogen, halogen and C₁-C₃ alkyl;
R⁵ is selected from hydrogen, halogen, CN and C₁-C₆ alkyl;
R^{a} is selected from hydrogen and C₁-C₃ alkyl;
and preferably, R⁶, R⁷ and R⁸ are each independently selected from hydrogen and C₁-C₃ alkyl.

In a preferred embodiment of the present disclosure, at least one of X¹, X², X³ and X⁴ is independently selected from Nor NR¹¹, and R¹ is hydrogen or halogen;
R² is selected from hydrogen, halogen, OH, C₁-C₃ alkyl and C₁-C₃ alkoxy;
R³ is selected from hydrogen, halogen, OH and C₁-C₃ alkoxy;
R⁴ is selected from hydrogen, halogens and C₁-C₃ alkoxy;
R⁵ is selected from hydrogen, halogen, CN and C₁-C₆ alkyl;
and R^{a} is selected from hydrogen and C₁-C₃ alkyl.

In another specific embodiment of the present disclosure, at least one of X¹, X², X³ and X⁴ is independently selected from N; specifically, the present disclosure provides compounds of the following formulas (Ia1), (Ia2), (Ia3) or (Ia4): 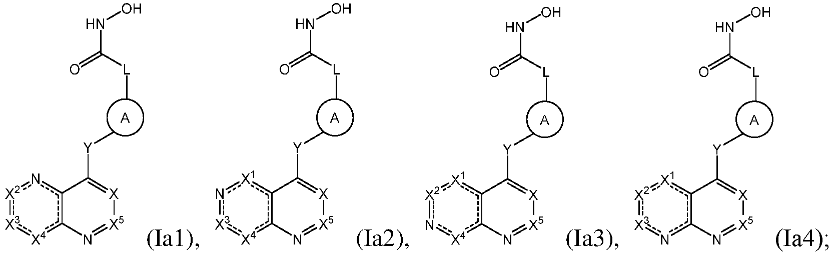
or each pharmaceutically acceptable salt thereof;
wherein, at least one of X¹, X², X³ and X⁴ is independently selected from N;
the other variables are defined as formula (I).

In a specific embodiment of the present disclosure, X¹ is N or CR¹. Preferably, X¹ is N or CR¹, and at least one of X², X³ and X⁴ is independently selected from N and NR¹¹.

In a specific embodiment of the present disclosure, X¹ is N. Preferably, X¹ is N, and X³ is CR³.

In another specific embodiment of the present disclosure, X² is N or NR². Preferably, X² is N; more preferably, X² is N, and X³ is CR³; further preferably, X² is N, X³ is CR³, and X⁴ is CR⁴; and most preferably, X² is N, X³ is CR³, X⁴ is CR⁴, and X¹ is CR¹.

In another specific embodiment of the present disclosure, X³ is N or NR³. Preferably, X³ is N; more preferably, X³ is N, and X⁴ is CR⁴; further preferably, X³ is N, X⁴ is CR⁴, and X² is CR²; and most preferably, X³ is N, X⁴ is CR⁴, X² is CR², and X¹ is CR¹.

In another specific embodiment of the present disclosure, X⁴ is N or NR⁴. Preferably, X⁴ is N; more preferably, X⁴ is N, and X³ is CR³; further preferably, X⁴ is N, X³ is CR³, and X² is CR²; and most preferably, X⁴ is N, X³ is CR³, X² is CR², and X¹ is CR¹.

In a specific embodiment of the present disclosure, only one of X¹, X², X³ and X⁴ is independently selected from N and NRⁿ; wherein Rⁿ is R¹ for X¹, R² for X², R³ for X³, and R⁴ for X⁴. In some specific embodiments, X¹ is N, and X² is CR², X³ is CR³, X⁴ is CR⁴.

In other specific embodiments, X² is N, O, S or NR², and X¹ is a bond or CR¹, X³ is CR³, X⁴ is CR⁴; preferably, X² is N or NR², and X¹ is a bond or CR¹, X³ is CR³, X⁴ is CR⁴.

In other specific embodiments, X³ is N, O, S or NR³, and X¹ is a bond or CR¹, X² is CR², X⁴ is CR⁴; preferably, X³ is N or NR³, and X¹ is a bond or CR¹, X² is CR², X⁴ is CR⁴.

In other specific embodiments, X⁴ is N, O, S or NR⁴, and X¹ is a bond or CR¹, X² is CR², X³ is CR³; preferably, X⁴ is N or NR⁴, and X¹ is a bond or CR¹, X² is CR², X³ is CR³.

In another specific embodiment of the present disclosure, at least two of X¹, X², X³ and X⁴ are independently selected from N and NR¹¹.

In some specific embodiments, X¹ is N, and X² is N or NR².

In other specific embodiments, X¹ is N, and X³ is N or NR³.

In other specific embodiments, X¹ is N, and X⁴ is N or NR⁴.

In other specific embodiments, X² is N or NR², and X³ is N or NR³.

In other specific embodiments, X² is N or NR², and X⁴ is N or NR⁴.

In other specific embodiments, X³ is N or NR³, and X⁴ is N or NR⁴.

In other specific embodiments, X² is N or NR², and X³ is N or NR³, X⁴ is N or NR⁴.

In a specific embodiment of the present disclosure, X³ is NR³, and X⁴ is carbonyl-C(=O)-. Preferably, X³ is NH, and X⁴ is -C(=O)-.

In a specific embodiment of the present disclosure, X⁴ is NR⁴, and X³ is carbonyl-C(=O)-. Preferably, X⁴ is NH, and X³ is -C(=O)-.

In another specific embodiment of the present disclosure, X¹ is a bond; specifically, the present disclosure provides a compound of formula (Ib): 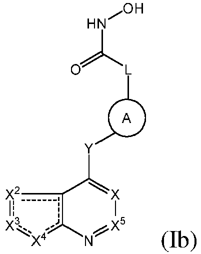
or a pharmaceutically acceptable salt thereof; wherein,
each of the variables X², X³, X⁴, X⁵, X, Y, A and L is defined as formula (I).

In a specific embodiment of the present disclosure, X¹ is a bond, and
R² is selected from hydrogen, halogen, C₁-C₃ alkyl and C₁-C₃ alkoxy;
R³ is selected from hydrogen, OH and halogen;
R⁴ is selected from hydrogen, halogen, C₁-C₃ alkyl, C₁-C₃ alkoxy, C₃-C₄ cycloalkyl and C₁-C₃ haloalkyl;
R⁵ is selected from hydrogen, halogen, CN and C₁-C₆ alkyl;
R⁶ is selected from hydrogen and C₁-C₃ alkyl;
and R⁷ and R⁸ are each independently selected from hydrogen and C₁-C₃ alkyl. In a specific embodiment of the present disclosure, X¹ is a bond, and at least one of X², X³ and X⁴ is independently selected from N, O, S or NRⁿ; when X¹ is a bond, in X², X³ and X⁴, at least X² is N, O, S or NR², or at least X³ is N, O, S or NR³, or at least X⁴ is N, O, S or NR⁴.

In a specific embodiment of the present disclosure, X¹ is a bond, and at least one of X², X³ and X⁴ is independently selected from N or NRⁿ; when X¹ is a bond, in X², X³ and X⁴, at least X² is N or NR², or at least X³ is N or NR³, or at least X⁴ is N or NR⁴.

In some specific embodiments, when X¹ is a bond, X² is N, O, S or NR²; preferably, X¹ is a bond, and X² is N or NR².

In other specific embodiments, when X¹ is a bond, X³ is N, O, S or NR³; preferably, X¹ is a bond, X³ is N or NR³; more preferably, X¹ is a bond, X³ is N.

In other specific embodiments, when X¹ is a bond, X⁴ is N, O, S or NR⁴. Preferably, X¹ is a bond, and X⁴ is N, O or NR⁴; more preferably, X¹ is a bond, and X⁴ is NR⁴.

In a preferred embodiment of the present disclosure, X¹ is a bond, and X³ is N;
R² is selected from hydrogen, halogen and C₁-C₃ alkyl;
R⁴ is selected from hydrogen, halogen, C₁-C₃ alkyl, C₁-C₃ alkoxy, C₃-C₄ cycloalkyl and C₁-C₃ haloalkyl;
and R⁵ is selected from hydrogen, halogen, CN and C₁-C₆ alkyl.

In another specific embodiment of the present disclosure, when X¹ is a bond, at least two of X², X³ and X⁴ are independently selected from N, O, S or NRⁿ; preferably, X¹ is a bond, at least two of X², X³ and X⁴ are independently selected from Nor NRⁿ.

In some specific embodiments, when X¹ is a bond, one of X², X³ and X⁴ is N, and at least another one is independently selected from N, O, S or NRⁿ.

In another specific embodiment of the present disclosure, when X¹ is a bond, X², X³ and X⁴ are independently selected from N, O, S and NRⁿ; preferably, X¹ is a bond, X², X³ and X⁴ are independently selected from N and NRⁿ.

In some specific embodiments, X¹ is a bond, X² is N, O, S or NR², and X³ is N or NR³. Preferably, X¹ is a bond, X² is O or NR², and X³ is N. More preferably, X¹ is a bond, X² is NR², X³ is N, and X⁴ is CR⁴.

In other specific embodiments, X¹ is a bond, X³ is N, O, S or NR³, and X⁴ is N or NR⁴. Preferably, X¹ is a bond, and X³ is N, X⁴ is NR⁴. More preferably, X¹ is a bond, X³ is N, X⁴ is NR⁴, and X² is CR².

In other specific embodiments, X¹ is a bond, X² is N, O, S or NR², and X⁴ is N, O, S or NR⁴. Preferably, X¹ is a bond, X² is O or N, and X⁴ is N, O, S or NR⁴. More preferably, X¹ is a bond, and X² is N, X⁴ is S, N or NR⁴.

In other specific embodiments, X¹ is a bond, X³ is N, and X⁴ is N or NR⁴, or X² is N, O or NR². Preferably, X¹ is a bond, X³ is N, and X⁴ is NR⁴; more preferably, X¹ is a bond, X³ is N, X⁴ is NR⁴, and X² is CR²;
or
X¹ is a bond, X³ is N, and X² is O or NR²; preferably, X¹ is a bond, X³ is N, X² is O or NR², and X⁴ is CR⁴.

In another specific embodiment of the present disclosure, when X¹ is a bond, X², X³ and X⁴ are independently selected from N and NRⁿ; that is, X¹ is a bond, X² is N or NR², and X³ is N or NR³, X⁴ is N or NR⁴. Preferably, X¹ is a bond, and X² is N, X³ is N, X⁴ is NR⁴.

In another specific embodiment of the present disclosure, X¹ is CR¹. Specifically, the present disclosure provides a compound of formula (Ic) 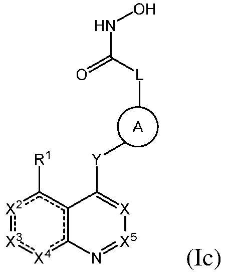
or a pharmaceutically acceptable salt thereof;
each of the variables R¹, X², X³, X⁴, X⁵, X, Y, A and L is defined as formula (I).

In a specific embodiment of the present disclosure, X¹ is CR¹, and at least one of X², X³ and X⁴ is independently selected from N or NRⁿ; that is, X¹ is CR¹, and in X², X³ and X⁴, at least X² is N or NR², or at least X³ is N or NR³, or at least X⁴ is N or NR⁴.

In some specific embodiments, when X¹ is CR¹, X² is N or NR². Preferably, X¹ is CR¹, when X² is N or NR², X³ is CR³. More preferably, X¹ is CR¹, X² is N, and X³ is CR³.

In other specific embodiments, when X¹ is CR¹, X³ is N or NR³. Preferably, X¹ is CR¹, and X³ is N.

In other specific embodiments, X¹ is CR¹, when X³ is N or NR³, X² is CR². Preferably, X¹ is CR¹, when X³ is N or NR³, X² is CR², and X⁴ is CR⁴. More preferably, X¹ is CR¹, X³ is N, X² is CR², and X⁴ is CR⁴.

In other specific embodiments, X¹ is CR¹, and X⁴ is N or NR⁴. Preferably, X¹ is CR¹, when X⁴ is N or NR⁴, X³ is CR³; more preferably, when X¹ is CR¹, X³ is CR³, and X⁴ is N.

In another specific embodiment of the present disclosure, X¹ is CR¹, and X², X³ and X⁴ are all CR¹¹; that is, X¹ is CR¹, and X² is CR², X³ is CR³, X⁴ is CR⁴. Specifically, the present disclosure provides a compound of formula (Id): 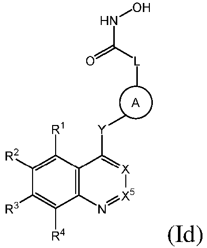
or a pharmaceutically acceptable salt thereof;
wherein, Y is O, S, -S(=O)-, -S(=O)₂-, -C(=O)- or CH₂;
each of the variables R¹, R², R³, R⁴, X⁵, X, A and L is defined as formula (I).

In a preferred specific embodiment of the present disclosure, X¹ is CR¹, X² is CR², X³ is CR³, and X⁴ is CR⁴;
Y is O, S, -S(=O)-, -S(=O)₂-, -C(=O)- or CH₂; and
R¹ is hydrogen or halogen;
R² is selected from hydrogen, halogen, OH and C₁-C₃ alkoxy;
R³ is selected from hydrogen, halogen, OH, C₁-C₃ alkyl and C₁-C₃ alkoxy;
R⁴ is selected from hydrogen, halogens and C₁-C₃ alkoxy;
R⁵ is selected from hydrogen, halogen, CN and C₁-C₆ alkyl;
and preferably, R⁶, R⁷ and R⁸ are each independently selected from hydrogen and C₁-C₃ alkyl.

In some specific embodiments, Y is O, S, -S(=O)-, -S(=O)₂- or -C(=O)-.

In other specific embodiments, Y is O, S, -S(=O)- or -S(=O)₂-.

In some specific embodiments, Y is O or CH₂.

In another specific embodiment of the present disclosure, X¹ is CR¹, X² is CR², X³ is CR³, and X⁴ is CR⁴;
Y is O, S, -S(=O)-, -S(=O)₂-, -C(=O)- or CH₂;
and R¹ is hydrogen or halogen;
R² is selected from hydrogen, halogen, OH and C₁-C₃ alkoxy;
R³ is selected from hydrogen, halogen, OH, C₁-C₃ alkyl and C₁-C₃ alkoxy;
R⁴ is selected from hydrogen, halogens and C₁-C₃ alkoxy;
R⁵ is selected from hydrogen, halogen, CN and C₁-C₆ alkyl;
R⁶ is selected from hydrogen and C₁-C₃ alkyl;
and R⁷ and R⁸ are each independently selected from hydrogen and C₁-C₃ alkyl; preferably, Y is O or CH₂.

In a preferred specific embodiment of the present disclosure, the present disclosure provides a compound of formula (I) as previously described or a pharmaceutically acceptable salt thereof; wherein: 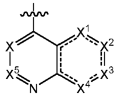 is selected from 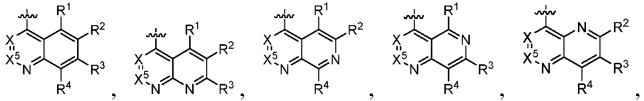 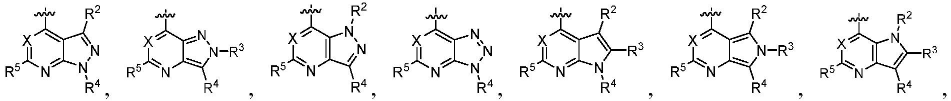 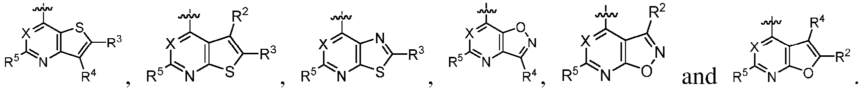 Preferably, selected from

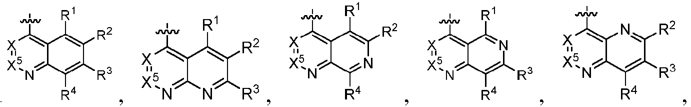

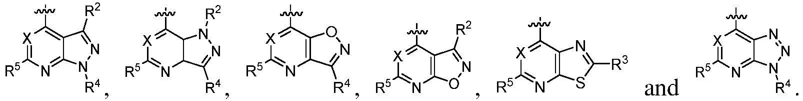

More preferably, selected from 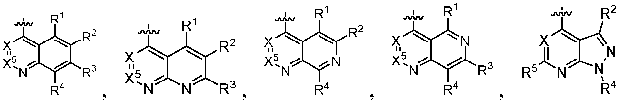 and

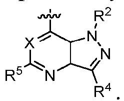

In a preferred embodiment of the present disclosure, a compound of formula (I) or a pharmaceutically acceptable salt thereof is provided, wherein, X¹ is a bond or CR¹. Specifically, the present disclosure provides the following (Ie1), (Ie2), (Ie3), (Ie4), (Ie5) and (Ie6) compounds: 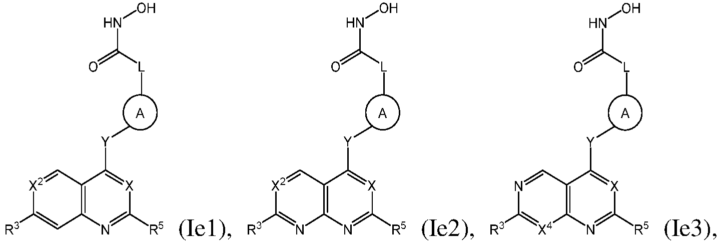 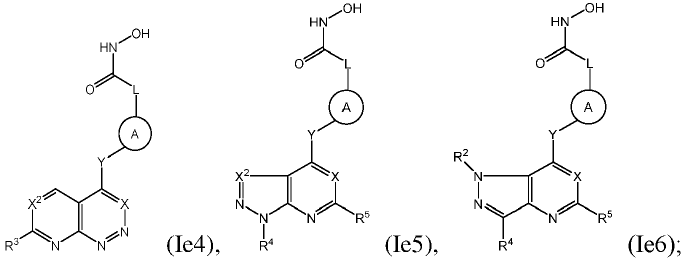
or a pharmaceutically acceptable salt thereof;
wherein, each of the variables X², R³, R⁴, R⁵, X, Y, A and L is defined as formula (I).

Preferably, L is NR^{a} or (CR⁹R¹⁰)ₘ; and m is 1 or 2. More preferably, L is CR⁹R¹⁰.

In an embodiment of the present disclosure, the compound of formula (I) is selected from the following embodiment compounds:

| Compo und | Structure | Compo und | Structure | Compo und | Structure |
|---|---|---|---|---|---|
| 1 | 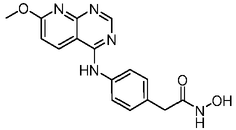 | 2 | 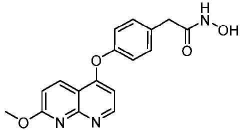 | 3 | 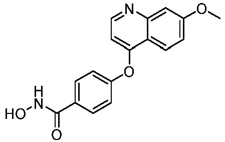 |
| 4 | 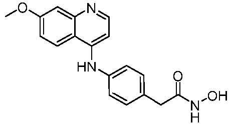 | 7 | 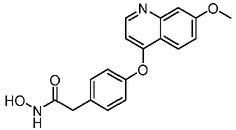 | 10 | 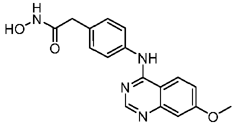 |
| 5 | 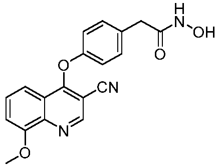 | 8 | 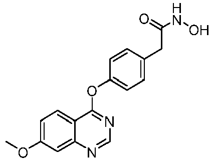 | 11 | 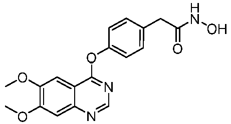 |
| 6 | 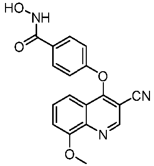 | 9 | 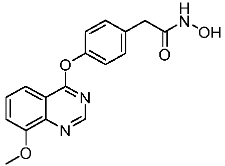 | 12 | 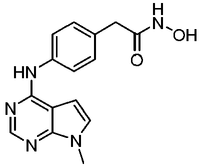 |
| 13 | 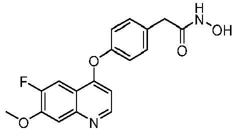 | 16 | 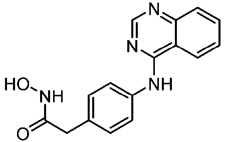 | 19 | 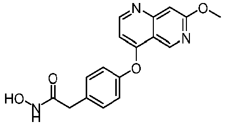 |
| 14 | 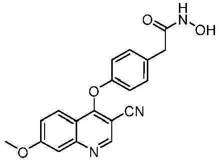 | 17 | 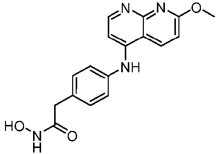 | 20 | 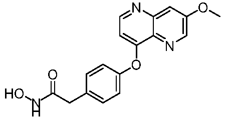 |
| 15 | 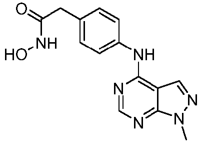 | 18 | 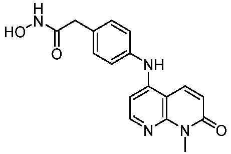 | 21 | 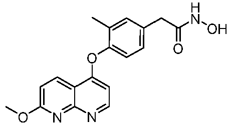 |
| 22 | 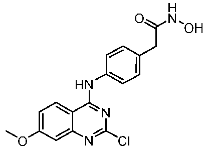 | 25 | 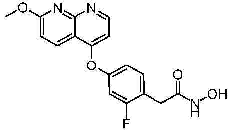 | 28 | 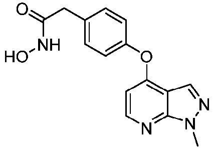 |
| 23 | 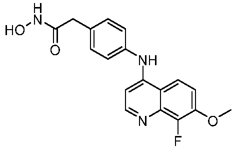 | 26 | 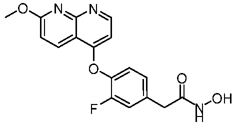 | 29 | 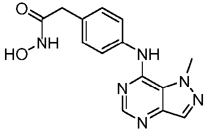 |
| 24 | 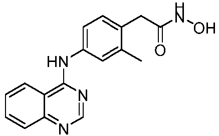 | 27 | 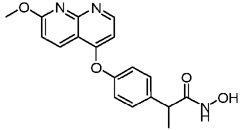 | 30 | 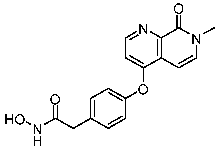 |
| 31 | 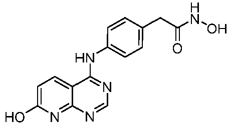 | 34 | 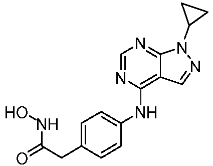 | 37 | 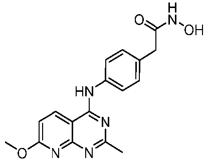 |
| 32 | 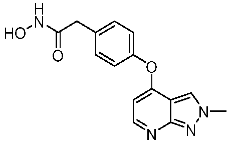 | 35 | 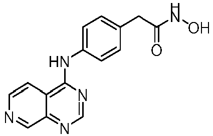 | 38 | 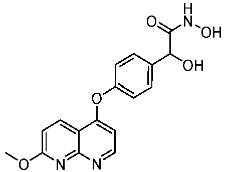 |
| 33 | 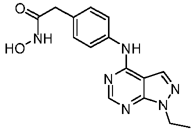 | 36 | 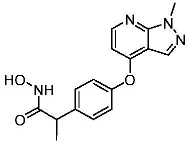 | 39 | 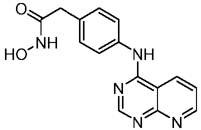 |
| 40 | 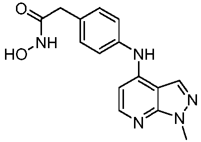 | 43 | 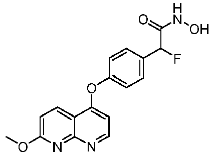 | 46 | 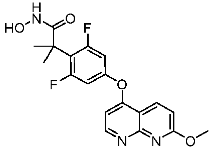 |
| 41 | 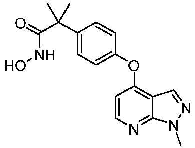 | 44 | 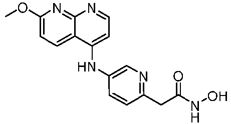 | 47 | 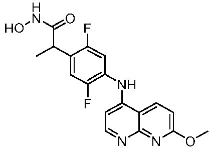 |
| 42 | 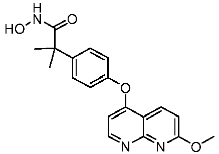 | 45 | 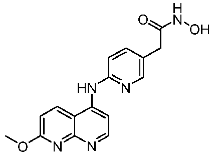 | 48 | 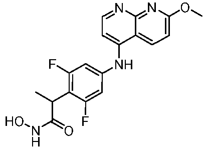 |
| 49 | 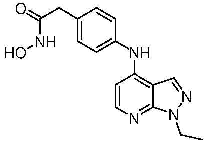 | 52 | 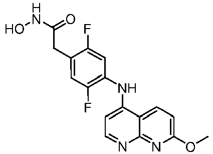 | 55 | 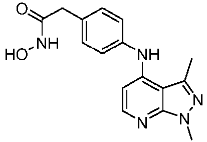 |
| 50 | 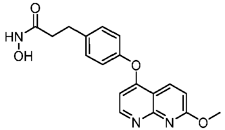 | 53 | 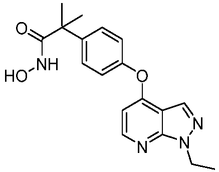 | 56 | 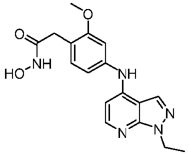 |
| 51 | 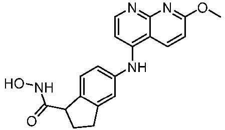 | 54 | 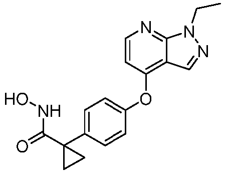 | 57 | 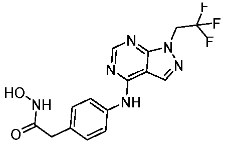 |
| 58 | 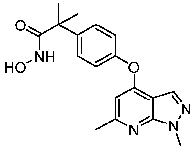 | 61 | 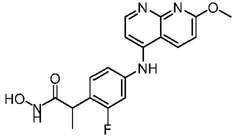 | 64 | 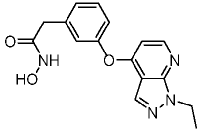 |
| 59 | 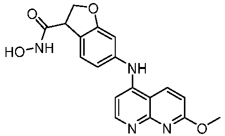 | 62 | 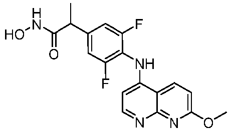 | 65 | 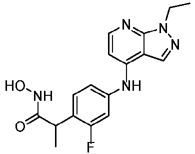 |
| 60 | 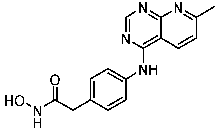 | 63 | 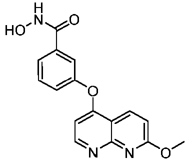 | 66 | 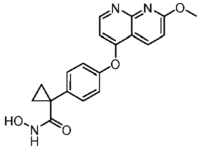 |
| 67 | 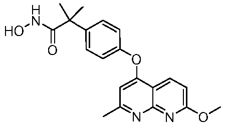 | 70 | 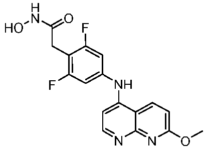 | 73 | 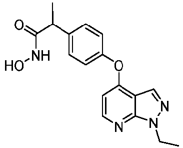 |
| 68 | 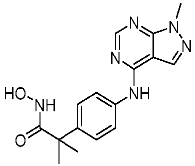 | 71 | 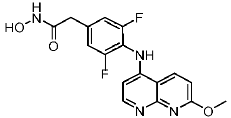 | 74 | 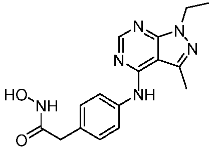 |
| 69 | 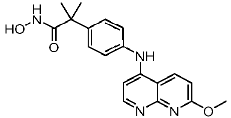 | 72 | 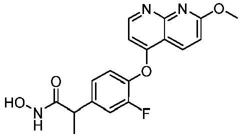 | 75 | 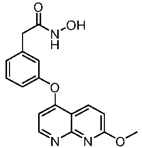 |
| 76 | 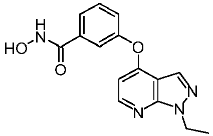 | 79 | 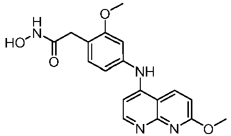 | 82 | 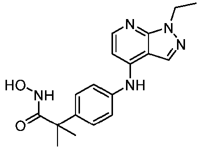 |
| 77 | 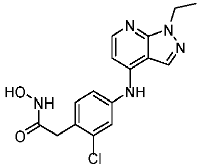 | 80 | 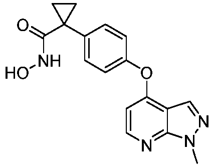 | 83 | 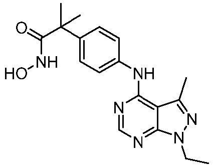 |
| 78 | 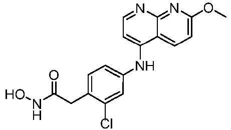 | 81 | 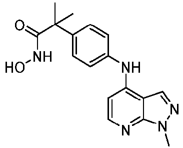 | 84 | 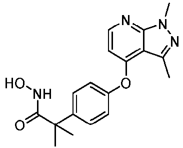 |
| 85 | 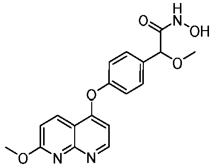 | 86 | 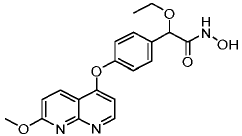 | 87 | 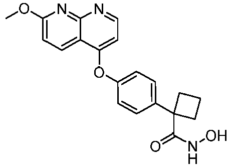 |
| 88 | 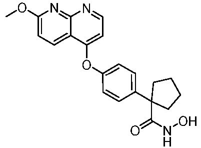 | 89 | 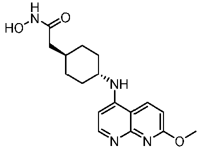 | 90 | 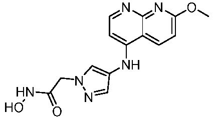 |
| 91 | 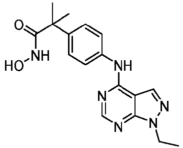 | 92 | 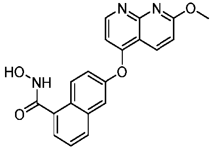 | 97 | 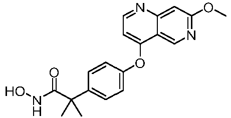 |
| 93 | 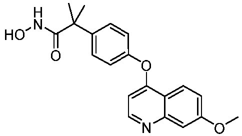 | 94 | 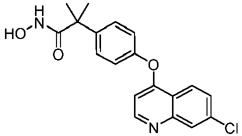 | 98 | 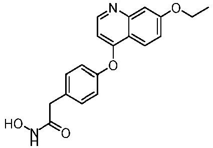 |
| 95 | 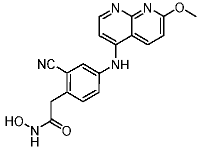 | 96 | 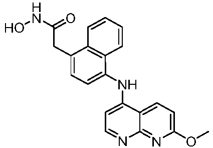 | 99 | 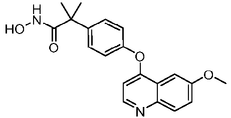 |
| 101 | 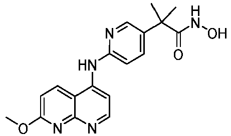 | 102 | 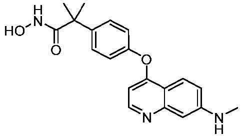 | 107 | 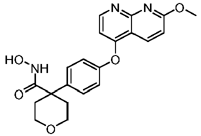 |
| 103 | 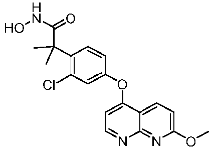 | 104 | 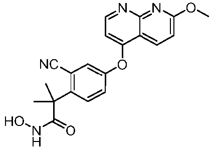 | 108 | 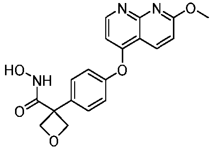 |
| 105 | 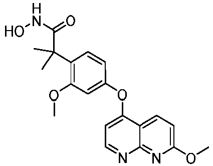 | 106 | 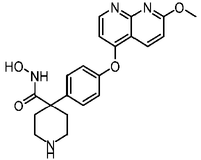 | 109 | 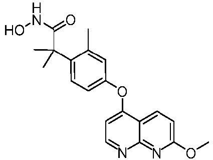 |
| 111 | 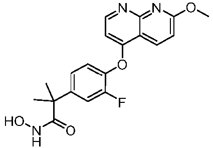 | 112 | 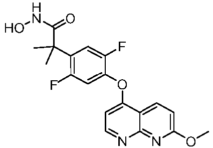 | 110 | 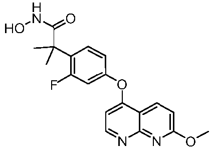 |
| 113 | 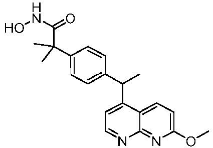 | 114 | 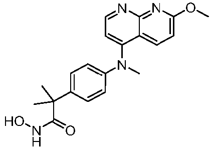 | 117 | 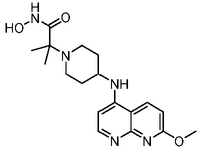 |
| 115 | 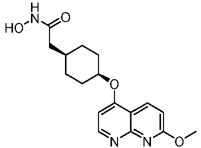 | 116 | 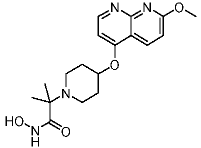 | 118 | 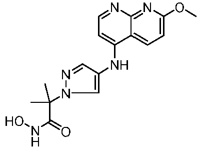 |
| 119 | 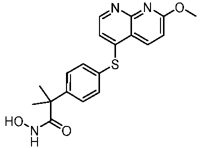 | 120 | 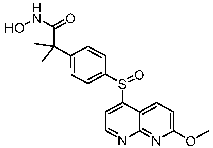 | 125 | 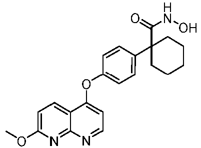 |
| 121 | 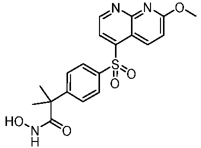 | 122 | 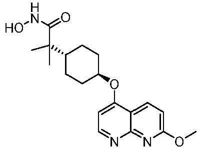 | 126 | 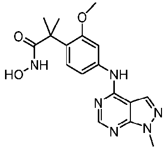 |
| 123 | 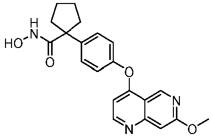 | 124 | 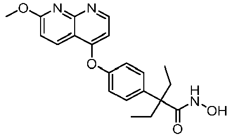 | 127 | 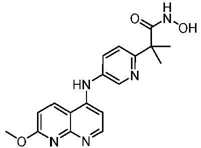 |
| 128 | 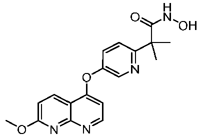 | 129 | 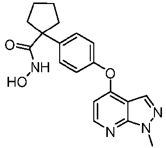 | 130 | 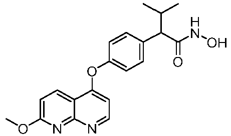 |
| 131 | 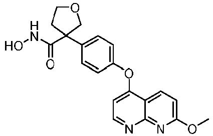 | 132 | 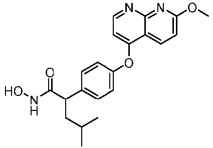 | 137 | 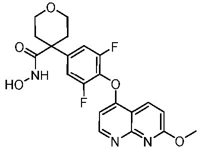 |
| 133 | 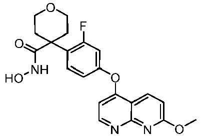 | 134 | 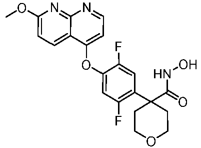 | 138 | 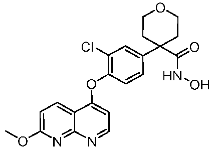 |
| 135 | 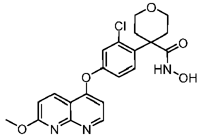 | 136 | 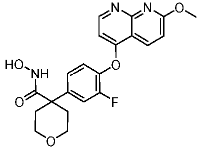 | 139 | 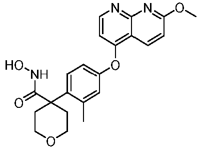 |
| 140 | 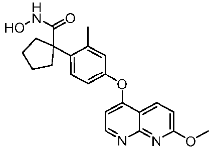 | 141 | 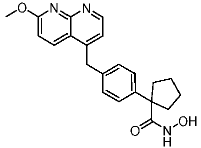 | 142 | 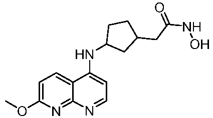 |
| 143 | 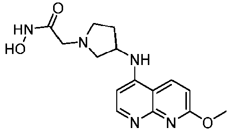 | 144 | 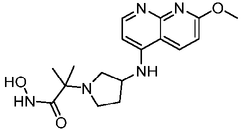 | 149 | 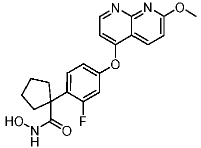 |
| 145 | 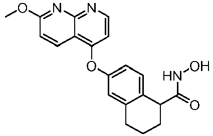 | 146 | 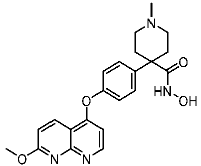 | 150 | 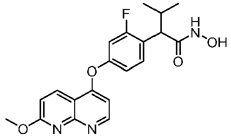 |
| 147 | 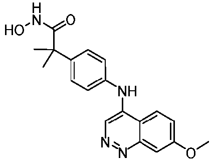 | 148 | 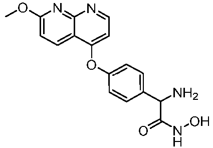 | 151 | 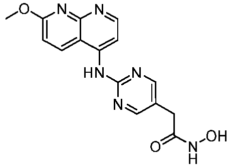 |
| 152 | 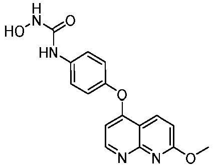 | 153 | 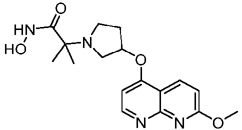 | 154 | 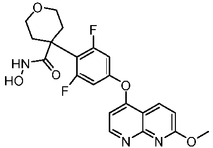 |
| 155 | 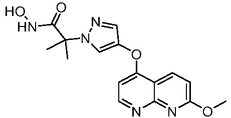 | 156 | 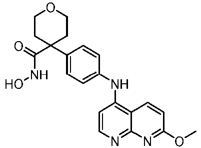 | 157 | 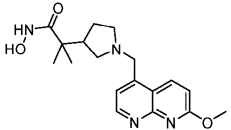 |
| 158 | 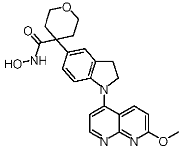 | 159 | 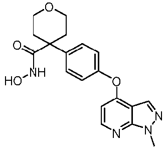 | 160 | 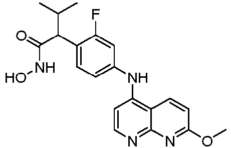 |
| 161 | 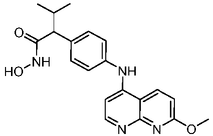 | 162 | 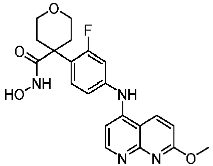 | 167 | 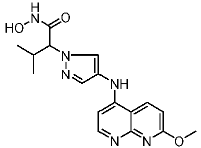 |
| 163 | 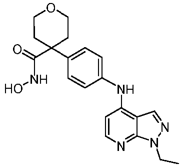 | 164 | 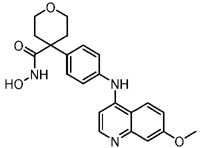 | 168 | 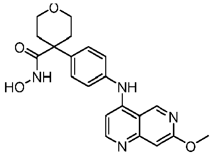 |
| 165 | 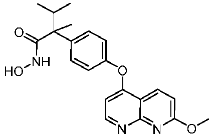 | 166 | 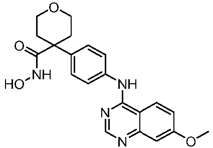 | 169 | 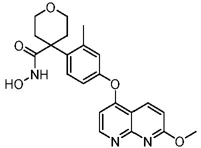 |
| 170 | 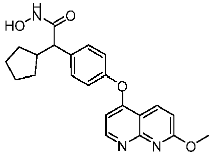 | 171 | 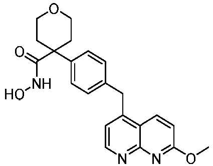 | 172 | 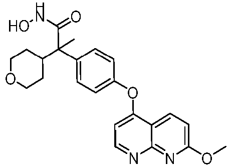 |
| 173 | 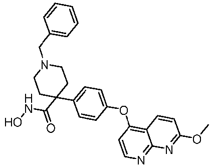 | 174 | 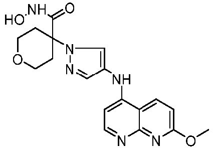 | 179 | 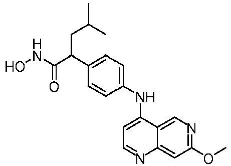 |
| 175 | 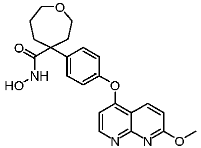 | 176 | 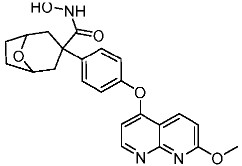 | 180 | 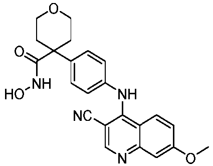 |
| 177 | 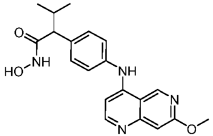 | 178 | 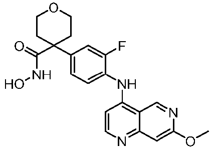 | 181 | 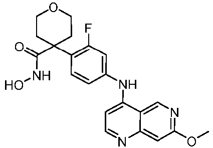 |
| 182 | 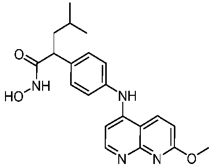 | 183 | 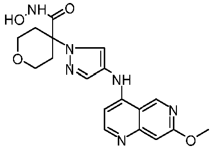 | 184 | 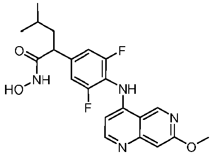 |
| 185 | 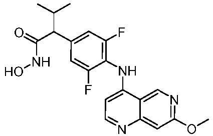 | 186 | 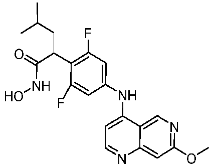 | 191 | 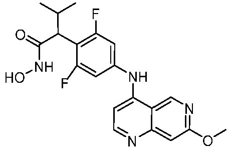 |
| 187 | 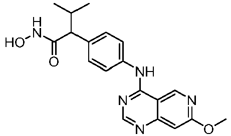 | 188 | 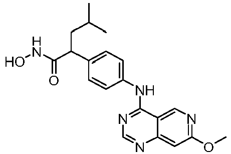 | 192 | 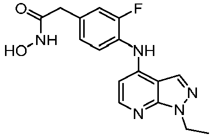 |
| 189 | 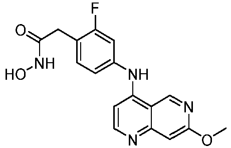 | 190 | 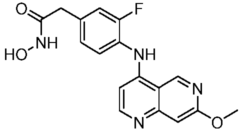 | 193 | 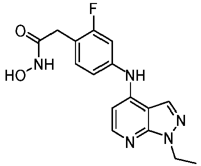 |
| 194 | 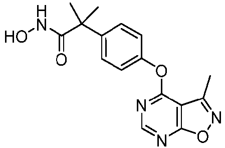 | 195 | 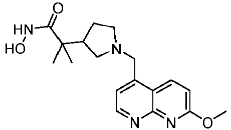 | 196 | 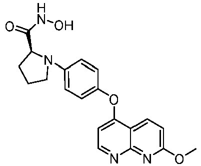 |
| 197 | 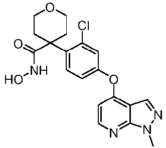 | 198 | 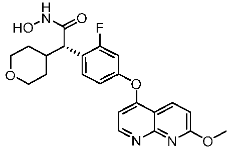 | 203 | 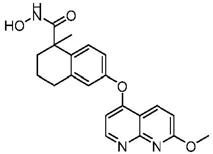 |
| 199 | 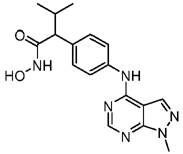 | 200 | 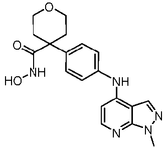 | 204 | 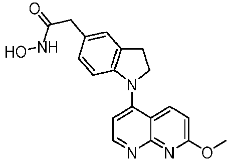 |
| 201 | 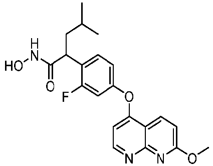 | 202 | 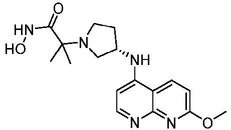 | 205 | 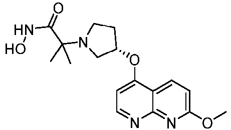 |
| 206 | 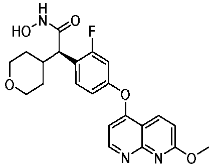 | 207 | 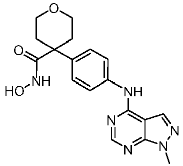 | 208 | 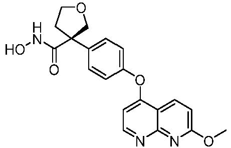 |
| 209 | 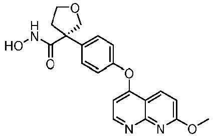 | 210 | 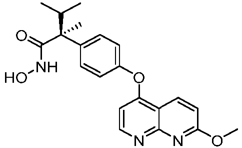 | 213 | 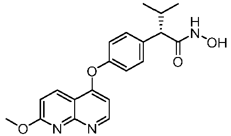 |
| 211 | 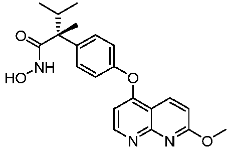 | 212 | 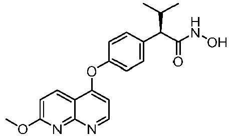 | 214 | 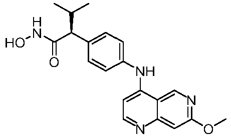 |
| 215 | 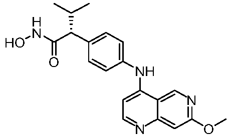 | | | | |

On the other hand, the present disclosure provides a method for preparing the compound of the present disclosure.

In an embodiment, the present disclosure also provides an intermediate for preparing the compound of the present disclosure and a preparation method thereof.

On the other hand, the present disclosure provides a composition comprising at least one compound or the pharmaceutically acceptable salt thereof of the present disclosure.

In an embodiment, the present disclosure provides a pharmaceutical composition comprising at least one compound or the pharmaceutically acceptable salt thereof of the present disclosure, as well as at least one pharmaceutically acceptable carrier, diluent or excipient.

In another embodiment, the present disclosure provides a pharmaceutical composition comprising a therapeutically effective amount of at least one compound of the present disclosure, or a stereoisomer, a geometric isomer, a tautomer, a pharmaceutically acceptable salt, a crystal form, a solvate, a hydrate or a prodrug thereof, and at least one pharmaceutically acceptable carrier, diluent or excipient. In certain embodiments of the pharmaceutical composition, the pharmaceutical composition is formulated for any suitable route of administration, for example, intravenous administration, intramuscular administration, oral administration, rectal administration, inhalation administration, nasal administration, local administration, eye administration or ear administration. In other embodiments of the pharmaceutical composition, the pharmaceutical composition is a tablet, a pill, a capsule, a liquid agent, an inhalant, a nasal spray solution agent, a suppository, a solution agent, an emulsion, an ointment, an eye drop or an ear drop.

On the other hand, the present disclosure provides a pharmaceutical combination product comprising at least one compound or the pharmaceutically acceptable salt thereof of the present disclosure, as well as one or more other active agents.

On the other hand, the present disclosure provides the use of a compound of formula (I), or a stereoisomer, a geometric isomer, a tautomer, a pharmaceutically acceptable salt, a crystal form, a solvate, a hydrate or a prodrug thereof in the preparation of a drug for preventing, treating or alleviating disorders or diseases caused by tumors or infections in a patient.

In an embodiment, the tumor or infection is caused by changes in interferon gene stimulating factor (STING).

In another embodiment, the present disclosure provides the use of a compound of formula (I), or a stereoisomer, a geometric isomer, a tautomer, a pharmaceutically acceptable salt, a crystal form, a solvate, a hydrate or a prodrug thereof in the preparation of a drug for preventing, treating or alleviating diseases or obstacles mediated by ENPP1, such as cancer or infectious diseases or conditions.

In another embodiment, the present disclosure provides the compound of the present disclosure, which can be used alone or optionally in combination with another compound of the present disclosure and/or at least one other therapeutic agent in therapy to treat ENPP1-mediated diseases or disorders.

In another embodiment, the present disclosure also provides the use of the compound of the present disclosure in the preparation of a medicament, wherein the medicament is used alone or optionally in combination with another compound of the present disclosure and/or at least one other therapeutic agent to treat ENPP1-mediated diseases or disorders.

In another embodiment, the present disclosure provides a combination preparation of the compound of the present disclosure and one or more additional therapeutic agents for use in therapy.

In another embodiment, the present disclosure provides a combination of the compound of the present disclosure and one or more additional therapeutic agents for simultaneous or separate use in therapy.

In another embodiment, the present disclosure provides a combination preparation of the compound of the present disclosure and one or more additional therapeutic agents for simultaneous, separate or sequential use in the treatment of ENPP1-mediated diseases or disorders. The compound can be administered as a pharmaceutical composition described herein.

On the other hand, the present disclosure provides a method for treating or preventing ENPP1-mediated diseases or disorders, including administering a therapeutically effective amount of the compound of the present disclosure, or a stereoisomer, a geometric isomer, a tautomer, a pharmaceutically acceptable salt, a crystal form, a solvate, a hydrate or a prodrug thereof or a pharmaceutical composition comprising them, to an individual in need thereof.

In one embodiment, the present disclosure provides a method for treating ENPP1-mediated diseases or disorders, including administering a therapeutically effective amount of at least one compound of the present disclosure alone or optionally in combination with another compound of the present disclosure and/or at least one other therapeutic agent to a patient in need of the treatment.

In another embodiment, the present disclosure provides a method for treating ENPP1-mediated diseases or disorders, including administering a first and second therapeutic agent in a therapeutically effective amount to a patient in need thereof, wherein the first therapeutic agent is the compound of the present disclosure, and the second therapeutic agent is another compound of the present disclosure and/or at least one other therapeutic agent.

In some embodiments of the present disclosure, ENPP1-mediated diseases or obstacles include but are not limited to tumors, infectious diseases or obstacles, especially cancers, such as recurrent or refractory cancers, metastatic cancers, etc.

In another embodiment, ENPP1-mediated diseases or disorders are recurrent or refractory cancers.

In another embodiment, ENPP1-mediated diseases or disorders are metastatic cancer.

In another embodiment, ENPP1-mediated diseases or disorders are tumors, especially solid tumors, for example, breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer.

In another embodiment, ENPP1-mediated diseases or disorders are tumors, especially hematological malignancies; especially leukemia, lymphoma or myeloma.

In another embodiment, ENPP1-mediated diseases or disorders are infectious diseases or disorders, including but not limited to herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.

In a specific embodiment, EED Alphascreen binding, LC-MS and/or ELISA analysis disclosed herein are used, and the compound of the present disclosure has a IC₅₀ value of ≤ 5µM, preferred a IC₅₀ value of ≤ 1 µM, more preferably a IC₅₀ value of ≤ 0.5 µM, even more preferably ≤ 0.125 µM, and most preferably a IC₅₀ value of ≤ 0.1 µM.

It should be understood that within the scope of the present disclosure, the technical features defined in each of the technical solutions the present disclosure and the specific technical features described in the following (as examples) can be combined with each other to form new or preferred technical solutions that are not described one by one in the specification. It is also understood that each individual element of the embodiment is its own independent embodiment.

Other features of the present disclosure should become apparent in the course of the above descriptions of exemplary embodiments that are given for illustration of the disclosure and are not intended to be limiting thereof.

### Terminology

In the present disclosure, unless otherwise explicitly stated, the terms used in the present disclosure have the meanings defined below. Terms not explicitly defined in the present disclosure have general meanings that are generally understood by those skilled in the art.

The term "a", "an", "the" and similar terms used in the context of the present disclosure (especially in the context of the claims) are to be construed to cover both the singular and plural unless otherwise indicated herein or clearly contradicted by the context.

A dash ("-") that is not between two letters or symbols is used to indicate a connecting site for a substituent. For example, -O(C₁-C₃ alkyl) indicates that the group is connected to the rest of the molecule through an oxygen atom. However, when the connecting site of the substituent is obvious to those skilled in the art, for example, for substituents such as halogen and hydroxyl, the "-" can be omitted.

When a group has a wavy line " 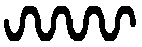 ", the wavy line represents the connection position between the group and the rest of the molecule.

As used herein, " - - - - :" represents single or double bonds. Those skilled in the art can determine - - - - whether to represent a single bond or a double bond based on the valence of the relevant ring atoms and the connected groups, which falls within the scope of their abilities. Those skilled in the art can understand that the relevant rings can be saturated, partially saturated or aromatic.

As used herein, "heteroatoms" refer to nitrogen (N), oxygen (O), or sulfur (S) atoms, particularly nitrogen or oxygen atoms, each of which can be substituted or unsubstituted, including their oxidized forms. Examples of heteroatoms include but are not limited to -O-, - N=, -NR-, -S-, -S(O)- and -S(O)₂-, wherein R is hydrogen, C₁-C₄ alkyl or nitrogen protective groups (for example, benzyloxycarbonyl, p-methoxybenzylcarbonyl, tert-butoxycarbonyl, acetyl, benzoyl, benzyl, p-methoxy-benzyl, p-methoxy-benzyl, 3,4-dimethoxybenzyl, etc.). Any heteroatom with an unsatisfied valence bond is considered to have a hydrogen atom sufficient to satisfy the valence bond, unless otherwise indicated.

As used herein, "halogen" or "halogenated" refers to fluorine, chlorine, bromine and iodine. The preferred halogen as a substituent is fluorine and chlorine.

As used herein, "alkyl" refers to a monovalent hydrocarbon group of completely saturated straight or branched chains. Alkyl preferably contains 1-20 carbon atoms, more preferably 1-16 carbon atoms, 1-10 carbon atoms, 1-8 carbon atoms, 1-6 carbon atoms, 1-4 carbon atoms or 1-3 carbon atoms. The number before alkyl represents the number of carbon atoms. For example, "C₁-C₆ alkyl" represents an alkyl with 1-6 carbon atoms, "C₁-C₄ alkyl" represents an alkyl with 1-4 carbon atoms, and "C₁-C₃ alkyl" represents an alkyl with 1-3 carbon atoms. Representative examples of alkyl include but are not limited to methyl, ethyl, n-propyl, isopropyl, n-butyl, secbutyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, etc. Whether the term "alkyl" appears alone or as part of other functional groups such as haloalkyl, alkoxy, etc., this definition applies.

As used herein, "alkenyl" refers to a monovalent hydrocarbon group of straight or branched chains containing at least one double bond. Alkenyl preferably contains 2-20 carbon atoms, more preferably 2-10 carbon atoms, 2-8 carbon atoms, 2-6 carbon atoms or 2-4 carbon atoms. The number before alkenyl represents the number of carbon atoms. Representative examples of alkenyl include but are not limited to vinyl, propylene, isopropenyl, butene, isobutenyl, pentenyl, isopentenyl, hexenyl, heptenenyl, octenyl, etc.

As used herein, "alkynyl" refers to a monovalent hydrocarbon group of straight or branched chains containing at least one triple bond. Alkynyl preferably contains 2-20 carbon atoms, more preferably 2-10 carbon atoms, 2-8 carbon atoms, 2-6 carbon atoms or 2-4 carbon atoms. The number before alkynyl represents the number of carbon atoms. Representative examples of alkynyl include but are not limited to acetylene, propargyl, isopropyne, butyrgyl, isobutyrgyl, pentyne, isopentyne, hexyne, heptanyne, octyne, etc.

As used herein, "alkoxy" refers to an alkyl, i.e. an alkyl-O group, connected by an oxygen bridge as defined herein. The number before alkoxy represents the number of carbon atoms. For example, "C₁-C₆ alkoxy" represents an alkoxy with 1-6 carbon atoms, i.e. -O-C₁₋₆ alkyl; "C₁-C₄ alkoxy" refers to an alkoxy with 1-4 carbon atoms, i.e. -O-C₁₋₄ alkyl; and "C₁-C₃ alkoxy" refers to an alkoxy with 1-3 carbon atoms, i.e. -O-C₁₋₃ alkyl. Representative examples of alkoxy include but are not limited to methoxy, ethoxy, propanoxy, 2-propanoxy, butoxy, tert butoxy, pentoxy, hexoxy, etc. Preferably, alkoxy contains about 1-6 or about 1-4 carbons, etc.

As used herein, "cycloalkyl" refers to saturated or partially saturated non-aromatic carbon rings, including monocyclo, bicyclo, or tricyclo, preferably with 3-12 ring carbon atoms, more preferably 3-10 ring carbon atoms, for example, 3-8, 3-7, 3-6, 4-10 or 4-8 ring carbon atoms. "C₃-C₈ cycloalkyl" is intended to include C₃, C₄, C₅, C₆, C₇ and C₈ cycloalkyl groups; "C₃-C₆ cycloalkyl" is intended to include C₃, C₄, C₅ and C₆ cycloalkyl; and so on. Exemplary monocyclic cycloalkyls include but are not limited to cyclopropyl, cyclobutyl, cyclopentyl, cyclopentenyl, cyclohexyl and cyclohexene. Exemplary bicyclic cycloalkyls include bornyl, tetrahydronaphthyl, decahydronaphthyl, bicyclo[2.1.1]hexyl, bicyclo[2.2.1]heptyl, bicyclo[2.2.1]heptyl, 6,6-dimethyldicyclo[3.1.1]heptyl, 2,6,6-trimethyldicyclo[3.1.1]heptyl, bicyclo[2.2.2]octyl, etc. Exemplary tricyclic cycloalkyls include diamond alkyl, etc.

As used herein, "haloalkyl" refers to an alkyl as defined herein where one or more hydrogen atoms, for example, 1, 2, 3, 4, 5, 6 or 7 hydrogen atoms, for example, 1, 2 or 3 hydrogen atoms, are replaced by a halogen, and when more than one hydrogen atom is replaced by a halogen atom, the halogen atom can be the same or different from each other. For example, "C₁-C₄ haloalkyl" is intended to include C₁, C₂, C₃ and C₄ haloalkyl groups, and "C₁-C₃ haloalkyl" is intended to include C₁, C₂ and C₃ haloalkyl groups. Examples of haloalkyl include but are not limited to fluoromethyl, difluoromethyl, trifluoromethyl, trichloromethyl, 1,1-difluoroethyl, 1,1-difluoropropyl and 1,1,1-trifluoropropyl. Examples of haloalkyl also include "fluoroalkyl", which is intended to include alkyl as defined herein where one or more hydrogen atoms are substituted by fluorine atoms. The "haloalkyl" herein is preferred that at most three hydrogen atoms in the alkyl are replaced by halogens.

As used herein, "haloalkoxy" refers to a haloalkyl as defined above with a specified number of carbon atoms connected by an oxygen bridge, where one or more hydrogen atoms, for example, 1, 2, 3, 4, 5, 6 or 7 hydrogen atoms, for example, 1, 2 or 3 hydrogen atoms, are replaced by halogens. For example, "C₁-C₆ haloalkoxy" or "C₁ to C₆ haloalkoxy" is intended to include C₁, C₂, C₃, C₄, C₅ and C₆ haloalkoxy groups. Examples of haloalkoxy include but are not limited to fluoromethoxy, difluoromethoxy, trifluoromethoxy, 2-fluoroethoxy, and 2,2,2-trifluoroethoxy. Examples of haloalkoxy also include "fluoroalkoxy".

As used herein, "aryl" refers to a monocyclic, bicyclic or tricyclic carbocyclic hydrocarbyl consisting of one or more rings fused together, with 6-20, preferably 6-14, more preferably 6-12, most preferably 6-10, such as 6-9 cyclic carbon atoms, wherein at least one ring is an aromatic ring, and the other rings (if present) can be aromatic or non-aromatic. The preferred aryl is an aryl with 6-10 cyclic carbon atoms, namely 6- to 10 membered aryl, which includes: monocyclic aryl (such as phenyl); or a fused double ring system, where one ring is an aromatic ring and the other ring is an aromatic ring (such as in naphthalene or biphenyl) or a non-aromatic ring (such as in dihydroindene or tetrahydronaphthalene). Non-limiting examples of aryl include phenyl, biphenyl, naphthyl, tetrahydronaphthyl, indenyl, dihydroindenyl or anthryl.

As used herein, "heteroaryl" refers to a 5-14 membered, preferably 5-10 membered, more preferably 5-7 membered, or 5-6 membered aromatic ring system containing 1-8, preferably 1-4, further preferably 1-3, more preferably 1 or 2 heteroatoms selected from N, O or S, including a single ring, a double ring, or a fused multi ring, with the remaining ring atoms being carbon atoms. The preferred heteroaryl is 5-10 membered heteroaryl, more preferably 5-7 membered heteroaryl or 5-6 membered heteroaryl, each containing 1, 2 or 3 heteroatoms selected from N, O or S. Examples of heteroaryl include but are not limited to pyrrolyl, furanyl, thienyl, pyrazolyl, imidazolyl, triazolyl, thiazolyl, isothiazolyl, oxazolyl, pyridyl, pyranyl, pyrazinyl, pyridazinyl, pyrimidinyl, oxazinyl, oxadiazinyl, quinolinyl, isoquinolinyl, cinnolinyl, quinazolinyl, quinoxalinyl, benzoxazinyl, 2H-chromone, benzopyranyl, benzothienyl, indolyl, indazolyl, benzoimidazolyl, benzooxazolyl, benzothiazolyl, 7-azaindolyl, 6-azaindolyl, 5-azaindolyl, 4-azaindolyl, 1H-benzo[d][1,2,3]triazolyl, etc.

As used herein, "heterocycloalkyl" refers to a cycloalkyl as defined in the present application, provided that one or more cyclic carbons are replaced by heteroatoms selected from N, O or S, the heteroatoms is, for example, -O-, -N=, -NR-, -S-, -S(O)- and -S(O)₂-, wherein R is hydrogen, C₁₋₄ alkyl or nitrogen protective groups (for example, benzyloxycarbonyl, p-methoxybenzylcarbonyl, tert-butoxycarbonyl, acetyl, benzoyl, benzyl, p-methoxy-benzyl, p-methoxy-benzyl, 3,4-dimethoxybenzyl, etc.). Preferably, heterocycloalkyl is saturated and partially unsaturated non-aromatic rings of a single, two, or three ring with 3-20 ring atoms, such as 3-12 ring atoms, such as 3-8 ring atoms, such as 3-6 ring atoms. More preferably, heterocycloalkyl preferably contains 4- to 12-membered heterocycloalkyl containing 1, 2, or 3 heteroatoms selected from N, O and S, preferably 4- to 8-membered heterocycloalkyl, more preferably 4- to 7-membered, 4- to 6-membered, or 5- to 6-membered heterocycloalkyl, wherein the heteroatoms are substituted or unsubstituted, such as being substituted by C₁-C₄ alkyl. For example, examples of heterocycloalkyl include but are not limited to oxiranyl, aziridinyl, azetidinyl, oxetanyl, azolidinyl (pyrrolidinyl), tetrahydrofuryl, tetrahydrothienyl, tetrahydrothienyl 1,1-dioxide, pyrazolidinyl, imidazolidinyl, oxazolidinyl, thiazolidinyl, isothiazolidinyl, pyrrolidyl-2-one, imidazolonyl, piperidyl (hexahydropyridine), N-methylpiperidyl, tetrahydropyranyl, oxazinanyl, 1,3-oxazinanyl, hexahydropyrimidinyl, piperazinyl, piperidinylone, 1,4-dioxa-8-aza-spiro[4.5]decane-8-yl, morpholino, thiomorpholino, sulfanomorpholino, sulfonomorpholino, octahydropyrrolo[3,2-b]pyrrolyl, etc.

As used herein, "partially saturated heterocycles" refer to partially hydrogenated non-aromatic rings that can exist as single or double rings (including fused rings). Unless otherwise stated, the partially saturated heterocycles are typically 3- to 12-membered, preferably 5- to 10-membered, more preferably 9- to 10- membered monocyclic system or bicyclic system containing at least one heteroatom, for example, 1 to 3 heteroatoms, preferably 1 or 2 heteroatoms, wherein the heteroatoms are selected from N, O or S, and the heteroatoms are - O-, -N=, -NR- or -S-, and wherein R is hydrogen, C₁₋₄ alkyl, or nitrogen protective groups. Partially saturated heterocycles include functional groups such as dihydropyrrolyl, dihydrofuranyl, dihydrooxazolyl, dihydropyridyl, imidazolin, 1H-dihydroimidazole, 2H-pyranyl, 2H-chromenyl, dihydrooxazinyl, etc. Partially saturated heterocycles also include heterocycles with fused aromatic or heteroaromatic rings, preferably with 9-10 ring members (such asdihydrobenzofuranyl, dihydroisobenzofuranyl, dihydroindolyl (or 2,3-dihydroindolyl), dihydrobenzothiophenyl, dihydrobenzothiazolyl, dihydrobenzopyranyl, tetrahydroquinolyl, tetrahydroisoquinolyl, tetrahydropyridino[3,4-b]pyrazinyl)

As used herein, "partially or completely saturated heterocycles" refer to non-aromatic rings that have been partially or completely hydrogenated and can exist as single rings, double rings (including fused rings) or spiral rings. Unless otherwise stated, heterocycles are typically 3- to 12-membered rings containing 1 to 3, preferably 1 or 2 independently selected heteroatoms from sulfur, oxygen and/or nitrogen, and preferably 5- to 10-membered rings. When the term "partially or completely saturated heterocycles" is used, it is intended to include "heterocycloalkyl" and "partially saturated heterocycles". Examples of spiral rings include 2,6-diazaspiro[3.3]heptanyl, 3-azaspiro[5.5]undecyl, 3,9-diazaspiro[5.5]undecyl, etc.

As used herein, "heterocycles" refer to completely saturated or unsaturated, aromatic or non-aromatic cyclic groups, which are intended to include "heterocycloalkyl", "partially or completely saturated heterocycles", "partially saturated heterocycles", "completely saturated heterocycles" and "heteroaryls". For example, it is a ring system consisting of 4- to 7-membered single rings, 7- to 12-membered double rings, or 10- to 15-membered triple rings, which contain at least one heteroatom on a ring containing at least one carbon atom. A ring containing heteroatoms of the heterocycle can contain 1-6, preferably 1, 2 or 3 heteroatoms selected from nitrogen, oxygen, or sulfur atoms, wherein nitrogen and sulfur heteroatoms can also be optionally oxidized. Preferably, heterocycle is 4-to-7-membered monocyclic heterocycle.

Exemplary monocyclic heterocycles include pyrrolidine, pyrrole, pyrazole, oxocyclobutane, pyrazoline, imidazole, imidazoline, imidazolidine, triazole, thiazole, thiadiazole, thiazolidine, isothiazole, isothiazolidine, furan, tetrahydrofuran, thiophene, piperidine, piperazine, 2-oxopiperazine, 2-oxopiperidine, 2-oxypyrrolidine, 4-piperidone, pyridine, pyrazine, pyrimidine, pyridazine, tetrahydropyran, morpholine, thiomorpholine, sulfanomorpholino, sulfonomorpholino, 1,3-dioxolane and tetrahydro-1,1-dioxothiophene, 1,1,4-trioxo-1,2,5-thiadiazolidin-2-, etc.

Exemplary bicyclic heterocycles include indole, dihydroindole, benzothiazole, etc.

As used herein, "heterocyclic group" refers to a group formed by the loss of one or more hydrogen atoms from the heterocycle defined above. Heterocyclic groups can be connected at heteroatoms or carbon atoms.

As used herein, "carbon ring" refers to a saturated or unsaturated monocyclic, bicyclic or tricyclic hydrocarbon group consisting of 3-12 carbon atoms. The carbon ring is preferably composed of 3-8 cyclic carbon atoms, such as 3-7 or 4-7 cyclic carbon atoms. Exemplary monocyclic carbon rings include but are not limited to cyclopropane, cyclobutane, cyclopentane, cyclopentene, cyclohexane, cyclohexene, cycloheptane, cycloheptene, etc. Exemplary bicyclic hydrocarbons include tetrahydronaphthalene, decahydronaphthalene, bicyclic[2.1.1]hexane, bicyclic[2.2.1]heptane, etc. Exemplary tricyclic hydrocarbon groups include diamond alkyl, etc.

As used herein, the term "-C(=O)" refers to carbonyl, "- S(=O)" refers to sulfoxide, and "-S(=O)₂" refers to sulfonyl. "=O" represents an oxo, which means that the oxygen atom is connected to other atoms through double bonds.

Alkyl, alkenyl, alkoxy, carbon ring, cycloalkyl, heteroaryl, heterocycle, heterocyclic, carbonyl, sulfonyl, sulfinyl, and other functional groups can be substituted by substituents, and the substituents include but not limited to OH, Boc, halogen, cyanide, substituted or unsubstituted alkyl, substituted or unsubstituted alkoxy, substituted or unsubstituted cycloalkyl, substituted or unsubstituted heterocyclic; NRR', C(O)R, SO₂R, C(O)NRR' or C(O)OR, and R and R' are each independently selected from H and substituted or unsubstituted alkyl.

The term "optionally" used herein refers to the events described subsequently that may or may not occur, and this description includes both the situations in which the event occurred and the situations in which it did not occur. For example, "optionally substituted alkyl" includes "unsubstituted alkyl" and "substituted alkyl" as defined herein. "Optionally substituted by halogens" includes situations where "substituted by halogens" and situations where "not substituted by halogens", such as being substituted by 0-3 halogens. Those skilled in the art should understand that for any functional group containing one or more substituents, the group does not include any spatially impractical, chemically incorrect, synthetic infeasible, and/or inherently unstable substitution patterns.

When any variable appears more than once in any composition or formula of a compound, its definition at each occurrence is independent of its definition at each other occurrence. Therefore, for example, if a group is shown to be substituted by 0-3 Rs, the group can be either unsubstituted or substituted by up to three Rs, and R is independently selected from the definition of R each time it appears. For example, when applied to one or more substitutions in the definition of ring A, that is, when ring A is C₄-C₈ cycloalkyl, 4- to 8-membered heterocycloalkyl, 6- to 10-membered aryl, and 5- to 6-membered heteroaryl, these groups are unsubstituted or substituted by one or more, for example, two substituent groups independently selected from the given definition each time they appear. This applies similarly to the definition of other similar situations.

When the valence bond of a substituent shows a valence bond connecting two atoms through the ring, the substituent can bond with any atom on the ring. When a substituent is listed without specifying the substituent connected to the rest of the compound of the formula, the substituent can be bonded through any atom in the substituent.

The combination of substituents and/or variables is allowed as long as such combination produces stable compounds.

When using dashed rings in a ring structure, it indicates that the ring structure can be saturated, partially saturated or unsaturated.

The term "substituted" used herein refers to one or more hydrogen atoms on a given atom or group being replaced by one or more substituents selected from a given substituent group, provided that they do not exceed the normal valence of the given atom. When the substituent is an oxo (i.e.=O), the two hydrogen atoms on a single atom are replaced by oxygen. There are no oxygen substituents present on the aromatic portion. When a ring system (such as a carbon ring or heterocycle) is replaced by a carbonyl group or double bond, it is intended that the carbonyl group or double bond is part of the ring (i.e., within the ring). Only when the combination of substituents and/or variables leads to chemically correct and stable compounds, such combinations are allowed. A chemically correct and stable compound means that it is sufficiently stable to be separated from the reaction mixture and its chemical structure can be determined, and subsequently formulated into a preparation with at least practical utility. For example, in the absence of a clear list of substituents, the terms "substituted" used herein refer to one or more hydrogen atoms on a given atom or group being independently replaced by one or more substituents, such as 1, 2, 3 or 4 substituents. When an atom or group is replaced by multiple substituents, the substituents can be the same or different.

The term "pharmaceutically acceptable" includes substances or compositions that must be chemically/toxicologically compatible with other ingredients, including preparations and/or mammals treated with them.

Unless otherwise specified, the terms "the compound of the present disclosure" refer to a compound comprising one or more of the formula (I) or its subforms defined herein, such as formulas (Ia), (Ia1), (Ia2), (Ia3), (Ia4), (Ib), (Ic), (Id), (Ie1), (Ie2), (Ie3), (Ie4), (Ie5) and (Ie6), or their pharmaceutically acceptable salts, and all isomers such as stereoisomers (including diastereoisomers, enantiomers and racemes), geometric isomers, conformational isomers (including rotamers and atropisomers), tautomers, internal addition products of isomers, prodrugs, and isomer labeled compounds (including deuterium substitution) and naturally formed parts (such as polymorphic forms, solvates, and/or hydrates). When there is a part that can form salt, it also includes salt, particularly a pharmaceutically acceptable salt. The presence of internal addition products of tautomers or isomers can be identified by those skilled in the art using tools such as NMR. The compound of formula (I) of the present disclosure can easily form internal addition products of tautomers and isomers as described herein.

When formula (I) is mentioned herein, the term also includes its subunits, such as formulas (Ia), (Ia1), (Ia2), (Ia3), (Ia4), (Ib), (Ic), (Id), (Ie1), (Ie2), (Ie3), (Ie4), (Ie5) and (Ie6).

Those skilled in the art will recognize that the compound of the present disclosure may contain chiral centers, allowing for the existence of different isomeric forms. As used herein, "isomers" refer to different compounds with the same molecular formula but different atomic arrangements and configurations.

As used herein, "enantiomers" are a pair of stereoisomers that are non-overlapping mirrors of each other. A 1:1 mixture of enantiomers is a "racemic" mixture. When appropriate, this term is used to refer to racemic mixtures. When indicating the stereochemistry of the compound of the present disclosure, a conventional RS system is used to specify a known relative and absolute configuration of a single stereoisomer with two chiral centers (e.g. (1S, 2S); single stereoisomers with known relative configurations but unknown absolute configurations are marked with asterisks (e.g. (1R*, 2R*); a racemic mixture with two letters (e.g. (1RS, 2RS) is a racemic mixture of (1R, 2R) and (1S, 2S); (1RS, 2SR) is a racemic mixture of (1R, 2S) and (1S, 2R). "Diastereomers" are stereoisomers that have at least two asymmetric atoms but are not mirror images of each other. According to the Cahn-lngold-Prelog R-S system, absolute stereochemistry is indicated. When a compound is a pure enantiomer, the stereochemistry at each chiral carbon can be explained by R or S. The split compounds with unknown absolute configurations can be specified as (+) or (-) based on the direction of polarized light (right rotation or left rotation) of rotation of plane at the sodium D-line wavelength. Alternatively, the split compounds can be defined by the respective retention times of enantiomers/diastereomers via chiral HPLC.

Some of the compounds described herein contain one or more asymmetric centers or axes, thus producing enantiomers, diastereomers, and other stereoisomers that can be defined as (R)- or (S)- in absolute stereochemistry.

When a compound contains a double bond or some other characteristic that gives the molecule a certain amount of structural rigidity, a geometric isomer can occur. If a compound contains a double bond, the substituent can be in the E or Z conformation. If a compound contains a disubstituted cycloalkyl, the cycloalkyl substituent can have a cis-configuration or transconfiguration.

A conformational isomer is an isomer that differs by the rotation of one or more valence bonds. A rotamer is a conformational isomer that differs by the rotation of only a single valence bond.

"Atropisomers" refers to a structural isomer based on axial or planar chirality generated by rotational confinement within a molecule.

Unless otherwise stated, the compound of the present disclosure is intended to include all such possible isomers, including racemic mixtures, optically active forms, and intermediate mixtures. (R)- and (S)- isomers with optical activity can be prepared using chiral synthons or chiral reagents, or split using conventional techniques.

The compound of the present disclosure can be separated into optically active or racemic forms. The compound in the optically active form can be split through racemic separation or synthesis from materials with optical activity. All methods for preparing the compound of the present disclosure and the intermediates prepared therein are considered to be part of the present disclosure. When preparing enantiomer or diastereomer products, they can be separated by conventional methods such as chromatography or stepwise crystallization.

According to the method conditions, the end product of the present disclosure is obtained in the form of free (neutral) or salt. The free and salt forms of these end products are within the scope of the present disclosure. If desired, one form of the compound can be transformed into another form. Free bases or acids can be converted into salts; salts can be converted into free compounds or other salts; the mixture of isomeric compounds of the present disclosure can be separated into individual isomers.

Pharmaceutically acceptable salts are preferred. However, other salts can be useful, for example, in separation or purification steps, which can be used during preparation and are therefore considered within the scope of the present disclosure.

As used herein, "pharmaceutically acceptable" and "medical" can be interchanged, referring to pharmaceutical compositions that are generally safe, non-toxic, and neither biologically nor otherwise undesirable, including those acceptable for veterinary medicine and human medicine.

As used herein, "pharmaceutically acceptable salts" refer to salts that maintain the biological effects and properties of the compound of the present disclosure, and such salts are not unexpected in terms of biology or other aspects. Non-limiting examples of the salt include non-toxic, inorganic or organic base or acid addition salts of the compound of the present disclosure. In many cases, the compound of the present disclosure can form acid salts and/or base salts due to the presence of amino and/or carboxyl or similar functional groups. Inorganic and organic acids can be used to form pharmaceutically acceptable acid addition salts. Inorganic acids from which salts can be derived include, for example, hydrochloric acid, hydrobromic acid, sulfuric acid, nitric acid, phosphoric acid, etc. Organic acids from which salts can be derived include, for example, acetic acid, propionic acid, hydroxyacetic acid, pyruvate, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, etc. Inorganic and organic bases can be used to form pharmaceutically acceptable base addition salts. Inorganic bases from which salts can be derived include, for example, sodium, potassium, lithium, ammonium, calcium, magnesium, iron, zinc, copper, manganese, aluminum, etc.; particularly preferred are ammonium, potassium, sodium, calcium and magnesium salts. Organic bases from which salts can be derived include, for example, primary amines, secondary amines and tertiary amines, substituted amines (including naturally occurring substituted amines), cyclic amines, alkaline ion exchange resins, etc., especially such as isopropylamine, trimethylamine, diethylamine, triethylamine, tripropylamine and ethanolamine. By conventional chemical methods, the pharmaceutically acceptable salt of the present disclosure can be synthesized from the parent compound (alkaline portion or acidic portion). In general, the salt can be prepared by reacting the free acid form of the compound with a stoichiometric quantity of an appropriate base (such as hydroxide of Na, Ca, Mg or K, carbonate, bicarbonate, etc.) or by reacting the free base form of the compound with a stoichiometric quantity of an appropriate acid. This type of reaction is usually carried out in water, organic solvents or a mixture of both. In general, non-aqueous media such as ether, ethyl acetate, ethanol, isopropanol or acetonitrile are preferred when feasible. Other suitable salts can be found in Remington's Pharmaceutical Sciences, 20th edition, Mack Publishing Company, East, Pa., (1985), which was introduced herein as a reference.

As used herein, "pharmaceutically acceptable excipients" include any and all solvents, dispersants, coatings, surfactants, antioxidants, preservatives (such as antibacterial agents, antifungal agents), isotopes, absorption retardants, salts, drugs, drug stabilizers, adhesives, excipients, disintegrants, lubricants, sweeteners, correctors, dyes, similar substances and their combinations, which is well-known to those skilled in the art (see, for example, Remington's Pharmaceutical Sciences, 18th edition, Mack Printing Company, 1990, pp 1289-1329, which was introduced herein as a reference). Unless any conventional carrier cannot coexist with the active ingredient, it can be considered for use in therapeutic or pharmaceutical compositions.

As used herein, "solvate" refers to the physical combination of the compound of the present disclosure with one or more organic or inorganic solvent molecules. This physical combination includes hydrogen bonds. In some cases, solvates will be able to separate, for example, when one or more solvent molecules are incorporated into the lattice of a crystalline solid. The solvent molecules in the solvate can exist in a regular and/or unordered arrangement. The solvate can contain solvent molecules with a stoichiometric quantity or a non-stoichiometric quantity. "Solvates" include solution phases and separable solvates. Exemplary solvates include but are not limited to hydrates, ethanolamides, methanolides and isopropanolides. The method of solvation is well-known in the art.

As used herein, "polymorphic form" refers to crystalline forms with the same chemical structure/composition, but with different spatial arrangements of molecules and/or ions that form crystals. The compound of the present disclosure can be provided as amorphous or crystalline solids. The freeze-drying method can be used to provide the solid compound of the present disclosure.

As used herein, "prodrug" refers to a chemically modified active or inactive compound that, after administration to an individual, undergoes physiological processes in the body (such as hydrolysis, neometabolism, etc.) to become the compound of the present disclosure. The adaptability and technology related to the manufacturing and use of prodrugs are well-known to those skilled in the art.

The arbitrary formula given herein is also intended to represent the unlabeled form of the compound and the isotopic labeled form. Isotopic labeled compounds have the structure described in the formula provided herein, except for one or more atoms replaced by atoms with the selected atomic mass or mass number. Examples of isotopes that can be incorporated into the compound of the present disclosure include isotopes of hydrogen, carbon, nitrogen, oxygen, phosphorus, fluorine and chlorine, such as ²H (i.e. D), ³H (i.e. T), ¹¹C, ¹³C, ¹⁴C, ¹⁵N, ¹⁸F, ³¹P, ³²P, ³⁵S, ³⁶Cl and ¹²⁵I, respectively. The present disclosure includes different isotopic labeled compounds as defined herein, such as those containing radioactive isotopes such as ³H, ¹³C and ¹⁴C. These isotopic labeled compounds can be used for metabolic studies (using ¹⁴C), reaction kinetics studies (such as using ²H or ³H), detection or imaging techniques, such as positron emission tomography (PET) or single photon emission computed tomography (SPECT), including drug or substrate tissue distribution measurements, or can be used for patient radiation therapy. Particularly, ¹⁸F or labeled compounds can be particularly desired for use in PET or SPECT studies. Typically, isotopic labeled compounds of the present disclosure can be prepared by using readily available isotope labeled reagents instead of non-isotope labeled reagents, as described in the following processes or examples and preparation examples.

Moreover, being replaced by heavier isotopes, particularly deuterium (i.e. ²H or D), can also yield certain therapeutic benefits due to greater metabolic stability, such as prolonging in vivo half-life, reducing dose requirements, or improving treatment indices. It can be understood that deuterium in the context can be regarded as a substituent of the compound of the present disclosure. The concentration of these heavier isotopes, particularly deuterium, can be defined by isotopic enrichment factors. "Isotope enrichment factor" represents the ratio between the isotopic abundance of a specified isotope and its natural abundance.

The isotope-labeled compound of the present disclosure can typically be prepared using conventional techniques known to those skilled in the art or by performing methods similar to those described herein, using appropriate isotope-labeled reagents instead of other unlabeled reagents used. These compounds have various potential applications, such as serving as standards and reagents for determining the ability of potential drug compounds to bind to target proteins or receptors, or for imaging the compound of the present disclosure that binds to biological receptors in vivo or in vitro.

As used herein, the "therapeutic effective amount" of the compound of the present disclosure refers to the amount of the compound of the present disclosure that can cause individual biological or medical reactions, improve symptoms, slow or delay disease progression, or prevent disease. The "therapeutic effective amount" can be determined by participating physicians or veterinary practitioners, and will vary depending on factors such as the compound, the disease status being treated, the severity of the disease being treated, the individual's age and relevant health status, the route and form of administration, and the judgment of the attending physician or veterinary practitioner.

As used herein, "individual" refers to animals. Preferably, animals are mammals. Individuals also refer to primates (for example, humans), cows, sheep, goats, horses, dogs, cats, rabbits, rats, mice, fish, birds, etc. In a preferred embodiment, the individual is a human.

As used herein, "inhibition" refers to the alleviation or inhibition of a specific patient, symptom or disease, or a significant decrease in biological activity or process baseline activity.

As used herein, the term "treatment" or any disease or condition in an embodiment refers to the improvement of diseases or conditions (i.e., preventing or slowing down the development of the disease or at least one clinical symptom). In another embodiment, "treatment" refers to improving at least one physical parameter that may not be perceived by the patient. In another embodiment, "treatment" refers to the regulation of diseases or conditions on the body (such as stable and perceptible symptoms) or physiology (such as stable body parameters) or both.

As used herein, "prevention" refers to the administration of one or more pharmaceutical substances, particularly the compound and/or the pharmaceutically acceptable salt thereof of the present disclosure, to individuals with a predisposition to the disease, in order to prevent them from developing the disease.

When it comes to chemical reactions, "treatment", "contact" and "reaction" refer to adding or mixing two or more reagents under appropriate conditions to produce the shown and/or desired products. It should be understood that the reaction that produces the shown and/or desired product may not necessarily come directly from a combination of the two reagents initially added, i.e., there may be one or more intermediates generated in the mixture that ultimately lead to the formation of the shown and/or desired product.

In general, the term "about" is used herein to adjust the given value to be 20% higher or lower than that value, such as 10%, such as 5%.

The undefined technical and scientific terms used herein have the meanings commonly understood by those skilled in the art to which the present disclosure belongs.

### Efficacy

The hydroxamic acid compound of the present disclosure is an ENPP1 inhibitor compounds with high selectivity and inhibitory activity towards ENPP1, fewer side effects, high resistance, high bioavailability, and high clinical application value. The compound of the present disclosure can be used in various applications that require inhibition of ENPP1.

The compound of the present disclosure can be used to treat, prevent or improve ENPP1-mediated diseases or obstacles, for example, ENPP1-mediated tumors (such as cancer) or infectious diseases or obstacles, especially recurrent or refractory cancers or metastatic cancers.

Particularly, the compound of the present disclosure can be used for treating, preventing or improving solid tumors, for example, selected from breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer.

The compound of the present disclosure can also be used for treating, preventing or improving hematopoietic malignancies, for example, selected from leukemia, lymphoma or myeloma.

Moreover, the compound of the present disclosure can also be used to treat, prevent or improve infectious diseases or conditions, particularly ENPP1-mediated infectious diseases or conditions, for example, selected from herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.

### Pharmaceutical Composition and Administration

The compound of the present disclosure can be administered to individuals in the form of pharmaceutical compositions, which may optionally comprise one or more pharmaceutically acceptable excipients.

The compound of the present disclosure can be administered through various known routes, including oral, rectal, gastric, intracranial and parenteral administration, for example, intravenous, intramuscular, nasal, dermal, subcutaneous and similar administration routes. Particularly preferred are oral, intranasal and parenteral administration. Depending on the administration route, different pharmaceutical preparations are required, and some of these administration routes may require the application of protective coatings to the pharmaceutical preparations to prevent degradation of the compound of the present disclosure, for example, in the digestive tract.

The compound of the present disclosure can be prepared into syrup, infusion or injection, spray, tablet, capsule, pastille, liposome or suppository, etc.

The particularly preferred drug form for administering the compound of the present disclosure is a form suitable for injection, including sterile aqueous solutions or dispersions and sterile powders for immediate preparation of sterile injection solutions or dispersions. In all cases, the final solution or dispersion form must be sterile and fluid. Typically, such solutions or dispersions will contain solvents or dispersion medium that contain, for example, waterbuffered aqueous solutions such as biocompatible buffering agents, ethanol or polyols such as glycerol, propylene glycol, polyethylene glycol, suitable mixtures thereof, surfactants or vegetable oils. The compound of the present disclosure can also be formulated into liposomes, particularly liposomes for parenteral administration. Liposomes provide the advantage of increased half-life in circulation (if compared to free drugs) and longer and more uniform release of encapsulated drugs.

The sterilization of infusion and injection can be achieved through recognized techniques in this field, including but not limited to the addition of preservatives such as antibacterial or antifungal agents, such as nipagin esters, tert butanol trichloride, phenol, sorbic acid or thiomersal. In addition, isotonic agents such as sugar or salt, particularly sodium chloride, can be added to infusion and injection.

The production of sterile injections containing one or more compounds of the present disclosure is completed by introducing the required amount of each compound into a suitable solvent with the various components listed above as appropriate, and then sterilizing. To obtain sterile powder, vacuum dry or freeze dry the above solution as needed. The preferred diluents of the present disclosure are water, physiologically acceptable buffering agents, physiologically acceptable buffer salt solutions or salt solutions. The preferred carriers are cocoa butter and vitebesole.

The excipients that can be used in conjunction with various drug forms of the compound of the present disclosure can be selected from the following non-limiting list: a) adhesives, such as lactose, mannitol, crystalline sorbitol, hydrogen phosphate, sugars, microcrystalline cellulose, carboxymethyl cellulose, hydroxyethyl cellulose and/or polyvinylpyrrolidone; b) lubricants, such as magnesium stearate, talc powder, calcium stearate, zinc stearate, stearic acid, hydrogenated vegetable oil, leucine, glyceride and sodium stearoyl fumarate; and c) disrupting agents, such as starch, cross-linked carboxymethyl cellulose, sodium methylcellulose, agar, bentonite, alginate, carboxymethyl cellulose and/or polyvinylpyrrolidone. Other suitable pharmaceutical carriers and their formulations are well-known in this field, such as those described in the following literature: Remington: The Science and Practice of Pharmacy 1995, edited by E. W. Martin, Mack Publishing Company, 19th edition, Easton, Pennsylvania.

In one embodiment, the preparation is used for oral administration and comprises one or more or all of the following ingredients: pre-gelatinized starch, talc powder, polyvinylpyrrolidone K30, cross-linked carboxymethyl cellulose sodium, sodium stearyl fumarate, gelatin, titanium dioxide, sorbitol, monosodium citrate, xanthan gum, titanium dioxide, flavoring agent, sodium benzoate and saccharin sodium.

In an embodiment, the compound of the present disclosure is administered intranasally, which can be administered in a dry powder inhaler or a spray in a pressurized container, pump, spray or atomizer using a suitable propellant such as dichlorodifluoromethane, trichlorofluoromethane, dichlorotetrafluoroethane, hydrofluorane such as 1,1,1,2-tetrafluoroethane (HFA 134A^{™}) or 1,1,1,2,3,3,3-hexafluoropropane (HFA 227EA^{™}), carbon dioxide, or other suitable gases. The pressurized container, pump, spray or atomizer can contain a solution or suspension of the compound of the present disclosure, such as a solution or suspension using ethanol and a propellant as the solvent, and it can also contain a lubricant, such as sorbitane trioleate.

The typical dosage range of the compound of the present disclosure is 0.001-1000 mg of active ingredient/kg body weight/day. The dose can be administered once a day or in multiple doses. The determination of appropriate dosage is determined by the attending physician based on the type and severity of the disease to be treated, the individual's health status and past medical history, shared medications, specific compounds to be administered, and routes of administration. As needed, the dosage of the compound of the present disclosure may exceed this dosage range.

### Pharmaceutical Combination

The compound of the present disclosure may be used alone or in combination with one or more other active agents or therapies for the purposes described herein, which may have or produce the same or different pharmacological effects. The compound of the present disclosure may be administered simultaneously, before, or after other active agents or therapies.

When the compound of the present disclosure is administered in combination with other active agents, the dosage of the other active agents administered in combination will naturally vary depending on factors such as the shared drug, the disease to be treated, the general health condition of the patient, and the judgment of a physician or veterinarian. The compound of the present disclosure can be administered simultaneously, separately or sequentially with other active agents in combination through the same or different administration routes. They can be comprised in the same pharmaceutical composition, or they can be a combination product in a separate form, for example, in the form of a medicine box. They can be prepared and/or formulated by the same or different manufacturers. Moreover, the compound of the present disclosure and other active agents may be added in combination therapy (i) before sending the combination product to physicians (for example, in the case of a medicine box containing the compound of the present disclosure and other drugs); (ii) prior to administration, by the physician themselves (or under the guidance of the physicians); (iii) by the patients themselves, for example, during the sequential administration of the compound of the present disclosure and other active agents.

In an embodiment, the present disclosure provides a pharmaceutical composition comprising the compound or the pharmaceutically acceptable salt thereof of the present disclosure, as well as one or more other active agents. Optionally, the pharmaceutical composition may comprise a pharmaceutically active excipient as described above.

In an embodiment, the present disclosure provides a medicine box comprising two or more separate pharmaceutical compositions, wherein at least one comprises a compound of formula (I) or a pharmaceutically acceptable salt thereof. In an embodiment, the medicine box comprises utensils for separately retaining the composition, such as containers, separate bottles, or separate foil bags. An example of this type of medicine box is a blister bag, which is usually used for packaging tablets, capsules, etc.

The medicine box of the present disclosure can be used for administering different dosage forms, such as oral and parenteral dosage forms, for administering individual compositions at different dose intervals, or for gradually increasing individual compositions relative to another. To aid compliance, the medicine box of the present disclosure typically includes an administration instruction.

In the combination therapy of the present disclosure, the compound of the present disclosure and other therapeutic agents may be prepared and/or formulated by the same or different manufacturers. Moreover, the compound of the present disclosure and other therapeutic agents can be introduced together into combination therapy, which can be done (i) before sending the combination product to physicians (for example, for a medicine box comprising the compound of the present disclosure and other therapeutic agents); (ii) prior to administration, by the physician themselves (or under the guidance of the physicians); (iii) by the patients themselves, for example, during the sequential administration of the compound of the present disclosure and other therapeutic agents.

### General Synthesis Methods

The compound of the present disclosure can be prepared by various methods, including the methods described below, the methods described in examples, or similar methods. The following text describes a suitable general synthesis scheme. The appropriate reaction conditions for each reaction step are known to those skilled in the art. The raw materials can be purchased and obtained, or can be prepared through the methods described below, similar methods to those provided below, or methods known in the art. The variables in the general formula have the same meanings as in the previous text, unless otherwise stated.

In an embodiment, the compound of the present disclosure can be synthesized through the following general synthesis scheme, where each variable is defined herein, and the specific reaction conditions are the same as in the example.

### Process 1

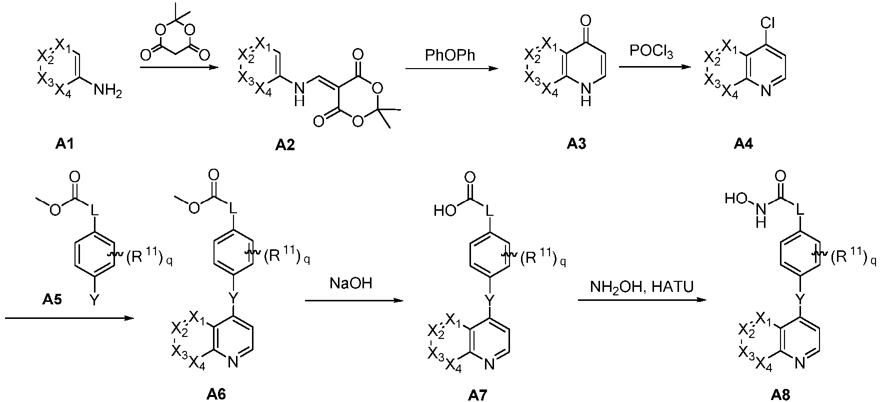

Compound of general formula A2 can be prepared by the following synthesis method: trimethyl orthoformate and 2,2-dimethyl-1,3-dioxan-4,6-dione are stirred at 100°C for 2 hours, followed by the addition of compound of general formula A1, and the reaction continues at that temperature for 2 hours. After cooling the reaction solution to room temperature, the solid was precipitated, filtered, and collected to obtain compound of general formula A2.

The compound of general formula A3 can be prepared by the following synthesis method: dissolving the compound of general formula A2 in diphenyl ether, stirring at 230°C for 1 hour, cooling the reaction solution to room temperature, adding petroleum ether, precipitating the solid, filtering, and collecting the solid to obtain the compound of general formula A3.

The compound of general formula A4 can be prepared by the following synthesis method: reacting the compound of general formula A3 with phosphorus oxychloride at 100°C for 1 hour, cooling to room temperature, removing excess phosphorus oxychloride, obtaining residue, adding ice water to the residue, adjusting the pH to 8 with ammonia water, extracting with dichloromethane, washing organic phase with water and saturated salt water sequentially, drying, filtering, concentrating to obtain crude product, purifying with silica gel column to obtain compound of general formula A4.

The general formula A6 compound can be prepared by the following synthesis method: in one case, compounds of general formula A4 and general formula A5 undergo nucleophilic substitution reaction under DIEA, potassium carbonate or hydrochloric acid conditions to generate the compound of general formula A6; in another case, compounds of general formula A4 and general formula A5 undergo coupling reaction under palladium catalytic conditions to obtain the compound of general formula A6.

The compound of general formula A7 can be prepared by the following synthesis method: the compound of general formula A6 and sodium hydroxide undergo hydrolysis reaction in an appropriate solvent to obtain the compound of general formula A7.

The compound of general formula A8 can be prepared by the following synthesis method: the compound of general formula A7 and hydroxylamine undergo a condensation reaction in the presence of the condensation agent HATU to obtain the compound of general formula A8.

### DETAILED DESCRIPTION OF THE INVENTION

In the present application, when the chemical name and structural formula are inconsistent, the one shown in the structural formula shall prevail, unless it can be inferred from the context that the chemical name rather than the structural formula is correct.

As those skilled in the art can clearly understand, for the sake of simplicity, not all hydrogen atoms are clearly labeled in the structural formulas of some compounds provided in the present application. When there is a vacant valence of carbon or nitrogen atoms in a compound, it indicates the presence of unmarked hydrogen.

The compound of the present disclosure can be prepared using various methods known to technicians in the field of organic synthesis according to the methods, reaction processes and examples provided herein. The compound of the present disclosure can be synthesized using the methods described below in combination with known synthesis methods in the field of organic chemistry, or through alternative methods that can be understood by those skilled in the art. The preferred methods include but are not limited to those described below. The reaction is carried out in a solvent or solvent mixture suitable for the reagents and materials used and suitable for the transformation being carried out. Technicians in the field of organic synthesis can understand that the functional groups present on the molecule should be consistent with the planned conversion. This sometimes requires judgment to change the sequence of synthesis steps or to choose a specific operational process relative to others to obtain the expected compound of the present disclosure.

The present disclosure will be further described in combination with specific examples. It should be understood that these examples are only used to illustrate the present disclosure and not to limit the scope of the present disclosure. The experimental methods without specific conditions in the following embodiments are usually under conventional conditions, or the conditions recommended by the manufacturer. Unless otherwise stated, percentages and parts are weight percentages, weight parts or volume percentages (when liquid).

For the purpose of explanation, the reaction process described below provides potential pathways and key intermediates for synthesizing the compound of the present disclosure. For a more detailed description of individual reaction steps, please refer to the following example section. Those skilled in the art will understand that other synthetic pathways can be used to synthesize the compound of the present disclosure. Although specific raw materials and reagents are described in the process and discussed below, other raw materials and reagents can be easily replaced to provide various derivatives and/or reaction conditions. In addition, most of the compounds prepared by the following methods can be further modified using conventional chemistry familiar to those skilled in the art according to the present disclosure.

In the preparation of the compound of the present disclosure, protection of the distal functional group of the intermediate may be necessary. The demand for such protection will vary depending on the properties of the remote functional groups and the conditions of the preparation method. The need for such protection is easily determined by those skilled in the art.

The experimental materials and reagents used in the following examples can be obtained from commercial channels unless otherwise stated. Raw materials can usually be obtained from commercial sources or easily prepared using methods known to those skilled in the art.

In each example, the experimental instrument description (for example, ¹H NMR was recorded by Varian Mercury-300 or Varian Mercury-400 nuclear magnetic resonance instrument, ¹³C NMR was recorded by Varian Mercury-400 or Varian Mercury-500 or Varian Mercury-600 nuclear magnetic resonance instrument, chemical shift was represented by δ (ppm); the mass spectrometry was recorded by Finnigan/MAT-95 (EI) and Finnigan LCQ/DECA and Micromass Ultra Q-TOF (ESI) mass spectrometers; reverse phase preparation of silica gel for HPLC separation was 200-300 meshes).

### Abbreviations

| | | | |
|---|---|---|---|
| AcOH | Acetic acid | NaH | Sodium hydride |
| BIANP | 1,1'-binaphthyl-2,2'-bis(diphenylphosphine) | NaOH | Sodium hydroxide |
| CH₃I | Iodomethane | NaOMe | Sodium methoxide |
| DCM | Dichloromethane | NBS | N-bromo succinimide |
| DIEA | N,N-diisopropylethylamine | nBuLi | N-butyl lithium |
| DMAP | 4-dimethylaminopyridine | nBuOH | N-butanol |
| DMF-DMA | Dimethylformamide dimethyl acetal | PdCl₂dppf | 1,1'-bis(diphenylphosphino)ferrocene palladium dichloride |
| DMF | N,N-dimethylformamide | Pd₂dba₃ | Tri(dibenzalacetone)dipalladium |
| EA | Ethyl acetate | POCl₃ | Phosphorus oxychloride |
| HC(OMe)₃ | Trimethyl orthoformate | PhOPh | Diphenyl ether |
| Bn | Benzyl | Pd/C | Palladium carbon |
| IPA | Isopropanol | PE | Petroleum ether |
| PMBNH₂ | 4-methoxybenzylamine | SOCl₂ | Dichlorosulfoxide |
| LDA | Lithium diisopropylamide | TFA | Trifluoroacetic acid |
| MeBF₃K | Potassium methyltrifluoroborate | TsOH | P-toluenesulfonic acid |
| MeOH | Methanol | THF | Tetrahydrofuran |
| HATU | 2-(7-azobenzotriazole)-N,N,N',N'-tetramethylurea hexafluorophosphate | | |

### Synthesis of Key Intermediates

### Intermediate 1a: 5-chloro-2-methoxy-1,8-naphthyridine

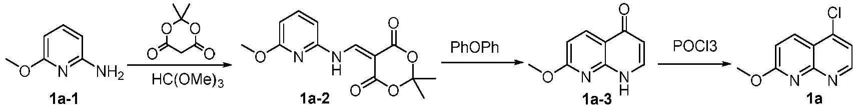

### Step 1: Synthesis of 5- (((6-methoxypyridin-2-yl)amino)methylene)-2,2-dimethyl-1,3-dioxane-4,6-dione

Compound 2,2-dimethyl-1,3-dioxan-4,6-dione (12.7 g, 88.61 mmol) was added to a single-necked bottle (250 mL) containing trimethyl orthoformate (100 mL). The reaction solution was stirred at 100°C for 2 hours, then compound **1a-1** (5.0 g, 40.28 mmol) was added to continue the reaction at this temperature for 2 hours. After cooling the reaction solution to room temperature, the solid was precipitated, filtered, and collected to obtain compound **1a-2** (10 g, yellow solid) with a yield of 89%. ¹H NMR (400 MHz, CDCl₃-*d*): δ 9.30 (d, *J =* 13.6 Hz, 1H), 7.63-7.59 (m, 1H), 6.63-6.57 (m, 2H), 3.99 (s, 3H), 1.76 (s, 6H).

### Step 2: Synthesis of 7-methoxy-1,8-naphthyridin-4(1H)-one

**1a-2** (5.0 g, 17.98 mmol) was added to a single-necked bottle (250 mL) containing 50 mL of diphenyl ether, stirred at 230°C for 1 hour. The reaction solution was cooled to room temperature, petroleum ether was added, and the solid was precipitated, filtered and collected to obtain compound **1a-3** (2.9 g, yellow solid), with a yield of 94%. ¹H NMR (400 MHz, CDCl₃-*d*): δ 11.97 (s, 1H), 8.30 (d, *J* = 8.8 Hz, 1H), 7.78-7.75 (m, 1H), 6.79 (d, *J* = 8.8 Hz, 1H), 6.05-6.03 (m, 1H), 3.96 (s, 3H).

### Step 3: Synthesis of 5-chloro-2-methoxy-1,8-naphthyridine

Compound **1a-3** (500 mg, 2.84 mmol) and 10 mL of phosphorus oxychloride were added sequentially to a single-necked bottle (100 mL). After a reaction at 100°C for 1 hour, the reaction solution was cooled to room temperature to remove excess phosphorus oxychloride. Ice water was added to the residue, and the pH was adjusted to 8 with ammonia water. The reaction solution was extracted with dichloromethane. The organic phase was washed sequentially with water and saturated salt water, dried with anhydrous sodium sulfate, filtered, and concentrated to obtain the crude product. The crude product was purified on a silica gel column (PE: EA = 5:1) to obtain compound **1a** (400 mg, white solid) with a yield of 73%. LCMS(ESI): m/z 195.1 [M+H]⁺; RT = 1.137 min (6.00 min).

### Intermediate 2a: 4-chloro-6-fluoro-7-methoxyquinoline

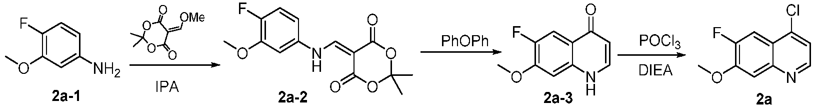

### Step 1: Synthesis of 5-((((4-fluoro-3-methoxyphenyl)amino)methylene)-2,2-dimethyl-1,3-dioxane-4,6-dione

Compound **2a-1** (3.00 g, 21.3 mmol) and 5-(methoxyethylene)-2,2-dimethyl-1,3-dioxane-4,6-dione (4.75 g, 25.6 mmol) were added to a single-necked bottle containing IPA (50 mL), stirred at 70°C for 2 hours, cooled to room temperature and filtered, and the solid was washed with methanol, dried to obtain compound **2a-2** (5.80 g, yellow solid) with a yield of 92.3%. LCMS(ESI): *m*/*z* 256.1 [M-40+H]⁺; RT = 1.08 min (2.00 min).

### Step 2: Synthesis of 6-fluoro-7-methoxyquinoline-4(1H)-one

Compound **2a-2** (5.80 g, 19.7 mmol) was added to a single-necked bottle containing 100 mL of diphenyl ether and stirred at 200°C for 2 hours. After cooling to room temperature, the reaction solution was poured into petroleum ether and filtered, and the filter cake was beaten with chloroform to obtain compound **2a-3** (2.80 g, gray solid) with a yield of 73.6%. LCMS(ESI): m/z 194.0 [M+H]⁺; RT = 1.08 min (2.00 min).

### Step 3: Synthesis of 4-chloro-6-fluoro-7-methoxy quinoline

Compound **2a-3** (1.00 g, 5.18 mmol) and phosphorus oxychloride (2 mL) were added to a single-necked bottle containing DIEA (7 mL) and stirred at 100°C for 1 hour. The reaction solution was poured into ice water, extracted with dichloromethane, the organic phase was washed with saturated salt water, dried with anhydrous sodium sulfate, concentrated, and purified using a chromatography plate to obtain compound **2a** (950 mg, white solid) with a yield of 86.9%. LCMS(ESI): *m*/*z* 212.0 [M+H]⁺; RT = 1.15 min (2.00 min).

### Intermediate 3a: 4-chloro-7-methoxy-1,6-naphthyridine

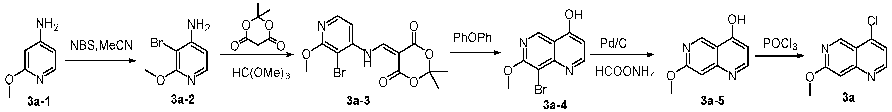

### Step 1: Synthesis of 3-bromo-2-methoxypyridin-4-amine

Compound **3a-1** (1.0 g, 8.06 mmol) was added to a single-necked bottle (100 mL) containing 10 mL of anhydrous acetonitrile. After cooling to 0°C, NBS (1.44 g, 8.06 mmol) was slowly added and stirred overnight at room temperature. The reaction solution was diluted with water, and extracted with ethyl acetate, and the organic phase was merged, washed sequentially with water and saturated salt water, dried with anhydrous sodium sulfate, filtered, concentrated and purified using a column (PE: EA = 8:1) to obtain compound **3a-2** (1.4 g, yellow solid) with a yield of 88%. LCMS(ESI): *m*/*z* 203.0 [M+H]⁺; RT = 0.967 min (2.50 min).

### Step 2: Synthesis of 5-(((3-bromo-2-methoxypyridin-4-yl)amino)methylene)-2,2-dimethyl-1,3-dioxane-4,6-dione

2,2-dimethyl-1,3-dioxan-4,6-dione (709 mg, 4.92 mmol) was added to a single-necked bottle (100 mL) containing HC(OMe)₃ (10 mL), stirred at 100°C for 2 hours, compound **3a-2** (500 mg, 2.46 mmol) was added, and the reaction was continued for 2 hours. After cooling the reaction solution to room temperature, petroleum ether was added, the solid was precipitated, filtered, and collected to obtain compound **3a-3** (679 mg, white solid) with a yield of 77%. LCMS(ESI): *m*/*z* 357.0 359.0 [M+H]⁺; RT = 1.517 min (2.50 min).

### Step 3: Synthesis of 8-bromo-7-methoxy-1,6-naphthyridin-4-ol

Compound **3a-3** (3.0 g, 8.40 mmol) was added to a single-necked bottle (100 mL) containing 20 mL of diphenyl ether, stirred at 230°C for 1 hour, the reaction solution was cooled to room temperature, diluted with petroleum ether, filtered, and filter cake was collected to obtain compound **3a-4** (2.27 g, brown solid) with a yield of 100%. LCMS(ESI): m/z 255.0 257.0 [M+H]⁺; RT = 0.957 min (2.50 min).

### Step 4: Synthesis of 7-methoxy-1,6-naphthyridin-4-ol

Compound **3a-4** (2.0 g, 7.84 mmol), ammonium formate (980 mg, 15.68 mmol), and palladium carbon (300 mg, wt%: 10%) were added sequentially to a single-necked bottle (50 mL) containing 10 mL of methanol, and stirred at 60°C for 1 hour. The reaction solution was filtered, the filtrate was diluted with ethyl acetate, washed with water and saturated salt water in sequence, dried with anhydrous sodium sulfate, filtered and concentrated to obtain compound **3a-5** (1.4 g, yellow solid) with a yield of 100%. LCMS(ESI): m/z 177.1 [M+H]⁺; RT = 0.757 min (2.50 min).

### Step 5: Synthesis of 4-chloro-7-methoxy-1,6-naphthyridine

Compound **3a-5** (900 mg, 0.644 mmol) and DIEA (1.7 mL, 5.62 mmol) were added sequentially to a three-necked bottle (100 mL) containing anhydrous acetonitrile (10 mL). The reaction solution was cooled to 0°C under nitrogen protection, and then phosphorus oxychloride (0.9 mL, 5.62 mmol) was slowly added dropwise. After stirring at 70°C for 30 minutes, the reaction solution was cooled to room temperature. Saturated sodium bicarbonate was used to adjust the pH to 8. The reaction solution was extracted with ethyl acetate, and the organic phase was combined. The organic phase was washed sequentially with water and saturated salt water, dried with anhydrous sodium sulfate, filtered, concentrated, and purified on a column (PE: EA = 10: 1) to obtain compound **3a** (1.0 g, yellow solid) with a yield of 100%. LCMS(ESI): m/z 195.1 [M+H]⁺; RT=1.287 min (2.50 min).

### Intermediate 4a: 8-chloro-3-methoxy-1,5-naphthyridine

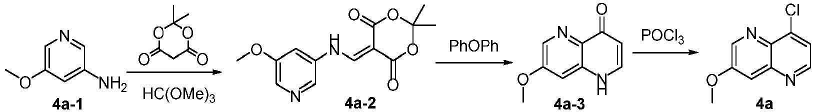

### Step 1: Synthesis of 5-((5-methoxypyridin-3-yl)amino)methylene)-2,2-dimethyl-1,3-dioxo-4,6-dione

2,2-dimethyl-1,3-dioxo-4,6-dione (4.64 g, 16.11 mmol) was added to a single-necked bottle (250 mL), and then trimethyl orthoformate (100 mL) was added. The reaction solution was stirred at 105°C for 2 hours. Compound **4a-1** (4.00 g, 16.11 mmol) was added and stirred overnight at 105°C. The reaction solution was diluted with petroleum ether at room temperature, filtered, and the filter cake was washed with petroleum ether. The filter cake is crude **4a-2** (7.0 g, yellow solid). LCMS (ESI): *m*/*z* 279.1[M-H]⁻; RT = 0.891 min (2.50 min).

### Step 2: Synthesis of 7-methoxy-1,5-naphthylidin-4(1H)-one

Compound **4a-2** (5.00 g, 17.97 mmol) was loaded into a three-necked bottle (250 mL), diphenyl ether (100 mL) was added, and stirred under nitrogen protection for 1 hour. A large amount of n-hexane was added at room temperature to dilute the reaction solution, which was filtered, and the filter cake was washed with n-hexane. The filter cake is crude **4a-3** (2.5 g, gray solid).

### Step 3: Synthesis of 8-chloro-3-methoxy-1,5-naphthyridine

Compound **4a-3** (3.50 g, 19.87 mmol) was loaded into a three-necked bottle (250 mL), phosphorus oxychloride (70 mL) was added, and stirred at 110°C for 12 hours. After cooling to room temperature, solvent was removed, then dichloromethane and a small amount of water were added. The pH was adjusted to 9-10 using sodium bicarbonate, and the reaction solution was extracted with ethyl acetate. The organic phase was washed with salt water, dried with anhydrous sodium sulfate, and the solvent was removed. The crude product was purified using a normal phase column (PE/EA = 3:1) to obtain compound **4a** (2.70 g, yellow solid). Yield: 70%. LCMS (ESI): *m*/*z* 195.1 [M+H]⁺; RT = 1.271 min (2.50 min).

### Intermediate 5a: 4-chloro-8-fluoro-7-methoxyquinoline

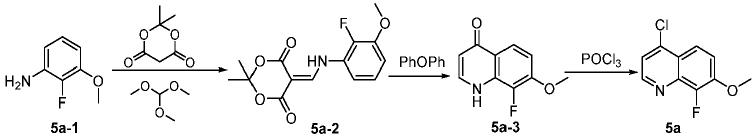

### Step 1: Synthesis of 5-(((2-fluoro-3-methoxyphenyl)amino)methylene)-2,2-dimethyl-1,3-dioxane-4,6-dione

At room temperature, compound **5a-1** (500 mg, 3.50 mmol), 2,2-dimethyl-1,3-dioxan-4,6-dione (655 mg, 4.55 mmol), trimethoxymethane (725 mg, 4.9 mmol), and acetonitrile (10 mL) were added sequentially to a dry single-necked bottle (50 mL), and heated to 85°C for overnight reaction. The reaction solution was concentrated under reduced pressure. The residue was beaten with n-pentane and dried to obtain crude product **5a-2** (1 g, white solid). LCMS (ESI): *m*/*z* 296.1 [M+H]⁺; RT = 6.324 min (15.00 min).

### Step 2: Synthesis of 8-fluoro-7-methoxyquinoline-4(1H)-one

At room temperature, compound **5a-2** (1 g, crude) and diphenyl ether (10 g) were added sequentially to a dry single-necked bottle (50 mL) and heated to 200°C to react for 2 hours. Reduced pressure concentration was carried out, the residue was beaten with n-pentane and dried to obtain compound **5a-3** (600 mg, brown solid) with a yield of 91.70%. LCMS (ESI): *m*/*z* 194.0 [M+H]⁺; RT = 0.94 min (3.00 min).

### Step 3: Synthesis of 4-chloro-8-fluoro-7-methoxyquinoline

At room temperature, compound **5a-3** (600 mg, 3.11 mmol), acetonitrile (20 mL), DIEA (1.5 mL), and phosphorus oxychloride (3 mL) were added sequentially to a dry single-necked bottle (50 mL), heated to 80°C and reacted overnight. The reaction solution was poured into ice water, adjusted to alkaline with saturated sodium bicarbonate solution, and extracted with ethyl acetate (30 mL). The organic phase was washed with salt water, dried with sodium sulfate, filtered, and concentrated under reduced pressure. Purification of residual silica gel column (PE : EA = 1:1) resulted in compound **5a** (350 mg, yellow solid) with a yield of 53.4%. LCMS (ESI): *m*/*z* 212.0 [M+H]⁺; RT = 1.51 min (3.00 min).

### Intermediate 6a: 4-chloro-7-methoxyquinoline-3-acetonitrile

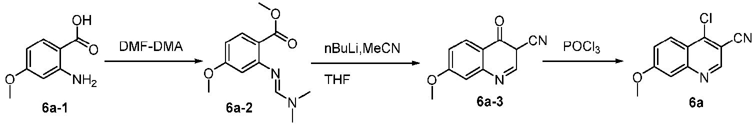

### Step 1: Synthesis of methyl (E)-2-(((dimethylamino)methylene)amino)-4-methoxybenzoate

Compound **6a-1** (3 g, 17.9 mmol) was added to a single-necked bottle (250 mL) containing DMF-DMA (30 mL) and stirred at 105°C for 4 hours. Concentration was carried out to obtain crude product **6a-2** (5.0 g, yellow solid) after cooling. LCMS (ESI): m/z 237.1[M+H]⁺; RT = 0.792 min (2.50 min).

### Step 2: Synthesis of 7-methoxy-4-oxo-3,4-dihydroquinoline-3-acetonitrile

N-butyl lithium (13.7 mL, 34.3 mmol) was added dropwise within ten minutes in a three-necked bottle containing anhydrous THF (40 mL) at -78°C; within half an hour, acetonitrile (1.46 g, 35.5 mmol) was added dropwise. When a white solid precipitated, 10 mL of THF solution of compound **6a-2** (3.0 g, 12.7 mmol) was added dropwise. The reaction was carried out for 40 minutes, quenched with acetic acid (3 mL). The reaction solution was concentrated, filtered with water, and the filter cake was dried to obtain crude product **6a-3** (1.6 g, yellow solid) with a yield of 24%. LCMS (ESI): *m*/*z* 201.1[M+H]⁺; RT = 1.032min (2.50 min).

### Step 3: Synthesis of 4-chloro-7-methoxyquinoline-3-acetonitrile

Compound **6a-3** (1.1 g, 5.5 mmol) was added to a single-necked bottle (100 mL) containing POCl₃ (15 mL) and stirred at 100°C for 1.5 hours. After cooling, the solvent was removed, ethyl acetate and water were added at room temperature, the pH was adjusted to 8 with saturated sodium bicarbonate aqueous solution, the organic phase was washed with sodium chloride, dried with anhydrous sodium sulfate, and filtered. Crude product **6a** (500 mg, yellow solid) was purified using prep-TCL (PE: EA = 5:1) in a yield of 42%. LCMS (ESI): *m*/*z* 219.1[M+H]⁺; RT = 1.662 min (2.50 min).

### Intermediate 7a: 4-chloro-7-methoxy-2-methylpyridino[2,3-d]pyrimidine

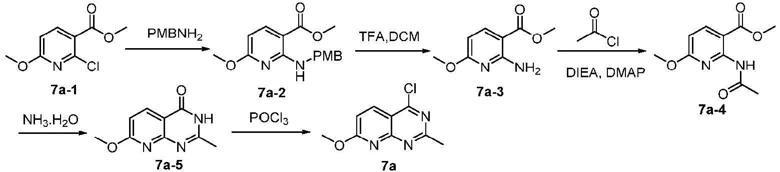

### Step 1: Synthesis of methyl 6-methoxy-2-((4-methoxybenzyl)amino)nicotinate

At room temperature, **7a-1** (5.0 g, 24.9 mmol), PMBNH₂ (4.1 g, 29.9 mmol), potassium carbonate (6.87 g, 49.8 mmol), and DMF (20 mL) were added sequentially to a dry single-necked bottle (100 mL). The reaction solution was heated to 80°C and reacted for 16 hours. After cooling to room temperature, the reaction solution was extracted with ethyl acetate and water, the organic phase was washed with salt water, concentrated, and purified (PE/EA = 2/1) to obtain compound **7a-2** (7g, colorless liquid) with a yield of 92%. LCMS (ESI): *m*/*z* 303.1[M+H]⁺; RT = 1.98 min (3 min).

### Step 2: Synthesis of methyl-2-amino-6-methoxynicotinate

At room temperature, compound 7a-2 (7.0 g, 24.0 mmol), dichloromethane (5 mL), and TFA (5 mL) were added sequentially to a dry single-necked bottle (100 mL) and reacted at room temperature for 16 hours. Concentration was carried out, the residue was diluted with dichloromethane, washed with sodium bicarbonate aqueous solution, the organic phase was dried with anhydrous sodium sulfate, filtered and concentrated, purified to obtain compound **7a-3** (3.5 g, colorless liquid) with a yield of 80%. LCMS (ESI): *m*/*z* 183.1[M+H]⁺; RT = 1.42 min (3 min).

### Step 3: Synthesis of methyl 2-acetamino-6-methoxynicotinate

At room temperature, compound **7a-3** (2.0 g, 11.0 mmol), DIEA (7.1 g, 55.0 mmol), DMAP (134 mg, 1.1 mmol), dichloromethane (20 mL), and acetyl chloride (1.7 g, 22.0 mmol) were added sequentially to a dry single-necked bottle (50 mL). The reaction solution was reacted at room temperature for 16 hours. Concentration was carried out, the residue was extracted with ethyl acetate and water, the organic phase was concentrated and purified (PE/EA = 10/1-1/1) to obtain compound **7a-4** (1.0 g, white solid). Yield: 45%. LCMS (ESI): *m*/*z* 224.8[M+H]⁺; RT = 1.541 min (3 min).

### Step 4: Synthesis of 7-methoxy-2-methylpyridino[2,3-d]pyrimidin-4(3H)-one

At room temperature, compound **7a-4** (1.0 g, 4.46 mmol), anhydrous methanol (10 mL), and ammonia solution (20 mL) were added sequentially to a dry single-necked bottle (50 mL). The reaction solution was reacted at room temperature for 16 hours. Concentration was carried out, the residue was extracted with ethyl acetate and water, the organic phase was concentrated, and then the residue was beaten with a small amount of ethyl acetate, filtered to obtain compound **7a-5** (200 mg, white solid) with a yield of 21%. LCMS (ESI): *m*/*z* 191.8[M+H]⁺; RT = 1.715 min (3 min).

### Step 5: Synthesis of 4-chloro-7-methoxy-2-methylpyridino[2,3-d]pyrimidine

At room temperature, compound **7a-5** (200 mg, 1.05 mmol) and phosphorus oxychloride (10 mL) were added sequentially to a dry single-necked bottle (50 mL). The reaction solution was heated to 100°C and reacted for 16 hours. Concentration was carried out, the residue was adjusted to pH=9 with sodium bicarbonate aqueous solution, extracted with ethyl acetate, the organic phase was dried, concentrated, and filtered to obtain compound **7a** (210 mg, white solid) with a yield of 90%. LCMS (ESI): *m*/*z* 209.8[M+H]⁺; RT = 2.44 min (5 min).

### Intermediate 8a: 7-chloro-1-methyl-1H-pyrazolo[4,3-d]pyrimidine

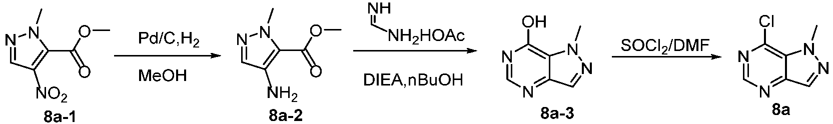

### Step 1: Synthesis of methyl 4-amino-1-methyl-1H-pyrazole-5-carboxylate

Compound **8a-1** (2.00 g, 10.8 mmol) and palladium carbon (400 mg) were added to a single-necked bottle containing 30 mL of methanol, and stirred at room temperature under hydrogen pressure (15 psi) for 3 hours. After filtering, the filtrate was concentrated to obtain compound **8a-2** (1.60 g, white solid) with a yield of 95.6%. LCMS(ESI): *m*/*z* 156.1 [M+H]⁺; RT = 0.94 min (2.00 min).

### Step 2: Synthesis of 1-methyl-1H-pyrazolo[4,3-d]pyrimidin-7-ol

Compound **8a-2** (1.60 g, 10.3 mmol) and acetamidine acetate (1.29 g, 12.4 mmol) were added to a single-necked bottle containing n-butanol (15 mL) and DIEA (15 mL), and stirred at 110°C for 6 hours. The reaction solution was cooled to room temperature, filtered, and the solid was washed with ether to obtain compound **8a-3** (1.30 g, white solid) with a yield of 84.1%; LCMS(ESI): *m*/*z* 151.2 [M+H]⁺; RT=0.623 min (2.00 min).

### Step 3: Synthesis of 7-chloro-1-methyl-1H-pyrazolo[4,3-d]pyrimidine

Compound **8a-3** (1.30 g, 8.67 mmol) and DMF (0.5 mL) were added to a single-necked bottle containing 20 mL of dichlorosulfoxide, and stirred at 90°C for 2 hours. Dichlorosulfoxide was removed, dichloromethane and water were added to separate the solution. The aqueous phase was extracted with dichloromethane, the organic phase was washed with saturated salt water, dried with anhydrous sodium sulfate, and concentrated to obtain compound **8a** (1.20 g, white solid) with a yield of 82.4%; LCMS(ESI): *m*/*z* 169.0 [M+H]⁺; RT = 1.20 min (2.00 min).

### Intermediate 9a: 4-chloro-1,3-dimethyl-1H-pyrazolo[3,4-b]pyridine

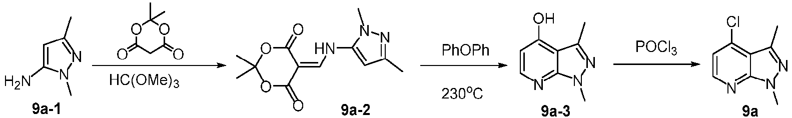

### Step 1: Synthesis of 5-(((1,3-dimethyl-1H-pyrazole-5-yl)amino)methylene)-2,2-dimethyl-1,3-dioxane-4,6-dione

2,2-dimethyl-1,3-dioxan-4,6-dione (2.33 g, 16.19 mmol) was added to a single-necked bottle (100 mL) containing 30 mL of trimethyl orthoformate. After reacting at 100°C for 2 hours, compound **9a-1** (1.5 g, 13.5 mmol) was added and stirred at 100°C for another 2 hours. After cooling the reaction solution to room temperature, the reaction solution was diluted with petroleum ether, filtered, and the solid was collected to obtain compound **9a-2** (2.6 g, yellow solid) with a yield of 74%; LCMS(ESI): *m*/*z* 264.1 [M-H]⁺; RT=1.004 min (2.50 min).

### Step 2: Synthesis of 1,3-dimethyl-1H-pyrazolo[3,4-b]pyridin-4-ol

Compound **9a-2** (2.6 g, 9.81 mmol) was added to a single-necked bottle (100 mL) containing 50 mL of diphenyl ether, stirred at 230°C for 2 hours, the reaction solution was cooled to room temperature, petroleum ether (100 mL) was added, filtered, the solid was collected and purified using a silica gel column (DCM : methanol = 15:1) to obtain compound **9a-3** (0.8 g, white solid); yield: 50%; LCMS(ESI): *m*/*z* 164.2 [M+H]⁺; RT=0.680 min (2.50 min).

### Step 3: Synthesis of 4-chloro-1,3-dimethyl-1H-pyrazolo[3,4-b]pyridine

**9a-3** (0.7 g, 4.30 mmol) was added to a single-necked bottle (100 mL) containing 10 mL of POCl₃, and the reaction solution was stirred at 100°C for 2 hours. After concentrating the reaction solution, the pH was adjusted to 8 with saturated sodium bicarbonate aqueous solution in an ice bath, the reaction solution was extracted with dichloromethane, and the organic phase was washed sequentially with water and saturated salt water, dried with anhydrous sodium sulfate, filtered and concentrated to obtain compound **9a** (700 g, gray solid) with a yield of 91%; LCMS(ESI): *m*/*z* 182.1 [M+H]⁺; RT=1.540 min (2.50 min).

### Intermediate 10a: methyl 2-(4-amino-2-methylphenyl)acetate

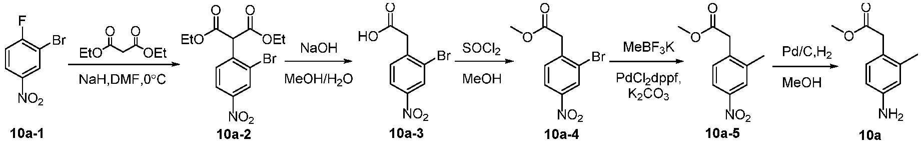

### Step 1: Synthesis of diethyl (2-bromo-4-nitrophenyl)malonate

Under nitrogen protection, sodium hydride (0.50 g, 2.27 mmol) and DMF (10 mL) were loaded sequentially into a three-necked bottle (100 mL), and diethyl malonate (0.47 mg, 2.95 mmol) was slowly added dropwise at 0°C to stir for 30 minutes. Compound **10a-1** (0.5 g, 2.27 mmol) was slowly added dropwise. The reaction solution was heated up to room temperature and stirred for 12 hours. 1 mol/L hydrochloric acid was added at room temperature to adjust the pH to 5-6, then extracted with ethyl acetate. The organic phase was washed with salt water, dried with anhydrous sodium sulfate, and the solvent was removed to obtain crude product **10a-2** (0.9 g, yellow oily substance). LCMS (ESI): *m*/*z* 359.9[M-H]⁻; RT = 1.040 min (2.50 min).

### Step 2: Synthesis of 2-(2-bromo-4-nitrophenyl)acetic acid

Compound **10a-2** (0.9 g, 2.49 mmol) and sodium hydroxide (0.4 g, 9.99 mmol) were added sequentially to a single-necked bottle (100 mL) containing 6 mL of methanol and 6 mL of water at room temperature, and reacted at room temperature for 12 hours. The solvent was removed, 2 mol/L hydrochloric acid aqueous solution was added to adjust the pH to 3-4, the solid was precipitated, filtered, and the filter cake was washed with a small amount of dichloromethane to obtain crude product **10a-3** (600 mg, yellow oily substance). LCMS (ESI): *m*/*z* 257.9[M-H]⁻; RT = 1.384 min (2.50 min).

### Step 3: Synthesis of methyl 2-(2-bromo-4-nitrophenyl)acetate

4 mL of dichlorosulfoxide was slowly added to a single-necked bottle (100 mL) containing anhydrous methanol (10 mL) at 0°C, stirred for 10 minutes. Compound **10a-3** (170 mg, 0.56 mmol) was added to a single-necked bottle and stirred at room temperature for 12 hours. Concentration was carried out to obtain crude product **10a-4** (150 mg, yellow solid). LCMS (ESI): *m*/*z* 273.2, 275.2 [M+H]⁺; RT = 1.369 min (2.50 min).

### Step 4: Synthesis of methyl 2- (2-methyl-4-nitrophenyl)acetate

Compound **10a-4** (150 mg, 0.48 mmol) was loaded into a 100 mL three-necked bottle, and a mixture of 1,4-dioxane and water (9 mL, 10/1), MeBF₃K (99 mg, 0.27 mmol), potassium carbonate (275 mg, 2.27 mmol), and PdCl₂dppf (45 mg, 0.02 mmol) were added respectively. The reaction solution was stirred at 80°C for 8 hours. Ethyl acetate and water were added for extraction. The organic phase was washed with salt water, dried with anhydrous sodium sulfate, and the solvent was removed. The crude product was purified using prep-TCL (PE/EA = 5:1) to obtain compound **10a-5** (165 mg, gray white solid). Yield: 97%. LCMS (ESI): *m*/*z* 210.2 [M+H]⁺; RT = 1.456 min (2.50 min).

### Step 5: Synthesis of methyl 2-(4-amino-2-methylphenyl)acetate

Compound **10a-5** (160 mg, 0.76 mmol) was added to a single-necked bottle (100 mL) containing 3 mL of methanol, then palladium carbon (16 mg) was added and stirred at 30°C under hydrogen protection for 12 hours. The reaction solution was filtered with diatomite, washed with methanol, and the organic phase was removed to obtain crude product **10a** (130 mg, gray white solid). LCMS (ESI): *m*/*z* 180.2 [M+H]⁺; RT = 0.756 min (2.50 min).

### Intermediate 11a: 2-(4-amino-2-fluorophenyl)methyl propionate

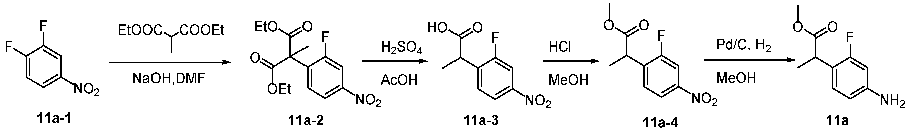

### Step 1: Synthesis of diethyl(2-fluoro-4-nitrophenyl) -2-methylmalonate

Compound **11a-1** (1 g, 6.23 mmol) was added to a single-necked bottle (100 mL) containing DMF (15 mL), followed by the addition of diethyl methylmalonate (1.3 g, 7.55 mmol) and sodium hydroxide (377 mg, 9.43 mmol). The reaction solution was stirred at room temperature for 2 hours. The reaction solution was poured into water, extracted with ethyl acetate, and the organic phase was washed with water, washed with saturated salt water, dried with anhydrous sodium sulfate, and concentrated. Compound **11a-2** (2.0 g, yellow oil) was purified using a positive column (PE: EA = 60:1). Yield: 100 %, LCMS (ESI): *m*/*z* 314.1 [M+H]⁺; RT = 1.832 min (2.50 min).

### Step 2: Synthesis of 2-(2-fluoro-4-nitrophenyl)propionic acid

At room temperature, compound **11a-2** (2.0 g, 6.04 mmol) was added to a single-necked bottle (100 mL) containing 7.5 mL of acetic acid and 5 mL of water, and 2 mL of concentrated sulfuric acid was added slowly. The reaction solution was reacted overnight at 130°C. The solvent was removed, 20 mL of water was added, the pH was adjusted to 11-12, the reaction solution was extracted with ethyl acetate, and the aqueous phase was retained; the pH of the aqueous phase was adjusted to 3-4, the reaction solution was extracted with ethyl acetate, and the organic phase was washed with water, washed with saturated salt water, dried with anhydrous sodium sulfate, and concentrated to obtain compound **11a-3** (1.0 g, colorless oil). Yield: 73.4%. ¹H NMR (400 MHz, CDCl₃): δ 8.06-8.03 (m, 1H), 7.97-7.94 (m, 1H), 7.55-7.51 (m, 1H), 4.16-4.11 (m, 1H), 1.59 (d, *J* = 7.2 Hz, 3H).

### Step 3: Synthesis of methyl 2-(2-fluoro-4-nitrophenyl)propanoate

Compound **11a-3** (1.0 g, 5.49 mmol) was added to a single-necked bottle (100 mL) containing 15 mL of methanol, then concentrated hydrochloric acid (0.2 mL) was added and stirred at 75°C for 3 hours. The reaction solution was concentrated directly to obtain crude compound **11a-4** (1.0 g, colorless oil). LCMS (ESI): *m*/*z* 226.1 [M-H]⁻; RT = 1.672 min (2.50 min).

### Step 4: Synthesis of methyl 2-(4-amino-2-fluorophenyl)propanoate

At room temperature, compound **11a-4** (1.0 g, 4.4 mmol) and palladium carbon catalyst (100 mg) were added sequentially to a single-necked bottle (100 mL) containing methanol (20 mL), and reacted at room temperature for 2 hours under hydrogen. The reaction solution was filtered and concentrated to obtain crude compound **11a** (900 mg, colorless oil). LCMS (ESI): m/z 239.2 [M+H+41]⁺; RT = 1.309 min (2.50 min).

### Intermediate 12a: ethyl 6-amino-2,3-dihydrobenzofuran-3-carboxylate

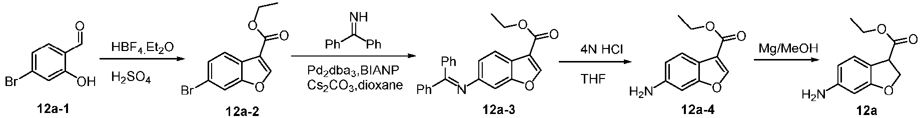

### Step 1: Synthesis of ethyl 6-bromobenzofuran-3-carboxylate

Compound **12a-1** (8 g, 40.0 mmol) and tetrafluoroborate ether solution (0.6 mL) were added to a single-necked bottle (250 mL) containing 40 mL of dichloromethane, and then 10 mL of dichloromethane solution of ethyl nitroacetate (9.20 g, 80.0 mmol) was added dropwise, and stirred at room temperature for 1 hour. The solvent was concentrated and removed, 4 mL of concentrated sulfuric acid was added, and stirred at room temperature for 1 hour. The reaction solution was poured into ice water, extracted with dichloromethane, and the organic phase was merged. The organic phase was washed with saturated sodium chloride, dried with anhydrous sodium sulfate, filtered, and concentrated to obtain crude product. After column purification (PE : EA = 3:1), compound **12a-2** (7.10 g, white solid) was obtained with a yield of 66%. ¹H NMR (400 MHz, CDCl₃): δ 8.21 (s, 1H), 7.92 (d, *J* = 8.0 Hz, 1H), 7.71 (d, *J* = 1.6 Hz, 1H), 7.48 (dd, *J* = 8.4, 1.6 Hz, 1H), 4.45-4.36 (m, 2H), 1.42 (t, *J* = 8.0 Hz, 3H).

### Step 2: Synthesis of ethyl 6-(diphenylmethyleneamino)benzofuran-3-carboxylate

**12a-2** (2.70 g, 10.0 mmol), benzophenone imine (2.72 g, 10.0 mmol), cesium carbonate (6.52 g, 20.0 mmol), BIANP (623 mg, 1.0 mmol), and Pd₂dba₃ (916 mg, 1.0 mmol) were added sequentially to a single-necked bottle (100 mL) containing 1,4-dioxane (50 mL), and reacted at 100°C under nitrogen protection for 16 hours. After removing the solvent, column purification (PE/EA = 1/1) was carried out to obtain compound **12a-3** (3.40 mg, yellow solid) with a yield of 92%; LCMS(ESI): *m*/*z* 370.3 [M+H]⁺; RT = 1.64 min (2.50 min).

### Step 3: Synthesis of ethyl 6-aminobenzofuran-3-carboxylate

**12a-3** (3.40 g, 9.21 mmol) and 4N hydrochloric acid (25 mL) were added to a single-necked bottle (250 mL) containing 50 mL of THF, and stirred at room temperature for 3 hours. After removing the solvent, the aqueous phase was washed with ethyl acetate and the pH was adjusted to 8 with a sodium bicarbonate solution. Further EA extraction was performed, the organic phase was washed with saturated sodium chloride, dried with anhydrous sodium sulfate, filtered, and concentrated to obtain compound **12a-4** (1.70 g, yellow solid) with a yield of 90%; LCMS(ESI): m/z 306.0 [M+H]⁺; RT = 1.476 min (2.50 min).

### Step 4: Synthesis of ethyl 6-amino-2,3-dihydrobenzofuran-3-carboxylate

Compound **12a-4** (1.30 g, 6.34 mmol) and magnesium shavings (1.54 g, 63.40 mmol) were added to a single-necked bottle (250 mL) containing methanol (100 mL), and stirred at room temperature for 4 hours. Ethyl acetate was added and concentrated to obtain crude product. After column purification (PE/EA = 1/1), compound **12a** (1.10 g, yellow oily substance) was obtained with a yield of 84%; LCMS(ESI): *m*/*z* 208.2 [M+H]⁺; RT=1.18 min (2.50 min).

### Intermediate 13a: ethyl 2-(6-aminopyridin-3-yl)-2-methylpropanoate

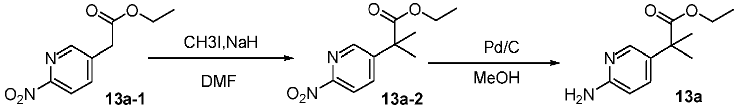

### Step 1: Synthesis of ethyl 2-methyl-2-(6-nitropyridin-3-yl)propanoate

Compound **13a-1** (1.5 g, 7.14 mmol) was dissolved in DMF (15 mL), add sodium hydride (857 mg, 21.42 mmol) in batches at 0°C, stirred at 0°C for half an hour, then iodomethane (3.04 g, 21.42 mmol) was slowly added dropwise. After dropwise addition, the reaction solution was stirred at room temperature for 3 hours. The reaction solution was poured into water to be quenched, extracted with ethyl acetate, and the organic phase was washed with saturated salt water, dried with anhydrous sodium sulfate, filtered, concentrated, and purified (PE/EA= 10/1) to obtain compound **13a-2** (1.0 g, yellow oily liquid) with a yield of 59%. LCMS (ESI): *m*/*z* 239.1[M+H]⁺; RT= 1.56 min (3 min).

### Step 2: Synthesis of ethyl 2-(6-aminopyridin-3-yl)-2-methylpropanoate

Compound **13a-2** (1 g, 4.2 mmol), anhydrous methanol (15 mL), and 10% palladium carbon (200 mg) were added sequentially to a dry single-necked bottle (50 mL) at room temperature, and reacted overnight under nitrogen protection. The reactant was filtered under reduced pressure, and the filtrate was concentrated to obtain compound 13a (600 mg, yellow oily liquid) with a yield of 68.7%. LCMS (ESI): *m*/*z* 209.1[M+H]⁺; RT= 0.99 min (3 min).

### Intermediate 14a: Methyl 4-(4-hydroxyphenyl)tetrahydro-2H-pyran-4-carboxylate

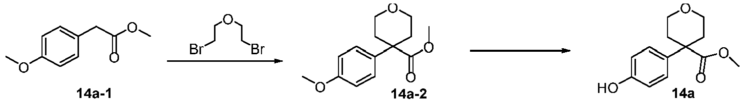

### Step 1: Synthesis of methyl 4-(4-methoxyphenyl)tetrahydro-2H-pyran-4-carboxylate

**14a-1** (5.0 g, 27.78 mmol) was added to a three-necked flask containing 20 mL of N-methylpyrrolidone, and sodium hydride (2.8 g, 69.45 mmol) was added under an ice bath. The reaction was carried out at 0°C for half an hour, 1-bromo-2-(2-bromoethoxy)ethane (7.1 g, 30.56 mmol) was added, and reacted at room temperature for 1 hour. The reaction solution was quenched with saturated ammonium chloride, extracted with ethyl acetate, the organic phase was washed with saturated salt water, dried with anhydrous sodium sulfate, and performed column chromatography (EA:PE = 1:3) to obtain compound **14a-2** (2.1 g, colorless oil); ¹H NMR(400 MHz, CDC1₃-*d*): δ 7.27 (d, *J* = 8.0 Hz, 2H), 6.88 (d, *J* = 8.8 Hz, 2H), 3.97-3.91 (m, 2H), 3.84 (s, 3H), 3.67 (s, 3H), 3.61-3.40 (m, 2H), 2.52-2.49 (m, 2H), 1.99-1.93 (m, 2H).

### Step 2: Synthesis of methyl 4-(4-hydroxyphenyl)tetrahydro-2H-pyran-4-carboxylate

Compound **14a-2** (1 g, 4.0 mmol) was added to a three-necked flask containing 15 mL of dichloromethane, and boron tribromide (5.0 g, 20.0 mmol) was added in an ice bath, and reacted at room temperature for 2 hours. The reaction solution was poured into water, extracted with dichloromethane, the organic phase was washed with water, washed with saturated salt water, dried with anhydrous sodium sulfate, concentrated to obtain crude product **14a** (900 mg, colorless oil); ¹H NMR(400 MHz, DMSO-*d₆*): δ 9.40 (s, 1H), 7.15 (d, *J* = 8.4 Hz, 2H), 6.73 (d, *J* = 8.8 Hz, 2H), 3.80-3.76 (m, 2H), 3.58 (s, 3H), 3.40-3.35 (m, 2H), 2.36-2.32 (m, 2H), 1.84-1.77 (m, 2H).

### Intermediate 15a: 6-hydroxy-1-methyl-1,2,3,4-tetrahydronaphthalene-1-carboxylic acid

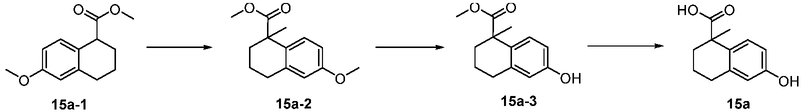

### Step 1: Synthesis of methyl 6-methoxy-1-methyl-1,2,3,4-tetrahydronaphthalene-1-carboxylate

Sodium hydride (270 mg, 6.81 mmol) and DMF (20 mL) were added sequentially to a dry three-necked flask (50 mL) at 0°C. Under nitrogen protection, compound **15a-1** (500 mg, 2.27 mmol) and a DMF (10 mL) solution of iodomethane (0.42 mL, 6.81 mmol) were added at 0°C. The reaction solution was stirred at 0°C for 30 minutes, then stirred at room temperature for 16 hours. The reaction solution was poured into water and extracted with ethyl acetate. The organic phase was washed with saturated salt water, dried with anhydrous sodium sulfate, filtered, and the filtrate was concentrated under reduced pressure. The crude product was purified using a preparation plate (PE: EA = 20:1) to obtain compound **15a-2** (500 mg, yellow oil) with a yield of 94.01%. LCMS (ESI): m/z 235.2 [M-H]⁻; RT = 1.567 min (2.50 min).

### Step 2: Synthesis of methyl 6-hydroxy-1-methyl-1,2,3,4-tetrahydronaphthalene-1-carboxylate

Aluminum trichloride (1420 mg, 10.67 mmol), dichloromethane (30 mL), and compound **15a-2** (500 mg, 2.13 mmol) were added sequentially to a dry three-necked flask (100 mL) at 0°C. The reaction solution was stirred at 0°C for 10 minutes, then ethyl mercaptan (0.79 mL, 10.67 mmol) was added. The reaction solution was stirred at room temperature for 2 hours. The reaction solution was poured into water and extracted with dichloromethane. The organic phase was washed with saturated salt water, dried with anhydrous sodium sulfate, filtered, and the filtrate was concentrated under reduced pressure to obtain compound **15a-3** (430 mg, yellow oil) with a yield of 91.48%, LCMS (ESI): m/z 221.2 [M+H]⁺; RT = 1.506 min (2.50 min).

### Step 3: Synthesis of 6-hydroxy-1-methyl-1,2,3,4-tetrahydronaphthalene-1-carboxylic acid

Compound **15a-3** (380 mg, 1.73 mmol), 1,4-dioxane (24 mL), water (8 mL), and potassium tert butanol (968 mg, 8.63 mmol) were added sequentially to a dry single-necked flask (25 mL) at room temperature. The reaction solution was heated to 60°C and reacted for 24 hours. The pH of the reaction solution was adjusted to 4 using 1 mol/L hydrochloric acid aqueous solution, and the reaction solution was extracted with ethyl acetate. The organic phase was collected, washed with saturated salt water, dried with anhydrous sodium sulfate, filtered, and the filtrate was concentrated under reduced pressure to obtain product **15a** (350 mg, white solid) with a yield of 98.37%, LCMS (ESI): m/z 205.2 [M-H]⁻; RT = 1.258 min (2.50 min).

### Intermediate 16a: methyl 2-(indol-5-yl)acetate

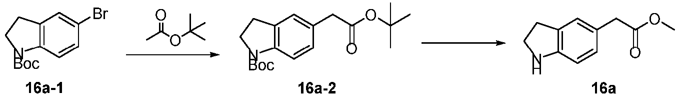

### Step 1: Synthesis of tert-butyl 5-(2-(tert-butoxy)-2-oxoethyl)indole-1-carboxylate

2.5 mL of lithium bis(trimethylsilyl)amide (2.50 mmol), palladium acetate (II) (catalytic amount), and tri-tert-butylphosphine (catalytic amount) were loaded into a 100 mL three-necked bottle under nitrogen protection, and then 5 mL of anhydrous toluene was added. Tert-butyl acetate (269 mg, 2.32 mmol) was slowly added dropwise at -10°C and stirred for 20 minutes. **16a-1** (300 g, 1.01 mmol) was added dropwise and heated up to 80°C to react for 2 hours. Ethyl acetate and water were added for extraction at room temperature. The organic phase was washed with salt water, dried with anhydrous sodium sulfate, concentrated and purified on a column (PE: EA = 10:1) to obtain **16a-2** (180 mg, yellow oily substance). Yield: 54%. ¹H NMR (400 MHz, CDCl₃): δ 7.65-7.44 (m, 1H), 7.05-7.03 (m, 1H), 3.99-3.96 (m, 2H), 3.44 (s, 2H), 3.08-3.05 (m, 2H), 1.56 (s, 9H), 1.47 (s, 9H).

### Step 2: Synthesis of methyl 2-(indol-5-yl)acetate

**16a-2** (160 mg, 0.48 mmol), 5 mL methanol, and 3 drops of concentrated sulfuric acid were added sequentially to a single-necked bottle (50 mL). The reaction solution was stirred overnight at 70°C. After cooling to room temperature, the solvent was removed, water and ethyl acetate were added, and the pH of the sodium bicarbonate aqueous solution was adjusted to 9. The organic phase was washed with salt water, dried with anhydrous sodium sulfate, and the solvent was removed to obtain **16a** (90 mg, yellow oily substance). LCMS (ESI): m/z 192.2 [M-H]⁻; RT = 0.645 min (2.50 min).

### Intermediate 17a: 3-(4-methoxyphenyl)tetrahydrofuran-3-carboxylic acid

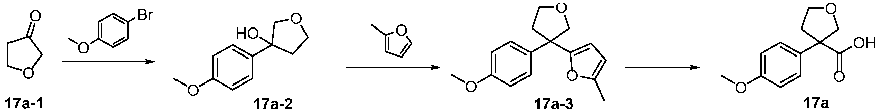

### Step 1: Synthesis of 3-(4-methoxyphenyl)tetrahydrofuran-3-ol

N-butyl lithium (8.7 ml, 13.48 mmol, 1.6 N) was slowly added dropwise to a 100 mL three-necked bottle containing anhydrous THF (25 mL) containing p-bromobenzyl ether (2.82 g, 14.6 mmol) under nitrogen protection at -78°C. After 30 minutes of reaction, THF solution of compound **17a-1** (1.0 g, 11.23 mmol) was slowly added dropwise, stirred at -78°C for 10 minutes, heated up to room temperature, and the reaction solution was quenched with saturated ammonium chloride solution, diluted with water, and washed with ethyl acetate and water. The organic phase was washed with sodium chloride, dried with anhydrous sodium sulfate, and filtered. Compound **17a-2** (2.0 g, white solid) was purified using a silica gel column (PE: EA = 1:1). Yield: 92%. ¹H NMR (400 MHz, CDCl₃): δ 7.41-7.38 (m, 2H), 6.92-6.88 (m, 2H), 4.22-4.07 (m, 2H), 3.99-3.95 (m, 1H), 3.86 (d, *J* = 9.2 Hz, 1H), 3.81 (s, 3H), 2.42-2.35 (m, 1H), 2.27-2.16 (m, 1H).

### Step 2: Synthesis of 2-(3-(4-methoxyphenyl)tetrahydrofuran-3-yl)-5-methylfuran

Compound **17a-2** (200 mg, 1.03 mmol), 2-methylfuran (422 mg, 5.1 mmol), bis((trifluoromethyl)sulfonyl)lithium amino (33 mg, 0.11 mmol), and tetrabutylhexafluorophosphate ammonium (22 mg, 0.062 mmol) were added sequentially to a single-necked bottle (50 mL) containing 5 mL of anhydrous toluene, reacted under nitrogen protection at 60°C for 2 hours. After cooling to room temperature, the reaction solution was diluted with water, extracted with ethyl acetate, and the organic phase was washed with sodium chloride, dried with anhydrous sodium sulfate, and filtered. Compound **17a-3** (220 mg, yellow oily substance) was purified using prep-TLC (PE: EA= 15:1). Yield: 83%. ¹H NMR (400 MHz, CDCl₃): δ 7.19-7.15 (m, 2H), 6.86-6.82 (m, 2H), 5.99 (d, *J* = 3.2 Hz, 1H), 5.87-5.86 (m, 1H), 4.35 (d, *J* = 8.4 Hz, 1H), 4.12 (d, *J* = 8.4 Hz, 1H), 4.02-3.97 (m, 2H), 3.78 (s, 3H), 2.72-2.66 (m, 1H), 2.42-2.35 (m, 1H), 2.22 (s, 3H).

### Step 3: Synthesis of 3-(4-methoxyphenyl)tetrahydrofuran-3-carboxylic acid

Compound **17a-3** (220 mg, 0.85 mmol), sodium periodate (422 mg, 5.1 mmol), and ruthenium trichloride (catalytic amount) were added sequentially to a single-necked bottle (50 mL) containing 8 mL of mixed solvent (n-hexane: EA: water = 1:1:2) at 0°C. The reaction was carried out overnight under nitrogen protection at room temperature. The reaction solution was diluted with water, extracted with ethyl acetate, and the organic phase was washed with sodium chloride, dried with anhydrous sodium sulfate, and filtered. The filtrate was concentrated to obtain compound **17a** (200 mg, yellow solid). ¹H NMR (400 MHz, DMSO-*d₆*): δ 12.51 (brs, 1H), 7.23-7.21 (m, 2H), 6.91-6.89 (m, 2H), 4.50 (d, *J* = 8.0 Hz, 1H), 3.86-3.76 (m, 2H), 3.73 (s, 3H), 3.68 (d, *J* = 8.4 Hz, 1H), 2.84-2.78 (m, 1H), 2.15-2.08 (m, 1H).

### Intermediate 18a: Methyl 6-hydroxy-1,2,3,4-tetrahydronaphthalene-1-carboxylate

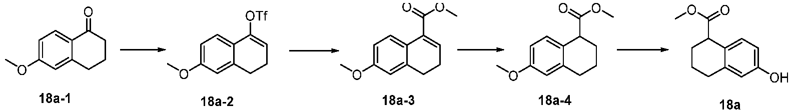

### Step 1: Synthesis of 6-methoxy-3,4-dihydronaphthalen-1-yl trifluoromethanesulfonate

Compound **18a-1** (500 mg, 2.84 mmol) and anhydrous THF (10 mL) were added sequentially to a dry single-necked flask (50 mL) at room temperature. Under nitrogen protection, the reaction solution was cooled to -78°C, lithium bis(trimethylsilyl)amide (4.26 mL, 4.26 mmol) was slowly added dropwise, and reacted for 1 hour. N-phenyl-bis(trifluoromethanesulfonimide) (2030 mg, 5.67 mmol) was added and reacted at -78°C for 2 hours. 20 mL of water was added and extracted with ethyl acetate. The organic phase was collected, washed with saturated salt water, dried with anhydrous sodium sulfate, and filtered The crude product was purified using column chromatography (PE:EA = 50:1) to obtain compound **18a-2** (800 mg, yellow solid) with a yield of 91.46%, ¹H NMR(400 MHz, CDCl₃): δ 7.42-7.38 (m, 1H), 6.78-6.73 (m, 2H), 5.86 (t, *J* = 4.8 Hz, 1H), 3.81 (s, 3H), 2.83 (t, *J* = 8.0 Hz, 2H), 2.50-2.45 (m, 2H).

### Step 2: Synthesis of methyl 6-methoxy-3,4-dihydronaphthalen-1-carboxylate

Compound **18a-2** (700 mg, 2.27 mmol), methanol (20 mL), triethylamine (1.58 mL, 11.35 mmol), and PdCl₂dppf (830 mg, 1.14 mmol) were added sequentially to a single-necked flask (50 mL) at room temperature. Carbon monoxide was replaced three times to stir at 90°C for 16 hours. Reduced pressure concentration was carried out. The crude product was purified using a preparation plate (PE:EA = 20:1) to obtain compound **18a-3** (300 mg, yellow oil) with a yield of 60.53%, ¹H NMR(400 MHz, CDCl₃): δ 7.74 (d, *J* = 8.4 Hz, 1H), 7.03 (t, *J* = 4.8 Hz, 1H), 6.77-6.71 (m, 2H), 3.83 (s, 3H), 3.81 (s, 3H), 2.74 (t, *J* = 8.0 Hz, 2H), 2.40-2.35 (m, 2H).

### Step 3: Synthesis of methyl 6-methoxy-1,2,3,4-dihydronaphthalen-1-carboxylate

Compound **18a-3** (300 mg, 1.37 mmol), methanol (10 mL), and palladium carbon catalyst (30 mg) were added sequentially to a dry single-necked flask (50 mL) at room temperature. Hydrogen was replaced three times to stir at room temperature for 16 hours. Concentrating under reduced pressure to obtain compounds **18a-4** (240 mg, yellow oil) with a yield of 79.27%, ¹H NMR(400 MHz, CDCl₃): δ 7.08 (d, *J* = 8.8 Hz, 1H), 6.72-6.70 (m, 1H), 6.63 (d, *J* = 2.4 Hz, 1H), 3.79-3.77 (m, 4H), 3.71 (s, 3H), 2.85-2.71 (m, 2H), 2.16-2.10 (m, 1H), 2.02-1.92 (m, 2H), 1.78-1.72 (m, 1H).

### Step 4: Synthesis of methyl 6-hydroxy-1,2,3,4-tetrahydronaphthalene-1-carboxylate

Aluminum trichloride (726 mg, 5.45 mmol), dichloromethane (15 mL), and compound **18a-4** (240 mg, 1.09 mmol) were added sequentially to a three-necked flask (50 mL) at 0°C. The reaction solution was stirred at 0°C for 10 minutes, then ethyl mercaptan (0.40 mL, 5.45 mmol) was added to react at room temperature for 2 hours. The reaction solution was poured into water and extracted with dichloromethane. The organic phase was collected, washed with saturated salt water, dried with anhydrous sodium sulfate, filtered, and the filtrate was concentrated under reduced pressure to obtain compound 18a (210 mg, yellow oil) with a yield of 93.45%, ¹H NMR(400 MHz, CDCl₃-d): δ 7.01 (d, *J* = 8.4 Hz, 1H), 6.61-6.58 (m, 1H), 6.54 (d, *J* = 2.4 Hz, 1H), 5.02 (s, 1H), 3.78-3.75 (m, 1H), 3.71 (s, 3H), 2.81-2.64 (m, 2H), 2.15-1.88 (m, 3H), 1.77-1.68 (m, 1H).

### Intermediate 19a: Methyl 2- (4-hydroxyphenyl) -3-methylbutanoate

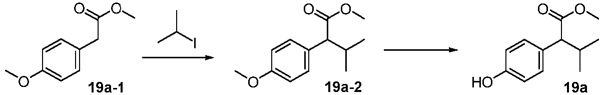

### Step 1: Synthesis of methyl 2-(4-methoxyphenyl)-3-methylbutanoate

Potassium tertbutanol-THF (1.0 M, 8.25 mL, 8.25 mmol), THF (20 mL), compound **19a-1** (1.00 g, 5.50 mmol), and iodoisopropane (1.50 g, 8.25 mmol) were added sequentially to a dry single-necked flask (100 mL) at 0°C. The reaction solution was reacted at room temperature for 2 hours. The reaction solution was poured into 50 mL of water and extracted with ethyl acetate. The organic phase was collected washed with saturated salt water, dried with anhydrous sodium sulfate, filtered, and the filtrate was concentrated under reduced pressure. The crude product was purified using chromatography column (PE: EA = 5:1) to obtain compound **19a-2** (0.86 g, yellow solid) with a yield of 69.70%, ¹H NMR(400 MHz, CDCl₃-d): δ 9.60 (d, *J* = 8.4 Hz, 2H), 9.20 (d, *J* = 8.4 Hz, 2H), 6.15 (s, 3H), 6.00 (s, 3H), 5.46 (d, *J* = 10.8 Hz, 1H), 4.68-4.62 (m, 1H), 3.38 (d, *J* = 6.4 Hz, 3H), 3.04 (d, *J* = 8.8 Hz, 3H).

### Step 2: Synthesis of methyl 2-(4-hydroxyphenyl)-3-methylbutanoate

Aluminum trichloride (2.58 g, 19.34 mmol), DCM (100 mL), and compound **19a-2** (0.86 g, 3.87 mmol) were added sequentially to a three-necked flask (100 mL) at 0°C. The reaction solution was stirred at 0°C for 10 hours. Ethanethiol (1.43 mL, 19.34 mmol) was added at 0°C. The reaction solution was reacted at room temperature for 2 hours. After the reaction was complete, the reaction solution was poured into 100 mL of water and extracted with ethyl acetate. The organic phase was collected, washed with saturated salt water, dried with anhydrous sodium sulfate, filtered, and the filtrate was concentrated under reduced pressure. The crude product was purified using chromatography column (PE: EA = 3:1) to obtain compound **19a** (0.68 g, yellow oil) with a yield of 84.40%, LCMS(ESI): m/z 209.2 [M+H]⁺; RT = 1.571 min (2.50 min).

### Intermediate 20a: 4-(4-methoxyphenyl)oxocycloheptane-4-carboxylic acid

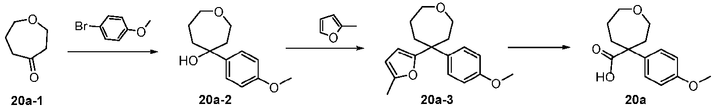

### Step 1: Synthesis of 4-(4-methoxyphenyl)oxocycloheptane-4-ol

1-bromo-4-methoxybenzene (5.9 g, 31.6 mmol) and n-butyl lithium (14.7 mL, 26.3 mmol) were added sequentially to a three-necked bottle (250 mL) containing 60 mL of THF at -78°C. Compound **20a-1** (3.0 g, 36.8 mmol) was added after half an hour of reaction and reacted at - 78°C for 2 hours. The reaction solution was quenched with saturated ammonium chloride, diluted with water, and the organic phase was extracted with ethyl acetate, washed with water, washed with saturated salt water, and concentrated to obtain crude product **20a-2** (4.2 g, colorless oil). Yield: 71.9%, ¹H NMR (400 MHz, CDCl₃): δ 7.42-7.40 (m, 2H), 6.88-6.86 (m, 2H), 3.94-3.88 (m, 2H), 3.80 (s, 3H), 3.75-3.67 (m, 2H), 2.29-2.12 (m, 4H), 1.93-1.88 (m, 2H).

### Step 2: Synthesis of 4-(4-methoxyphenyl)-4-(5-methylfuran-2-yl)oxocycloheptane

**20a-2** (4.2 g, 18.9 mmol), 2-methylfuran (7.76 g, 94.6 mmol), lithium bis(trifluoromethanesulphonyl)imide (597 mg, 2.08 mmol), and ammonium tetrabutylhexafluorophosphate (366 mg, 0.95 mmol) were added to a 100 mL single-necked bottle containing 50 mL of toluene, and reacted at 40°C for 2 hours. The reaction solution was filtered, and the filtrate was concentrated and carried out column chromatography to obtain compound **20a-3** (4.7 g, colorless oil). Yield: 86.9%, ¹H NMR (400 MHz, CDCl₃): δ 7.09-7.06 (m, 2H), 6.82-6.80 (m, 2H), 5.97-5.90 (m, 2H), 3.76-3.71 (m, 7H), 2.40-2.29 (m, 4H), 2.23 (s, 3H), 1.75-1.72 (m, 2H).

### Step 3: Synthesis of 4- (4-methoxyphenyl)oxocycloheptane-4-carboxylic acid

**20a-3** (4.7 g, 16.4 mmol) was added to a single-necked bottle (2000 mL) containing a mixture of 200 mL n-hexane, 200 mL ethyl acetate, and 400 mL water at room temperature. Sodium periodate (24.6 g, 115 mmol) was added to an ice bath, stirred for 5 minutes, and then ruthenium trichloride (catalytic amount) was added. The reaction solution was filtered, diluted with water, extracted with ethyl acetate, and the organic phase was washed sequentially with water and saturated salt water, dried with anhydrous sodium sulfate, and concentrated to obtain crude product **20a** (2.6 g, yellow oil). LCMS (ESI): m/z 251.1 [M+H]⁺; RT = 1.064 min (2.50 min).

### Intermediate 21a: 3- (4-methoxyphenyl)-8-oxadiacyclo[3.2.1]octane-3-carboxylic acid

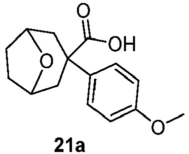

Referring to the synthesis method of intermediate **20a,** 8-oxadiacyclo[3.2.1]octane-3-one was used as the starting material. ¹H NMR (400 MHz, DMSO-d*₆*): δ 12.21-12.17 (m, 1H), 7.40 (d, *J* = 8.8 Hz, 2H), 6.88 (d, *J* = 8.8 Hz, 2H), 4.33-4.29 (m, 2H), 3.74 (s, 1H), 2.73-2.68 (m, 2H), 1.96-1.92 (m, 2H), 1.69-1.66 (m, 2H), 1.20-1.17 (m, 2H).

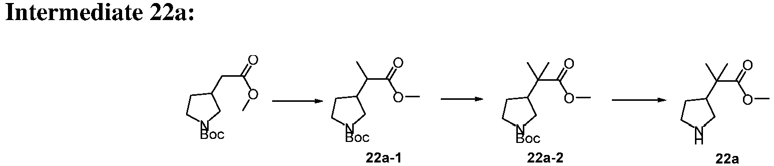

### Step 1: tert-butyl 3-(1-methoxy-1-oxopropan-2-yl)pyrrolidin-1-carboxylate

Under nitrogen protection, 3-(2-methoxy-2-oxoethyl)pyrrolidin-1-tert butyl formate (500 mg, 2.05 mmol) and THF (5 mL) were added sequentially to a three-necked bottle (100 mL). Diisopropylamine lithium (2 M, 3 mL) was added dropwise at -78°C. After 1 hour of reaction, a THF solution of iodomethane (1.75 g, 12.3 mmol) (5 mL) was added. The reaction was carried out at room temperature for 1 hour, the reaction solution was quenched with saturated ammonium chloride solution, diluted with water, extracted with ethyl acetate, and the organic phase was washed sequentially with water and saturated salt water, dried with anhydrous sodium sulfate, filtered, and concentrated to obtain the crude product **22a-1** (150 mg, yellow solid).

### Step 2: tert-butyl 3-(1-methoxy-2-methyl-1-oxopropan-2-yl)pyrrolidin-1-carboxylate

Under nitrogen protection, **22a-1** (150 mg, 0.58 mmol) and THF (3 mL) were added sequentially to a three-necked bottle (50 mL). Lithium diisopropylamine (2 M, 0.88 mL) was added dropwise at -78°C. After 1 hour of reaction, THF solution of iodomethane (123 mg, 0.87 mmol) was added. The reaction was carried out at room temperature for 1 hour, the reaction solution was quenched with saturated ammonium chloride solution, diluted with water, extracted with ethyl acetate, and the organic phase was washed sequentially with water and saturated salt water, dried with anhydrous sodium sulfate, filtered, and concentrated to obtain compound **22a-2** (105 mg, transparent oil); ¹H NMR (400 MHz, DMSO-*d₆*): δ 3.68 (d, *J*=6.0 Hz, 3H), 3.55-3.36 (m, 2H), 3.26-3.20 (m, 1H), 3.10-2.99 (m, 1H), 2.45-2.37 (m,1H),1.87-1.81 (m, 1H), 1.46 (s, 9H), 1.19 (s, 6H).

### Step 3: methyl 2-methyl-2-(pyrrolidin-3-yl)propanoate hydrochloride

Compound **22a-2** (800 mg, 2.95 mmol) and TFA (2 mL) were added sequentially to a single ended bottle (50 mL) containing 6 mL of dichloromethane at room temperature. The reaction solution was reacted at room temperature for 4 hours, adjusted to pH = 9, diluted with water, extracted with dichloromethane, and the organic phase was washed sequentially with water and saturated salt water, dried with anhydrous sodium sulfate, filtered, and concentrated to obtain the crude compound **22a** (500 mg, yellow oil).

### Example 1 Preparation of Compound 1

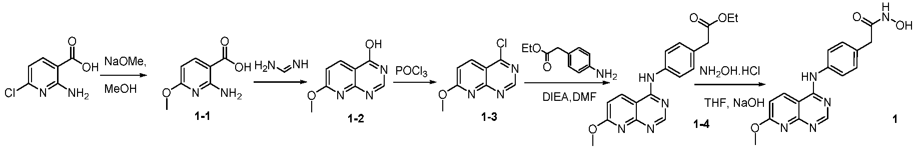

### Step 1: Synthesis of 2-amino-6-methoxynicotinic acid

2-amino-6-chloro-nicotinic acid (5.0 g, 29.0 mmol), sodium methoxide (15.7 g, 290 mmol), and methanol (50 mL) were added sequentially to a dry single-necked bottle (100 mL) at room temperature. The reaction solution was heated to 80°C and reacted for 48 hours. After cooling to room temperature, the reaction solution was filtered under reduced pressure, and the filter cake was washed with methanol, vacuum dried to obtain compound **1-1** (2.0 g, white solid) with a yield of 40%. LCMS (ESI): *m*/*z* 169.0 [M+H]⁺; RT = 1.10min (3 min).

### Step 2: Synthesis of 7-methoxypyridino[2,3-d]pyrimidin-4-phenol

**1-1** (1.0 g, 6.0 mmol), acetamidine acetate (624 mg, 17.8 mmol), and ethylene glycol methyl ether (10 mL) were added sequentially to a dry single-necked bottle (50 mL) at room temperature. The reaction solution was heated to 100°C and reacted for 18 hours. The reaction solution was concentrated and the residue was purified on a silica gel column (DCM:methanol = 10:1) to obtain compound **1-2** (200 mg, white solid) with a yield of 20%. LCMS (ESI): *m*/*z* 177.9 [M+H]⁺; RT = 1.502min (3 min).

### Step 3: Synthesis of 4-chloro-7-methoxypyridino[2,3-d]pyrimidine

**1-2** (200 mg, 1.13 mmol) and phosphorus oxychloride (10 mL) were added sequentially to a dry single-necked bottle (50 mL) at room temperature. The reaction solution was heated to 100°C and reacted for 16 hours. The reaction solution was concentrated, the residue was alkalized with sodium bicarbonate solution to pH 9, extracted with dichloromethane, and the organic phase was dried with sodium sulfate, concentrated to obtain compound **1-3** (200 mg, white solid) with a yield of 90%. LCMS (ESI): *m*/*z* 339.7[M+H]⁺; RT = 1.521 min (3 min).

### Step 4: Synthesis of ethyl 2-(4-((7-methoxypyridino[2,3-d]pyrimidin-4-yl) amino)phenyl) acetate

**1-3** (200 mg, 1.02 mmol) and 2-(4-aminophenyl)ethyl acetate (275 mg, 1.54 mmol) were added sequentially to a dry single-necked bottle(50 mL) at room temperature. The reaction solution was heated up to 80°C and reacted for 16 hours. The reaction solution was extracted with ethyl acetate and water, the organic phase was washed with saturated salt water, dried with sodium sulfate, concentrated and purified (PE: EA = 10:1-1:1) to obtain compound **1-4** (100 mg, white solid) with a yield of 29%. LCMS (ESI): *m*/*z* 339.1 [M+H]⁺; RT = 1.33 min (3 min).

### Step 5: Synthesis of N-hydroxy-2-(4-((7-methoxypyridino[2,3-d]pyrimidin-4-yl)amino)phenyl)acetamide

**1-4** (100 mg, 0.30 mmol), 50% hydroxylamine aqueous solution (1 mL), methanol (2 mL), THF (2 mL), and sodium hydroxide (60 mg, 1.5 mmol) were added sequentially to a dry single-necked bottle (50 mL) at room temperature. The reaction solution was reacted at room temperature for 2 hours. The reaction solution was acidified with 1 M dilute hydrochloric acid to pH 7, concentrated, and the precipitated solid was washed with methanol and dried to obtain compound **1** (20 mg, white solid) with a yield of 20%. LCMS (ESI): m/z 326.1[M+H]⁺; RT = 0.92 min (3min). ¹H NMR (400 MHz, DMSO-*d₆*): δ 10.67 (s, 1H), 9.90 (s, 1H), 8.33-8.62 (m, 2H), 8.61 (m, 1H), 7.70-7.69 (m, 2H), 7.28-7.26 (m, 2H), 7.12-7.11 (m, 1H), 4.01 (s, 2H), 3.28 (s, 2H).

### Example 2 Preparation of Compound 2

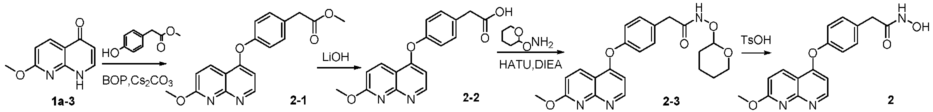

### Step 1: Synthesis of methyl 2-(4-((7-methoxy-1,8-naphthylidin-4-yl)oxy)phenyl)acetate

**1a-3** (0.5 g, 2.84 mmol), 1H-benzotriazol-1-yloxytris(dimethylamino)phosphonium hexafluorophosphate (2.51 g, 5.68 mmol), and anhydrous cesium carbonate (3.7 g, 11.36 mmol) were added sequentially to a single-necked bottle (100 mL) containing 20 mL of anhydrous DMF. After stirring at room temperature for 1 hour, methyl 2-(4-hydroxyphenyl)acetate (940 mg, 5.68 mmol) and anhydrous cesium carbonate (3.7 g, 11.36 mmol) were added. After 6 hours of reaction at 70°C, the reaction solution was cooled to room temperature and filtered. The filtrate was concentrated and purified on a column (EA/PE = 2/1) to obtain compound **2-1** (280 mg, yellow solid) with a yield of 30%. LCMS(ESI): *m*/*z* 325.2 [M+H]⁺; RT = 1.307 min (2.50 min).

### Step 2: Synthesis of 2-(4-((7-methoxy-1,8-naphthylidin-4-yl)oxy)phenyl)acetic acid

**2-1** (280 mg, 0.864 mmol) and lithium hydroxide (42 mg, 1.73 mmol) were added sequentially to a single-necked bottle (50 mL) containing 5 mL of mixed solvent (THF/water = 3/1), and stirred overnight at room temperature. After concentrating the reaction solution, the pH was adjusted to 4 with 1 N hydrochloric acid, the solid was precipitated, filtered, collected to obtain compound **2-2** (200 mg, yellow solid) with a yield of 75%. LCMS(ESI): m/z 311.1 [M+H]⁺; RT = 0.960 min (2.50 min).

### Step 3: Synthesis of 2-(4-((7-methoxy-1,8-naphthylidin-4-yl)oxy)phenyl)-N-((tetrahydro-2H-pyran-2-yl)oxy)acetamide

**2-2** (200 mg, 0.644 mmol), O-(tetrahydro-2H-pyran-2-yl)hydroxylamine (114 mg, 0.966 mmol, and DIEA (250 mg, 1.93 mmol) were added sequentially to a single-necked bottle (100 mL) containing 8 mL of anhydrous DMF, stirred well, and slowly added HATU (490 mg, 1.29 mmol) to stir to react at room temperature for 1 hour. The reaction solution was concentrated, and compound **2-3** (200 mg, yellow solid) was obtained by purifying with prep-TLC (DCM : methanol = 20:1) with a yield of 76%. LCMS(ESI): *m*/*z* 410.2 [M+H]⁺; RT = 1.237 min (2.50 min).

### Step 4: Synthesis of N-hydroxy-2-(4-((7-methoxy-1,8-naphthylidin-4-yl)oxy)phenyl)acetamide

**2-3** (200 mg, 0.49 mmol) and TsOH (252 mg, 1.47 mmol) were added sequentially to a single-necked bottle (50 mL) containing 5 mL of acetonitrile, and stirred to react at room temperature for 2 hours. The reaction solution was concentrated, and the crude product was separated and purified by reverse phase preparation to obtain compound **2** (30 mg, white solid) with a yield of 19%. LCMS(ESI): *m*/*z* 326.0 [M+H]⁺; RT = 3.245 min (6.00 min). ¹H NMR (400 MHz, DMSO-*d₆*): δ 8.69 (d, *J* = 6.4 Hz, 1H), 8.58 (d, *J* = 8.8 Hz, 1H), 7.41 (d, *J* = 8.4 Hz, 2H), 7.24 (d, *J* = 8.8 Hz, 2H), 7.15 (d, *J* = 9.2 Hz, 1H), 6.54 (d, *J* = 5.2 Hz, 1H), 4.04 (s, 3H), 3.35 (s, 2H).

### Example 3 Preparation of Compound 3

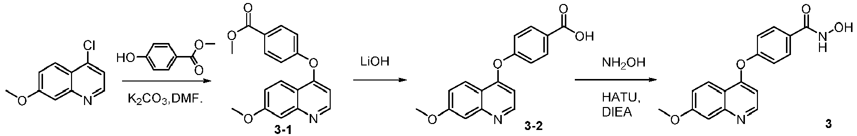

### Step 1: Synthesis of methyl 4-((7-methoxyquinolin-4-yl)oxy)benzoate

4-chloro-7-methoxyquinoline (100 mg, 0.52 mmol), methylparaben (95 mg, 0.62 mmol), potassium carbonate (143 mg, 1.04 mmol), and DMF (5 mL) were added sequentially to a dry single-necked bottle (50 mL) at room temperature, heated to 130°C to react overnight. 10 mL of water was added, the reaction solution was extracted with 30 mL of ethyl acetate, the organic phase was washed with 10 mL of saturated salt water, dried with anhydrous sodium sulfate, filtered, and concentrated under reduced pressure. Purification of residual silica gel column (PE: EA = 3:1) resulted in compound **3-1** (90 mg, light yellow solid) with a yield of 56.00%. LCMS (ESI): *m*/*z* 310.1 [M+H]⁺; RT = 1.81 min (3.00 min).

### Step 2: Synthesis of 4-((7-methoxyquinoline-4-yl)oxy)benzoic acid

**3-1** (90 mg, 0.29 mmol), THF (3 mL), water (1 mL), and lithium hydroxide monohydrate (36 mg, 0.67 mmol) were added sequentially to a dry single-necked bottle (25 mL) at room temperature. The reaction solution was stirred overnight at room temperature. The reaction solution was adjusted to pH 6 with 1 M dilute hydrochloric acid, THF was removed under reduced pressure, the reaction solution was filtered, the filter cake was washed with water, and dried to obtain compound **3-2** (70 mg, light yellow solid) with a yield of 81.39%. LCMS (ESI): *m*/*z* 296.1 [M+H]⁺; RT = 1.39 min (3.00 min).

### Step 3: Synthesis of N-hydroxy-4-((7-methoxyquinolin-4-yl)oxy)benzamide

**3-2** (30 mg, 0.10 mmol), DIEA (39 mg, 0.30 mmol), HATU (57 mg, 0.15 mmol), and DMF (3 mL) were added sequentially to a dry single-necked bottle (25 mL) at room temperature. The reaction solution was stirred at room temperature for 10 minutes. 50% hydroxylamine aqueous solution (7 mg, 0.20 mmol) was added, and the reaction solution was stirred for 2 hours. The residue was concentrated and purified using a high-performance liquid reverse phase preparation column to obtain compound **3** (11 mg, milky white solid) with a yield of 35.48%. LCMS (ESI): *m*/*z* 310.7 [M+H]⁺; RT = 3.199 min (15.00 min). ¹H NMR (400 MHz, DMSO-*d₆):* 11.27 (s, 1H), 9.08 (s, 1H), 8.67-8.66 (m, 1H), 8.16-8.14 (m, 1H), 7.90-6.88 (m, 2H), 7.43 (s, 1H), 7.34-7.28 (m, 3H), 7.61-7.60 (m, 1H), 3.94 (s, 3H).

### Example 4 Preparation of Compound 4

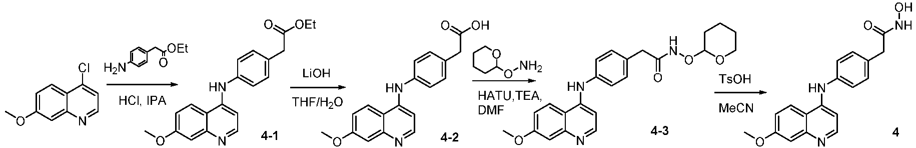

### Step 1: Synthesis of ethyl 2-(4-(((7-methoxyquinolin-4-yl)amino)phenyl)acetate

4-chloro-7-methoxyquinoline (1.50 g, 10.33 mmol) was added to a single-necked bottle (100 mL) containing IPA (10 mL), then ethyl 2-(4-aminophenyl)acetate (1.39 mg, 10.33 mmol) and concentrated hydrochloric acid (1.95 mL, 30.99 mmol) were added, and stirred under 90°C nitrogen protection for 1.5 hours. After cooling, sodium bicarbonate was added at room temperature to adjust the pH to 9-10, the reaction solution was then beaten with petroleum ether-dichloromethane (10:1, 100 mL), filtered, and the filter cake was washed with petroleum ether to obtain crude **4-1** (2.0 g, yellow solid) with a yield of 58%. LCMS (ESI): m/z 337.0 [M+H]⁺; RT = 1.120 min (2.50 min).

### Step 2: Synthesis of 2-(4-((7-methoxyquinoline-4-yl)amino)phenyl)acetic acid

**4-1** (700 mg, 2.08 mmol) and lithium hydroxide (98 mg, 4.14 mmol) were added sequentially at room temperature to a single-necked bottle (100 mL) containing a mixed solution of THF (12 mL) and water (4 mL), and reacted at room temperature for 16 hours. The solvent was removed and 1mol/L hydrochloric acid aqueous solution was added to adjust pH to 3-4. The aqueous phase was freeze-dried to obtain crude product **4-2** (700 mg, white solid). LCMS (ESI): *m*/*z* 309.1[M+H]⁺; RT = 0.762 min (2.50 min).

### Step 3: Synthesis of 2-(4-((7-methoxyquinolin-4-yl)amino)phenyl)-N-((tetrahydro-2H-pyran-2-yl)oxy)acetamide

**4-2** (200 mg, 0.65 mmol) was added to a single-necked bottle (100 mL) containing DMF (5 mL), and then O-(tetrahydro-2H-pyran-2-yl)hydroxylamine (92 mg, 0.78 mmol), TEA (132 mg, 1.30 mmol), and HATU (370 mg, 0.97 mmol) were added in batches. The reaction solution was stirred at room temperature for 3 hours. Ethyl acetate and water were added for extraction at room temperature. The organic phase was washed with sodium chloride, dried with anhydrous sodium sulfate, and filtered. Compound **4-3** (200 mg, white solid) was purified using prep-TCL (DCM : methanol = 20:1) with a yield of 76%. LCMS (ESI): m/z 408.2[M+H]⁺; RT = 1.137 min (2.50 min).

### Step 4: Synthesis of N-hydroxy-2-(4-(((7-methoxyquinolin-4-yl)amino)phenyl)acetamide

**4-3** (100 mg, 0.25 mmol) and TsOH (94 mg, 0.49 mmol) were added sequentially to a single-necked bottle (50 mL) containing acetonitrile (10 mL) at room temperature, and reacted at room temperature for 3 hours. The reaction solution was purified using prep-HPLC (formic acid system) to obtain compound **4** (43 mg, white solid) with a yield of 53%. LCMS (ESI): m/z 324.0[M+H]⁺; RT = 3.155 min (6.00 min). ¹H NMR (400 MHz, MeOD-*d₄*): δ 8.43 (d, *J* = 9.2 Hz, 1H), 8.24 (d, *J* = 7.2 Hz, 1H), 7.41-7.39 (m, 3H), 7.24-7.23 (m, 3H), 6.76 (d, *J* = 7.2 Hz, 1H), 4.02 (s, 3H), 3.50 (s, 2H).

**Examples 5-17** Compound **5-17** was synthesized using the methods described in Examples 1-4, respectively. The structural formulas of the compounds in each example are shown in the previous table.

| Compound | LCMS (ESI): [M+H]⁺ | ¹H NMR (400 MHz) |
|---|---|---|
| 5 | 350.1 | (DMSO-*d₆*): δ 10.66 (s, 1H), 9.07 (s, 1H), 8.85 (s, 1H), 7.76-7.63 (m, 1H), 7.57-7.55 (m, 1H), 7.46-7.44 (m, 1H), 7.29-7.27 (m, 2H), 7.06-7.04 (m, 2H), 4.01 (s, 3H), 3.26 (s, 2H). |
| 6 | 335.7 | (DMSO-*d₆*): δ 11.23 (s, 1H), 9.12 (s, 1H), 9.04 (s, 1H), 7.81-7.79 (m, 2H), 7.68-7.64 (m, 1H), 7.55-7.53 (m, 1H), 7.48-7.46 (m, 1H), 7.19-7.17 (m, 2H), 4.02 (s, 3H). |
| 7 | 324.7 | (DMSO-*d₆*): δ 10.70 (s, 1H), 8.87 (s, 1H), 8.61-8.60 (m, 1H), 8.20-8.18 (m, 1H), 7.41-7.39 (m, 3H), 7.30-7.27 (m, 1H), 7.22-7.20 (m, 2H), 6.45-6.44 (m, 1H), 3.94 (s, 3H), 3.32 (s, 2H). |
| 8 | 325.8 | (DMSO-*d₆*): δ 10.72 (s, 1H), 8.86 (s, 1H), 8.63 (s, 1H), 8.28-8.25 (m, 1H), 7.38-7.35 (m, 4H), 7.25-7.23 (m, 2H), 3.97 (s, 3H), 3.34 (s, 2H). |
| 9 | 325.7 | (DMSO-*d₆*): δ 10.70 (s, 1H), 8.87 (s, 1H), 8.66 (s, 1H), 7.89-7.88 (m, 1H), 7.71-7.68 (m, 1H), 7.51-7.49 (m, 1H), 7.38-7.25 (m, 4H), 3.99 (s, 3H), 3.34 (s, 2H). |
| 10 | 325.1 | (DMSO-*d₆*): δ 11.29 (s, 1H), 10.79 (s, 1H), 8.82 (s, 1H), 8.64 (d, *J* = 9.2 Hz, 1H), 7.59 (d, *J* = 8.4 Hz, 2H), 7.52-7.49 (m, 1H), 7.37 (d, *J* = 8.4 Hz, 2H), 7.27 (s, 1H), 7.13 (d, *J* = 8.0 Hz, 1H), 3.98 (s, 3H), 3.36 (s, 2H). |
| 11 | 356.0 | (MeOD-*d₄*): δ 8.93 (s, 1H), 7.81 (s, 1H), 7.49-7.47 (m, 3H), 7.41-7.31 (m, 2H), 4.09 (s, 3H), 4.01 (s, 3H), 3.49 (s, 2H). |
| 12 | 298.0 | (MeOD-*d₄*): δ 8.20 (s, 1H), 7.69 (d, *J* = 8.0 Hz, 2H), 7.52-7.48 (m, 1H), 7.44-7.40 (m, 1H), 7.21 (d, *J* = 7.6 Hz, 2H), 3.89 (s, 3H), 3.51 (s, 2H). |
| 13 | 343.0 | (MeOD-*d₄*): δ 8.77 (d, *J* = 6.6 Hz, 1H), 8.27 (d, *J* = 10.9 Hz, 1H), 7.61 (d, *J* = 8.0 Hz, 1H), 7.54 (d, *J* = 8.6 Hz, 2H), 7.34 - 7.27 (m, 2H), 6.92 (d, *J =* 6.6 Hz, 1H), 4.16 (s, 3H), 3.52 (s, 2H). |
| 14 | 350.0 | (DMSO-*d₆*): δ 9.06 (s, 1H), 7.95 (d, *J* = 9.2 Hz, 1H), 7.56 (s, 1H), 7.38-7.35 (m, 1H), 7.28 (d, *J* = 8.8 Hz, 2H), 7.04 (d, *J* = 8.8 Hz, 2H), 3.98 (s, 3H), 3.28 (s, 2H). |
| 15 | 299.0 | (DMSO-*d₆*): δ 10.03 (s, 1H), 8.83 (s, 1H), 8.41 (s, 1H), 7.76-7.74 (m, 2H), 7.28 (d, *J* = 8.4 Hz, 2H), 3.95 (s, 3H), 3.29 (s, 2H). |
| 16 | 295.0 | (MeOD-*d₄*): δ 8.76 (s, 1H), 8.63 (d, *J* = 8.4 Hz, 1H), 8.12-8.11 (m, 1H), 7.89-7.85 (m, 2H), 7.69 (d, *J* = 7.6 Hz, 2H), 7.44 (d, *J* = 8.0 Hz, 2H), 3.48 (s, 2H). |
| 17 | 325.0 | (MeOD-*d₄*): δ 8.76 (d, *J* = 9.2 Hz, 1H), 8.23 (d, *J* = 7.2 Hz, 1H), 7.50 (d, *J* = 8.0 Hz, 2H), 7.40 (d, *J* = 8.8 Hz, 2H), 7.21 (d, *J* = 8.8 Hz, 1H), 6.84 (d, *J* = 7.6 Hz, 1H), 4.15 (s, 3H), 3.50 (s, 2H). |

### Example 18 Preparation of Compound 18

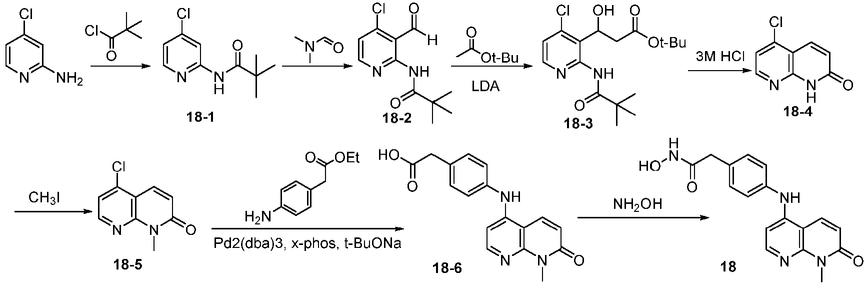

### Step 1: Synthesis of N-(4-chloropyridin-2-yl)neopentanamide

4-chloropyridin-2-amine (12.8 g, 100 mmol), triethylamine (13.1 g, 130 mmol), pyridine (15 mL), and dichloromethane (15 mL) were added sequentially to a dry single-necked bottle (100 mL) at room temperature. Pivaloyl chloride (13.2 g, 110 mmol) was added dropwise under ice bath conditions, and stirred overnight. The reaction solution was concentrated under reduced pressure. Purification of residual silica gel column (PE/EA = 10/1) resulted in compound **18-1** (18.5 g, pink solid) with a yield of 87.00%. ¹H NMR(400 MHz, CDCl₃): 8.36-8.35 (m, 1H), 8.16-8.14 (m, 1H), 8.10 (s, 1H), 7.05-7.03 (m, 1H), 1.30 (s, 9H).

### Step 2: Synthesis of N-(4-chloro-3-formylpyridin-2-yl)neopentanamide

**18-1** (11.4 g, 53.8 mmol) and THF (70 mL) were added sequentially at room temperature to a dry three-necked bottle (250 mL). Under nitrogen protection, n-butyl lithium (54 mL, 2.5 M THF) was added dropwise at -78°C, and the reaction solution was stirred at -78°C for half an hour. THF (30 mL) solution containing DMF (4.0 g, 54.0 mmol) was added to the above reaction solution to continue stirring for 1 hour at -78°C. The reaction solution was heated up to room temperature, and saturated ammonium chloride solution (200 mL) was added to react at room temperature for 30 minutes. The reaction solution was extracted with 100 mL of ethyl acetate, the organic phase was washed with 50 mL of saturated salt water, dried with anhydrous sodium sulfate, filtered, and concentrated under reduced pressure. Purification of residual silica gel column (PE/EA = 3/1) resulted in compound **18-2** (4 g, yellow oily substance) with a yield of 31.00%. LCMS (ESI): *m*/*z* 240.8 [M+H]⁺; RT = 1.768 min (3.00 min).

### Step 3: Synthesis of tert-butyl 3- (4-chloro-2-neopentanamideaminopyridin-3-yl) -3-hydroxypropanoate

**18-2** (2.28 g, 9.5 mmol) and THF (40 mL) were added sequentially at room temperature to a dry three-necked bottle (250 mL). Under nitrogen protection, LDA (10.5 mL, 2 M THF) was added dropwise at -78°C, and the reaction solution was stirred at -78°C for half an hour. THF (20 mL) solution of tert-butyl acetate (1.1 g, 9.5 mmol) was added to the above reaction solution to continue stirring for 1 hour at -78°C. The reaction solution was heated up to room temperature, 40 mL of water was added, extracted with 50 mL of ethyl acetate, the organic phase was washed with saturated salt water (20 mL), dried with anhydrous sodium sulfate, filtered, and concentrated under reduced pressure. Purification of residual silica gel column (PE/EA = 2/1) resulted in compound **18-3** (2.2 g, yellow solid) with a yield of 65.00%, LCMS (ESI): m/z 356.7 [M+H]⁺; RT = 1.871 min (3.00 min).

### Step 4: Synthesis of 5-chloro-1,8-naphthylidin-2(1H)-one

**18-3** (1.9 g, 5.34 mmol) and 3 M hydrochloric acid (20 mL) were added sequentially at room temperature to a dry single-necked bottle (50 mL), and reacted at 100°C for 8 hours. The reaction solution was cooled and filtered, washed sequentially with saturated sodium bicarbonate solution (5 mL) and water (5 mL), and dried to obtain compound **18-4** (440 mg, white solid) with a yield of 46.00%. LCMS (ESI): *m*/*z* 181.0 [M+H]⁺; RT = 1.20 min (3.00 min).

### Step 5: Synthesis of 5-chloro-1-methyl-1,8-naphthyridin-2(1H)-one

**18-4** (150 mg, 0.84 mmol), iodomethane (153 mg, 1.08 mmol), potassium carbonate (231 mg, 1.58 mmol), and DMF (3 mL) were added sequentially to a dry single-necked bottle (25 mL) at room temperature, and stirred overnight at room temperature. 10 mL of water was added, the reaction solution was extracted with 20 mL of ethyl acetate, the organic phase was washed with 20 mL of saturated salt water, dried with anhydrous sodium sulfate, filtered, and concentrated under reduced pressure. Purification of residual silica gel column (PE:EA = 2:1) resulted in compound **18-5** (150 mg, white solid) with a yield of 92.60%. LCMS (ESI): *m*/*z* 194.8 [M+H]⁺; RT = 1.621 min (3.00 min).

### Step 6: Synthesis of 2-(4-((8-methyl-7-oxy-7,8-dihydro-1,8-naphthyridin-4-yl)amino)phenyl)acetic acid

**18-5** (110 mg, 0.57 mmol), 4-aminophenylethyl acetate (101 mg, 0.57 mmol), Pd₂(dba)₃ (52 mg, 0.057 mmol), x-PHOS (54 mg, 0.11 mmol), sodium tertbutanol (109 mg, 1.14 mmol), and dioxane (5 mL) were added sequentially to a dry single-necked bottle (50 mL) at room temperature. The reaction solution was heated under nitrogen protection to 100°C and stirred overnight. The reaction solution was concentrated under reduced pressure. Purification of residual silica gel column (DCM: MeOH = 40:1) resulted in compound **18-6** (70 mg, yellow solid) with a yield of 40.00%. LCMS (ESI): *m*/*z* 309.7 [M+H]⁺; RT = 1.433 min (3.00 min).

### Step 7: Synthesis of N-hydroxy-2-(4-((8-methyl-7-oxy-7,8-dihydro-1,8-naphthyridin-4-yl)amino)phenyl)acetamide

At room temperature, **18-6** (70 mg, 0.23 mmol), DIEA (89 mg, 0.69 mmol), HATU (131 mg, 0.34 mmol), and DMF (3 mL) were added sequentially to a dry single-necked bottle (25 mL). The reaction solution was stirred at room temperature for 10 minutes. 50% hydroxylamine aqueous solution (30 mg, 0.45 mmol) was added and stirred for 1 hour. The reaction solution was concentrated and the residue was purified using a high-performance liquid reverse phase preparation column (0.1% formic acid/acetonitrile/water gradient elution) to obtain compound **18** (15 mg, yellow solid) with a yield of 20.00%. LCMS (ESI): *m*/*z* 325.0 [M+H]⁺; RT = 3.16 min (15.00 min). ¹HNMR (400 MHz, DMSO-*d₆*): 10.66 (s, 1H), 9.04 (s, 1H), 8.84 (s, 1H), 8.34-8.32 (m, 1H), 8.17-8.16 (m, 1H), 7.31-7.22 (m, 4H), 6.69-6.68 (m, 1H), 6.59-6.57 (m, 1H), 3.63 (s, 3H), 3.29 (s, 2H).

### Example 19 Preparation of Compound 141

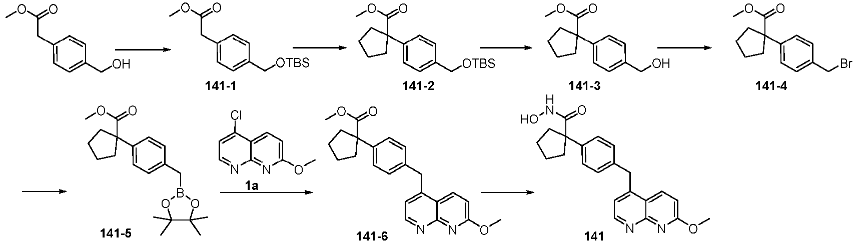

### Step 1: Synthesis of methyl 2-(4-(((tert-butyldimethylsilyl)oxy)methyl)phenyl)acetate

Methyl 2-(4-(hydroxymethyl)phenyl)acetate (4 g, 22.22 mmol) and imidazole (3.02g, 44.44 mmol) were dissolved in DMF (25 mL), nitrogen gas, and DMF (10 mL) solution of TBSCl (4.03 g, 26.67 mmol) was slowly added dropwise under ice bath. After adding, the reaction solution was stirred at room temperature for 2 hours. The reaction solution was diluted with water, extracted with ethyl acetate, the organic phase was washed with water, dilute hydrochloric acid (1 M) aqueous solution, and saturated sodium chloride aqueous solution. Compound **141-1** (5.3 g, 18.03 mmol) was obtained by drying with anhydrous sodium sulfate, concentrating, and column chromatography purification (EA:PE = 1:20). It was a colorless oily substance with a yield of 81%. ¹H NMR (400 MHz, DMSO-*d₆*) δ 7.20-7.12 (m, 4H), 4.61 (s, 2H), 3.58 (s, 2H), 3.53 (s, 3H), 0.86-0.78 (m, 9H), 0.03 -0.05 (m, 6H).

### Step 2: Synthesis of methyl 1-(4-(((tert-butyldimethylsilyl)oxy)methyl)phenyl)cyclopentane-1-carboxylate

Compound **141-1** (2 g, 6.80 mmol) was dissolved in DMF (20 mL), NaH (680 mg, 17 mmol) was added under an ice salt bath, 1,4-dibromobutane (1.47 g, 6.8 mmol) was added dropwise, and stirred at room temperature for 3 hours. Water was slowly added dropwise to the reaction solution to quench, then extracted with ethyl acetate, the organic phase was concentrated, and purified by column chromatography (EA:PE = 1:20) to obtain compound **141-2** (1.1 g, 3.16 mmol), a colorless oily substance with a yield of 46%. ¹H NMR (600 MHz, DMSO-*d₆*) δ 7.23-7.16 (m, 4H), 4.60 (s, 2H), 3.46 (s, 3H), 2.47-2.43 (m, 2H), 1.77 (ddd, *J =* 12.4, 8.2, 6.0 Hz, 2H), 1.64-1.50 (m, 4H), 0.84-0.81 (m, 9H), 0.02-0.01 (m, 6H).

### Step 3: Synthesis of methyl 1-(4-(hydroxymethyl)phenyl)cyclopentane-1-carboxylate

Compound **141-2** (500 mg, 1.436 mmol) was dissolved in methanol (10 mL), HCl/MeOH (2 mL, 4 M) was added at 0°C, stirred at 0°C for 1 hour, concentrated under reduced pressure to remove solvent and excess hydrochloric acid, and vacuum dried to obtain compound **141-3** (336 mg, 1.436 mmol) with a yield of 100%. LCMS (ESI): *m*/*z* 235.1[M+H]⁺; RT = 1.54 min (3.0 min)

### Step 4: Synthesis of methyl 1-(4-(bromomethyl)phenyl)cyclopentane-1-carboxylate

Compound **141-3** (500 mg, 2.14 mmol) was dissolved in dichloromethane (15 mL), PBr₃ (577 mg, 2.14 mmol) was slowly added dropwise at 0°C, and stirred in an ice bath for 2 hours. Water was added to the reaction solution to quench, the aqueous phase was extracted with dichloromethane, and the organic phase was concentrated, and purified by column chromatography (EA:PE = 1:20) to obtain compound **141-4** (530 mg, 1.78 mmol), a colorless oily substance with a yield of 83%. ¹H NMR (400 MHz, DMSO) δ 7.44-7.37 (m, 2H), 7.36 - 7.28 (m, 2H), 4.69 (s, 2H), 3.55 (s, 3H), 2.58 - 2.51 (m, 2H), 1.86 (m, 2H), 1.74-1.53 (m, 4H).

### Step 5: Synthesis of methyl 1-(4-((4,4,5,5-tetramethyl-1,3,2-dioxaborolan-2-yl)methyl)phenyl)cyclopentane-1-carboxylate

**141-4** (500 mg, 1.69 mmol), bis(pinacolato)diboron (858 mg, 3.38 mmol), tetrakis(triphenylphosphine)palladium (195 mg, 0.169 mmol), potassium carbonate (700 mg, 5.07 mmol), and 25 mL of dioxane were added to a 50 mL single-necked bottle, and heated under argon protection to 100°C and stirred for 4 hours. The reaction solution was cooled to room temperature, diluted with water, extracted with ethyl acetate, the organic phase was concentrated, and purified by column chromatography (MeOH: DCM = 1:10) to obtain compound **141-5** (350 mg, 1.017 mmol), a white solid with a yield of 60%. ¹H NMR (400 MHz, DMSO-*d₆*) δ 7.23-7.17 (m, 2H), 7.09 (m, 2H), 3.58 (s, 3H), 2.54-2.50 (m, 2H), 2.20 (s, 2H), 1.86 (m, 2H), 1.74-1.62 (m, 4H), 1.22 (s, 12H).

### Step 6: Synthesis of methyl 1-(4-((7-methoxy-1,8-naphthyridin-4-yl)methyl)phenyl)cyclopentane-1-carboxylate

Compound **141-5** (480 mg, 1.395 mmol), **1a** (271 mg, 1.395 mmol), PdCl₂dppf (102 mg, 0.140 mmol), potassium carbonate (578 mg, 4.185 mmol), and dioxane/water (15 mL/5 mL) were added to a 50 mL single-necked bottle, and heated under argon protection to 100°C and stirred for 8 hours. The reaction solution was cooled to room temperature, diluted with water, extracted with ethyl acetate, the organic phase was concentrated, and purified by column chromatography (EA:PE = 1:2) to obtain compound **141-6** (80 mg, 0.212 mmol), a yellow oily substance with a yield of 15.2%. LCMS (ESI): *m*/*z* 377.1 [M+H]⁺; RT = 1.85 min (3.0 min).

### Step 7: Synthesis of N-hydroxy-1-(4-((7-methoxy-1,8-naphthylidin-4-yl)methyl)phenyl)cyclopentane-1-formamide

**141-6** (80 mg, 0.265 mmol) was added to a single-necked bottle (10 mL), methanol/THF (1 mL/1 mL) was added, stirred, 50% hydroxylamine aqueous solution (0.5 mL) and sodium hydroxide (21 mg, 0.53 mmol) were added sequentially, reacted at room temperature for 8 hours, the pH was adjusted to 7-8 with 1 M dilute hydrochloric acid, and the reaction solution was purified on a reverse phase column (acetonitrile: water (1 %o HCOOH) = 35:65) to obtain compound **141** (30 mg, 0.0796 mmol), a white solid with a yield of 30%. LCMS (ESI): *m*/*z* 378.2 [M+H]⁺; RT = 1.39 min (3.0 min). ¹H NMR (400 MHz, DMSO-*d₆*) δ 10.38 (s, 1H), 8.82 (d, *J* = 4.6 Hz, 1H), 8.62 - 8.51 (m, 2H), 8.14 (s, 1H), 7.28 (m, 3H), 7.18 (m, 2H), 7.12 (d, *J* = 9.0 Hz, 1H), 4.41 (s, 2H), 4.01 (s, 3H), 2.46 (s, 2H), 1.73 (m, 2H), 1.57 (m, 4H).

### Example 20 Preparation of Compound 194

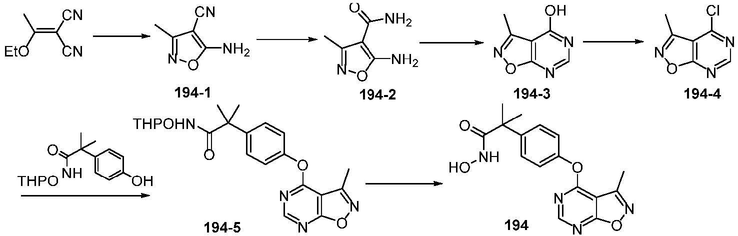

### Step 1: Synthesis of 5-amino-3-methylisoxazol-4-acetonitrile

Hydroxylamine hydrochloride (2.55 g, 36.72 mmol) was dissolved in 25 mL of sodium hydroxide solution (1.50 M), 50 mL of ethanol was added, and 2-(1-ethoxyethylidene)acetonitrile (5.00 g, 36.72 mmol) was slowly added while stirring. The reaction solution was heated to 50°C to react for 30 minutes, then reacted overnight at room temperature. The solvent was removed, the reaction solution was filtered to collect the solid, which was washed with clean water, and dried to obtain compound **194-1** (3.75 g, white solid) with a yield of 82.85%; LCMS(ESI): *m*/*z* 124.3 [M+H]⁺; RT=0.897 min (2.50 min). ¹H NMR(400 MHz, DMSO-*d₆*): δ 8.33 (s, 2H), 2.13 (s, 3H).

### Step 2: Synthesis of 5-amino-3-methylisoxazol-4-formamide

Compound **194-1** (3.6 g, 29.24 mmol) was dissolved in 100 mL of water and 100 mL of ethanol, while sodium hydroxide (9.36 g, 233.93 mmol) was slowly added to react at 100°C for 3 hours. Ethanol was removed, the reaction solution was diluted with water to adjust its pH to 8, extracted with ethyl acetate, the organic phase was collected, dried, and filtered to obtain compound **194-2** (1.07 g, white solid) with a yield of 25.90%; LCMS(ESI): *m*/*z* 142.3 [M+H]⁺; RT=0.557 min (2.50 min).

### Step 3: Synthesis of 3-methylisoxazol[5,4-d] pyrimidin-4-ol

Compound **194-2** (1.00 g, 7.09 mmol) and triethyl orthoformate (2.10 g, 14.17 mmol) were dissolved in 20 mL of acetic anhydride and heated at 120°C for 3 hours. After cooling to room temperature, ice water was added to dilute the reaction solution, pH was adjusted to 8 with NaOH (2M), the reaction solution was extracted with ethyl acetate, the organic phase was washed with saturated salt water, dried with anhydrous sodium sulfate, concentrated, and purified to obtain compound **194-3** (260 mg, white solid) with a yield of 24.30%; LCMS(ESI): m/z 152.2 [M+H]⁺; RT=0.827 min (2.50 min). ¹H NMR(400 MHz, CDCl₃): δ 11.38 (s, 1H), 8.17 (s, 1H), 2.63 (s, 3H).

### Step 4: Synthesis of 4-chloro-3-methylisoxazolo[5,4-d]pyrimidine

Compound **194-3** (260 mg, 1.72 mmol) was dissolved in 10 mL of phosphorus oxychloride and stirred at 90°C for 2 hours. After cooling to room temperature, solvent was removed, ice water was added to dilute the reaction solution, and pH was adjusted to 8 with 2 M sodium hydroxide solution. The reaction solution was extracted with ethyl acetate. The organic phase was collected, washed with saturated salt water, dried with anhydrous sodium sulfate, filtered, concentrated, and purified with prep-TLC (PE: EA = 3:1) to obtain compound **194-4** (227 mg, brown solid) with a yield of 77.90%; LCMS(ESI): *m*/*z* 170.2 [M+H]⁺; RT = 1.337min (2.50 min).

### Step 5: Synthesis of 2-methyl-2-(4-((3-methylisoxazol[5,4-d]pyrimidin-4-yl)oxy)phenyl)-N-((tetrahydro-2H-pyran-2-yl)oxy)propionamide

Compound **194-4** (50 mg, 0.29 mmol), 2-(4-hydroxyphenyl)-2-methyl-N-((tetrahydro-2H-pyran-2-yl)oxy)propionamide (99 mg, 0.35 mmol), and cesium carbonate (192 mg, 0.59 mmol) were dissolved in 3 mL of DMF to react at 80°C for 2 hours. After cooling to room temperature, the reaction solution was extracted with ethyl acetate. The organic phase was collected, washed with saturated salt water, dried with anhydrous sodium sulfate, filtered, concentrated, and purified with prep-TLC (PE : EA = 1:1) to obtain compound **194-5** (92 mg, transparent oil) with a yield of 75.65%; LCMS(ESI): m/z 411.1 [M-H]⁻; RT=1.571min (2.50 min).

### Step 6: Synthesis of N-hydroxy-2-methyl-2-(4-((3-methylisoxazol[5,4-d]pyrimidin-4-yl)oxy)phenyl)propanamide

Compound **194-5** (65 mg, 0.16 mmol) and TFA (1 mL) were dissolved in 3 mL of anhydrous dichloromethane and stirred at room temperature for 2 hours. The reaction solution was extracted with ethyl acetate. The organic phase was collected, washed with saturated salt water, dried with anhydrous sodium sulfate, filtered, concentrated, and purified with pre-HPLC (FA) to obtain compound **194** with a yield of 30.92%; LCMS(ESI): m/z 328.95 [M+H]⁺; RT=3.933 min (6 min). ¹H NMR(400 MHz, DMSO-*d₆* ): δ 10.42 (s, 1H), 8.71 (s, 1H), 8.70 (s, 1H), 7.44 (d, *J* =8.4 Hz, 2H), 7.28 (d, *J* =8.8 Hz, 2H), 2.68 (s, 3H), 1.49 (s, 6H).

### Example 21 Preparation of Compound 195

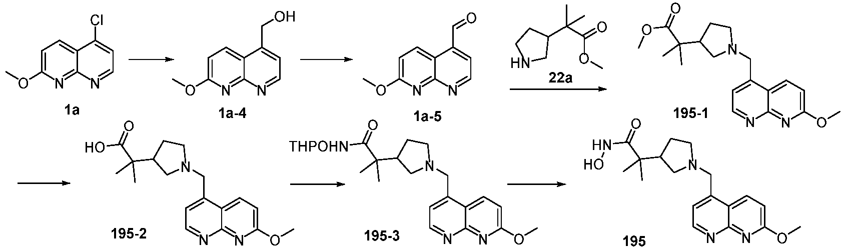

### Step 1: (7-methoxy-1,8-naphthyridin-4-yl) methanol

Compound **1a** (808 mg, 4.2 mmol), potassium fluoride (732 mg, 12.6 mmol), and (tributyl)methanol (1.6 g, 5.0 mmol) were added sequentially to a three-necked bottle (50 mL) containing 10 mL of dioxane at room temperature. Then, PdCl₂dppf (307 mg, 0.42 mmol) was added and reacted overnight at 100°C. After filtration, the filtrate was concentrated and purified by column chromatography (EA:PE = 1:2) to obtain compound **1a-4** (200 mg, yellow solid), LCMS (ESI): m/z 191.1 [M+H]⁺; RT = 0.307 min (2.50 min).

### Step 2: 7-methoxy-1,8-naphthyridin-4-formaldehyde

Compound **1a-4** (90 mg, 0.26 mmol) and Dess-Martin periodinane (535 mg, 1.26 mmol) were added sequentially to a single-necked bottle (50 mL) containing 7 mL of dichloromethane at room temperature. The reaction was carried out at room temperature for 3 hours. The reaction solution was diluted with water, extracted with dichloromethane. The organic phase was washed with water and saturated saline water, dried with anhydrous sodium sulfate, filtered, and the filtrate was concentrated to obtain compound **1a-5** (180 mg, white solid). Yield: 90.9%, ¹H NMR (400 MHz, DMSO-*d₆*): δ 10.41 (s, 1H), 9.25-9.23 (m, 2H), 7.73 (d, *J* = 4.0 Hz, 1H), 7.17 (d, *J* = 9.2 Hz, 1H), 4.19 (s, 3H).

### Step 3: methyl 2-(1-((7-methoxy-1,8-naphthyridin-4-yl) methyl)pyrrolidin-3-yl)-2-methylpropanoate

Compound **1a-5** (60 mg, 0.33 mmol), compound **22a** (82 mg, 0.48 mmol), and sodium cyanide borohydride (42 mg, 0.66 mmol) were added sequentially to a single-necked bottle (50 mL) containing 3 mL of 1,2-dichloroethane. The reaction solution was reacted at room temperature for 2 hours, diluted with water, extracted with dichloromethane. The organic phase was washed with water and saturated salt water, dried with anhydrous sodium sulfate, filtered, and purified by concentrated column chromatography (PE: EA = 4:1) to obtain compound **195-1** (105 mg, transparent oil), LCMS (ESI): *m*/*z* 344.1 [M+H]⁺; RT = 0.880 min (2.50 min).

### Step 4: 2-(1-((7-methoxy-1,8-naphthylidin-4-yl) methyl)pyrrolidin-3-yl)-2-methylpropionic acid

Compound **195-1** (80 mg, 0.23 mmol) and lithium hydroxide (11 mg, 0.47 mmol) were added sequentially to a single ended bottle (50 mL) containing 1,4-dioxane/water (3:1, 3mL) at room temperature. Reaction was carried out at 80°C for 10 hours, the pH was adjusted to 5 with hydrochloric acid aqueous solution (1 M), the reaction solution was diluted with water, extracted with ethyl acetate, the organic phase was washed sequentially with water and saturated salt water, dried with anhydrous sodium sulfate, filtered, and the filtrate was concentrated to obtain crude product **195-2** (50 mg, white solid), LCMS(ESI): m/z 320.2 [M+H]⁺; RT=0.807 min (2.50 min).

### Step 5: 2-(1-((7-methoxy-1,8-naphthylidin-4-yl)methyl)pyrrolidin-3-yl)-2-methyl-N-((tetrahydro-2H-pyran-2-yl)oxy)propionamide

Compound **195-2** (60 mg, 0.18 mmol), O-(tetrahydro-2H-pyran-2-yl)hydroxylamine (26 mg, 0.22 mmol), diisopropylethylamine (46 mg, 0.36 mmol), and HATU (102 mg, 0.27 mmol) were added to a single-necked flask containing 15 mL of DMF, stirred at room temperature for 3 hours, the reaction solution was poured into water, extracted with ethyl acetate, and the organic phase was washed with water and saturated salt water, dried with anhydrous sodium sulfate, concentrated, and purified by chromatography plate (EA) to obtain compound **195-3** (30 mg, white solid) with a yield of 38.4%; LCMS(ESI): m/z 429.2 [M+H]⁺; RT = 0.360 min (2.50 min).

### Step 6: N-hydroxy-2-(1-((7-methoxy-1,8-naphthyridin-4-yl)methyl)pyrrolidin-3-yl)-2-methylpropionamide

2 mL of dichloromethane, **195-3** (40 mg, 0.09 mmol), and TFA (0.6 mL) were added to a single-necked flask and stirred at room temperature for 3 hours. After removing the solvent, the crude product was purified by reverse phase column (formic acid) to obtain compound **195** (8.5 mg, white solid) with a yield of: 26.4%; LCMS(ESI): m/z 345.05 [M+H]⁺; RT = 1.978 min (6.00 min). ¹H NMR (400 MHz, DMSO-*d₆*): δ 10.41 (s, 1H), 8.91 (s, 1H), 8.69-8.61 (m, 2H), 7.53 (s, 1H), 7.19 (d, *J* = 7.6 Hz, 1H), 4.04 (s, 3H), 3.34 (s, 2H), 2.51 (s, 4H), 1.79-1.63 (m, 3H), 1.03 (d, *J* = 6.0 Hz, 6H).

### Example 22 Preparation of Compound 196

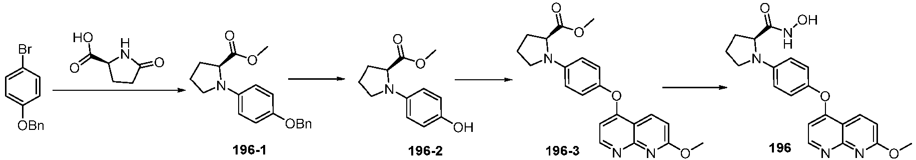

### Step 1: Synthesis of (4-(benzyloxy)phenyl)-L-proline methyl ester

1-(benzyloxy)-4-bromobenzene (500 mg, 1.9 mmol), (S)-5-oxopyrrolidin-2-carboxylic acid (549 mg, 4.8 mmol), cuprous iodide (72 mg, 0.38 mmol), potassium phosphate (1.6 g, 7.6 mmol), and dimethyl sulfoxide (10 mL) were added sequentially to a dry 50 mL single-necked flask, heated under nitrogen protection to 100°C overnight, DMF (10 mL) and iodomethane (1.3 g, 9.5 mmol) were added, and heated to 60°C for 2 hours. Water was added to the reaction solution, which was extracted with ethyl acetate, washed with salt water, dried with anhydrous sodium sulfate, concentrated under reduced pressure, and the residue was purified on a silica gel column (PE: EA = 1:10) to obtain compound **196-1** (350 mg, yellow solid) with a yield of 59.0%. LCMS (ESI): m/z 312.1 [M+H]⁺; RT = 1.94 min (3.00 min).

### Step 2: Synthesis of (4-hydroxyphenyl)-L-proline methyl ester

**196-1** (350 mg, 1.1 mmol), 10% palladium carbon (35 mg), and methanol (10 mL) were added sequentially to a dry 50 mL single-necked flask at room temperature, stirred at room temperature for 2 hours under hydrogen protection, and the filtrate was concentrated to obtain compound **196-2** (220 mg, yellow solid) with a yield of 88.0%. LCMS (ESI): m/z 222.1 [M+H]⁺; RT = 1.36 min (3.00 min).

### Step 3: Synthesis of (4-((7-methoxy-1,8-naphthyridin-4-yl)oxy)phenyl)-L-proline methyl ester

**196-2** (220 mg, 1.0 mmol), 5-chloro-2-methoxy-1,8-naphthalidine (194 mg, 1.0 mmol), potassium carbonate (276 mg, 2 mmol), and DMF (5 mL) were added sequentially to a dry 50 mL single-necked flask at room temperature, heated to 100°C and stirred overnight. Water was added to the reaction solution, which was extracted with ethyl acetate, washed with salt water (10 mL), dried with anhydrous sodium sulfate, concentrated under reduced pressure, and the residue was purified on a silica gel column (PE: EA = 1:1) to obtain compound **196-3** (100 mg, yellow oily substance) with a yield of 26.0%. LCMS (ESI): m/z 380.1 [M+H]⁺; RT = 1.66 min (3.00 min).

### Step 4: Synthesis of (S)-N-hydroxy-1-(4-((7-methoxy-1,8-naphthylidin-4-yl)oxy)phenyl)pyrrolidin-2-formamide

**196-3** (100 mg, 0.26 mmol), THF (1.0 mL), methanol (1.0 mL), 50% hydroxylamine aqueous solution (0.5 mL), and sodium hydroxide (21 mg, 0.52 mmol) were added sequentially to a dry 25 mL single-necked flask at room temperature. The reaction solution was stirred at room temperature for 1 hour. The reaction solution was adjusted to pH = 8 with 1 M dilute hydrochloric acid, and the residue was purified using a high-performance liquid reverse phase preparation column (0.1% formic acid). Compound **196** (17 mg, yellow solid) was obtained by drying, with a yield of 17%. LCMS (ESI): m/z 381.1 [M+H]⁺; RT = 4.27 min (15.00 min). ¹H NMR(400 MHz, DMSO-*d₆*): δ 10.70 (s, 1H), 8.70 (s, 1H), 8.68-8.59 (m, 2H), 7.15-7.12 (m, 3H), 6.61-6.59 (m, 2H), 6.45-6.44 (m, 1H), 4.05-3.98 (m, 4H), 3.58-3.55 (m, 1H), 3.23-3.19 (m, 1H), 2.08-2.00 (m, 1H), 1.99-1.96 (m, 3H).

**Examples 23-207:** Compounds **19-140, 142-193 and 197-207** were synthesized using the methods described in Examples 1-4 and 18-22, respectively. The structural formulas of the compounds in each example are shown in the previous table.

| Compound | LCMS (ESI): [M+H]⁺ | ¹H NMR (400 MHz) |
|---|---|---|
| 19 | 326.0 | (DMSO-*d₆*): δ 9.45 (s, 1H), 8.77 (d, *J* = 5.6 Hz, 1H), 7.44 (d, *J* = 8.8 Hz, 2H), 7.30 (d, *J* = 8.4 Hz, 2H), 7.23 (s, 1H), 6.46 (d, *J* = 5.2 Hz, 1H), 4.02 (s, 3H), 3.37 (s, 2H). |
| 20 | 326.0 | (DMSO-*d₆*): δ 8.81-8.78 (m, 2H), 7.81 (s, 1H), 7.43 (d, *J* = 8.4 Hz, 2H), 7.24 (d, *J* = 8.4 Hz, 2H), 6.80 (s, 1H), 4.03 (s, 3H), 3.36 (s, 2H). |
| 21 | 340.0 | (DMSO-*d₆*): δ 10.83 (s, 1H), 8.89 (dd, *J* = 7.9, 6.2 Hz, 2H), 7.49 (d, *J* = 8.0 Hz, 1H), 7.39 (s, 1H), 7.36 - 7.25 (m, 2H), 6.76 (d, *J* = 6.8 Hz, 1H), 4.16 (s, 3H), 3.38 (s, 2H), 2.14 (s, 3H). |
| 22 | 358.6 | (DMSO-*d₆*): δ 10.67 (s, 1H), 10.03 (s, 1H), 8.83 (s, 1H), 8.47-8.44 (m, 1H), 8.61 (s, 1H), 7.65-7.63 (m, 2H), 7.31-7.24 (m, 3H), 7.15 (s, 1H), 3.92 (s, 3H), 3.29 (s, 2H). |
| 23 | 324.1 | (DMSO-*d₆*): δ 10.67 (s, 1H), 9.00 (s, 1H), 8.85 (s, 1H), 8.40-8.38 (m, 1H), 8.20-8.18 (m, 1H), 7.54-7.50 (m, 1H), 7.32-7.28 (m, 4H), 6.77-6.75 (m, 1H), 4.00 (s, 3H), 3.30 (s, 2H). |
| 24 | 309.0 | (MeOD-*d₄*): δ 8.73 (s, 1H), 8.57 (d, *J* = 8.0 Hz, 1H), 8.11-8.08 (m, 1H),7.86-7.83 (m, 2H), 7.52 (d, *J* = 8.8 Hz, 2H), 7.34 (d, *J* = 8.0 Hz, 1H), 3.52 (s, 2H), 2.40 (s, 3H). |
| 25 | 344.0 | (MeOD-*d₄*): δ 8.88 - 8.79 (m, 2H), 7.60 (t, *J* = 8.2 Hz, 1H), 7.41 (d, *J* = 9.0 Hz, 1H), 7.29-7.16 (m, 2H), 7.09 (d, *J* = 6.8 Hz, 1H), 4.23 (s, 3H), 3.58 (s, 2H). |
| 26 | 344.1 | (MeOD-*d₄*): δ 8.83-8.80 (m, 2H), 7.48-7.38 (m, 3H), 7.33 (d, *J* = 8.4 Hz, 1H), 7.02 (d, *J* = 6.8 Hz, 1H), 4.22 (s, 3H), 3.53 (s, 2H). |
| 27 | 340.0 | (MeOD-*d₄*): δ 8.81-8.70 (m, 2H), 7.60 (d, *J* = 6.8 Hz, 2H), 7.37-7.30 (m, 3H), 6.92 (s, 1H), 4.21 (s, 3H), 3.62 (d, *J* = 6.4 Hz, 1H), 1.52 (d, *J* = 7.2 Hz, 3H). |
| 28 | 299.1 | (DMSO-*d₆*): δ 10.77 (s, 1H), 8.89 (s, 1H), 8.38 (s, 1H), 7.70 (s, 1H), 7.42-7.40 (m, 2H), 7.26-7.23 (m, 2H), 6.47-6.46 (m, 1H), 4.03 (s, 3H), 3.36 (s, 2H). |
| 29 | 299.0 | (MeOD-*d₄*): δ 8.44 (s, 1H), 8.08 (s, 1H), 7.57 (d, *J* = 8.4 Hz, 2H), 7.41 (d, *J* = 8.4 Hz, 2H), 4.47 (s, 3H), 3.47 (s, 2H). |
| 30 | 326.0 | (MeOD-*d₄*): δ 8.62 (d, *J* = 5.6 Hz, 1H), 7.66 (d, *J* = 7.6 Hz, 1H), 7.49 (d, *J* = 8.4 Hz, 2H), 7.23 (d, *J* = 8.4 Hz, 2H), 7.28-7.06 (m, 2H), 3.72 (s, 3H), 3.49 (s, 2H). |
| 31 | 312.1 | (DMSO-*d₆*): δ 12.13 (s, 1H), 10.64 (s, 1H), 9.80 (s, 1H), 8.33-8.62 (m,1H), 8.61 (m,1H), 7.70-7.69 (m, 2H), 7.28-7.27 (m, 2H), 7.12-7.11 (m, 1H), 3.27 (s, 2H). |
| 32 | 299.1 | (DMSO-*d₆*): δ 10.69 (s, 1H), 8.87 (s, 1H), 8.39-8.34 (m, 2H), 7.40-7.38 (m, 2H), 7.22-7.20 (m, 2H), 6.21-6.20 (m, 1H), 4.16 (s, 3H), 3.35 (s, 2H). |
| 33 | 313.0 | (MeOD-*d₄*): δ 8.35 (s, 1H), 8.01 (s, 1H), 7.68 (d, *J* = 8.0 Hz, 2H), 7.35 (d, *J* = 8.0 Hz, 2H), 4.45-4.39 (m, 2H), 3.43 (s, 2H), 1.47-1.44 (s, 3H). |
| 34 | 325.0 | (MeOD-*d₄*): δ 8.37 (s, 1H), 7.96 (s, 1H), 7.68 (d, *J* = 8.0 Hz, 2H), 7.34 (d, *J* = 8.0 Hz, 2H), 3.76-3.75 (m, 1H), 3.43 (s, 2H), 1.24-1.22 (m, 2H), 1.17-1.14 (m, 2H). |
| 35 | 296.0 | (DMSO-*d₆*): δ 10.68 (s, 1H), 10.09 (s, 1H), 9.18 (s, 1H), 8.85 (s, 1H), 8.74-8.71 (m, 2H), 8.44 (d, *J* = 6.4 Hz, 1H), 7.77 (d, *J* = 8.4 Hz, 2H), 7.32 (d, *J* = 8.4 Hz, 2H), 3.31 (s, 2H). |
| 36 | 312.7 | (DMSO-*d₆*): δ 10.70 (s, 1H), 8.86 (s, 1H), 8.39-8.38 (m, 1H), 7.71 (s, 1H), 7.47-7.45 (m, 2H), 7.26-7.23 (m, 2H), 6.46-6.44 (m, 1H), 4.03 (s, 3H), 3.55-3.49 (m, 1H), 1.39-1.37 (m, 3H). |
| 37 | 340.1 | (DMSO-*d₆*): δ 10.66 (s, 1H), 9.68 (s, 1H), 8.83 (s, 1H), 8.78 (d, *J* = 9.2Hz, 1H), 7.74 (d, *J* = 8.4Hz, 2H), 7.26 (d, *J* = 8.8Hz, 2H), 7.2 (d, *J* = 8.8Hz, 1H), 3.98 (s, 3H), 3.28 (s, 2H), 2.50-2.49 (m, 3H). |
| 38 | 341.9 | (MeOD-*d₄*): δ 8.83 (t, *J* = 8.0 Hz, 2H), 7.72 (d, *J* = 8.0 Hz, 2H), 7.52-7.32 (m, 3H), 6.98 (d, *J* = 6.8 Hz, 1H), 5.20 (s, 1H), 4.23 (s, 3H). |
| 39 | 295.9 | (MeOD-*d₄*): δ 9.13-9.11 (m, 1H), 9.03 (d, *J* = 7.2 Hz, 1H), 8.85 (s, 1H), 7.90-7.86 (m, 1H), 7.71 (d, *J* = 8.4 Hz, 2H), 7.45 (d, *J* = 8.4 Hz, 2H), 3.47 (s, 2H). |
| 40 | 298.1 | (DMSO-*d₆*): δ 10.66 (s, 1H), 9.24 (s, 1H), 8.84 (s, 1H), 8.10-8.06 (m, 2H), 7.31-7.25 (m, 4H), 6.58-6.57 (m, 1H), 3.95 (s, 3H), 3.29 (s, 2H). |
| 41 | 327.1 | (DMSO-*d₆*): δ 10.37 (s, 1H), 8.74 (s, 1H), 8.40-8.38 (m 1H), 7.72 (s, 1H), 7.47-7.45 (m, 2H), 7.26-7.23 (m, 2H), 6.49-6.47 (m, 1H), 4.03 (s, 3H), 1.49 (s, 6H). |
| 42 | 354.1 | (DMSO-*d₆*): δ 8.71 (d, *J* = 5.4 Hz, 1H), 8.57 (d, *J* = 8.8 Hz, 1H), 7.47 (d, *J* = 8.4 Hz, 2H), 7.24 (d, *J* = 8.4 Hz, 2H), 7.15 (d, *J* = 8.8 Hz, 1H), 6.57 (d, *J* = 5.4 Hz, 1H), 4.04 (s, 3H), 1.49 (s, 6H). |
| 43 | 343.9 | (MeOD-*d₄*): δ 8.66 - 8.53 (m, 2H), 7.66 (d, *J* = 8.0 Hz, 2H), 7.33 (d, *J* = 8.0 Hz, 2H), 7.12 (d, *J* = 8.0 Hz, 1H), 6.64 (d, *J* = 8.0 Hz, 1H), 5.92 (d, *J* = 48.0 Hz, 1H), 4.13 (s, 3H). |
| 44 | 325.7 | (DMSO-*d₆*): δ 10.70 (s, 1H), 9.16 (s, 1H), 8.88 (s, 1H), 8.68 (d, *J*=9.0 Hz, 1H), 8.47 (d, *J*=10.4 Hz, 2H), 7.74 (d, *J*=6.8 Hz, 1H), 7.39 (d, *J*=8.2 Hz, 1H), 7.06 (d, *J*=8.8 Hz, 1H), 6.80 (s, 1H), 3.99 (s, 3H), 3.50 (s, 2H). |
| 45 | 325.7 | (DMSO-*d₆*): δ 10.68 (s, 1H), 9.49 (s, 1H), 8.86 (s, 1H), 8.80 (d, *J* = 9.1 Hz, 1H), 8.62 (d, *J* = 5.4 Hz, 1H), 8.20 (dd, *J* = 7.4, 3.8 Hz, 2H), 7.65 (dd, *J* = 8.4, 2.2 Hz, 1H), 7.26 (d, *J* = 8.4 Hz, 1H), 7.08 (d, *J* = 9.0 Hz, 1H), 4.00 (s, 3H), 3.28 (s, 2H). |
| 46 | 390.0 | (MeOD-*d*₄): δ 8.81 (d, J = 6.4 Hz, 1H), 8.72 (d, J = 9.2 Hz, 1H), 7.32 (d, J = 8.8 Hz, 1H), 7.09-7.05 (m, 3H), 4.20 (s, 3H), 1.67 (s, 6H). |
| 47 | 375.0 | (MeOD-*d₄*): δ 8.81-8.79 (m, 1H), 8.34 (s, 1H), 7.56 (s, 1H), 7.35-7.25 (m, 2H), 6.70 (s, 1H), 4.16 (s, 3H), 3.92 (s, 1H), 1.52 (s, 3H). |
| 48 | 375.0 | (MeOD-*d₄*): δ 8.80 (d, *J* = 9.2 Hz, 1H), 8.36 (d, *J* = 6.0 Hz, 1H), 7.26-7.06 (m, 4H), 4.16 (s, 3H), 4.06-4.03 (m, 1H), 1.57 (d, *J* = 6.4 Hz, 3H). |
| 49 | 312.0 | (DMSO-*d₆*): δ 11.20 (s, 1H), 10.78 (s, 1H), 8.66 - 8.00 (m, 2H), 7.40 (dd, *J* = 23.0, 8.4 Hz, 4H), 6.63 (d, *J* = 6.6 Hz, 1H), 4.53 (q, *J* = 7.2 Hz, 2H), 3.38 (s, 2H), 1.40 (t, *J* = 7.2 Hz, 3H). |
| 50 | 340.0 | (DMSO-*d₆*): δ 8.90 (d, *J* = 7.2 Hz, 1H), 8.84 (d, *J* = 9.2 Hz, 1H), 7.49-7.45 (m, 3H), 7.35 (d, *J* = 8.4 Hz, 2H), 6.88 (d, *J* = 6.8 Hz, 1H), 4.16 (s, 3H), 2.94-2.90 (m, 2H), 2.37-2.33 (m, 2H). |
| 51 | 351.0 | (DMSO-*d₆*): δ 10.77 (s, 1H), 8.70 (d, *J* = 9.2 Hz, 1H), 8.41 (d, *J* = 5.6 Hz, 1H), 8.20 (s, 1H), 7.22-7.20 (m, 2H), 7.15-7.12 (m, 1H), 7.02 (d, *J* = 8.8 Hz, 1H), 6.80 (d, *J* = 5.6 Hz, 1H), 3.99 (s, 3H), 3.75-3.71 (m, 1H), 3.04-3.04 (m, 1H), 2.90-2.88 (m, 1H), 2.28-2.23 (m, 2H). |
| 52 | 361.0 | (MeOD-*d₄*): δ 8.78 (d, *J* = 9.2 Hz, 1H), 8.35 (d, *J* = 7.2 Hz, 1H), 7.46-7.33 (m, 2H), 7.27 (d, *J* = 9.2 Hz, 1H), 6.73-6.71 (m, 1H), 4.17 (s, 3H), 3.57 (s, 2H). |
| 53 | 341.1 | (MeOD-d₄): δ 8.54 (d, *J* = 6.4 Hz, 1H), 7.91 (s, 1H), 7.59 (d, *J* = 8.8 Hz, 2H), 7.32 (d, *J* = 8.8 Hz, 2H), 6.88 (d, J = 6.4 Hz, 1H), 4.60-4.55 (m, 2H), 1.61 (s, 6H), 1.56-1.52 (m, 3H). |
| 54 | 339.15 | (MeOD-d₄): δ 8.34 (d, *J*=5.6 Hz, 1H), 7.83 (s, 1H), 7.59-7.49 (m, 2H), 7.30-7.13 (m, 2H), 6.60 (d, *J* = 5.6 Hz, 1H), 4.53 (q, *J* = 7.2 Hz, 2H), 1.53 (q, *J*=4.0 Hz, 2H), 1.47 (t, *J*= 7.2 Hz, 3H), 1.12 (q, *J* = 4.0 Hz, 2H). |
| 55 | 312.0 | (DMSO-*d₆*): δ 10.77 (s, 1H), 9.55 (s, 1H), 8.11 (d, *J* = 6.8 Hz, 1H), 7.43 (d, *J* = 8.0 Hz, 2H), 7.37 (d, *J* = 8.0 Hz, 2H), 6.38 (d, *J* = 6.8 Hz, 1H), 4.03 (s, 3H), 3.44 (s, 2H), 2.69 (s, 3H). |
| 56 | 342.0 | (MeOD-*d₄*): δ 8.09 (d, *J* = 4.0 Hz, 1H), 8.03 (s, 1H), 7.26 (d, *J* = 8.0 Hz, 1H), 7.00-6.88 (m, 2H), 6.71 (d, *J* = 4.0 Hz, 1H), 4.52-4.37 (m, 2H), 3.84 (s, 3H), 3.44 (s, 2H), 1.45 (t, *J* = 8.0 Hz, 3H). |
| 57 | 367.1 | (DMSO-*d₆*): δ 10.65 (s, 1H), 10.19 (s, 1H), 8.82 (s, 1H), 8.46 (s, 1H), 7.74-7.72 (m, 2H), 7.30-7.28 (m, 2H), 5.30-5.23 (m, 2H), 3.28 (s, 2H). |
| 58 | 341.1 | (DMSO-*d₆*): δ 7.56 (s, 1H), 7.45 (d, *J* = 8.8 Hz, 2H), 7.22 (d, *J* = 8.8 Hz, 2H), 6.39 (s, 1H), 3.98 (s, 3H), 2.50 (s, 3H), 1.49 (s, 6H). |
| 59 | 352.9 | (MeOD-*d₄*): δ 8.67 (d, *J* = 8.0 Hz, 1H), 8.32 (d, *J* = 8.0 Hz, 1H), 7.35 (d, *J* = 8.0 Hz, 1H), 7.11 (d, *J* = 8.0 Hz, 1H), 6.98-6.88 (m, 2H), 6.86 (s, 1H), 4.85-4.72 (m, 2H), 4.26-4.18 (m, 1H), 4.14 (s, 3H). |
| 60 | 310.0 | (MeOD-*d₄*): δ 8.73-8.63 (m, 2H), 7.70 (s, 2H), 7.53 (s, 1H), 7.36 (d, J=6.0 Hz, 2H), 3.42 (s, 2H), 2.73 (s, 3H). |
| 61 | 357.0 | (MeOD-*d₄*): δ 8.77 (d, *J* = 9.2 Hz, 1H), 8.29 (d, *J* = 7.2 Hz, 1H), 7.72-7.68 (m, 1H), 7.31-7.22 (m, 3H), 6.94 (d, *J* = 7.2 Hz, 1H), 4.16 (s, 3H), 3.95-3.89 (m, 1H), 1.52 (d, *J* = 6.8 Hz, 3H). |
| 62 | 375.0 | (MeOD-*d₄*): δ 8.81 (d, *J* = 8.8 Hz, 1H), 8.36 (d, *J* = 7.2 Hz, 1H), 7.30-7.27 (m, 3H), 6.58 (d, *J* = 7.2 Hz, 1H), 4.17 (s, 3H), 3.65-3.60 (m, 1H), 1.53 (d, *J* =7.2 Hz, 3H). |
| 63 | 311.9 | (MeOD-*d₄*): δ 8.85-8.68 (m, 2H), 7.83 (d, *J* = 7.8 Hz, 1H), 7.78-7.73 (m, 1H), 7.70 (t, *J* = 8.0 Hz, 1H), 7.55 (dd, *J* = 7.2, 2.0 Hz, 1H), 7.36 (d, *J* = 9.0 Hz, 1H), 6.92 (d, *J* = 6.4 Hz, 1H), 4.21 (s, 3H). |
| 64 | 313.0 | (MeOD-d₄): δ 8.34 (d, *J* =5.2 Hz, 1H), 7.68 (s, 1H), 7.46 (t, *J* =8.0 Hz, 1H), 7.31 (d, *J* = 7.6 Hz, 1H), 7.22 (s, 1H), 7.16-7.13 (m, 1H), 6.53 (d, *J* = 5.6 Hz, 1H), 4.54-4.49 (m, 2H), 3.47 (s, 2H), 1.47 (t, *J* = 7.2 Hz, 3H). |
| 65 | 344.0 | (MeOD-d₄): δ 8.14 (d, *J* = 5.6 Hz, 1H), 8.06 (s, 1H), 7.54-7.50 (m, 1H), 7.17 (d, *J* = 6.0 Hz, 1H), 7.09 (d, *J* = 13.6 Hz, 1H), 6.74 (d, *J* = 5.6 Hz, 1H), 4.48-4.43 (m, 2H), 3.86-3.84 (m, 1H), 1.50-1.43 (m, 6H). |
| 66 | 352.0 | (MeOD-d₄): δ 8.82 (dd, *J* = 12.6, 8.0 Hz, 2H), 7.69-7.54 (m, 2H), 7.42 (d, *J* = 9.0 Hz, 1H), 7.38-7.31 (m, 2H), 7.18 (d, *J* = 6.8 Hz, 1H), 4.23 (s, 3H), 1.56 (q, *J* = 4.0 Hz, 2H), 1.14 (q, *J* = 4.2 Hz, 2H). |
| 67 | 368.2 | (DMSO-*d₆*): δ 10.38 (s, 1H), 8.74 (s, 1H), 8.49-8.47 (m, 1H), 7.47-7.44 (m, 2H), 7.24-7.21 (m, 2H), 7.07-7.05 (m, 1H), 6.48 (s, 1H), 4.01 (s, 3H), 2.50 (s, 3H), 1.49 (s, 6H). |
| 68 | 327.1 | (DMSO-*d₆*): δ 10.05 (s, 1H), 8.40 (s, 1H), 8.18 (s, 1H), 7.73 (d, *J* = 8.6 Hz, 2H), 7.34 (d, *J* = 8.7 Hz, 2H), 3.95 (s, 3H), 1.46 (s, 6H). |
| 69 | 353.1 | (DMSO-*d₆*): δ 9.05 (s, 1H), 8.69 (d, *J* = 9.0 Hz, 1H), 8.41 (d, *J* = 4.9 Hz, 1H), 7.37 (d, *J* = 8.5 Hz, 2H), 7.27 (d, *J* = 8.4 Hz, 2H), 7.02 (d, *J* = 9.0 Hz, 1H), 6.82 (d, *J* = 5.4 Hz, 1H), 3.99 (s, 6H), 1.46 (s, 6H). |
| 70 | 360.9 | (DMSO-*d₆*): δ 8.62 (d, *J* = 9.2 Hz, 1H), 8.42 (d, *J* = 6.4 Hz, 1H), 7.13-7.10 (m, 2H), 7.07-7.05 (m, 2H), 4.12 (s, 3H), 3.54 (s, 2H). |
| 71 | 360.9 | (MeOD-*d₄*): δ 8.70 (d, *J*=8.0 Hz, 1H), 8.43 (s, 1H), 8.35 (d, *J*=8.0 Hz, 1H), 7.23-7.07 (m, 3H), 6.44 (d, *J*=4.0 Hz, 1H), 4.13 (s, 3H), 3.51 (s, 2H). |
| 72 | 358.0 | (MeOD-*d₄*): δ 8.87-8.82 (m, 2H), 7.51-7.39 (m, 4H), 7.05 (d, *J* = 7.2 Hz, 1H), 4.23 (s, 3H), 3.64-3.62 (m, 1H), 1.53 (d, *J* = 7.2 Hz, 3H). |
| 73 | 326.9 | (MeOD-*d₄*): δ 8.33 (d, *J* = 5.6 Hz, 1H), 7.66 (s, 1H), 7.51 (d, *J* = 8.8 Hz, 2H), 7.21-7.19 (m, 2H), 6.50 (d, *J* = 5.6 Hz, 1H), 4.54-4.49 (m, 2H), 3.60-3.58 (m, 1H), 1.51 (d, *J* = 7.2 Hz, 3H), 1.48-1.44 (m, 3H). |
| 74 | 327.1 | (MeOD-d₄): δ 8.17 (s, 1H), 7.56 (d, *J* = 8.4 Hz, 2H), 7.48 (d, *J* = 8.8 Hz, 2H), 4.46-4.42 (m, 2H), 3.53 (s, 2H), 2.72 (s, 3H), 1.50-1.46 (m, 3H). |
| 75 | 326.0 | (MeOD-*d₄*): δ 8.85-8.77 (m, 2H), 7.57 (t, *J* = 8.0 Hz, 1H), 7.42-7.40 (m, 2H), 7.34 (s, 1H), 7.29-7.27 (m, 1H), 7.01 (d, *J* = 6.8 Hz, 1H), 4.23 (s, 3H), 3.52 (s, 2H). |
| 76 | 298.9 | (MeOD-*d₄*): δ 8.61 (d, *J*= 6.6 Hz, 1H), 8.01 (s, 1H), 7.90-7.82 (m, 1H), 7.81-7.75 (m, 1H), 7.72 (t, *J*= 7.8 Hz, 1H), 7.58 (dd, *J*= 8.0, 1.6 Hz, 1H), 6.92 (d, *J* = 6.6 Hz, 1H), 4.61 (q, *J*= 7.2 Hz, 2H), 1.56 (t, *J*= 7.2 Hz, 3H). |
| 77 | 345.9 | (MeOD-*d₄*): δ 8.12 (d, *J* = 7.2 Hz, 1H), 7.58-7.53 (m, 2H), 7.40-7.37 (m, 1H), 6.79 (d, *J* = 7.2 Hz, 1H), 4.50-4.45 (m, 2H), 3.68 (s, 2H), 1.54-1.50 (m, 3H). |
| 78 | 359.0 | (MeOD-*d₄*): δ 8.77 (d, *J* = 9.2 Hz, 1H), 8.29 (d, *J* = 7.2 Hz, 1H), 7.59-7.55 (m, 2H), 7.41-7.38 (m, 1H), 7.23 (d, *J* = 9.2 Hz, 1H), 6.93 (d, *J* = 7.2 Hz, 1H), 4.16 (s, 3H), 3.68 (s, 2H). |
| 79 | 354.9 | (MeOD-d₄): δ 8.71-8.59 (m, 1H), 8.31 (d, *J* = 8.0 Hz, 1H), 7.32 (d, *J* = 8.0 Hz, 1H), 7.07 (d, *J* = 8.0 Hz, 1H), 7.00 - 6.88 (m, 3H), 4.11 (s, 3H), 3.85 (s, 3H), 3.46 (s, 2H). |
| 80 | 325.1 | (MeOD-d₄): δ 8.59 (d, *J* = 6.6 Hz, 1H), 8.11 (s, 1H), 7.69-7.56 (m, 2H), 7.44-7.19 (m, 2H), 7.00 (d, *J* = 6.6 Hz, 1H), 4.23 (s, 3H), 1.58 (q, *J* = 4.0 Hz, 2H), 1.17 (q, *J* = 4.2 Hz, 2H). |
| 81 | 326.1 | (DMSO-*d₆*): δ 11.14 (s, 1H), 10.42 (s, 1H), 8.20-8.18 (m, 1H), 7.48-7.36 (m, 4H), 6.66-6.64 (m, 1H), 4.09 (s, 3H), 1.49 (s, 6H). |
| 82 | 340.1 | (DMSO-*d₆*): δ 10.38 (s, 1H), 9.86 (s, 1H), 8.69 (s, 1H), 8.14-8.11 (m, 2H), 7.41-7.30 (m, 4H), 6.62-6.61 (m, 1H), 4.44-4.39 (m, 2H), 1.48 (s, 6H), 1.38 (t, *J* = 7.2 Hz, 3H). |
| 83 | 355.2 | (DMSO-*d₆*): δ 10.35 (s, 1H), 8.72 (s, 1H), 8.28 (s, 1H), 7.59 (m, 2H), 7.33-7.31 (m, 2H), 4.31-4.26 (m, 2H), 2.66 (s, 3H), 1.47(s, 6H), 1.36 (t, *J* = 7.2, 3H). |
| 84 | 341.1 | (600 MHz, DMSO-*d₆*): δ 10.36 (s, 1H), 8.81 (s, 1H), 8.30 (d, *J* = 5.4 Hz, 1H), 7.45 (d, *J* = 8.6 Hz, 2H), 7.27-7.16 (m, 2H), 6.24 (d, *J* = 5.4 Hz, 1H), 3.96 (s, 3H), 2.58 (s, 3H), 1.48 (s, 6H). |
| 85 | 356.0 | (MeOD-d₄): δ 8.85-8.72 (m, 2H), 7.69 (d, *J* = 8.0 Hz, 2H), 7.42- 7.31 (m, 3H), 6.92 (d, *J* = 8.0 Hz, 1H), 4.82 (s, 1H), 4.22 (s, 3H), 3.45 (s, 3H). |
| 86 | 370.2 | (MeOD-d₄): δ 8.89 - 8.77 (m, 2H), 7.72 (d, *J* = 8.0 Hz, 2H), 7.45- 7.37 (m, 3H), 6.99 (d, *J* = 8.0 Hz, 1H), 4.93 (s, 1H), 4.25 (s, 3H), 3.70 - 3.52 (m, 2H), 1.31 (t, *J* = 6.0 Hz, 3H). |
| 87 | 366.2 | (MeOD-d₄): δ 8.79-8.73 (m, 2H), 7.61-7.59 (m, 2H), 7.34-7.31 (m, 3H), 6.89 (d, *J* = 6.4 Hz, 1H), 4.20 (s, 3H), 2.86-2.79 (m, 2H), 2.58-2.51 (m, 2H), 2.05-1.90 (m, 2H). |
| 88 | 380.2 | (MeOD-d₄): δ 8.84-8.78 (m, 2H), 7.64 (d, *J =* 8.8 Hz, 2H), 7.72-7.32 (m, 3H), 6.99 (d, *J =* 6.8 Hz, 1H), 4.23 (s, 3H), 2.58-2.54 (m, 2H), 2.04-2.01 (m, 2H), 1.82-1.77 (m, 4H). |
| 89 | 331.1 | (MeOH-d₄): δ 8.63 (d, *J* = 9.2 Hz, 1H), 8.43 (d, *J* = 6.8 Hz, 1H), 7.02 (d, *J* = 8.8 Hz, 1H), 6.76 (d, J=7.2 Hz, 1H), 4.08 (s, 3H), 3.72-3.67 (m, 1H), 2.15-2.11 (m, 2H), 2.05-2.04 (m, 2H), 1.92-1.88 (m, 3H), 1.57-1.53 (m, 2H), 1.26-1.23 (m, 2H) . |
| 90 | 314.9 | (DMSO-*d₆*): δ 10.94 (s, 1H), 10.64 (s, 1H), 9.11 (s, 1H), 8.97 (d, *J =* 9.2 Hz, 1H), 8.41 (d, *J* = 9.2 Hz, 1H), 8.08 (s, 1H), 7.70 (s, 1H), 7.35 (d, *J* = 9.2 Hz, 1H), 6.79 (d, *J* = 7.2 Hz, 1H), 4.77 (s, 2H), 4.09 (s, 3H) . |
| 91 | 341.1 | (DMSO-*d₆*): δ 10.33 (s, 1H), 10.04 (s, 1H), 8.66 (s, 1H), 8.39 (s, 1H), 8.23 (s, 1H), 7.75-7.72 (m, 2H), 7.35-7.32 (m, 2H), 4.39-4.34 (m, 2H), 1.46 (s, 6H), 1.39 (t, *J* = 7.2, 3H). |
| 92 | 362.1 | (DMSO-*d₆*): δ 11.17 (s, 1H), 8.72 - 8.63 (m, 2H), 8.36-8.34 (m, 1H), 8.05-8.04 (m, 1H), 7.91-7.90 (m, 1H), 7.61-7.56 (m, 3H), 7.19 - 7.17 (m, 1H), 6.68-6.67 (m, 1H), 4.05 (s, 3H). |
| 93 | 353.1 | (DMSO-*d₆*): δ 8.62 (d, *J* = 5.2 Hz, 1H), 8.18 (d, *J* = 9.2 Hz, 1H), 7.49-7.44 (m, 2H), 7.41 (d, *J* = 2.4 Hz, 1H), 7.29 (dd, *J* = 9.2, 2.6 Hz, 1H), 7.24-7.19 (m, 2H), 6.48 (d, *J* = 5.2 Hz, 1H), 3.94 (s, 3H), 1.49 (s, 6H). |
| 94 | 357.1 | (DMSO-*d₆*): δ 8.74-8.73 (m, 1H), 8.34-8.32 (m, 1H), 8.10-8.09 (m, 1H), 7.71-7.68 (m, 1H), 7.49-7.47 (m, 2H), 7.28-7.25 (m, 2H), 6.65-6.63 (m, 1H), 1.50 (s, 6H). |
| 95 | 350.0 | (MeOD-*d₄*): δ 8.78-8.74 (m, 1H), 8.33 (s, 1H), 7.90-7.83 (m, 1H), 7.74-7.66 (m, 2H), 7.25 (s, 1H), 6.95 (s, 1H), 4.16 (s, 3H), 3.77 (s, 2H). |
| 96 | 375.0 | (DMSO-*d₆*): δ 11.27-11.21 (m, 1H), 10.89 (d, *J* = 5.4 Hz, 1H), 9.22 (d, *J* = 9.2 Hz, 1H), 8.93 (s, 1H), 8.29 (d, *J* = 8.0 Hz, 1H), 8.24 (d, *J* = 7.2 Hz, 1H), 7.93 (d, *J* = 8.4 Hz, 1H), 7.71-7.56 (m, 4H), 7.41-7.38 (m, 1H), 6.15 (d, *J* = 6.8 Hz, 1H), 4.12 (s, 3H), 3.91 (s, 2H). |
| 97 | 354.2 | (DMSO-*d₆*): δ 9.43 (s, 1H), 8.78 (d, *J* = 4.8 Hz, 1H), 7.51-7.49 (m, 3H), 7.31 (d, *J* = 7.2 Hz, 2H), 7.23 (s, 1H), 6.47 (m, 1H), 4.03 (s, 3H), 1.51 (s, 6H). |
| 98 | 339.1 | (DMSO-*d₆*): δ 10.70 (s, 1H), 8.60 (d, *J*= 5.2 Hz, 1H), 8.18 (m, 2H), 7.39 (m, 3H), 7.27 (dd, *J*=9.0, 2.4 Hz, 1H), 7.23-7.18 (m, 2H), 6.44 (d, *J* = 5.2 Hz, 1H), 4.21 (q, *J*=7.0 Hz, 2H), 3.35 (s, 2H), 1.42 (t, *J*=7.0 Hz, 3H). |
| 99 | 353.1 | (DMSO-*d₆*): δ 10.39 (s, 1H), 9.21-8.57 (m, 1H), 8.55 (d, *J* = 5.0 Hz, 1H), 7.95 (d, *J*=9.2 Hz, 1H), 7.55 (d, *J* = 2.8 Hz, 1H), 7.49-7.41 (m, 3H), 7.27-7.18 (m, 2H), 6.60 (d, *J* = 5.0 Hz, 1H), 3.91 (s, 3H), 1.50 (s, 6H). |
| 100 | 323.1 | (DMSO-*d₆*): δ 10.61 (s, 1H), 8.73 (m, 2H), 8.07 (d, *J* = 9.2 Hz, 1H), 7.39 (d, *J* = 1.8 Hz, 1H), 7.28-7.09 (m, 6H), 4.39 (s, 2H), 3.90 (s, 3H), 3.22 (s, 2H). |
| 101 | 354.1 | (DMSO-*d₆*): δ 10.41 (m, 1H), 9.52 (m, 1H), 8.81 (d, J = 9.0 Hz, 1H), 8.62 (d, *J* = 5.4 Hz, 1H), 8.26 (m, *J* = 13.4, 3.8 Hz, 3H), 7.70 (dd, *J* = 8.6, 2.5 Hz, 1H), 7.27 (d, J = 8.6 Hz, 1H), 7.08 (d, *J* = 9.0 Hz, 1H), 4.00 (s, 3H), 1.47 (s, *J* = 12.6 Hz, 6H). |
| 102 | 352.1 | (DMSO-*d₆*): δ 10.37 (s, 1H), 8.44-8.42 (m, 1H), 8.20 (s, 1H), 7.94-7.92 (m, 1H), 7.44-7.41 (m, 2H), 7.18-7.15 (m, 2H), 7.03-7.00 (m, 1H), 6.76-6.75 (m, 1H), 6.44-6.43 (m, 1H), 6.25-6.24 (m, 1H), 2.81-2.80 (m, 3H), 1.48 (s, 6H). |
| 103 | 387.9 | (DMSO-*d₆*): δ 10.10 (s, 1H), 8.75 (d, J=5.2 Hz, 1H), 8.56 (d, J=8.8 Hz, 1H), 7.60 (d, J=8.8 Hz, 1H), 7.42 (d, J=2.8 Hz, 1H), 7.29-7.26 (m, 1H), 7.17 (d, J=9.2Hz, 1H), 6.72 (d, J=5.6 Hz, 1H), 4.04 (s, 3H), 1.53 (s, 6H). |
| 105 | 384.1 | (DMSO-*d₆*): δ 9.88 (s, 1H), 8.83 (d, *J* = 6.0 Hz, 1H), 8.72 (d, *J* = 8.4 Hz, 1H), 7.42-7.35 (m, 2H), 7.02 (d, *J* = 2.0 Hz, 1H), 6.91-6.88 (m, 2H), 4.11 (s, 3H), 3.71 (s, 3H), 1.44 (s, 6H). |
| 107 | 396.2 | (DMSO-*d₆*): δ 8.71 (d, *J* = 5.2 Hz, 1H), 8.57 (d, *J* = 9.2 Hz, 1H), 7.51 (d, *J* = 8.8 Hz, 2H), 7.29 (d, *J* = 8.8 Hz, 2H), 7.15 (d, *J* = 8.8 Hz, 1H), 6.57 (d, *J* = 5.2 Hz, 1H), 4.04 (s, 3H), 3.78-3.75 (m, 2H), 3.52-3.46 (m, 2H), 2.51-2.49 (m, 2H), 1.90-1.83 (m, 2H). |
| 108 | 368.0 | (MeOD-*d₄*): δ 8.64-8.61 (m, 2H), 7.56 (d, *J* = 9.6 Hz, 1H), 7.31-7.24 (m, 2H), 7.13-7.11 (m, 1H), 6.65-6.64 (m, 1H), 5.25-5.24 (m, 2H), 5.04-4.98 (m, 2H), 4.08 (s, 3H). |
| 109 | 368.1 | (MeOD-*d₄*): δ 8.62-8.60 (m, 2H), 7.56 (d, *J* = 9.6 Hz, 1H), 7.11-7.06 (m, 3H), 6.67 (d, *J* = 5.6 Hz, 1H), 4.12 (s, 3H), 2.34 (s, 3H), 1.59 (s, 6H). |
| 110 | 372.2 | (MeOD-*d₄*): δ 8.66 (d, *J* = 5.6 Hz, 1H), 8.59 (d, *J* = 9.2 Hz, 1H), 7.59-7.55 (m, 1H), 7.13-7.05 (m, 3H), 6.75 (d, *J* =5.6 Hz, 1H), 4.12 (s, 3H), 1.59 (s, 6H). |
| 111 | 372.2 | (MeOD-*d₄*): δ 8.74-8.71 (m, 2H), 7.44-7.35 (m, 3H), 7.25 (d, *J* = 9.2 Hz, 1H), 6.81 (d, *J* = 2.0 Hz, 1H), 4.17 (s, 3H), 1.60 (s, 6H). |
| 112 | 390.2 | (MeOD-*d₄*): δ 8.86-8.80 (m, 2H), 7.57-7.52 (m, 1H), 7.41-7.37 (m, 2H), 7.15-7.14 (m, 1H), 4.17 (s, 3H), 1.60 (s, 6H). |
| 113 | 366.1 | (DMSO-*d₆*): δ 10.28 (s, 1H), 8.88 (d, *J* = 6 Hz, 1H), 8.56 (m, 2H), 7.46 (d, *J* = 6 Hz, 1H), 7.22 (s, 4H), 7.08 (d, *J* = 8.0 Hz, 1H), 4.97 (d, *J* = 8.0 Hz, 1H), 3.99 (s, 3H), 1.65 (d, *J* = 8.0 Hz, 3H), 1.38 (m, 6H). |
| 114 | 367.2 | (DMSO-*d₆*): δ 8.68 (d, J = 8.8 Hz, 1H), 8.30 (s, 2H), 7.72 (d, *J* = 8.0 Hz, 1H), 7.32 (d, *J* = 8.4 Hz, 2H), 6.94-6.90 (m, 3H), 6.11 (d, *J* = 7.8 Hz, 1H), 4.02 (s, 3H), 3.76 (d, *J* = 6.2 Hz, 3H), 1.46 (s, 6H). |
| 116 | 361.1 | (DMSO-*d₆*): δ 10.30 (s, 1H), 8.70 (d, *J* = 5.6 Hz, 1H), 8.60 (s, 1H), 8.40 (d, *J* = 8.8 Hz, 1H), 7.07 (dd, *J* = 7.2, 4.2 Hz, 2H), 4.87-4.69 (m, 1H), 4.00 (s, 3H), 2.70 (d, *J* = 7.8 Hz, 2H), 2.42 (t, *J* = 8.4 Hz, 2H), 2.14-2.00 (m, 2H), 1.93-1.79 (m, 2H), 1.14 (s, 6H). |
| 117 | 360.1 | (DMSO-*d₆*): δ 10.22 (s, 1H), 8.90-8.88 (m, 1H), 8.70 (s, 1H), 8.43-8.41 (m, 1H), 8.22 (s, 1H), 7.91 (s, 1H), 7.16-7.14 (m, 1H), 6.80-6.78 (m, 1H), 4.08 (s, 3H), 3.69-3.68 (m, 1H), 2.90-2.88 (m, 2H), 2.39-2.33 (m, 2H), 2.01-1.81 (m, 2H), 1.79-1.76 (m, 2H), 1.17 (s, 6H). |
| 118 | 343.0 | (DMSO-*d₆*): δ 10.63 (s, 1H), 10.57 (s, 1H), 8.98 (s, 1H), 8.92 (d, *J*= 6.0 Hz, 1H), 8.41 (d, *J*= 6.8 Hz, 1H), 8.15 (s, 1H), 7.71 (s, 1H), 7.33 (d, *J*= 9.2 Hz, 1H), 6.87 (d, *J*= 7.2 Hz, 1H), 4.09 (s, 3H), 1.81 (s, 6H). |
| 123 | 380.2 | (MeOD-d₄): δ 9.69 (s, 1H), 6.87 (d, *J* = 6.8Hz, 1H), 7.64 (d, *J* = 8.4 Hz, 2H), 7.55 (d, *J* = 8.8 Hz, 2H), 7.22 (s, 1H), 6.81 (d, *J* = 6.8 Hz, 1H), 4.17 (s, 3H), 2.58-2.54 (m, 2H), 2.04-2.01 (m, 2H), 1.79-1.78 (m, 4H). |
| 124 | 382.2 | (DMSO-*d₆*): δ 10.30 (s, 1H), 8.71 (d, *J* = 5.6 Hz, 1H), 8.58 (d, *J* = 8.8 Hz, 1H), 7.37 (d, *J* = 8.8 Hz, 2H), 7.25 (d, *J* = 8.4 Hz, 2H), 7.15 (d, *J* = 9.2 Hz, 1H), 6.55 (d, *J* = 5.2 Hz, 1H), 4.04 (s, 3H), 2.04-1.87 (m, 4H), 0.70-0.67 (m, 6H). |
| 125 | 394.2 | (MeOD-d₄): δ 8.80-8.73 (m, 2H), 7.66 (d, *J* =8.8 Hz, 2H), 7.37-7.31 (m, 3H), 6.90 (d, *J* =6.8 Hz, 1H), 4.21 (s, 3H), 2.46-2.43 (m, 2H), 1.90-1.84 (m, 2H), 1.71-1.58 (m, 5H), 1.40-1.36 (m, 1H). |
| 126 | 357.0 | (DMSO-*d₆*): δ 10.44 (s, 1H), 9.85 (s, 1H), 8.43 (s, 1H), 8.24 (s, 1H), 7.48 (s, 1H), 7.42 (d, *J* = 7.6 Hz, 1H), 7.24 (d, *J* = 8.4 Hz, 1H), 3.96 (s, 3H), 3.74 (s, 3H), 1.41 (s, 6H). |
| 127 | 354.1 | (DMSO-*d₆*): δ 10.34 (s, 1H), 9.15 (s, 1H), 8.68 (d, *J* = 9.0 Hz, 2H), 8.51 (s, 1H), 8.47 (d, *J* = 4.8 Hz, 1H), 7.74 (d, *J* = 7.2 Hz, 1H), 7.40 (d, *J* = 8.6 Hz, 1H), 7.07 (d, *J* = 8.8 Hz, 1H), 6.83 (d, *J* = 4.8 Hz, 1H), 3.99 (s, 3H), 1.50 (s, 6H). |
| 128 | 355.1 | (DMSO-*d₆*): δ 8.73 (d, *J* = 5.4 Hz, 1H), 8.60 (d, *J* = 8.8 Hz, 1H), 8.53 (d, *J* = 2.8 Hz, 1H), 7.74 (dd, *J* = 8.7, 2.8 Hz, 1H), 7.52 (d, *J* = 8.8 Hz, 1H), 7.18 (d, *J* = 8.8 Hz, 1H), 6.63 (d, *J* = 5.4 Hz, 1H), 4.04 (s, 3H), 1.51 (s, 6H). |
| 129 | 353.2 | (MeOD-d₄): δ 8.34 (d, *J* = 5.6 Hz, 1H), 7.66 (s, 1H), 7.53 (d, *J* = 8.8 Hz, 2H), 7.19 (d, *J* = 8.4 Hz, 2H), 6.51 (d, *J* = 5.2 Hz, 1H), 4.07 (s, 3H), 2.56-2.51 (m, 2H), 2.03-2.00 (m, 2H), 1.77-1.75 (m, 4H). |
| 130 | 366.1 | (MeOD-d₄): δ 8.84-8.77 (m, 2H), 7.63 (d, *J* = 8.4 Hz, 2H), 7.41-7.32 (m, 3H), 6.93 (d, *J* = 6.8 Hz, 1H), 4.23 (s, 3H), 2.96-2.42 (m, 1H), 2.44-2.38 (m, 1H), 1.09-1.07 (m, 3H), 0.79-0.77 (m, 3H). |
| 131 | 382.2 | (DMSO-*d₆*): δ 10.72 (s, 1H), 8.90 (s, 1H), 8.73-8.71 (m, 1H), 8.57 (d, *J* = 8.8 Hz, 1H), 7.46 (d, *J* = 8.4 Hz, 2H), 7.29 (d, *J* = 6.8 Hz, 2H), 7.16 (d, *J* = 8.8 Hz, 1H), 6.59 (d, *J* = 3.6 Hz, 1H), 4.51 (d, *J* = 8.8 Hz, 1H), 4.04 (s, 3H), 3.85-3.81 (m, 3H), 2.89-2.87 (m, 1H), 2.24-2.18 (m, 1H). |
| 132 | 382.1 | (DMSO-*d₆*): δ 10.84 (s, 1H), 8.86-8.79 (m, 2H), 7.56 (d, *J* = 8.4 Hz, 2H), 7.44-7.34 (m, 3H), 6.82 (d, *J* = 6.4 Hz, 2H), 4.15 (s, 3H), 3.53-3.51 (m, 1H), 1.91-1.89 (m, 1H), 1.54-1.51 (m, 1H), 0.92-0.88 (m, 6H). |
| 133 | 414.0 | (DMSO-*d₆*): δ 10.41 (s, 1H), 8.75 (d, *J*=5.2 Hz, 1H), 8.54 (d, *J*=7.6 Hz, 1H), 7.55-7.50 (m, 1H), 7.26-7.21 (m, 1H), 7.15-7.13 (m, 2H), 6.72 (d, *J*=5.6 Hz, 1H), 4.04 (s, 3H), 3.70-3.62 (m, 4H), 2.37 (d, *J*= 14.0 Hz, 1H), 2.05-1.98 (m, 2H). |
| 134 | 432.0 | (MeOD-*d₄*): δ 8.77-8.70 (m, 2H), 7.57-7.53 (m, 1H), 7.38-7.33 (m, 1H), 7.27 (d, *J*=9.2 Hz, 1H), 6.92 (d, *J*=6.0 Hz, 1H), 4.18 (s, 3H), 3.88-3.79 (m, 4H), 2.47-2.43 (m, 2H), 2.19-2.12 (m, 2H). |
| 135 | 430.1 | (DMSO-*d₆*): δ 10.07 (m, 1H), 8.86-8.72 (m, 2H), 8.62 (d, *J*=8.8 Hz, 1H), 7.73 (d, *J*=8.8 Hz, 1H), 7.50 (d, *J*=2.6 Hz, 1H), 7.36 (dd, *J*= 8.6, 2.4 Hz, 1H), 7.23 (d, *J*= 8.8 Hz, 1H), 6.80 (d, *J*= 5.4 Hz, 1H), 4.10 (s, 3H), 3.95-3.83 (m, 2H), 3.72-3.61 (m, 2H), 2.49-2.39 (m, 2H), 2.21 -2.09 (m, 2H). |
| 136 | 414.1 | (DMSO-*d₆*): δ 8.74 (d, *J*=5.2 Hz, 1H), 8.61 (d, *J*=8.8 Hz, 1H), 7.53-7.45 (m, 2H), 7.34 (d, *J*=8.8 Hz, 1H), 7.19 (d, *J*=9.2 Hz, 1H), 6.57 (d, *J*=5.2 Hz, 1H), 4.05 (s, 3H), 3.78 (d, *J*=11.6 Hz, 2H), 3.53-3.30 (m, 2H), 2.52-2.45 (m, 2H),1.93-1.86 (m, 2H). |
| 137 | 431.9 | (MeOD-*d₄*): δ 8.86-8.81 (m, 2H), 7.40-7.37 (m, 3H), 7.08 (d, *J*=6.4 Hz, 1H), 4.22 (s, 3H), 3.91-3.87 (m, 2H), 3.70-3.65 (m, 2H), 2.50-2.46 (m, 2H), 2.07-2.00 (m, 2H). |
| 138 | 430.1 | (DMSO-*d₆*): δ 8.90 (s, 1H), 8.72 (d, *J* = 5.4 Hz, 1H), 8.61 (d, *J* = 8.8 Hz, 1H), 7.63 (d, *J* = 2.0 Hz, 1H), 7.49 (m, 2H), 7.19 (d, *J* = 8.8 Hz, 1H), 6.46 (d, *J* = 5.4 Hz, 1H), 4.05 (s, 3H), 3.78 (dd, *J* = 8.0, 3.6 Hz, 2H), 3.49 (t, *J* = 10.4 Hz, 2H), 2.47 (m, 2H), 1.93 - 1.84 (m, 2H). |
| 139 | 409.9 | (DMSO-*d₆*): δ 10.11 (s, 1H), 8.76 (d, *J*=5.6 Hz, 1H), 8.60 (d, *J*=8.8 Hz, 1H), 7.53 (d, *J*=8.4 Hz, 1H), 7.21 (d, *J*=9.2 Hz, 1H), 7.15-7.10 (m, 2H), 6.68 (d, *J*=5.6 Hz, 1H), 4.06 (s, 3H), 3.81-3.76 (m, 2H), 3.70-3.66 (m, 2H), 2.37-2.33 (m, 5H), 1.98-1.92(m, 2H). |
| 140 | 361.2 | (MeOD-*d₄*): δ 8.56-8.45 (m, 2H), 8.38 (s, 1H), 6.98 (d, *J* = 8.9 Hz, 1H), 4.25 (td, *J* = 11.0, 5.4 Hz, 1H), 4.07 (m, 3H), 3.01 (d, *J* = 11.4 Hz, 2H), 2.50 (t, *J* = 11.2 Hz, 2H), 2.13 (d, *J* = 11.6 Hz, 2H), 1.80 (td, *J* = 11.4, 9.0 Hz, 2H), 1.32 (s, 6H). |
| 142 | 317.0 | (DMSO-*d₆*): δ 10.37 (d, *J* = 6.4 Hz, 1H), 8.69-8.65 (m, 1H), 8.19 (s, 1H), 7.19-7.12 (m, 1H), 6.93-6.90 (m, 1H), 6.47-6.43 (m, 1H), 4.05-3.95 (m, 1H), 2.24-2.14 (m, 2H), 2.07-2.00 (m, 2H), 1.92-1.60 (m, 3H), 1.42-1.19 (m, 3H). |
| 143 | 318.1 | (MeOD-*d₄*): δ 8.67 (d, *J* = 9.2 Hz, 1H), 8.52 (s, 1H), 8.29 (d, *J* = 6.8 Hz, 1H), 7.07 (d, *J* = 9.2 Hz, 1H), 6.75 (d, *J* = 6.8 Hz, 1H), 4.43-4.41 (m, 1H), 4.10 (s, 3H), 3.23 (s, 3H), 2.97-2.94 (m, 3H), 2.58-2.54 (m, 2H), 2.49-2.47 (m, 1H). |
| 144 | 346.1 | (DMSO-*d₆*): δ 10.41 (s, 1H), 8.77-8.76 (m, 1H), 8.70 (s, 1H), 8.41-8.39 (m, 1H), 8.14 (s, 1H), 7.13-7.11 (m, 1H), 6.69-6.67 (m, 1H), 4.29 (s, 1H), 4.02 (s, 3H), 3.02-3.00 (m, 1H), 2.84-2.80 (m, 1H), 2.73-2.65 (m, 2H), 2.28-2.23 (m, 1H), 1.91-1.86 (m, 1H), 1.25 (d, *J* = 8.4 Hz, 6H). |
| 145 | 366.2 | (MeOD-*d₄*): δ 8.82-8.76 (m, 2H), 7.39 (d, *J*=9.2 Hz, 1H), 7.30 (d, *J*= 8.0 Hz, 1H), 7.13-7.11 (m, 2H), 7.03 (d, *J*=7.2 Hz, 1H), 4.22 (s, 3H), 3.68-3.65 (m, 1H), 2.96-2.81 (m, 2H), 2.12-2.06 (m, 3H), 1.82-1.73 (m, 1H). |
| 146 | 409.1 | (DMSO-*d₆*): δ 10.57 (s, 1H), 8.81-8.72 (m, 2H), 7.70 (d, *J*=8.8 Hz, 2H), 7.41 (d, *J*=8.4 Hz, 2H), 7.28 (d, *J*=9.2 Hz, 1H), 6.59 (d, *J*=5.6 Hz, 1H), 4.10 (s, 3H), 3.67-3.52 (m, 4H), 3.21 (s, 3H), 2.87-2.84 (m, 2H),2.50-2.40 (m, 2H). |
| 147 | 353.1 | (DMSO-*d₆*): δ 10.43 (s, 1H), 9.29 (s, 1H), 8.81 (s, 2H), 8.38 (d, *J* = 9.0 Hz, 1H), 8.22 (s, 1H), 7.72-7.28 (m, 5H), 4.03 (s, 3H), 1.55 (s, 6H). |
| 148 | 341.0 | (MeOD-*d₄*): δ 8.81-8.79 (m, 2H), 7.80-7.76 (m, 2H), 7.52-7.49 (m, 2H), 7.38-7.36 (m, 1H), 6.93-6.91 (m, 1H), 5.02 (s, 1H), 4.22 (s, 3H). |
| 150 | 386.1 | (MeOD-*d₄*): δ 8.70-8.63 (m, 2H), 7.88-7.84 (m, 1H), 7.19-7.11 (m, 3H), 6.75 (d, *J* = 6.0 Hz, 1H), 4.15 (s, 3H), 3.37 (d, *J* = 10.4 Hz, 1H), 2.42-2.36 (m, 1H), 1.10 (d, *J* = 6.4 Hz, 3H), 0.81 (d, *J* = 6.8 Hz, 3H). |
| 151 | 326.9 | (MeOD-*d₄*): δ 9.02-8.97 (m, 2H), 8.72 (s, 2H), 8.64 (d, *J*=7.2 Hz, 1H), 7.31 (d, *J*=9.2 Hz, 1H), 4.19 (s, 3H), 3.51 (s, 2H). |
| 152 | 327.1 | (DMSO-*d₆*): δ 8.99-8.98 (m, 2H), 8.90 (s, 1H), 8.69-8.58 (m, 2H), 7.78-7.76 (m, 2H), 7.21-7.13 (m, 3H), 6.54-6.53 (m, 1H), 4.03 (s, 3H). |
| 154 | 432.0 | (DMSO-*d₆*): δ 8.80 (d, *J*=5.6 Hz, 1H), 8.51 (d, *J*=9.2 Hz, 1H), 7.19-7.14 (m, 3H), 6.87 (d, *J*=5.2 Hz, 1H), 4.05 (s, 3H), 3.79-3.77 (m, 2H), 3.56-3.54 (m, 2H), 2.24 (s, 4H). |
| 155 | 344.0 | (DMSO-*d₆*): δ 10.53 (s, 1H), 8.81 (d, *J*=5.6 Hz, 1H), 8.62 (d, *J*=9.2 Hz, 1H), 8.15 (s, 1H), 7.65 (s, 1H), 7.23 (d, *J*=9.2 Hz, 1H), 7.01 (d, *J*=6.0 Hz, 1H), 4.07 (s, 3H), 1.73 (s, 6H). |
| 156 | 395.1 | (DMSO-*d₆*): δ 10.59 (s, 1H), 8.82 (s, 1H), 8.78 (s, 1H), 8.70-8.68 (m, 1H), 8.44-8.43 (m, 1H), 8.14 (s, 1H), 7.42-7.40 (m, 2H), 7.33-7.31 (m, 1H), 7.06-7.03 (m, 1H), 6.86-6.85 (m, 1H), 4.00 (s, 3H), 3.78-3.75 (m, 2H), 3.51-3.45 (m, 2H), 2.34-2.32 (m, 2H), 1.89-1.82 (m, 2H). |
| 157 | 345.1 | (DMSO-*d₆*): δ 10.41 (s, 1H), 8.91 (s, 1H), 8.69-8.61 (m, 2H), 7.53 (s, 1H), 7.19 (d, *J*=7.6 Hz, 1H), 4.04 (s, 3H), 3.34 (s, 2H), 2.51 (s, 4H), 1.79-1.63 (m, 3H), 1.03 (d, *J*=6.0 Hz, 6H). |
| 158 | 421.0 | (DMSO-*d₆*): δ 10.55 (s, 1H), 8.76 (s, 1H), 8.64 (d, *J*=6.0 Hz, 1H), 8.36 (d, *J*=9.2 Hz, 1H), 7.38-7.34 (m, 2H), 7.13-7.10 (m, 2H), 6.86 (d, *J*=8.4 Hz, 1H), 4.32-4.28 (m, 2H), 4.07 (s, 3H), 3.76-3.73 (m, 2H), 3.49-3.43 (m, 2H), 3.22-3.18 (m, 2H), 2.50-2.43 (m, 2H), 1.83-1.80(m, 2H). |
| 159 | 369.1 | (DMSO-*d₆*): δ 10.64 (s, 1H), 8.83 (s, 1H), 8.40 (d, *J*=5.6 Hz, 1H), 7.72 (s, 1H), 7.50 (d, *J*=8.8 Hz, 2H), 7.29 (d, *J*=8.8 Hz, 2H), 6.47 (d, *J*=5.6 Hz, 1H), 4.04 (s, 3H), 3.78-3.75 (m, 2H), 3.52-3.49 (m, 2H), 2.49-2.45 (m, 2H), 1.88-1.87 (m, 2H). |
| 160 | 385.1 | (DMSO-*d₆*): δ 10.77 (s, 1H), 8.87 (s, 1H), 8.79 (d, *J*=9.2 Hz, 1H), 8.42 (d, *J*=6.0 Hz, 1H), 7.76-7.72 (m, 1H), 7.24-7.23 (m, 3H), 6.90 (d, *J*=6.4 Hz, 1H), 4.05 (s, 3H), 3.24 (d, *J*=10.4 Hz, 1H), 2.27-2.21 (m, 1H), 0.99 (d, *J*=6.4 Hz, 3H), 0.71 (d, *J*=6.8 Hz, 3H). |
| 161 | 367.1 | (DMSO-*d₆*): δ 8.76 (d, *J* = 9.2 Hz, 1H), 8.23 (d, *J* = 7.2, 1H), 7.58 (d, *J* = 8.8 Hz, 2H), 7.40 (d, *J* = 8.4 Hz, 2H), 7.21 (d, *J* = 9.2 Hz, 1H), 6.85 (d, *J* = 7.2 Hz, 1H), 4.15 (s, 3H), 2.92 (d, *J* = 10.8 Hz, 1H), 2.44-2.38 (m, 1H), 1.08 (d, *J* = 6.4 Hz, 3H), 0.76 (d, *J* = 6.8 Hz, 3H). |
| 162 | 413.0 | (DMSO-*d₆*): δ 10.46 (s, 1H), 8.89-8.32 (m, 2H), 8.44 (d, J=7.2 Hz, 1H), 7.57-7.52 (m, 1H), 7.35-7.27 (m, 3H), 6.93 (d, J=6.8 Hz, 1H), 4.09 (s, 3H), 3.71-3.65 (m, 4H), 2.41-2.38 (m, 2H), 2.07-1.98 (m, 2H). |
| 163 | 382.1 | (DMSO-*d₆*): δ 10.58 (s, 1H), 9.26 (s, 1H), 8.77 (s, 1H), 8.10 (m, 2H), 7.40 (d, *J* = 8.6 Hz, 2H), 7.31 (d, *J* = 8.6 Hz, 2H), 6.62 (d, *J* = 5.4 Hz, 1H), 4.38 (q, *J* = 7.2 Hz, 2H), 3.82 - 3.71 (m, 2H), 3.48 (t, *J* = 10.4 Hz, 2H), 2.45 (m, 2H), 1.91 - 1.78 (m, 2H), 1.38 (t, *J* = 7.2 Hz, 3H). |
| 164 | 394.1 | (DMSO-*d₆*): δ 10.58 (s, 1H), 8.93 (s, 1H), 8.38-8.37 (m, 1H), 8.28-8.26 (m, 1H), 8.16 (s, 1H), 7.40-7.38 (m, 2H), 7.33-7.31 (m, 2H), 7.25 (s, 1H),7.18-7.16 (m, 1H), 6.81-6.80 (m, 1H), 3.90 (s, 3H), 3.76-3.74 (m, 2H), 3.50-3.45 (m, 2H), 2.46-2.44 (m, 2H), 1.87-1.83 (m, 2H). |
| 165 | 381.2 | (DMSO-*d₆*): δ 10.39 (m, 1H), 8.71-8.38 (m, 3H), 7.55 (d, *J* = 8.6 Hz, 2H), 7.25 (d, *J* = 8.4 Hz, 2H), 7.15 (d, *J* = 8.8 Hz, 1H), 6.53 (d, *J* = 5.2 Hz, 1H), 4.02 (s, 3H), 2.70 (dt, *J* = 13.2, 6.4 Hz, 1H), 1.41 (s, 3H), 0.91 (d, *J* = 6.4 Hz, 3H), 0.56 (d, J = 6.8 Hz, 3H). |
| 166 | 395.1 | (DMSO-*d₆*): δ 10.59-10.54 (m, 2H), 8.78 (s, 1H), 8.70 (s, 1H), 8.58-8.56 (m, 1H), 7.70-7.68 (m, 2H), 7.43-7.38 (m, 3H), 7.20 (s, 1H), 3.96 (s, 3H), 3.77-3.74 (m, 2H), 3.52-3.45 (m, 2H), 2.50-2.45 (m, 2H), 1.90-1.84 (m, 2H). |
| 167 | 357.1 | (DMSO-*d₆*): δ 11.08 (s, 1H), 8.70 (d, *J* = 8.8 Hz, 1H), 8.40 (d, *J* = 5.6 Hz, 1H), 7.95 (s, 1H), 7.55 (s, 1H), 7.00 (d, *J* = 8.8 Hz, 1H), 6.55 (d, *J* = 5.6 Hz, 1H), 4.32-4.30 (d, *J*=11.2 Hz, 1H), 3.99 (s, 3H), 2.47-2.45 (m, 1H), 0.96 (d, *J*=6.8 Hz, 3H), 0.74 (d, *J*=6.4 Hz, 3H). |
| 168 | 395.0 | (DMSO-*d₆*): δ 10.61 (s, 1H), 9.49 (s, 1H), 8.80 (d, *J* = 4.0 Hz, 1H), 8.45 (d, *J* = 5.2 Hz, 1H), 7.46 (d, *J* = 8.8 Hz, 2H), 7.38 (d, *J* = 8.4 Hz, 2H), 7.02 (s, 1H), 6.72 (d, *J* = 5.6 Hz, 1H), 3.97 (s, 3H), 3.79-3.76 (m, 2H), 3.53-3.48 (m, 2H), 2.46 (s, 2H), 1.90-1.84 (m, 2H). |
| 169 | 409.9 | (DMSO-*d₆*): δ 10.11 (s, 1H), 8.76 (d, *J*=5.6 Hz, 1H), 8.60 (d, *J*=8.8 Hz, 1H), 7.53 (d, *J*=8.4 Hz, 1H), 7.21 (d, *J*=9.2 Hz, 1H), 7.15-7.10 (m, 2H), 6.68 (d, *J*=5.6 Hz, 1H), 4.06 (s, 3H), 3.81-3.76 (m, 2H), 3.70-3.66 (m, 2H), 2.37-2.33 (m, 5H), 1.98-1.92(m, 2H). |
| 170 | 394.1 | (DMSO-*d₆*): δ 8.65-8.62 (m, 2H), 7.56 (d, *J*=8.4 Hz, 2H), 7.22-7.18 (m, 3H), 6.59 (d, *J*=5.6 Hz, 1H), 4.13 (s, 3H), 3.08-3.06 (m, 1H), 2.69-2.65 (m, 1H), 1.93-1.89 (m, 1H), 1.73-1.49 (m, 5H), 1.33-1.28 (m, 1H), 1.10-1.05 (m, 1H). |
| 171 | 394.1 | (DMSO-*d₆*): δ 10.49 (s, 1H), 8.82 (d, *J* = 4.6 Hz, 1H), 8.75 (m, 1H), 8.54 (d, *J* = 9.0 Hz, 1H), 7.29 (m, 3H), 7.21 (m, 2H), 7.11 (d, *J* = 9.0 Hz, 1H), 4.42 (s, 2H), 4.00 (s, 3H), 3.70 (d, *J* = 11.6 Hz, 2H), 3.43 (t, *J* = 10.7 Hz, 2H), 2.38 (d, *J* = 13.4 Hz, 2H), 1.81-1.68 (m, 2H). |
| 172 | 424.2 | (DMSO-*d₆*): δ 8.71 (d, *J* = 5.4 Hz, 1H), 8.57 (d, *J* = 8.8 Hz, 1H), 7.53 (d, *J* = 8.6 Hz, 2H), 7.25 (d, *J* = 8.6 Hz, 2H), 7.15 (d, *J* = 8.8 Hz, 1H), 6.54 (d, J = 5.2 Hz, 1H), 4.03 (s, 3H), 3.86 (d, *J* = 9.2 Hz, 1H), 3.75 (d, *J* = 11.4 Hz, 1H), 3.31-3.19 (m, 2H), 2.55-2.50 (m, 1H), 1.52-1.35 (m, 5H), 1.23-1.22 (m, 1H), 0.85-0.82 (m, 1H). |
| 173 | 485.1 | (DMSO-*d₆*): δ 8.75 (s, 1H), 8.71 (d, J = 5.6 Hz, 1H), 8.57-8.55 (m, 1H), 7.50 (d, J = 8.8 Hz, 2H), 7.34-7.23 (m, 7H), 7.15 (d, J = 8.8 Hz, 1H), 6.57 (d, J = 5.2 Hz, 1H), 4.03 (s, 3H), 3.42 (s, 2H), 2.67-2.60 (m, 4H), 2.22-2.16 (m, 2H), 1.88-1.85 (m, 2H). |
| 174 | 385.0 | (DMSO-*d₆*): δ 10.67 (s, 1H), 8.99 (s, 1H), 8.82 (s, 1H), 8.69 (d, J = 9.2 Hz, 1H), 8.41 (d, J = 5.6 Hz, 1H), 8.05 (s, 1H), 7.65 (s, 1H), 7.00 (d, J = 8.8 Hz, 1H), 6.37 (d, J = 5.6 Hz, 1H), 3.99 (s, 3H), 3.70-3.67 (m, 2H), 3.53-3.48 (m, 2H), 2.45-2.34 (m, 4H). |
| 175 | 410.0 | (DMSO-*d₆*): δ 8.62-8.60 (m, 2H), 7.51 (d, *J*=8.8 Hz, 2H), 7.23 (d, *J*=8.8 Hz, 2H), 7.10 (d, *J*=8.8 Hz, 1H), 6.10 (d, *J*=5.6 Hz, 1H), 4.12 (s, 3H), 3.91-3.73 (m, 4H), 2.56-2.44 (m, 2H), 2.35-2.26 (m, 1H), 2.16-2.09 (m, 1H), 2.00-1.81 (m, 2H). |
| 176 | 421.9 | (DMSO-*d₆*): δ 10.30 (s, 1H), 8.83 (d, *J*=6.4 Hz, 1H), 8.72 (d, *J*=8.8 Hz, 1H), 7.73 (d, *J*=8.8 Hz, 2H), 7.33 (d, *J*=8.8 Hz, 3H), 6.74 (d, *J*=6.0 Hz, 1H), 4.35 (s, 2H), 4.11 (s, 3H), 2.55-2.52 (m, 2H), 2.34-2.30 (m, 2H), 1.66-1.63 (m, 2H), 1.41-1.36 (m, 2H). |
| 177 | 367.1 | (DMSO-*d₆*): δ 10.70 (s, 1H), 9.66 (s, 2H), 8.86 (s, 1H), 8.43 (d, J = 6.8 Hz, 1H), 7.51 (d, J = 8.4 Hz, 2H), 7.41 (d, J = 8.4 Hz, 2H), 7.08 (s, 1H), 6.23 (d, J = 7.2 Hz, 1H), 4.04 (s, 3H), 2.90 (d, J = 10.4 Hz, 1H), 2.34-2.25 (m, 1H), 0.98 (d, J = 6.8 Hz, 3H), 0.68 (d, J = 6.8 Hz, 3H). |
| 178 | 413.2 | (DMSO-*d₆*): δ 10.66 (s, 1H), 9.56 (s, 1H), 8.88 (s, 1H), 8.43 (d, *J*=6.0 Hz, 1H), 7.49 (t, *J*=8.4 Hz, 1H), 7.42-7.32 (m, 2H), 7.04 (s, 1H), 6.28-6.26 (m, 1H), 4.01 (s, 3H), 3.79-3.76 (m, 2H), 3.53-3.48 (m, 2H), 2.50-2.45 (m, 2H), 1.93-1.86 (m, 2H). |
| 179 | 381.2 | (DMSO-*d₆*): δ 13.85-13.81 (m, 1H), 11.34 (s, 1H), 10.77 (s, 1H), 9.70 (s, 1H), 8.94-8.86 (m, 1H), 8.45 (d, J = 7.2 Hz, 1H), 7.54 (d, J = 8.0 Hz, 2H), 7.43 (d, J = 8.4 Hz, 2H), 7.10 (s, 1H), 6.64 (d, J = 7.2 Hz, 1H), 4.06 (s, 3H), 1.95-1.87 (m, 1H), 1.59-1.38 (m, 2H), 0.93-0.89 (m, 6H). |
| 180 | 419.2 | (DMSO-*d₆*): δ 10.54 (s, 1H), 8.79 (s, 1H), 8.45 (d, J = 9.2 Hz, 1H), 7.44-7.39 (m, 3H), 7.35-7.31 (m, 3H), 3.98 (s, 3H), 3.73-3.70 (m, 2H), 3.54-3.51 (m, 2H), 2.42-2.39 (m, 2H), 1.95-1.90 (m, 2H). |
| 181 | 413.1 | (DMSO-*d₆*): δ 10.48 (s, 1H), 9.71 (s, 1H), 8.86-8.85 (m, 1H), 8.54 (d, J = 7.2 Hz, 1H), 7.62-7.60 (m, 1H), 7.38-7.35 (m, 2H), 7.14 (s, 1H), 6.81 (d, J = 7.2 Hz, 1H), 4.06 (s, 3H), 3.72-3.64 (m,4H), 2.46-2.39 (m, 2H), 2.09-2.02 (m, 2H). |
| 182 | 381.2 | (DMSO-*d₆*): δ 10.71 (s, 1H), 9.99 (s, 1H), 8.85-8.81 (m, 2H), 8.37 (d, J = 4.2,1H), 7.45-7.40(m, 5H), 6.75 (d, J = 8.2,1H), 4.05(s, 3H), 3.43-3.33 (m,2H), 1.89 (m, 1H), 1.52-1.42 (m, 1H), 0.9 (m, 6H). |
| 183 | 385.2 | (DMSO-*d₆*): δ 10.76 (s, 1H), 9.64 (s, 1H), 8.51 (d, *J* = 6.8 Hz, 1H), 8.05 (s, 1H), 7.80 (s, 1H), 7.06 (s, 1H), 6.75 (d, *J* = 6.8 Hz, 1H), 4.04 (s, 3H), 3.79-3.70 (m, 2H), 3.51-3.46 (m, 2H), 2.46-2.41 (m, 4H). |
| 184 | 417.3 | (DMSO-*d₆*): δ 10.79 (s, 1H), 9.68 (s, 1H), 8.99 (s, 1H), 8.50-8.49 (m, 1H), 7.33 (d, J = 9.2Hz, 2H), 7.10 (s, 1H), 6.35 (d, J = 5.6Hz, 1H), 4.05 (s, 3H), 3.53-3.49 (m, 1H), 1.94-1.87 (m, 1H), 1.58-1.51 (m, 1H), 1.47-1.42 (m, 1H), 0.91 (dd, J1 = 6.8Hz, J2 = 8.4Hz, 6H). |
| 185 | 403.1 | (DMSO-*d₆*): δ 10.76 (s, 1H), 9.68 (s, 1H), 8.98 (s, 1H), 8.52-8.46 (m, 1H), 7.37-7.32 (m, 2H), 7.11 (s, 1H), 6.34 (d, J = 10.0 Hz, 1H), 4.06 (s, 3H), 2.96 (d, J = 10.8 Hz, 1H), 2.34-2.25 (m, 1H), 0.98 (d, J = 6.4 Hz, 3H), 0.74 (d, J = 6.8 Hz, 3H). |
| 186 | 417.3 | (DMSO-*d₆*): δ 10.57 (s, 1H), 9.56 (s, 1H), 9.41 (s, 1H), 8.77 (s, 1H), 8.58 (d, J = 5.2 Hz, 1H), 7.08 (s, 1H), 3.98 (s, 3H), 3.77-3.70 (m, 1H), 1.85-1.82 (m, 2H), 1.40-1.33(m, 1H), 0.90-0.85 (m, 6H). |
| 187 | 368.2 | (DMSO-*d₆*): δ 11.15 (s, 1H),10.66 (s, 1H), 9.62 (s, 1H), 8.73 (s, 1H), 7.63 (d, *J*=8.4 Hz, 2H), 7.41 (d, *J*=9.2 Hz, 2H), 6.95 (s, 1H), 4.03 (s, 3H), 2.85 (d, *J*=10.8 Hz, 1H), 2.32-2.28 (m, 1H), 0.96 (d, *J*=6.4 Hz, 3H), 0.67 (d, *J*=6.4 Hz, 3H). |
| 188 | 382.3 | (DMSO-*d₆*): δ 11.05 (s, 1H), 10.71 (s, 1H), 9.61 (s, 1H), 8.71 (s, 1H), 7.63 (d, *J*=8.4 Hz, 2H), 7.41 (d, *J*=8.8 Hz, 2H), 6.95 (s, 1H), 4.03 (s, 3H), 3.44-3.40 (m, 1H), 1.90-1.84 (m, 1H), 1.56-1.39 (m, 2H), 0.91-0.87 (m, 6H). |
| 189 | 343.1 | (DMSO-*d₆*): δ 10.73 (s, 1H), 9.68 (s, 1H), 8.91 (s, 1H), 8.50 (d, J=6.8 Hz, 1H), 7.57-7.53 (m, 1H), 7.39-7.36 (m, 1H), 7.32-7.29 (m, 1H), 7.10 (s, 1H), 6.74 (d, J=7.2 Hz, 1H), 4.05 (s, 3H), 3.44 (s, 2H). |
| 190 | 343.0 | (DMSO-*d₆*): δ 10.74 (s, 1H), 9.71 (s, 1H), 8.93 (s, 1H), 8.50 (d, J=6.8 Hz, 1H), 7.55-7.51 (m, 1H), 7.41 (d, J=11.2 Hz, 1H), 7.32-7.30 (m, 1H), 7.10 (s, 1H), 6.40-6.38 (m, 1H), 4.06 (s, 3H), 3.43 (s, 2H). |
| 191 | 403.1 | (DMSO-*d₆*): δ 9.57 (s, 1H), 8.39 (d, *J*=7.2 Hz, 1H), 7.20 (d, *J*=9.2 Hz, 2H), 7.03 (s, 1H), 6.93 (d, *J*=7.2 Hz, 1H), 4.13 (s, 3H), 4.48 (d, J=6.8 Hz, 1H), 2.85-2.79 (m, 1H), 1.10 (d, *J*=6.4 Hz, 3H), 0.87 (d, *J*=6.4 Hz, 3H). |
| 192 | 330.1 | (DMSO-*d₆*): δ 9.14 (s, 1H), 8.07 (d, J = 5.6 Hz, 1H), 7.97 (s, 1H), 7.41-7.37 (m, 1H), 7.28-7.24 (m, 1H), 7.16-7.14 (m, 1H), 6.20-6.18 (m, 1H), 4.01-3.54 (m, 2H), 3.34 (d, J = 12.8 Hz, 2H), 1.39-1.36 (m, 3H). |
| 193 | 330.1 | (DMSO-*d₆*): δ 8.14 (d, J = 6.8 Hz, 1H), 7.55-7.50 (m, 1H), 7.27-7.22 (m, 2H), 6.81 (d, J = 6.8 Hz, 1H), 4.51-4.46 (m, 2H), 3.56 (s, 2H), 1.55-1.51 (m, 3H). |
| 197 | 403.1 | (DMSO-*d₆*): δ 10.16 (s, 1H), 8.77 (s, 1H), 8.50 (d, J = 5.2 Hz, 1H), 7.92 (s, 1H), 7.72 (d, J = 8.8 Hz, 1H), 7.49 (d, J = 2.6 Hz, 1H), 7.37 (dd, J = 8.6, 2.6 Hz, 1H), 6.68 (d, J = 5.2 Hz, 1H), 4.12 (s, 3H), 3.95 - 3.83 (m, 2H), 3.72-3.61 (m, 2H), 2.43 (m, 2H), 2.21-2.09 (m, 2H). |
| 198 | 428.1 | (CD₃OD): δ 8.65 (d, J = 5.2 Hz, 1H), 8.58 (d, J = 9.2 Hz, 1H), 7.87-7.83 (m, 1H), 7.12-7.08 (m, 3H), 6.67 (d, J = 5.2 Hz, 1H), 4.12 (s, 3H), 3.99-3.87 (m, 2H), 3.50-3.36 (m, 3H), 2.34-2.29 (m, 1H), 1.78 (d, J = 12.0 Hz, 1H), 1.48-1.21 (m, 3H). |
| 199 | 340.9 | (DMSO-*d₆*): δ 8.41 (s, 1H), 8.20 (s, 1H), 7.74 (d, J = 8.4 Hz, 2H), 7.34 (d, J = 8.8 Hz, 2H), 3.95 (s, 3H), 2.82-2.81 (m, 1H), 2.03-2.25 (m, 1H), 0.97-0.95 (m, 3H), 0.68-0.66 (m, 3H). |
| 200 | 368.1 | (DMSO-*d₆*): δ 10.64 (m, 1H), 9.33 (s, 1H), 8.84 (s, 1H), 8.24 - 8.11 (m, 2H), 7.46 (d, J = 8.6 Hz, 2H), 7.37 (d, J = 8.6 Hz, 2H), 6.69 (d, J = 5.4 Hz, 1H), 4.02 (s, 3H), 3.82 (m, 2H), 3.54 (t, J = 10.4 Hz, 2H), 2.51 (m, 2H), 1.92 (dd, J = 17.2, 6.8 Hz, 2H). |
| 201 | 400.1 | (DMSO-*d₆*): δ 10.82 (s, 1H), 8.90 (s, 1H), 8.74-8.73 (m, 1H), 8.56-8.54 (m, 1H), 7.70-7.68 (m, 1H), 7.27-7.24 (m, 1H), 7.17-7.12 (m, 2H), 6.67-6.65 (m, 1H), 4.04 (s, 3H), 3.77-3.74 (m, 1H), 1.88-1.83 (m, 1H), 1.55-1.40 (m, 2H), 0.93-0.88 (m, 6H). |
| 202 | 346.1 | (DMSO-*d₆*): δ 10.45 (s, 1H), 8.69 (d, J= 9.0 Hz, 1H), 8.45 (d, J= 5.6 Hz, 1H), 8.27 (s, 1H), 7.22 (d, J= 6.8 Hz, 1H), 7.00 (d, J= 9.0 Hz, 1H), 6.52 (d, J= 5.6 Hz, 1H), 4.23-4.15 (m, 1H), 4.02 (s, 3H), 3.03 (dd, J= 9.2, 6.6 Hz, 1H), 2.88-2.81 (m, 1H), 2.73-2.64 (m, 2H), 2.33-2.24 (m, 1H), 1.88 (d, J = 6.0 Hz, 1H), 1.33-1.28 (m, 1H), 1.24 (d, J = 11.7 Hz, 6H). |
| 203 | 380.2 | (MeOD-*d₄*): δ 8.64-8.62 (m, 2H), 7.39-7.37 (m, 1H), 7.15-7.13 (d, *J* =9.2 Hz, 1H), 7.07-7.02 (m, 2H), 6.79 (d, *J* =6.0 Hz, 1H), 4.13 (s, 3H), 2.92-2.81 (m, 2H), 2.32-2.26 (m, 1H), 1.90-1.72 (m, 3H), 1.56 (s, 3H). |
| 204 | 351.0 | (DMSO-*d₆*): δ 8.59 (d, J=6.0 Hz, 1H), 8.32 (d, J=9.2 Hz, 1H), 7.28 (d, J=6.0 Hz, 2H), 6.97 (d, J=9.2 Hz, 2H), 6.70 (d, J=8.0 Hz, 1H), 4.24-4.20 (m, 2H), 4.13 (s, 3H), 3.36 (s, 2H), 3.24-3.21 (m, 2H). |
| 205 | 347.2 | (DMSO-*d₆*): δ 10.38 (s, 1H), 8.78-8.77 (m, 1H), 8.68 (s, 1H), 8.48-8.46 (m, 1H), 7.12-7.10 (m, 1H), 7.01-7.00 (m, 1H), 5.20-5.18 (m, 1H), 4.06 (s, 3H), 3.36-3.26 (m, 1H), 2.89-2.86 (m, 2H), 2.77-2.76 (m, 1H), 2.38-2.34 (m, 1H), 2.02-2.00 (m, 1H), 1.25 (d, *J* = 13.8 Hz, 6H). |
| 206 | 428.1 | (CD₃OD): δ 8.65 (d, *J* = 5.6 Hz, 1H), 8.58 (d, *J* = 9.2 Hz, 1H), 7.87-7.83 (m, 1H), 7.12-7.08 (m, 3H), 6.67 (d, *J* = 5.2 Hz, 1H), 4.12 (s, 3H), 3.99-3.87 (m, 2H), 3.50-3.33 (m, 3H), 2.35-2.32 (m, 1H), 1.78 (d, *J* = 12.0 Hz, 1H), 1.33-1.24 (m, 3H). |
| 207 | 369.1 | (DMSO-*d₆*): δ 10.54 (s, 1H), 10.07 (s, 1H), 8.75 (s, 1H), 8.40 (s, 1H), 8.34 (s, 1H), 7.79-7.77 (m, 2H), 7.39-7.36 (m, 2H), 3.95 (s, 3H), 3.76-3.73 (m, 2H), 3.51-3.45 (m, 2H), 2.49-2.42 (m, 2H), 1.88-1.81 (m, 2H). |

### Example 208 Preparation of Compounds 208 and 209

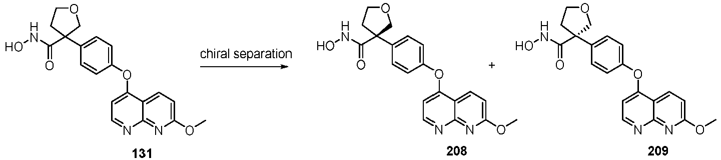

### Step: compound 131 was split to obtain compound 208 (isomer 1) and compound 209 (isomer 2)

Compound **131** (92 mg) was split through a chiral column, column model CHIRALCEL OZ-H(OZH0CE-RJ014), 0.46 cm I.D.×25 cm L, mobile phase: Hexane/EtOH/TFA/DEA = 50/50/0.1/0.05, resulting in compound **208** (20 mg) and compound **209** (22 mg);
Compound **208:** peak time 9.688 min; ee% > 99, LCMS (ESI): m/z 382.20 [M+H]⁺; ¹H NMR (400 MHz, DMSO-d₆): δ 10.80 (s, 1H), 8.97 (s, 1H), 8.79-8.80 (m, 1H), 8.65 (d, *J* = 9.2 Hz, 1H), 7.53 (d, *J* = 8.8 Hz, 2H), 7.36 (d, *J* = 8.8 Hz, 2H), 7.24 (d, *J* = 9.2 Hz, 1H), 6.66 (d, *J* = 5.6 Hz, 1H), 4.58 (d, *J =* 8.8 Hz, 1H), 4.04 (s, 3H), 3.94-3.86 (m, 3H), 2.97-2.59 (m, 1H), 2.32-2.27 (m, 1H).

Compound **209:** peak time 14.023 min; ee% > 99, LCMS (ESI): m/z 382.20 [M+H]⁺; ¹H NMR (600 MHz, DMSO-d*₆*): δ 10.73 (s, 1H), 8.97 (s, 1H), 8.74 (d, *J* = 3.6 Hz, 1H), 8.61 (d, *J* = 5.6 Hz, 1H), 7.48-7.46 (m, 2H), 7.32 (d, *J* = 8.8 Hz, 2H), 7.21 (d, *J* = 6.0 Hz, 1H), 6.63 (d, *J* = 4.0 Hz, 1H), 4.50 (d, *J* = 5.6 Hz, 1H), 4.07 (s, 3H), 3.87-3.78 (m, 3H), 2.90-2.86 (m, 1H), 2.23-2.18 (m, 1H).

### Example 209 Preparation of Compounds 210 and 211

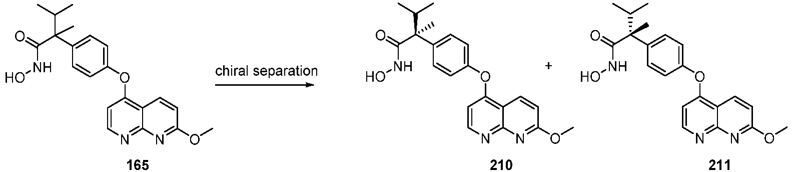

### Step: compound 165 was split to obtain compound 210 (isomer 1) and compound 211 (isomer 2)

Compound **165** (420 mg) was split through a chiral column, column model DAICELCHIRALPAK^{®}OZ, 250*25mm 10 µm, mobile phase: methanol(+0.1% 7.0 mol/L ammonia methanol), resulting in compound **210** (101.74 mg) and compound **211** (117.52 mg);
Compound **210:** peak time 4.478 min; ee% > 86, LCMS (ESI): m/z 382.00 [M+H]⁺; (DMSO-*d₆):* δ 10.38 (s, 1H), 8.71-8.67 (m, 2H), 8.58 (d, *J* = 9.2 Hz, 1H), 7.35 (d, *J* = 8.8 Hz, 2H), 7.25 (d, *J* = 8.8 Hz, 2H), 6.53 (d, *J* **=** 5.2 Hz, 1H), 4.04 (s, 3H), 2.74-2.67 (m, 1H), 1.41 (s, 3H), 0.91 (d, *J* = 6.4 Hz, 3H), 0.56 (d, *J* = 6.8 Hz, 3H);
Compound **211:** peak time 5.364 min; ee% > 97, LCMS (ESI): m/z 382.00 [M+H]⁺; (DMSO-*d₆):* δ 10.38 (s, 1H), 8.71-8.68 (m, 2H), 8.58 (d, *J* = 8.8 Hz, 1H), 7.55 (d, *J* = 8.8 Hz, 2H), 7.25 (d, *J* = 8.8 Hz, 2H), 7.15 (d, *J* = 8.8 Hz, 1H), 6.53 (d, *J* = 5.2 Hz, 1H), 4.04 (s, 3H), 2.74-2.67 (m, 1H), 1.41 (s, 3H), 0.91 (d, *J* = 6.8 Hz, 3H), 0.56 (d, *J* = 7.2 Hz, 3H).

### Example 210 Preparation of Compounds 212 and 213

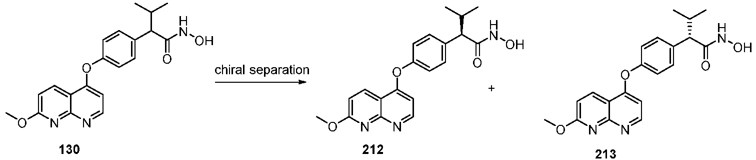

### Step: compound 130 was split to obtain compound 212 (isomer 1) and compound 213 (isomer 2)

Compound **130** (100 mg) was split through a chiral column, column model DAICELCHIRALPAK^{®}IG, 250*25mm 10 µm, mobile phase: methanol(+0.1% 7.0 mol/L ammonia methanol), resulting in compound **212** (18.03 mg) and compound **213** (24.55 mg);
Compound **212:** peak time 4.970 min; ee% > 99, LCMS (ESI): m/z 368.05 [M+H]⁺; (MeOD-*d₄*): δ 8.63-8.60 (m, 2H), 7.54 (d, *J* = 8.4 Hz, 2H), 7.20 (d, *J* = 8.4 Hz, 2H), 7.10 (d, *J* = 9.2 Hz, 1H), 6.56 (d, *J* = 1.6 Hz, 1H), 4.12 (s, 3H), 2.90 (d, *J* = 10.0 Hz, 1H), 2.43-2.37 (m, 1H), 1.07 (d, *J* = 6.4 Hz, 3H), 0.77 (d, *J* = 6.8 Hz, 3H);
Compound **213:** peak time 5.665 min; ee% > 99, LCMS (ESI): m/z 368.00 [M+H]⁺; (MeOD-*d₄):* δ 8.63-8.60 (m, 2H), 7.54 (d, *J* = 8.8 Hz, 2H), 7.20 (d, *J* = 8.4 Hz, 2H), 7.10 (d, *J* = 8.8 Hz, 1H), 6.57 (d, *J* = 5.6 Hz, 1H), 4.12 (s, 3H), 2.90 (d, *J* = 11.2 Hz, 1H), 2.43-2.37 (m, 1H), 1.07 (d, *J* = 6.4 Hz, 3H), 0.77 (d, *J* = 6.8 Hz, 3H).

### Example 211 Preparation of Compounds 214 and 215

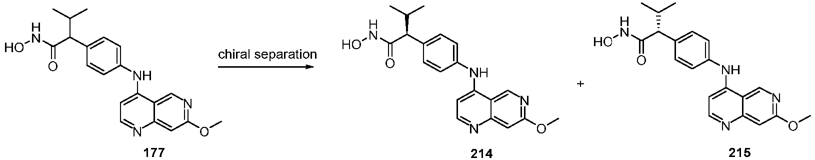

### Step: compound 177 was split to obtain compound 214 (isomer 1) and compound 215 (isomer 2)

Compound **177** (500 mg) was split through a chiral column, column model DAICELCHIRALPAK^{®}OM, 250*25mm 10 µm, mobile phase: methanol (+0.1% 7.0 mol/l ammonia methanol), resulting in compound **214** (112 mg) and compound **215** (232 mg);
Compound **214:** peak time 2.174 min; ee% > 99, LCMS (ESI): m/z 367.20 [M+H]⁺; (DMSO-*d₆*): δ 10.65 (s, 1H), 9.44 (d, *J* = 20.4 Hz, 2H), 8.83 (s, 1H), 8.43 (d, *J* = 2.4 Hz, 1H), 7.40 (d, *J* = 8.0 Hz, 2H), 7.32 (d, *J* = 8.0 Hz, 2H), 7.00 (s, 1H), 6.67 (d, *J* = 4.0 Hz, 1H), 3.97 (s, 3H), 2.84 (d, *J* = 10.8 Hz, 1H), 2.33-2.08 (m, 1H), 0.97 (d, *J* = 6.4 Hz, 3H), 0.687 (d, *J* = 6.8 Hz, 3H);
Compound **215:** peak time 7.057 min; ee% > 99, LCMS (ESI): *m*/*z* 367.10 [M+H]⁺; (DMSO-*d₆*): δ 10.70 (s, 1H), 9.66 (s, 2H), 8.86 (s, 1H), 8.43 (d, *J* = 6.8 Hz, 1H), 7.51 (d, *J* = 8.4 Hz, 2H), 7.41 (d, *J* = 8.4 Hz, 2H), 7.08 (s, 1H), 6.23 (d, *J* = 7.2 Hz, 1H), 4.04 (s, 3H), 2.90 (d, *J* = 10.4 Hz, 1H), 2.34-2.25 (m, 1H), 0.98 (d, *J* = 6.8 Hz, 3H), 0.68 (d, *J* = 6.8 Hz, 3H).

### Biological Example

### Evaluation of ENPP1 Inhibitory Activity of Compounds

The ENPP1 inhibitory activity of the compound of the present disclosure was tested in substrate 2',3'-cGAMP detection.

**Experimental objective:** based on the established experimental method, the inhibitory IC₅₀ value of the compound in the present application on ENPP1 was detected using 2',3'-cGAMP as the substrate. STF-32 was used as a positive control compound (STF-32 is derived from literature: Cell Chemical Biology 2020 (27), 1347-1358).

**Experimental reagent:** hENPP1-ECD-His (ChemPartner, cat. 202103121201); AMP-Glo^{™} Assay Kit (Promega, cat: V5011); DMSO (Sigma, cat. D8418-1L); 384-well white color board (PerkinElmer, cat. 6007290), 2'3'- cGAMP (MCE, cat. HY-100564A).

### Experimental method:

Preparation of 1 × reaction solution: 50 mM Tris-HCl (pH 7.5), 10 mM NaCl, 0.5 mM CaCl₂, 1 µM ZnCl₂, 0.01% Tween-20, 0.01% BSA.

Preparation of compound concentration gradient: the tested compound was dissolved in DMSO (dimethyl sulfoxide) to an initial concentration of 10 µM, diluted at 3 times, with 10 concentrations, single well or multi-well testing was set. The initial concentration in the positive control compound STF-32 test was 1 µM, diluted at 3 times, with 10 concentrations, and multi-well testing was set for each concentration. The compound was diluted by gradient in a 384 well plate to a solution with the final concentration of 1000 times (1000, 333.33, 111.11, 37.04, 12.35, 4.12, 1.37, 0.46, 0.15 nM), and then 5 nL of the solution was transferred to a 384 well reaction plate for testing using Echo550. 5 nL of 100% DMSO was transferred from the smallest well (with only substrate, no enzyme, minimum signal value) and the largest well (with substrate, with enzyme, no inhibitor, maximum signal value).

20 nM of 2 × hENPP1-ECD-His protein solution was prepared using 1 × reaction solution.

40 µM of 2 × 2'3'-cGAMP substrate solution was prepared using 1 × reaction solution.

2.5 µL of 2 × protein solution was added to each compound well and the maximum pore of the reaction plate, and 2.5 µL of 1 × reaction solution was added to the smallest well.

The reaction solution was centrifuged at 1000 rpm for 1 minute and incubated at room temperature for 15 minutes.

2.5 µL 2 × 2'3'-cGAMP substrate solution was added to each well of the reaction plate, centrifuged at 1000 rpm for 1 minute, and incubated at room temperature for 60 minutes.

5 µL of R1 solution (from AMP-Glo^{™} Kit) was added to each well of the reaction plate, centrifuged at 1000 rpm for 1 minute and incubated at room temperature for 120 minutes.

10 µL of R2 solution (from AMP-Glo^{™} Kit) was added to each well of the reaction plate, centrifuged at 1000 rpm for 1 minute and incubated at room temperature for 30 minutes.

A multifunctional enzyme-linked immunosorbent assay reader (2104EnVision) was used to detect and the test results were recorded.

### Data analysis

The inhibition rate is calculated using the following formula: inhibition rate% = (maximum signal - compound signal) / (maximum signal - minimum signal) x 100 wherein the "minimum signal" represents the mean value of the negative control well, and the "maximum signal" represents the mean value of the positive control well.

### Fitting the dose-response curve:

Using the log value of concentration as the X-axis and the percentage inhibition rate as the Y-axis, the dose-response curve was fitted using the log(inhibitor) vs. response-variable slope of analysis software GraphPadPrism5 to obtain the inhibitory IC₅₀ value of the compound of the present disclosure on enzyme activity.

The fitting formula is: Y = bottom + (top - bottom) / (1 + 10^((logIC₅₀ - X) * HillSlope)), wherein, the bottom and top are the minimum and maximum values of the fitting, respectively. In this experiment, under the same detection conditions, the sulfonamide compound Ex58 reported in known literature (see WO2019/046778 A1) and the inhibitory IC50 values of compounds A-1 and A-2 on ENPP1 were also detected. As shown in Table 1, the hydroxamic acid compound of the present disclosure exhibits a good inhibitory effect on ENPP1 kinase.

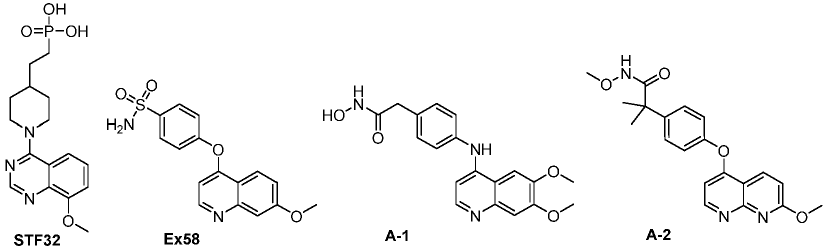

**Table 1. The inhibitory effect of the compound of the present disclosure on ENPP1 kinase**

| Compound | c-GAMP (IC₅₀, nM) | Compound | c-GAMP (IC₅₀, nM) | Compound | c-GAMP (IC₅₀, nM) |
|---|---|---|---|---|---|
| 1 | 1.8 | 28 | 14 | 55 | 4.3 |
| 2 | 1.9 | 29 | 14 | 56 | 2.3 |
| 3 | 317.8 | 31 | 20 | 57 | 513 |
| 4 | 2.62 | 33 | 2.4 | 58 | 41 |
| 5 | 150.2 | 34 | 376 | 59 | 1.8 |
| 7 | 0.2 | 35 | 49 | 60 | 673 |
| 8 | 2.17 | 36 | 24 | 61 | 1.7 |
| 10 | 2.7 | 37 | 2.4 | 62 | 1.6 |
| 11 | 28 | 38 | 4.4 | 63 | 212 |
| 13 | 1.37 | 39 | 205 | 65 | 1.9 |
| 14 | 1.2 | 40 | 4.8 | 66 | 1.8 |
| 15 | 19 | 41 | 3 | 67 | 1.7 |
| 16 | 64 | 42 | 1 | 68 | 4.8 |
| 17 | 0.71 | 43 | 5.3 | 69 | 1.9 |
| 18 | 343 | 44 | 2.7 | 70 | 1.7 |
| 19 | 2.9 | 45 | 3.1 | 71 | 1.8 |
| 20 | 324 | 46 | 16 | 72 | 2.2 |
| 21 | 3.1 | 47 | 4.5 | 73 | 8.6 |
| 22 | 1.8 | 48 | 2.7 | 74 | 7.8 |
| 23 | 20 | 49 | 1.4 | 75 | 112 |
| 24 | 36 | 50 | 289 | 76 | 289 |
| 25 | 1.9 | 51 | 2.5 | 77 | 1.2 |
| 26 | 1.3 | 52 | 2.2 | 78 | 1.8 |
| 27 | 2.1 | 53 | 2.9 | 79 | 1.9 |
| 80 | 1.9 | 54 | 1.6 | 81 | 1.9 |
| 82 | 1.7 | 83 | 4.4 | 84 | 6.1 |
| 87 | 1.8 | 85 | 4.8 | 86 | 3.7 |
| 90 | 4.5 | 88 | 1.5 | 89 | 24 |
| 93 | 1.5 | 91 | 1.2 | 92 | 169 |
| 96 | 6.8 | 94 | 257 | 95 | 70 |
| 99 | 63 | 97 | 1.5 | 98 | 125 |
| 102 | 389 | 100 | 1.6 | 101 | 1.1 |
| 111 | 1.1 | 103 | 21 | 107 | 2.2 |
| 112 | 2.5 | 109 | 2.3 | 110 | 2.2 |
| 116 | 21 | 113 | 219 | 114 | 162 |
| 117 | 2.1 | 118 | 1.5 | 124 | 1.6 |
| 125 | 0.63 | 123 | 1.2 | 127 | 1.6 |
| 128 | 2.5 | 126 | 21 | 130 | 6.9 |
| 133 | 2.1 | 129 | 5.7 | 131 | 1.5 |
| 132 | 6.6 | 134 | 3.3 | 135 | 7.1 |
| 136 | 4.4 | 137 | 2.6 | 138 | 6 |
| 139 | 2.3 | 140 | 6.3 | 141 | 1.4 |
| 144 | 259 | 146 | 13 | 143 | 4.7 |
| 145 | 4.2 | 142 | 13 | 148 | 2.8 |
| 151 | 70 | 147 | 1.5 | 150 | 7.2 |
| 154 | 3.5 | 152 | 396 | 156 | 2.7 |
| 157 | 81 | 155 | 28 | 159 | 8.1 |
| 160 | 15 | 158 | 9.9 | 162 | 3 |
| 163 | 2.9 | 161 | 16 | 165 | 23 |
| 166 | 10 | 164 | 3.4 | 168 | 4 |
| 169 | 2.3 | 167 | 103 | 171 | 1.8 |
| 172 | 3.1 | 170 | 18 | 174 | 2.1 |
| 175 | 1.9 | 173 | 1.6 | 177 | 7.9 |
| 178 | 1.2 | 176 | 2.9 | 180 | 1.6 |
| 181 | 1.9 | 179 | 1 | 183 | 6.8 |
| 184 | 2.2 | 182 | 1.7 | 186 | 3.8 |
| 187 | 27 | 185 | 3.6 | 189 | 1.8 |
| 190 | 3.9 | 188 | 4.6 | 192 | 1.4 |
| 193 | 2.2 | 191 | 11 | 195 | 81 |
| 196 | 130 | 194 | 284 | 198 | 61 |
| 199 | 317 | 197 | 111 | 201 | 11 |
| 202 | 3.3 | 200 | 1.8 | 204 | 24 |
| 205 | 39 | 203 | 1.8 | 207 | 9.3 |
| 208 | 3.4 | 206 | 12 | 209 | 3.2 |
| Ex58 | 152 | A-1 | 89 | A-2 | 3283 |

As shown in Table 1, the hydroxamic acid compound of the present disclosure is a good ENPP1 inhibitor with an IC₅₀ value of ≤ 5 µM, preferably an IC₅₀ value of ≤ 1 µM, more preferably an IC₅₀ value of ≤ 0.5 µM, or even more preferably an IC₅₀ value of ≤ 0.125 µM, and most preferably an IC₅₀ value of ≤ 0.1 µM. The inhibitory activity of ENPP1 by the hydroxamic acid compound of the present disclosure is significantly better than that of compound A-2.

### Preliminary pharmacokinetic testing experiment

1. 6 healthy ICR mice, male, weighing 30-35 g, were randomly divided into 2 groups with 3 mice in each group. The tested compound was administered by gavage (5 mg/kg) and intravenous injection (1 mg/kg), respectively:
   fasting for 12 hours before the experiment and drinking water freely. Feeding uniformly 4 hours after administration.
2. Blood collection time and sample processing

Intragastric administration: 0.25 h, 0.5 h, 1.0 h, 2.0 h, 3.0 h, 4.0 h, 6.0 h, 8.0 h and 24 h after administration.

Intravenous administration: 5 min, 0.25 h, 0.5 h, 1.0 h, 2.0 h, 4.0 h, 6.0 h, 8.0 h and 24 h after administration.

Blood was collected continuously, with 3 animals collected at each time point. Plasma collection and processing: at the above set time points, 30-40 µL of venous blood was collected from the posterior venous plexus of the mouse eyeball, placed in EDTA-K2 test tube, centrifuged at 3500 rpm for 10 min, plasma was separated, and frozen at -20°C in a refrigerator.

### 3. Sample testing and data analysis

LC/MS/MS method was used to determine the concentration of compounds in rat plasma. The pharmacokinetic parameters after administration were calculated using a non-compartmental model using Phoenix 8.3 software (Pharsight, USA).

### 4. Experimental results

The compound in the present invention has a high oral bioavailability, and the oral bioavailability is greater than 20%, as shown in Example 2 and Example 110. It can be seen that the hydroxamic acid compound of the present disclosure has a significant advantage in oral absorption compared to known phosphate compounds. The pK data of some compounds are shown in Table 2.

**Table 2. Oral administration (5 mg/kg) pharmacokinetics of the compound of the present disclosure**

| Compound | T_{1/2} (hr) | Cmax (ng/mL) | AUC 0-t (hr*ng/mL) | F% |
|---|---|---|---|---|
| 2 | 3.26 | 736 | 619 | 57.2 |
| 42 | 1.30 | 1488 | 887 | 46.0 |
| 68 | 2.44 | 244 | 161 | 20.2 |
| 107 | 2.0 | 640 | 463 | 23.5 |
| 110 | 3.9 | 2370 | 1589 | 46.3 |

## Claims

1. A compound of general formula (I): 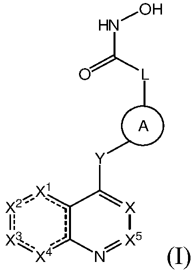
or a pharmaceutically acceptable salt thereof, wherein,
- - - - : is a single or double bond;
X is N or CR⁰;
X¹ is N, O, S, CR¹ or a bond;
X² is N, O, S, NR² or CR²;
X³ is N, O, S, NR³ or CR³;
X⁴ is N, O, S, NR⁴ or CR⁴;
X⁵ is N or CR⁵;
Y is O, S, -S(=O)-, -S(=O)₂-, -C(=O)-, NR⁶ or CR⁷R⁸;
L is a bond, NR^{a}, -NR^{x}-CHR^{Y}- or (CR⁹R¹⁰)ₘ; m is 1 or 2;
R⁰ and R⁵ are each independently selected from hydrogen, halogen, CN, OH, NO₂, NR^{b}R^{c}, C₁-C₆ alkyl, C₃-C₆ cycloalkyl, -SO₂R^{a}, -C(O)OR^{a}, -C(O)NR^{b}R^{c} and C₁-C₆ alkoxy; wherein each of the alkyl, the cycloalkyl and the alkoxy is optionally substituted with halogen;
R¹ and R² are each independently selected from hydrogen, halogen, CN, OH, NO₂, NR^{b}R^{c}, C₁-C₄ alkyl, C₃-C₆ cycloalkyl and C₁-C₄ alkoxy; wherein each of the alkyl, the cycloalkyl and the alkoxy is optionally substituted with halogen;
R³ and R⁴ are each independently selected from hydrogen, halogen, CN, OH, NO₂, NR^{b}R^{c}, =O, C₁-C₄ alkyl, C₃-C₆ cycloalkyl and C₁-C₄ alkoxy; wherein each of the alkyl, the cycloalkyl and the alkoxy is optionally substituted with halogen;
R⁶ is selected from hydrogen, C₁-C₄ alkyl and C₃-C₆ cycloalkyl; wherein each of the alkyl and the cycloalkyl is optionally substituted with halogen;
R⁷ and R⁸ are each independently selected from hydrogen, halogen, OH, CN, NO₂, NR^{a}, C₁-C₆ alkyl, C₃-C₆ cycloalkyl and C₁-C₆ alkoxy; wherein each of the alkyl, the cycloalkyl and the alkoxy is optionally substituted with halogen; or R⁷ and R⁸ together with the carbon atom bound thereto form C₃-C₆ cycloalkyl optionally substituted with halogen, or form 4- to 12-membered heterocycloalkyl containing 1-2 heteroatoms selected from N, NR^{a}, O and S(O)ₚ;
R⁹ and R¹⁰ are each independently selected from hydrogen, halogen, CN, OH, NO₂, NR^{a}R^{b}, C₁-C₈ alkyl, C₃-C₈ cycloalkyl, C₂-C₈ alkenyl, C₂-C₈ alkynyl, C₁-C₈ alkoxy, 4- to 12-membered heterocycloalkyl containing carbon atoms and 1-2 heteroatoms selected from N, NR^{a}, O and S(O)ₚ, 6- to 10-membered aryl and 5- to 10-membered heteroaryl; each of the alkyl, the alkenyl, the alkynyl, the alkoxy, the cycloalkyl and the heterocycloalkyl is substituted with 0-3 substituents independently selected from halogen, hydroxyl and CN; wherein each of the aryl and the heteroaryl is substituted with 0-3 substituents independently selected from halogen, Ci-C₄ alkyl, C₃-C₆ cycloalkyl and OR^{a};
or R⁹ and R¹⁰ together with the carbon atom bound thereto form C₃-C₆ cycloalkyl optionally substituted with halogen, or form 4- to 12-membered heterocycloalkyl containing 1-2 heteroatoms selected from N, NR^{a}, O and S(O)ₚ;
ring A is selected from C₄-C₁₀ cycloalkyl, 4- to 12-membered heterocycloalkyl, 6- to 10-membered aryl and 5- to 10-membered heteroaryl; wherein, ring A is optionally substituted one or more times by substituents independently selected from halogen, CN, OH, NO₂, NR^{b}R^{c}, C₁-C₄ alkyl, C₁-C₄ alkoxy, C₃-C₆ cycloalkyl, 4- to 7-membered heterocycloalkyl, 6- to 10-membered aryl, 5- to 10-membered heteroaryl and C₁-C₃ haloalkyl;
or
L together with ring A forms C₄-C₈ cycloalkyl, 5- to 10-membered partially saturated heterocycle containing at least one heteroatom selected from N, O and S, 6- to 10-membered aryl or 5- to 7-membered heteroaryl; wherein the cycloalkyl, the heterocycle, the aryl and the heteroaryl are optionally substituted one or more times by substituents independently selected from halogen, C₁-C₄ alkyl and C₁-C₄ alkoxy; or
Y together with ring A forms 5- to 10-membered partially saturated heterocycle, 6- to 10-membered aryl or 5- to 10-membered heteroaryl; wherein the heterocycle, the aryl and the heteroaryl are optionally substituted one or more times by substituents independently selected from halogen, C₁-C₄ alkyl and C₁-C₄ alkoxy;
R^{a}, R^{b} and R^{c} are each independently selected from hydrogen, C₁-C₄ alkyl, C₃-C₆ cycloalkyl and benzyl; wherein each of the alkyl and the cycloalkyl is optionally substituted with halogen; or R^{b} and R^{c} together with the nitrogen atom bound thereto form 3- to 6-membered heterocycloalkyl optionally substituted with halogen;
R^{x} and R^{y} are each independently selected from hydrogen and C₁-C₄ alkyl, or R^{x} and R^{y} together with the carbon and nitrogen atoms bound thereto form 4- to 8-membered heterocycloalkyl; and
p is 1 or 2;
with the proviso that when X₁ to X₄ are each CRⁿ, Y is O, S, -S(=O)-, -S(=O)₂-, -C(=O)- or CH₂; wherein Rⁿ is R¹ for X¹, R² for X², R³ for X³, and R⁴ for X⁴.

2. The compound of general formula (I) of claim 1: 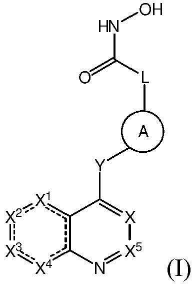
or a pharmaceutically acceptable salt thereof, wherein,
- - - - is a single or double bond;
X is N or CR⁰;
X¹ is N, O, S, CR¹ or a bond;
X² is N, O, S, NR² or CR²;
X³ is N, O, S, NR³ or CR³;
X⁴ is N, O, S, NR⁴ or CR⁴;
X⁵ is N or CR⁵;
Y is O, S, -S(=O)-, -S(=O)₂-, -C(=O)-, NR⁶ or CR⁷R⁸;
L is a bond, NR^{a} or (CR⁹R¹⁰)ₘ; m is 1 or 2;
R⁰ and R⁵ are each independently selected from hydrogen, halogen, CN, OH, NO₂, NR^{b}R^{c}, C₁-C₆ alkyl, C₃-C₆ cycloalkyl, -SO₂R^{a}, -C(O)OR^{a}, -C(O)NR^{b}R^{c} and C₁-C₆ alkoxy; wherein each of the alkyl, the cycloalkyl and the alkoxy is optionally substituted with halogen;
R¹ and R² are each independently selected from hydrogen, halogen, CN, OH, NO₂, NR^{b}R^{c}, C₁-C₄ alkyl, C₃-C₆ cycloalkyl and C₁-C₄ alkoxy; wherein each of the alkyl, the cycloalkyl and the alkoxy is optionally substituted with halogen;
R³ and R⁴ are each independently selected from hydrogen, halogen, CN, OH, NO₂, NR^{b}R^{c}, =O, C₁-C₄ alkyl, C₃-C₆ cycloalkyl and C₁-C₄ alkoxy; wherein each of the alkyl, the cycloalkyl and the alkoxy is optionally substituted with halogen;
R⁶ is selected from hydrogen, C₁-C₄ alkyl and C₃-C₆ cycloalkyl; wherein each of the alkyl and the cycloalkyl is optionally substituted with halogen;
R⁷ and R⁸ are each independently selected from hydrogen, halogen, OH, CN, NO₂, NR^{a}, C₁-C₆ alkyl, C₃-C₆ cycloalkyl and C₁-C₆ alkoxy; wherein each of the alkyl, the cycloalkyl and the alkoxy is optionally substituted with halogen; or R⁷ and R⁸ together with the carbon atom bound thereto form C₃-C₆ cycloalkyl optionally substituted with halogen, or form 4- to 12-membered heterocycloalkyl containing 1-2 heteroatoms selected from N, NR^{a}, O and S(O)ₚ;
R⁹ and R¹⁰ are each independently selected from hydrogen, halogen, CN, OH, NO₂, NR^{a}R^{b}, C₁-C₈ alkyl, C₃-C₈ cycloalkyl, C₂-C₈ alkenyl, C₂-C₈ alkynyl, C₁-C₈ alkoxy, 4- to 12-membered heterocycloalkyl containing carbon atoms and 1-2 heteroatoms selected from N, NR^{a}, O and S(O)ₚ, 6- to 10-membered aryl and 5- to 10-membered heteroaryl; each of the alkyl, the alkenyl, the alkynyl, the alkoxy, the cycloalkyl and the heterocycloalkyl is substituted with 0-3 substituents independently selected from halogen, hydroxyl and CN; wherein each of the aryl and the heteroaryl is substituted with 0-3 substituents independently selected from halogen, Ci-C₄ alkyl, C₃-C₆ cycloalkyl and OR^{a};
or R⁹ and R¹⁰ together with the carbon atom bound thereto form C₃-C₆ cycloalkyl optionally substituted with halogen, or form 4- to 12-membered heterocycloalkyl containing 1-2 heteroatoms selected from N, NR^{a}, O and S(O)ₚ;
ring A is selected from C₄-C₁₀ cycloalkyl, 4- to 12-membered heterocycloalkyl, 6- to 10-membered aryl and 5- to 10-membered heteroaryl; wherein, ring A is optionally substituted one or more times by substituents independently selected from halogen, CN, OH, NO₂, NR^{b}R^{c}, C₁-C₄ alkyl, C₁-C₄ alkoxy, C₃-C₆ cycloalkyl, 4- to 7-membered heterocycloalkyl, 6- to 10-membered aryl, 5- to 10-membered heteroaryl and C₁-C₃ haloalkyl;
or
L together with ring A forms C₄-C₈ cycloalkyl, 5- to 10-membered partially saturated heterocycle containing at least one heteroatom selected from N, O and S, 6- to 10-membered aryl or 5- to 7-membered heteroaryl; wherein the cycloalkyl, the heterocycle, the aryl and the heteroaryl are optionally substituted one or more times by substituents independently selected from halogen, C₁-C₄ alkyl and C₁-C₄ alkoxy;
R^{a}, R^{b} and R^{c} are each independently selected from hydrogen, C₁-C₄ alkyl and C₃-C₆ cycloalkyl; wherein each of the alkyl and the cycloalkyl is optionally substituted with halogen; or R^{b} and R^{c} together with the nitrogen atom bound thereto form 3- to 6-membered heterocycloalkyl optionally substituted with halogen; and
p is 1 or 2;
with the proviso that when X₁ to X₄ are each CRⁿ, Y is O, S, -S(=O)-, -S(=O)₂-, -C(=O)- or CH₂; wherein Rⁿ is R¹ for X¹, R² for X², R³ for X³, and R⁴ for X⁴.

3. The compound or the pharmaceutically acceptable salt thereof of the preceding claims, wherein
R⁰ and R⁵ are each independently selected from hydrogen, halogen, CN, OH, NO₂, NH₂, C₁-C₄ alkyl, C₃-C₆ cycloalkyl, -SO₂Rₐ, -C(O)OR^{a}, -C(O)NR^{b}R^{c} and C₁-C₄ alkoxy, wherein each of the alkyl, the cycloalkyl and the alkoxy is optionally with halogen; preferably, R⁰ and R⁵ are each independently selected from hydrogen, CN, halogen and C₁-C₄ alkyl;
R¹ and R² are each independently selected from H, halogen, CN, OH, NO₂, NH₂, C₁-C₄ alkyl, C₁-C₃ alkoxy and C₁-C₃ haloalkyl, preferably from H, halogen, C₁-C₄ alkyl and C₁-C₃ alkoxy;
R³ and R⁴ are each independently selected from H, halogen, CN, OH, NO₂, NH₂, =O, Ci-C₄ alkyl, C₃-C₆ cycloalkyl, C₁-C₃ alkoxy and C₁-C₃ haloalkyl, preferably from H, halogen, OH, =O, C₁-C₄ alkyl, C₃-C₆ cycloalkyl, C₁-C₃ alkoxy and C₁-C₃ haloalkyl;
R⁶ is selected from hydrogen, C₁-C₄ alkyl and C₃-C₆ cycloalkyl, preferably from hydrogen and C₁-C₄ alkyl; and
R⁷ and R⁸ are each independently selected from hydrogen, C₁-C₄ alkyl, C₃-C₆ cycloalkyl and C₁-C₄ alkoxy, preferably from hydrogen and C₁-C₄ alkyl.

4. The compound or the pharmaceutically acceptable salt thereof of the preceding claims, wherein,
at least one of X¹, X², X³ and X⁴ is independently selected from N, O, S and, if any, NRⁿ; preferably, at least one of X¹, X², X³ and X⁴ is independently selected from N and, if any, NRⁿ; wherein Rⁿ is R² for X², R³ for X³, and R⁴ for X⁴;
R¹ is hydrogen or halogen;
R² is selected from hydrogen, halogen, C₁-C₃ alkyl and C₁-C₃ alkoxy;
R³ is selected from hydrogen, halogen, OH, =O, NR^{b}R^{c}, C₁-C₃ alkyl and C₁-C₃ alkoxy;
R⁴ is selected from hydrogen, halogen and C₁-C₃ alkyl; and
R⁵ is selected from hydrogen, halogen, CN and C₁-C₆ alkyl.

5. The compound or the pharmaceutically acceptable salt thereof of any one of the preceding claims, wherein, at least one of X¹, X², X³ and X⁴ is independently selected from N; or, only one of X¹, X², X³ and X⁴ is independently selected from N and, if any, NRⁿ.

6. The compound or the pharmaceutically acceptable salt thereof of the preceding claims, wherein, X² is N or NR²; preferably, X² is N; more preferably, X² is N, and X³ is CR³; further preferably, X² is N, X³ is CR³, and X⁴ is CR⁴; most preferably, X² is N, X³ is CR³, X⁴ is CR⁴, and X¹ is CR¹.

7. The compound or the pharmaceutically acceptable salt thereof of the preceding claims, wherein, X⁴ is N or NR⁴; preferably, X⁴ is N; more preferably, X⁴ is N, and X³ is CR³; further preferably, X⁴ is N, X³ is CR³, and X² is CR²; most preferably, X⁴ is N, X³ is CR³, X² is CR², and X¹ is CR¹.

8. The compound or the pharmaceutically acceptable salt thereof of claim 1 or 2, wherein,
X¹ is a bond;
R² is selected from hydrogen, halogen, C₁-C₃ alkyl and C₁-C₃ alkoxy;
R³ is selected from hydrogen, OH and halogen;
R⁴ is selected from hydrogen, halogen, C₁-C₃ alkyl, C₁-C₃ alkoxy, C₃-C₄ cycloalkyl and C₁-C₃ haloalkyl; and
R⁵ is selected from hydrogen, halogen, CN and C₁-C₆ alkyl.

9. The compound or the pharmaceutically acceptable salt thereof of any one of the preceding claims, wherein, when X¹ is a bond, at least two of X², X³ and X⁴ are independently selected from N and NR¹¹;
for example, X¹ is a bond, X³ is N, and X⁴ is NR⁴; preferably, X¹ is a bond, X³ is N, X⁴ is NR⁴, and X² is CR²;
or
for example, X¹ is a bond, X³ is N, and X² is NR²; preferably, X¹ is a bond, X³ is N, X² is NR², and X⁴ is CR⁴.

10. The compound or the pharmaceutically acceptable salt thereof of claim 1 or 2, wherein
X¹ is CR¹, X² is CR², X³ is CR³, and X⁴ is CR⁴;
Y is O, S, -S(=O)-, -S(=O)₂-, -C(=O)- or CH₂;
R¹ is hydrogen or halogen;
R² is selected from hydrogen, halogen, OH and C₁-C₃ alkoxy;
R³ is selected from hydrogen, halogen, OH, C₁-C₃ alkyl and C₁-C₃ alkoxy;
R⁴ is selected from hydrogen, halogens and C₁-C₃ alkoxy;
R⁵ is selected from hydrogen, halogen, CN and C₁-C₆ alkyl.

11. The compound or the pharmaceutically acceptable salt thereof of any one of the preceding claims, wherein, 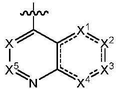 is selected from 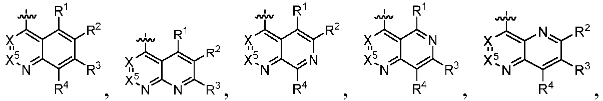 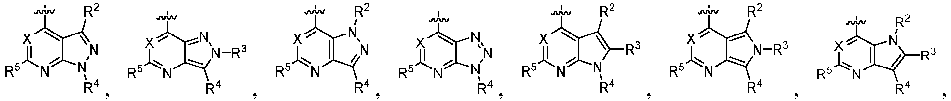 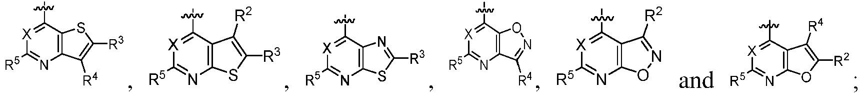 preferably, from

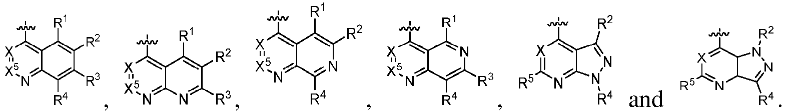

12. The compound or the pharmaceutically acceptable salt thereof of any one of the preceding claims, wherein,
Y is O, NR⁶ or CR⁷R⁸, preferably O, NH, N(C₁-C₃ alkyl), CH₂, CH(C₁-C₃ alkyl) or C(C₁-C₃ alkyl)(C₁-C₃ alkyl); or
Y is O, S, -S(=O)-, -S(=O)₂-, -C(=O)-, -NH-, -N(CH₃)-, -N(ethyl)-, -N(propyl)-, - N(cyclopropyl)-, -N(cyclobutyl)-, -N(cyclopentyl)-, -CH₂-, -CHF-, -CH(OH)-, -CH(CH₃)-, - CH(OCH₃)-, -CH(OEt)- or -C(CH₃)₂-; or
Y is O, S, -S(=O)-, -S(=O)₂-, -C(=O)-, -NH-, -N(CH₃)-, -CH(CH₃)- or -CH₂-.

13. The compound or the pharmaceutically acceptable salt thereof of any one of the preceding claims, wherein L is a bond, NR^{a}, -NR^{x}-CHR^{y}-, CR⁹R¹⁰ or (CR⁹R¹⁰)₂, wherein R^{a} is selected from hydrogen, C₁-C₄ alkyl, C₃-C₆ cycloalkyl and benzyl, preferably from hydrogen, C₁-C₃ alkyl and benzyl; R^{x} and R^{y} are each independently selected from hydrogen and C₁-C₄ alkyl, or R^{x} and R^{y} together with the carbon and nitrogen atoms bound thereto form 4- to 8-membered heterocycloalkyl; R⁹ and R¹⁰ are each independently selected from hydrogen, halogen, OH, NH₂, C₁-C₈ alkyl, C₃-C₈ cycloalkyl, C₁-C₈ alkoxy, 4- to 8-membered heterocycloalkyl containing carbon atoms and 1-2 heteroatoms selected from N, NR^{a}, O and S(O)ₚ; or R⁹ and R¹⁰ together with the carbon atom bound thereto form C₃-C₆ cycloalkyl, or 4-to 8-membered heterocycloalkyl containing 1-2 heteroatoms selected from N, NR^{a}, O and S(O)ₚ;
or, L is CR⁹R¹⁰, wherein R⁹ and R¹⁰ are each independently selected from hydrogen, halogen, CN, OH, NO₂, NH₂, C₁-C₆ alkyl, C₃-C₆ cycloalkyl, and C₁-C₆ alkoxy, 4- to 7-membered heterocycloalkyl containing carbon atoms and 1-2 heteroatoms selected from N, O and S, or R⁹ and R¹⁰ together with the carbon atom bound thereto form C₃-C₆ cycloalkyl substituted with 0-3 halogens (for example, cyclopropane, cyclobutane, cyclopentane or cyclohexane) or 4- to 12-membered heterocycloalkyl containing 1-2 heteroatoms selected from N, NR^{a}, O and S(O)ₚ (for example, oxocyclobutane, azacyclobutane, thiocyclobutane, tetrahydrofuran ring, pyrrolidine, tetrahydrothiophene ring, pyrazolidine, imidazolidine, thiazolidine, oxazolidine, piperidine ring, tetrahydropyran ring, piperazine ring, hexahydropyrimidine ring, oxazinane, pyridazinane, morpholine ring, thiomorpholine ring, thiophane, tetrahydropyran ring, thiocyclohexane, oxocycloheptane, azacycloheptane or thiocycloheptane);
or, L is CR⁹R¹⁰, wherein R⁹ and R¹⁰ are each independently selected from hydrogen, halogen, CN, OH, C₁-C₆ alkyl and C₁-C₆ alkoxy, or R⁹ and R¹⁰ together with the carbon atom bound thereto form C₃-C₆ cycloalkyl (for example, cyclopropane, cyclobutane, cyclopentane or cyclohexane);
or, L is selected from -CH₂-, -CH(OH)-, -CHF-, -CH(CH₃)-, -CH(OCH₃)-, -CH(OEt)-, - C(CH₃)₂-, -CH(CH(CH₃)₂)-, -CH(CH₂CH(CH₃)₂)-, -C(CH₂CH(CH₃)₂)₂-, -C(CH₂CH₃)₂-,

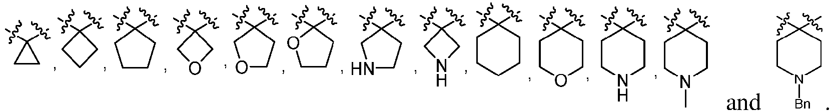

14. The compound or the pharmaceutically acceptable salt thereof of any one of the preceding claims, wherein ring A is selected from C₄-C₁₀ cycloalkyl, 4- to 12-membered heterocycloalkyl, 6- to 10-membered aryl and 5- to 10-membered heteroaryl, and the ring A is optionally substituted one or more times by substituents independently selected from halogen, CN, OH, NH₂, C₁-C₄ alkyl, C₁-C₄ alkoxy and C₁-C₃ haloalkyl, preferably substituents selected from halogen, CN, C₁-C₄ alkyl, and C₁-C₄ alkoxy; or
ring A is selected from cyclopentane, cyclohexane, pyrazolidine, imidazolidine, pyrrole ring, furan ring, thiophene ring, pyrazole ring, imidazole ring, tetrahydrofuran ring, pyrrolidine, tetrahydrothiophene ring, pyran ring, tetrahydropyran ring, piperidine ring, hexahydropyrimidine, piperazine ring, benzene ring, pyridine ring, pyrimidine ring, naphthalene ring, quinoline ring, isoquinoline ring, indole ring, isoindole ring, indazole ring and benzimidazole ring; preferably selected from cyclopentane, cyclohexane, pyrrolidine, pyrrole ring, pyridine ring, pyrimidine ring, benzene ring, pyridine ring, piperazine ring, imidazole ring, pyrazole ring and naphthalene ring; wherein, ring A is optionally substituted one or more times, for example once or twice, by substituents independently selected from halogen, CN, OH, NO₂, NH₂, C₁-C₃ alkyl, C₁-C₃ alkoxy and C₁-C₃ haloalkyl; preferably, ring A is optionally substituted one or more times, for example once or twice, by substituents independently selected from F, Cl, CN, OH, NO₂, NH₂, C₁-C₃ alkyl and C₁-C₃ alkoxy.

15. The compound or the pharmaceutically acceptable salt thereof of any one of the preceding claims, wherein, ring A is selected from: 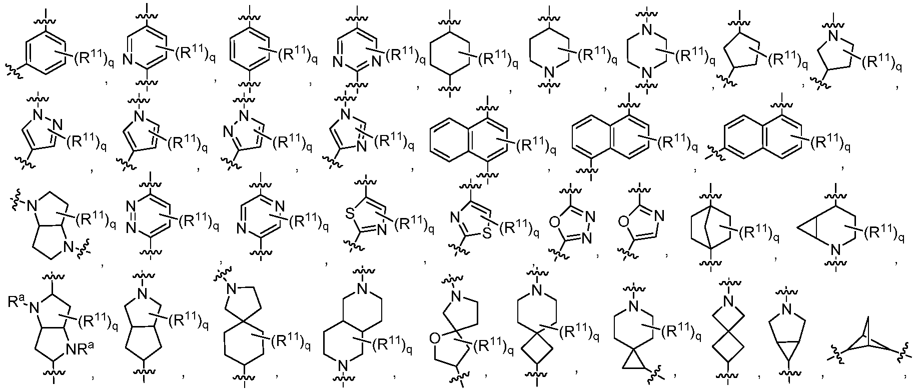 wherein, each R¹¹ is capable of being the same or different, and independently selected from halogen, CN, OH, NO₂, NH₂, C₁-C₃ alkyl, C₁-C₃ alkoxy and C₁-C₃ haloalkyl, preferably from F, Cl, CN, C₁-C₃ alkyl and C₁-C₃ alkoxy; and q is 0, 1 or 2.

16. The compound or the pharmaceutically acceptable salt thereof of any one of the preceding claims, wherein, L together with ring A forms dihydrobenzofuran ring, dihydroisobenzofuran ring, dihydrobenzothiophene ring, dihydroindole ring, dihydroisoindole ring, dihydrobenzimidazole ring, dihydrobenzoxazole ring, dihydrobenzothiazole ring, dihydrobenzopyran ring, dihydroisobenzopyran ring, tetrahydroquinoline ring, tetrahydroisoquinoline ring, 2,3-dihydro-1H-pyrrolo[2,3-b]pyridine ring, 2,3-dihydro-1H-pyrrolo[3,2-b]pyridine ring, tetrahydropyridino[3,4-b]pyrazine ring, dihydroindene ring, tetrahydronaphthalene ring, 2,3-dihydrofurano[2,3-b]pyridine ring or 2,3-dihydrofurano[3,2-h]pyridine ring, and each of which is optionally substituted one or more times by substituents independently selected from halogen, C₁-C₄ alkyl and C₁-C₄ alkoxy;
preferably, L together with ring A forms dihydrobenzofuran ring, dihydroisobenzofuran ring, dihydroindole ring, dihydroisoindole ring, dihydrobenzopyran ring, dihydroisobenzopyran ring, tetrahydroquinoline ring, tetrahydroisoquinoline ring, dihydroindene ring, tetrahydronaphthalene ring, 2,3-dihydrofurano[2,3-b]pyridine ring or 2,3-dihydro-1H-pyrrolo[2,3-b]pyridine ring, and each of which is optionally substituted one or more times by substituents independently selected from halogen, C₁-C₄ alkyl and C₁-C₄ alkoxy.

17. The compound or the pharmaceutically acceptable salt thereof of any one of the preceding claims, wherein, Y together with ring A forms 7- to 10-membered partially saturated bicyclic heterocycle, naphthyl or 7- to 10-membered bicyclic heteroaryl, wherein the heterocycle, the aryl and the heteroaryl are optionally substituted one or more times by substituents independently selected from halogen, C₁-C₄ alkyl and C₁-C₄ alkoxy;
preferably, Y together with ring A forms a group selected from the following groups: 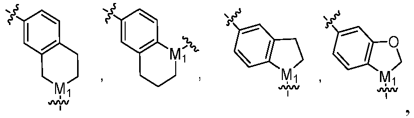
wherein, each M₁ is independently N, CH or C(C₁-C₄ alkyl);
and more preferably, Y together with ring A forms a group selected from the following groups:

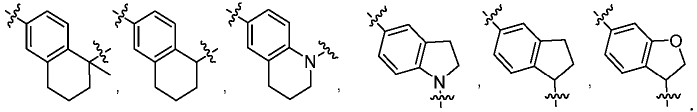

18. A compound or pharmaceutically acceptable salt thereof, wherein, the compound is selected from:
| Compo und | Structure | Compo und | Structure | Compo und | Structure |
|---|---|---|---|---|---|
| 1 | 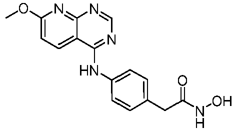 | 2 | 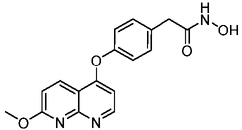 | 3 | 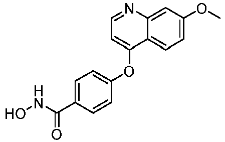 |
| 4 | 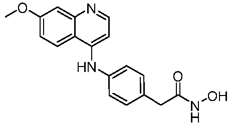 | 7 | 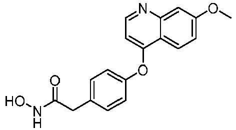 | 10 | 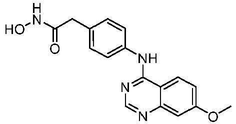 |
| 5 | 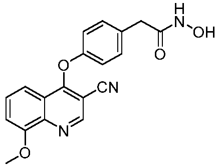 | 8 | 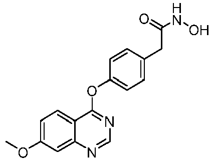 | 11 | 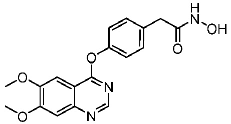 |
| 6 | 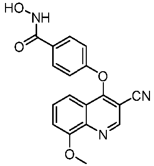 | 9 | 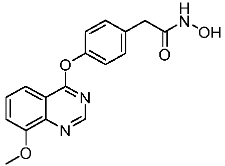 | 12 | 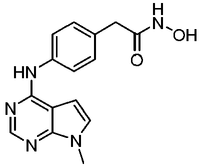 |
| 13 | 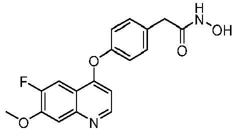 | 16 | 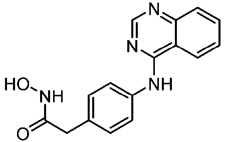 | 19 | 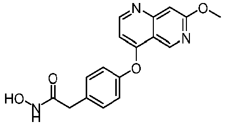 |
| 14 | 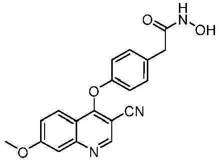 | 17 | 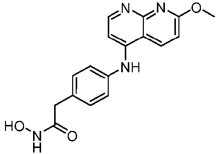 | 20 | 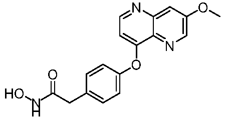 |
| 15 | 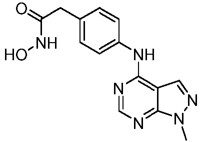 | 18 | 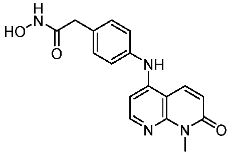 | 21 | 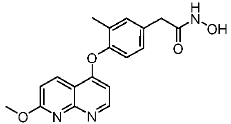 |
| 22 | 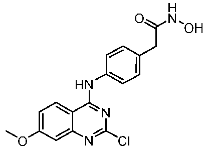 | 25 | 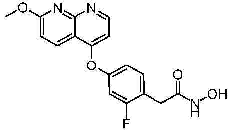 | 28 | 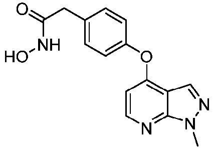 |
| 23 | 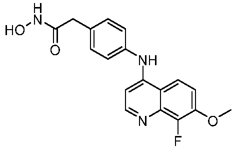 | 26 | 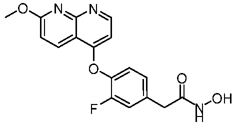 | 29 | 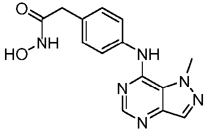 |
| 24 | 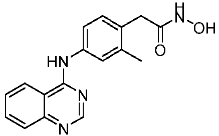 | 27 | 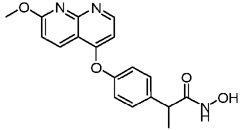 | 30 | 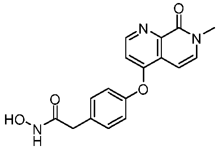 |
| 31 | 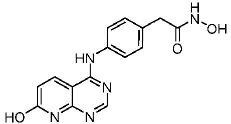 | 34 | 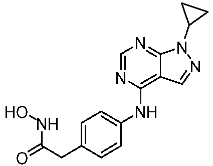 | 37 | 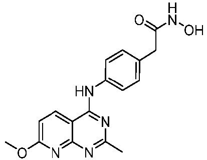 |
| 32 | 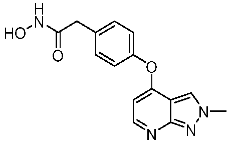 | 35 | 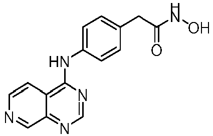 | 38 | 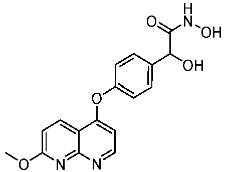 |
| 33 | 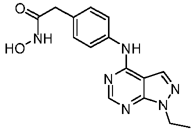 | 36 | 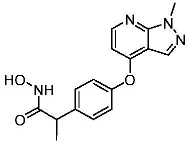 | 39 | 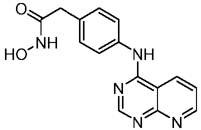 |
| 40 | 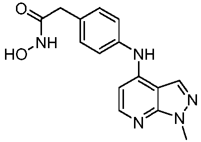 | 43 | 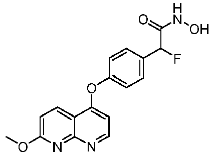 | 46 | 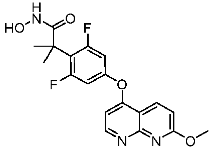 |
| 41 | 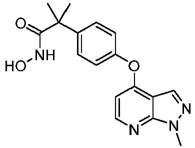 | 44 | 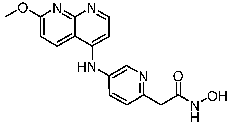 | 47 | 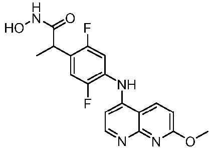 |
| 42 | 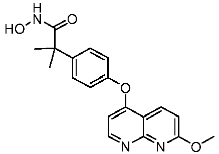 | 45 | 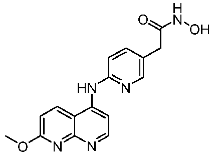 | 48 | 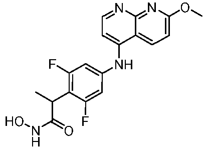 |
| 49 | 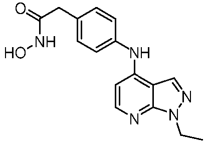 | 52 | 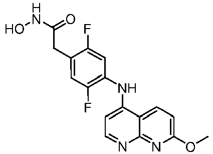 | 55 | 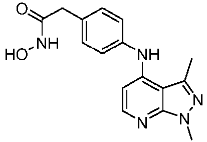 |
| 50 | 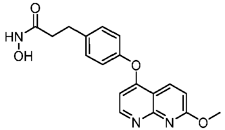 | 53 | 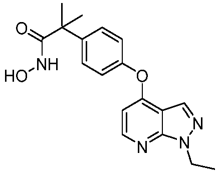 | 56 | 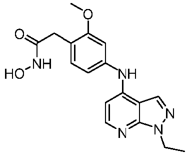 |
| 51 | 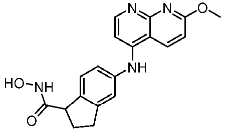 | 54 | 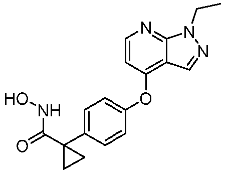 | 57 | 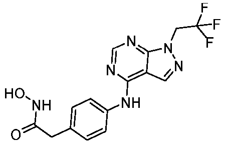 |
| 58 | 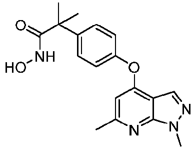 | 61 | 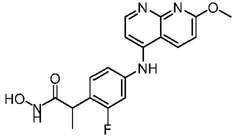 | 64 | 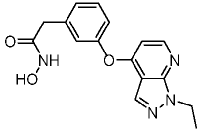 |
| 59 | 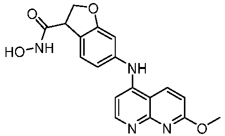 | 62 | 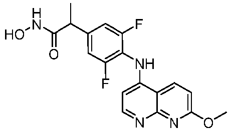 | 65 | 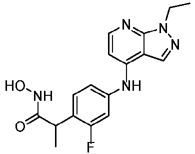 |
| 60 | 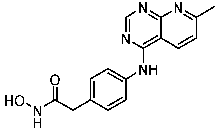 | 63 | 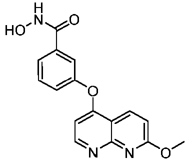 | 66 | 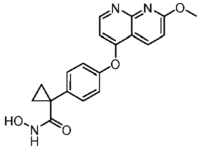 |
| 67 | 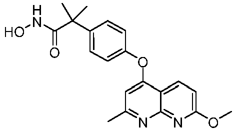 | 70 | 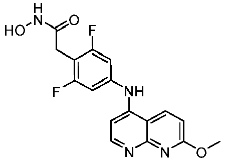 | 73 | 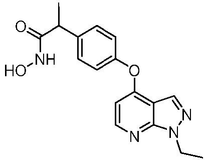 |
| 68 | 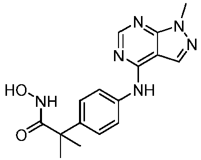 | 71 | 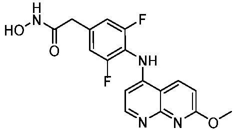 | 74 | 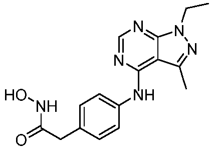 |
| 69 | 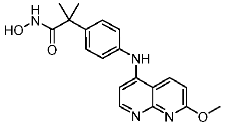 | 72 | 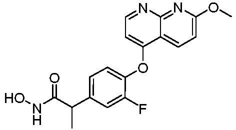 | 75 | 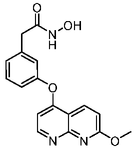 |
| 76 | 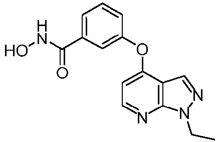 | 79 | 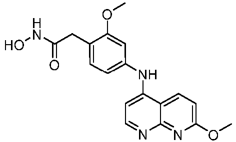 | 82 | 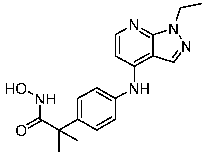 |
| 77 | 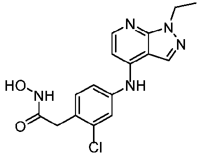 | 80 | 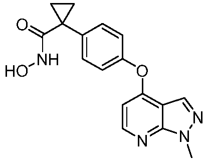 | 83 | 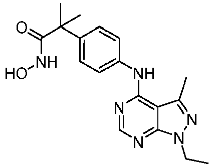 |
| 78 | 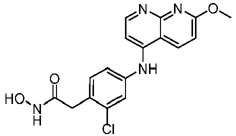 | 81 | 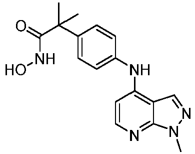 | 84 | 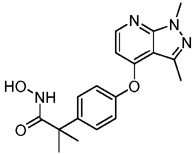 |
| 85 | 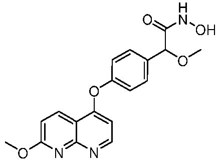 | 86 | 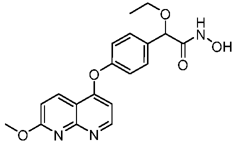 | 87 | 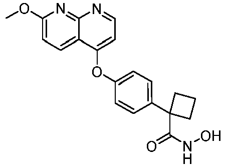 |
| 88 | 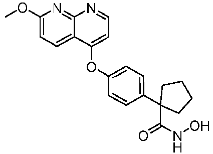 | 89 | 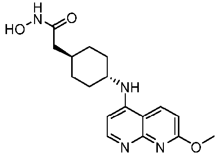 | 90 | 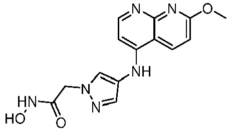 |
| 91 | 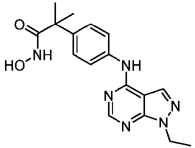 | 92 | 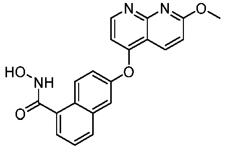 | 97 | 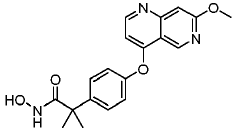 |
| 93 | 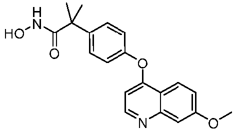 | 94 | 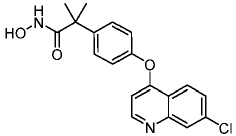 | 98 | 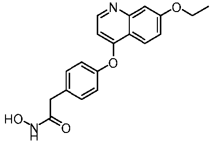 |
| 95 | 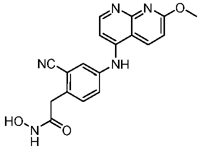 | 96 | 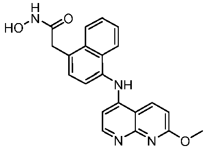 | 99 | 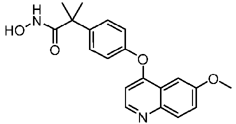 |
| 101 | 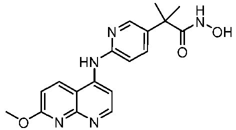 | 102 | 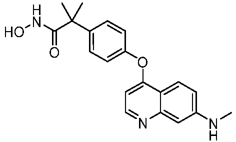 | 107 | 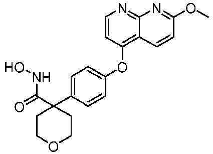 |
| 103 | 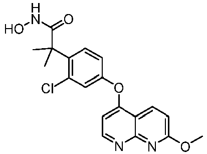 | 104 | 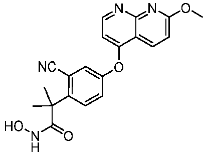 | 108 | 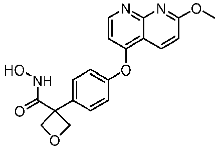 |
| 105 | 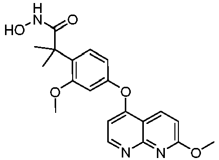 | 106 | 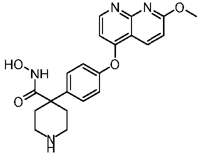 | 109 | 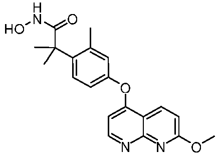 |
| 111 | 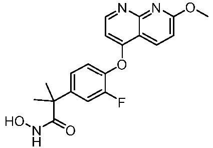 | 112 | 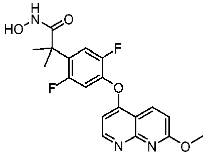 | 110 | 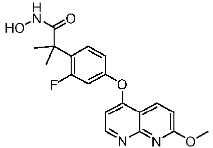 |
| 113 | 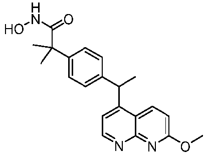 | 114 | 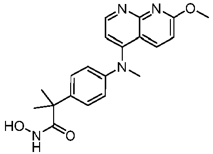 | 117 | 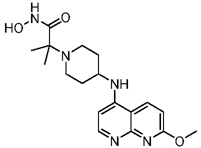 |
| 115 | 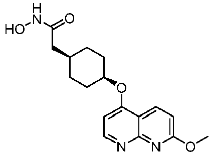 | 116 | 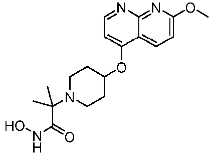 | 118 | 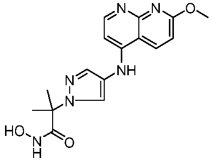 |
| 119 | 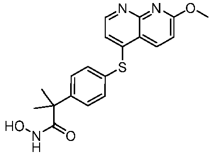 | 120 | 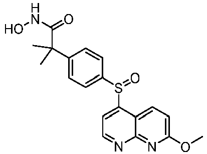 | 125 | 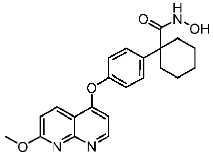 |
| 121 | 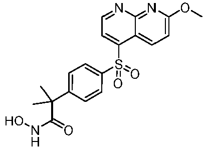 | 122 | 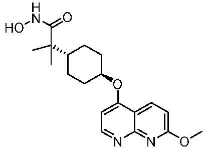 | 126 | 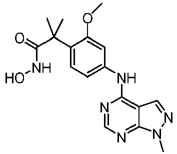 |
| 123 | 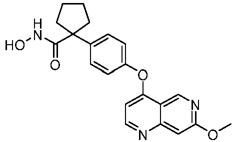 | 124 | 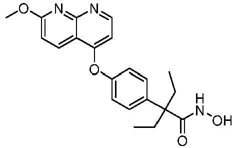 | 127 | 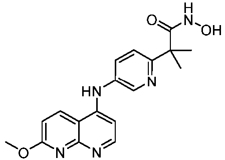 |
| 128 | 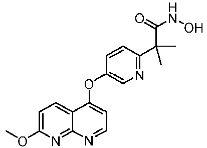 | 129 | 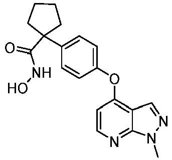 | 130 | 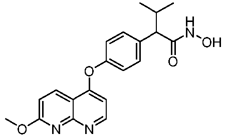 |
| 131 | 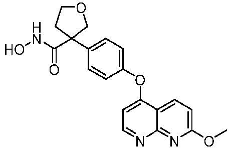 | 132 | 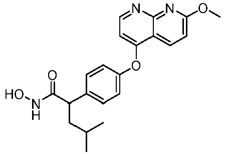 | 137 | 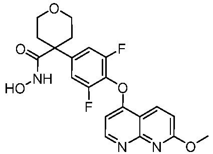 |
| 133 | 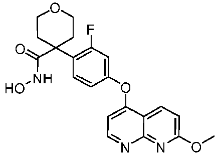 | 134 | 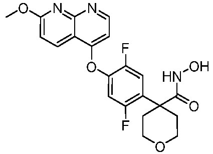 | 138 | 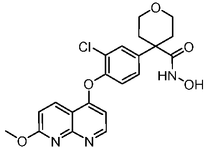 |
| 135 | 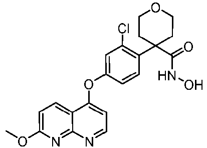 | 136 | 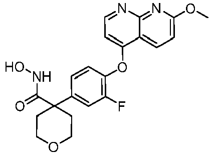 | 139 | 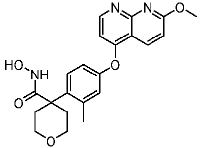 |
| 140 | 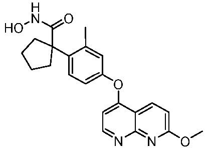 | 141 | 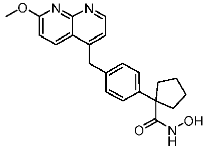 | 142 | 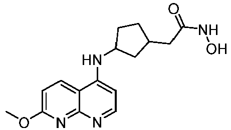 |
| 143 | 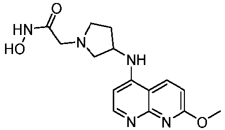 | 144 | 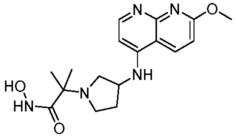 | 149 | 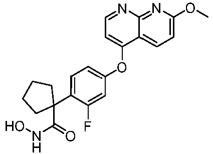 |
| 145 | 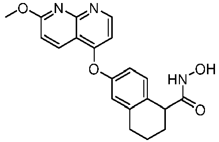 | 146 | 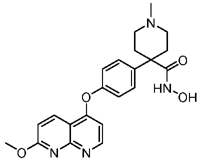 | 150 | 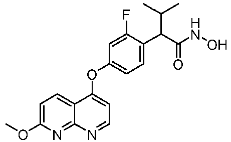 |
| 147 | 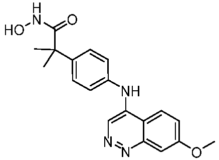 | 148 | 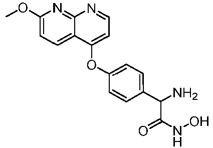 | 151 | 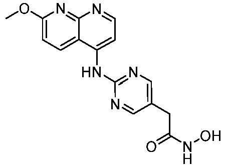 |
| 152 | 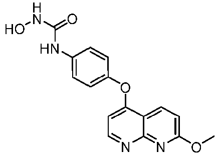 | 153 | 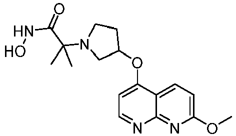 | 154 | 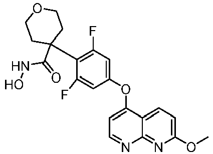 |
| 155 | 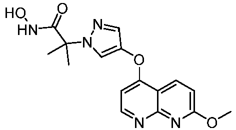 | 156 | 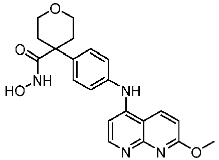 | 157 | 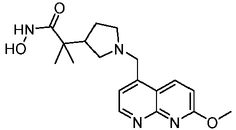 |
| 158 | 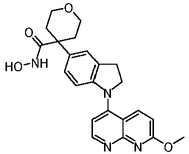 | 159 | 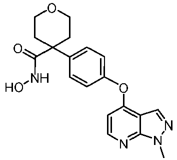 | 160 | 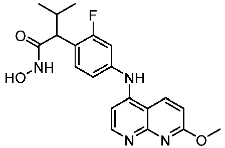 |
| 161 | 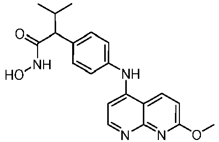 | 162 | 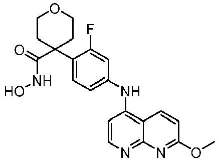 | 167 | 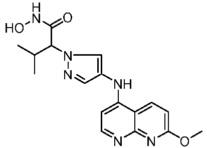 |
| 163 | 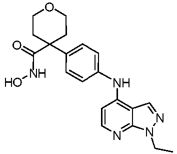 | 164 | 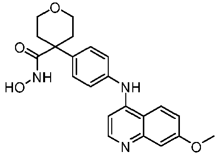 | 168 | 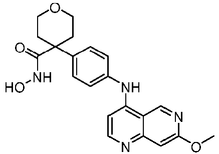 |
| 165 | 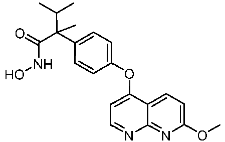 | 166 | 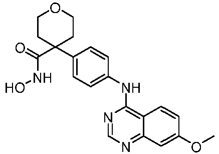 | 169 | 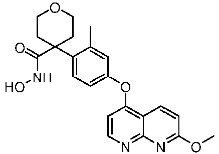 |
| 170 | 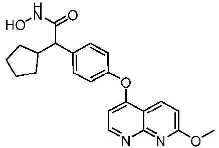 | 171 | 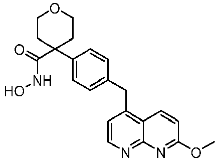 | 172 | 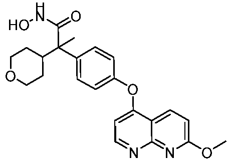 |
| 173 | 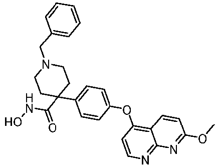 | 174 | 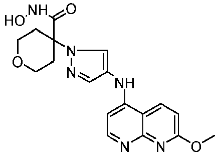 | 179 | 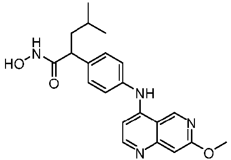 |
| 175 | 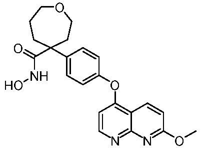 | 176 | 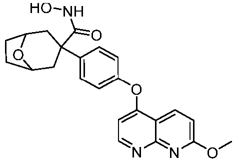 | 180 | 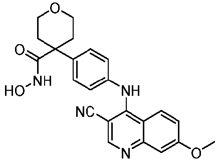 |
| 177 | 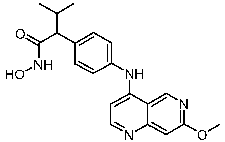 | 178 | 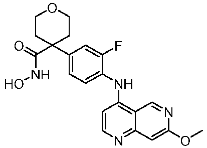 | 181 | 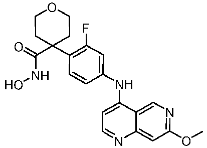 |
| 182 | 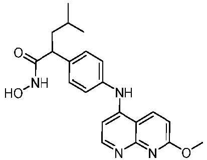 | 183 | 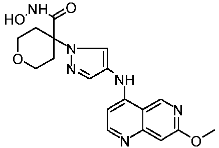 | 184 | 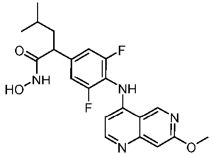 |
| 185 | 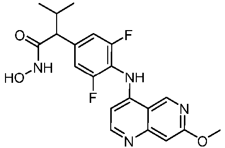 | 186 | 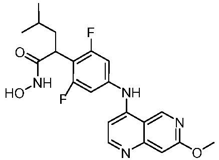 | 191 | 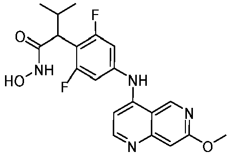 |
| 187 | 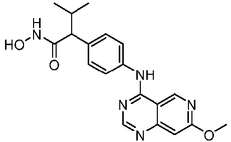 | 188 | 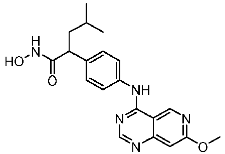 | 192 | 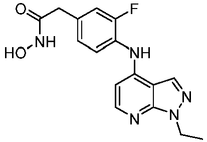 |
| 189 | 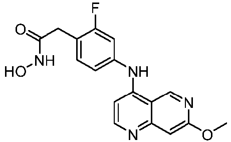 | 190 | 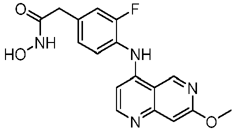 | 193 | 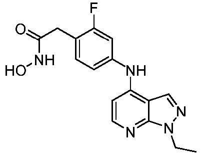 |
| 194 | 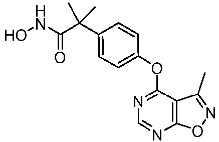 | 195 | 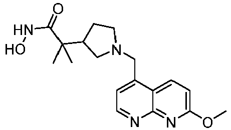 | 196 | 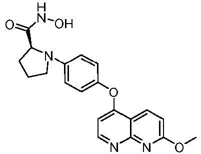 |
| 197 | 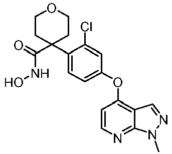 | 198 | 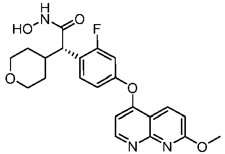 | 203 | 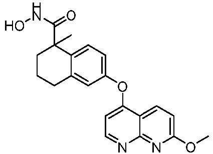 |
| 199 | 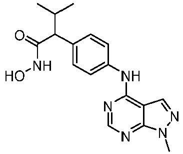 | 200 | 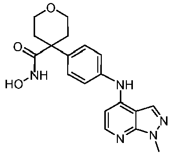 | 204 | 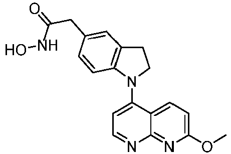 |
| 201 | 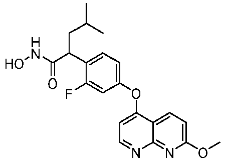 | 202 | 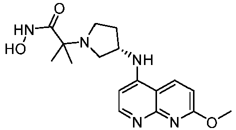 | 205 | 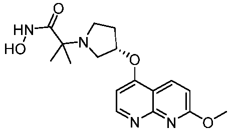 |
| 206 | 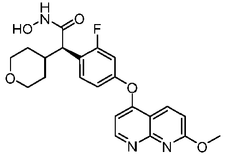 | 207 | 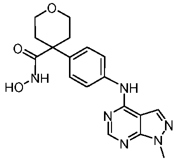 | 208 | 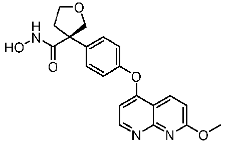 |
| 209 | 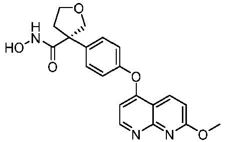 | 210 | 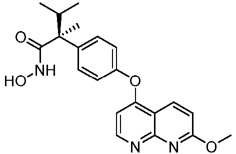 | 213 | 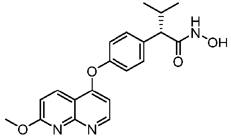 |
| 211 | 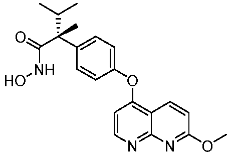 | 212 | 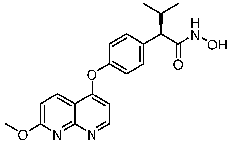 | 214 | 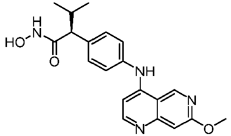 |
| 215 | 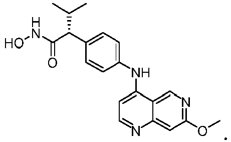 | | | | |

19. A pharmaceutical composition, comprising the compound or the pharmaceutically acceptable salt thereof of any one of the preceding claims, and one or more pharmaceutically acceptable carriers.

20. Use of the compound or the pharmaceutically acceptable salt thereof of any one of the preceding claims 1-18 in preparing a drug for preventing or treating ENPP1-mediated diseases or disorders.

21. A method for treating or preventing ENPP1-mediated diseases or disorders, including administering a therapeutically effective amount of the compound or the pharmaceutically acceptable salt thereof of any one of the preceding claims 1-18 to an individual in need thereof.

22. The use of claim 20 or the method of claim 21, wherein, the ENPP1-mediated diseases or disorders are solid tumors, for example, selected from breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and unknown primary cancer.

23. The use of claim 20 or the method of claim 21, wherein, the ENPP1-mediated diseases or disorders are hematopoietic malignancies, for example, selected from leukemia, lymphoma and myeloma.

24. The use of claim 20 or the method of claim 21, wherein the ENPP1-mediated diseases or disorders are infectious diseases, for example, selected from herpes simplex virus infection, cowpox virus infection, adenovirus infection, human papillomavirus infection, hepatitis B virus infection, hepatitis D virus infection, human immunodeficiency virus infection, human cytomegalovirus infection, dengue virus infection, Ebola virus infection, Marburg virus infection, Zika virus infection, Listeria monocytogenes infection, Mycobacterium tuberculosis infection, Francisella novicida infection, Legionella pneumophila infection, Chlamydia trachomatis infection, Streptococcus pneumoniae infection and Neisseria gonorrhoeae infection.

25. A drug combination product, comprising the compound or the pharmaceutically acceptable salt thereof of any one of the preceding claims 1-18, and one or more other active agents.
